# EUROPEAN PATENT APPLICATION

(11) **EP 1 792 987 A1**
(43) Date of publication of application: **06.06.2007**
(21) Application number: 05768941.6
(22) Date of filing: 05.08.2005
(51) Int. Cl.: C12N 15/11, C12N 5/10, C12P 21/02, C12P 21/08, C07K 16/00, C07K 2/00, A61K 39/395

(54) **METHOD OF PRODUCING GLYCOPROTEIN COMPOSITION**

(30) Priority: 05.08.2004 JP 2004228928; 31.08.2004 JP 2004252682; 09.05.2005 JP 2005136410
(71) Applicant: KYOWA HAKKO KOGYO CO., LTD., Chiyoda-ku, Tokyo 100-8185 (JP)
(72) Inventor: NISHIYA, Harue, c/o Kyowa Hakko Kogyo Co.,Ltd., Machida-shi, Tokyo 194-8533 (JP); SATOH, Mitsuo, c/o Kyowa Hakko Kogyo Co.,Ltd., Machida-shi, Tokyo 194-8533 (JP); MORI, Katsuhiro, c/o Kyowa Hakko Kogyo Co.,Ltd., Machida-shi, Tokyo 194-8533 (JP)
(74) Representative: Vossius & Partner
(86) International application number: PCT/JP2005/014408
(87) International publication number: WO 2006/013964

(57) **Abstract**

The present invention relates to a cell into which an RNA capable of suppressing the function of an enzyme catalyzing a reaction which converts GDP-mannose into GDP-4-keto,6-deoxy-GDP-mannose is introduced; a process for producing a glycoprotein using the cell; a cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme protein relating to synthesis of an intracellular sugar nucleotide, GDP-fucose, or an RNA capable of suppressing the function of a protein relating to transport of an intracellular sugar nucleotide, GDP-fucose, to the Golgi body are introduced; a process for producing a glycoprotein composition using the cell; and the like.

## Description

### TECHNICAL FIELD

The present invention relates to a cell into which an RNA capable of suppressing the function of an enzyme catalyzing a reaction which converts GDP-mannose into GDP-4-keto,6-deoxy-GDP-mannose is introduced; a process for producing a glycoprotein, which comprises using the cell; an RNA used for preparing the cell; a DNA corresponding to the RNA; and a vector comprising the DNA and its complementary DNA. Also, the present invention relates to a cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme protein relating to synthesis of an intracellular sugar nucleotide, GDP-fucose, or an RNA capable of suppressing the function of a protein relating to transport of an intracellular sugar nucleotide, GDP-fucose, to the Golgi body are introduced; and a process for producing a glycoprotein composition using the cell. Furthermore, the present invention relates to a DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme protein relating to synthesis of an intracellular sugar nucleotide, GDP-fucose, or an RNA capable of suppressing the function of a protein relating to transport of an intracellular sugar nucleotide, GDP-fucose, to the Golgi body and its complementary DNA; a vector comprising the DNA; a cell into which the vector is introduced; a cell into which a vector comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a vector comprising a DNA corresponding to an RNA capable of suppressing the function of an enzyme protein relating to synthesis of an intracellular sugar nucleotide, GDP-fucose, or an RNA capable of suppressing the function of a protein relating to transport of an intracellular sugar nucleotide, GDP-fucose, to the Golgi body and its complementary DNA are introduced; and a process for producing a glycoprotein composition using the cell.

### BACKGROUND ART

As a result of rapid development of genetic engineering or cell engineering techniques, physiologically active proteins which are present at a trace amount in the living body can be provided stably in a large amount to medical sites, so that they can be applied to treatments of many patients. Such protein medicaments are manufactured and sold as genetically engineered medicaments or cell culture medicaments. These protein medicaments are classified into a simple protein medicament in which a sugar chain is not concerned with its pharmacological activity and a glycoprotein medicament in which a sugar chain plays an important role in its physiological activity.

Erythropoietin is exemplified as a typical example of the glycoprotein medicament in which a sugar chain plays an important role in its pharmacological activity. Erythropoietin surely has various sugar chain structures, and it is known that it has three complex type N-glycoside-linked tetraantenary sugar chains in which a fucose is bound to three core structures, and one O-glycoside-linked sugar chain. The sugar chain structures are deeply related with the *in vivo* physiological activity of erythropoietin, and the physiological activity is not influenced by removing the O-glycoside-linked sugar chain (Non-patent references 1 and 2), but the physiological activity is lost by removing the N-glycoside-linked sugar chains (Non-patent reference 3). Furthermore, the pharmacological activity is influenced by addition of sialyic acid to the N-glycoside-linked sugar chains and difference of sugar chain structures such as a branched structure (Non-patent references 4, 5 and 6). Moreover, it is shown that a protein having a sugar chain structure in which fucose is modified generally has a shorten half-life in blood (Non-patent reference 7).

Regarding antibodies, it is known that the pharmacological activity is greatly influenced by the sugar chain structures.

In the Fc region of an IgG type antibody molecule, two N-glycoside-linked sugar chain binding sites are present. In serum IgG, a complex type sugar chain has plural branches in which sialic acid or bisecting N-acetylglucosamine are added at a low ratio is bound to the sugar chain binding site. The addition of galactose to the non-reducing end of the complex sugar chain and the addition of fucose to the N-acetylglucosamine in the reducing end is diversity (Non-patent reference 8). The sugar chain structure, that is, fucose which is added to N-acetylglucosamine in the reducing end in the N-glycoside-linked sugar chain which is bound to the antibody Fc region, plays an important role in effector functions of an antibody, such as antibody-dependent cell-mediated cytotoxic activity (hereinafter referred to as "ADCC activity") and complement-dependent cytotoxic activity (hereinafter referred to as "CDC activity") (Patent references 1 and 2, and Non-patent references 9 and 10).

Many of glycoproteins which are considered to be applied to medicaments are produced by using recombinant DNA techniques, and manufactured by using, as a host cell, an animal cell such as a CHO cell derived from a Chinese hamster ovary tissue. However, the sugar chain structures of the glycoproteins produced by using the recombinant DNA techniques are different depending on the host cells (Non-patent references 10 and 11). Accordingly, sugar chains are not always added to the glycoprotein produced by the recombinant DNA techniques so as to exert suitable pharmacological activity.

Application of inhibitors of an enzyme relating to the modification of a sugar chain has been attempted as a method for controlling the activity of an enzyme relating to the modification of a sugar chain in a cell and modifying the sugar chain structure of the produced glycoprotein. However, since the inhibitors have low specificity and it is difficult to sufficiently inhibit the target enzyme, it is difficult to surely control the sugar chain structure of the produced antibody.

Furthermore, modification of a sugar chain structure of a produced glycoprotein has been attempted by introducing a gene encoding an enzyme relating to the modification of a sugar chain (Non-patent references 12 and 13). When an antibody is expressed by using a CHO cell into which β1,4-N-acetylglucosamine transferase III (GnTIII) is introduced, the antibody had ADCC activity 16 times higher than the antibody expressed by using the parent cell (Non-patent reference 11 and Patent reference 3). However, since it has been reported that excess expression of GnTIII or β-1,4-N-acetylglucosamine transferase V (GnTV) shows toxicity for CHO cells, it is not suitable for the production of therapeutic antibodies.

Production examples of a glycoprotein in which the produced sugar chain structure was changed by using, as the host cell, a mutant in which the activity of a gene encoding an enzyme relating to the modification of a sugar chain was changed have been reported. The mutant in which the activity of an enzyme relating to the modification of a sugar chain is changed has been obtained, for example, as clones showing resistance to a lectin such as WGA (wheat-germ agglutinin derived from *T. vulgaris*), ConA (concanavalin A derived from C. *ensiformis*), RIC (a toxin derived from *R. communis*), L-PHA (leukoagglutinin derived from *P. vulgaris*), LCA (lentil agglutinin derived from L. *culinaris*), PSA (pea lectin derived from *P. sativum*) (Non-patent reference 14). A case has been reported in which a glycoprotein having a changed sugar chain structure is produced by using, as the host cell, such a mutant in which the activity of an enzyme relating to the modification of a sugar chain was changed. Specific examples include a report on the production of an antibody having a high mannose type sugar chain structure using a CHO cell mutant clone in which the activity of N-acetylglucosamine transferase I (GnTI) was deleted (Non-patent reference 15). In addition, a case has been reported on the expression of an antibody having a sugar chain structure in which sialic acid is not added to the non-reducing end in the sugar chains or an antibody without addition of galactose thereto, using a CMP-sialic acid transporter- or UDP-galactose transporter-deficient clone, but expression of an antibody having improved effector activity suitable for application to a medicament has been unsuccessful (Non-patent reference 15).

Under such a situation, it has been recently reported that an antibody having high ADCC activity which is suitable for medical applications can be produced by using, as the host cell, a clone having decreased activity of GDP-mannose 4,6-dehydratase (hereinafter also referred to as "GMD"), which is an enzyme catalyzing a reaction which converts GDP-mannose into GDP-4-keto,6-deoxy-GDP-mannose in the de novo pathway of the intracellular sugar nucleotide, GDP-fucose (Patent reference 1, and Non-patent references 9 and 10). In these reports, a clone resistant to a lectin which can recognize a sugar chain structure in which 1-position of fucose is bound to 6-position of N-acetylglucosamine in the reducing end in the complex type N-glycoside-linked sugar chain through α-bond, such as clone CHO-AAL which is resistant to AAL (a lectin derived from *Aleuria aurantia*), clone CHO-LCA which is resistant to LCA (lentil agglutinin derived from *L. culinaris*) or clone Lec 13 is used as the host cell. In addition to these, PL^{R}1.3 established as a PSA (pea lectin derived from *P. sativum*)-resistant mutant of a mouse leukemia-derived clone BW 5147 is also known as a clone having decreased activity of GDP-mannose 4,6-dehydratase (Non-patent reference 16).

However, since each of these clones is not a complete gene deficient clone, it is difficult to allow an antibody to carry a sugar chain structure which is a cause of showing high ADCC activity by the antibody, i.e. it is difficult to completely suppress an addition of fucose to the N-acetylglucosamine in the reducing end in the N-glycoside-linked sugar chains. Also, since mutants such as PL^{R}1.3 and Lec13 are obtained by randomly introducing mutation through a mutagen treatment, they are not suitable as clones to be used in the production of pharmaceutical preparations.

As is described above, attempts have been made for controlling the activity of an enzyme or protein relating to the modification of a sugar chain in a host cell in order to modify the sugar chain structure of a produced glycoprotein. However, since the modification mechanism of the sugar chain is various and complicated and the physiological functions of the sugar chain have not been sufficiently solved, trial and error are repeated at present. Especially, although a clone in which the activity of an enzyme catalyzing a reaction which converts GDP-mannose into GDP-4-keto,6-deoxy-GDP-mannose has been obtained and an antibody composition having high effector activity has been produced, the activity cannot be sufficiently controlled.

As an example of attempts for simply controlling the activity of an enzyme or protein relating to the modification of a sugar chain in a host cell, a method for controlling the function of a specific gene using siRNA (small interfering RNA) is known (Patent reference 4). Also, it is reported that a method for designing an RNA molecule used for suppressing the function of a gene (Non-patent reference 17). However, the RNA molecule designed by such a method is not always a molecule which can efficiently suppress the function of a target gene (Non-patent reference 18), and the design of an RNA molecule showing effective functional suppressive effect on a specific gene involves trial and error.

Also, it is shown that modification of binding of a fucose to a sugar chain which is added to a produced glycoprotein can be controlled by using a cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is introduced (Patent references 2 and 4). These reports show that the ratio of a sugar chain in which fucose is not bound among sugar chains bound to a produced antibody molecule can be increased by introducing an RNA capable of suppressing the function of α1,6-fucosyltransferase into a cell line which produces an antibody molecule.
Patent reference 1: WO00/61739
Patent reference 2: WO02/31140
Patent reference 3: WO99/54342
Patent reference 4: WO03/85118
Non-patent reference *1:* Biochemistry, 31, 9872 (1992)
Non-patent reference 2: J. BioL Chem., 267, 7703 (1992)
Non-patent reference 3: J. Biol. Chem., 265, 12127 (1990)
Non-patent reference 4: Blood, 73, 84, (1989)
Non-patent reference 5: Proc. Natl. Acad. Sci. U.S.A., 86, 7819 (1989)
Non-patent reference 6: British J. Cancer, 84, 3, (2001)
Non-patent reference 7: Science, 295, 1898 (2002)
Non-patent reference 8: Biochemistry, 36, 130 (1997)
Non-patent reference 9: J. BioL Chem., 277, 26733 (2002)
Non-patent reference 10: J. Biol. Chem., 278, 3466 (2003)
Non-patent reference 11: Glycobiology, 5, 813, (1995)
Non-patent reference 12: J. Biol. Chem., 261, 13848 (1989)
Non-patent reference 13: Science, 252, 1668 (1991)
Non-patent reference 14: Somatic Cell Mol. Genet., 12, 51 (1986)
Non-patent reference 15: J. Immunol., 160, 3393 (1998)
Non-patent reference 16: J. Biol. Chem., 255, 9900 (1980)
Non-patent reference 17: Nature Biotech., 22, 326 (2004)
Non-patent reference 18: Current Opinion in Molecular Therapeutics, 6, 129 (2004)

### DISCLOSURE OF THE INVENTION

### Problems to be solved by the invention:

An object of the present invention is to provide a cell into which an RNA capable of suppressing the function of an enzyme catalyzing a reaction which converts GDP-mannose into GDP-4-keto,6-deoxy-GDP-mannose is introduced; a process for producing a glycoprotein composition using the cell; an RNA used for preparing the cell; a DNA corresponding to the RNA; and a vector comprising the DNA and its complementary DNA.

Also, an object of the present invention is to provide a cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme protein relating to synthesis of an intracellular sugar nucleotide, GDP-fucose, or an RNA capable of suppressing the function of a protein relating to transport of an intracellular sugar nucleotide, GDP-fucose, to the Golgi body are introduced; and a process for producing a glycoprotein composition using the cell.

Furthermore, an object of the present invention is to provide a DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme protein relating to synthesis of an intracellular sugar nucleotide, GDP-fucose, or an RNA capable of suppressing the function of a protein relating to transport of an intracellular sugar nucleotide, GDP-fucose, to the Golgi body and its complementary DNA; a vector comprising the DNA; a cell into which the vector is introduced; a cell into which a vector comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a vector comprising a DNA corresponding to an RNA capable of suppressing the function of an enzyme protein relating to synthesis of an intracellular sugar nucleotide, GDP-fucose, or an RNA capable of suppressing the function of a protein relating to transport of an intracellular sugar nucleotide, GDP-fucose, to the Golgi body and its complementary DNA are introduced; and a process for producing a glycoprotein composition using the cell.

### Means for solving the problems:

The present invention relates to the following (1) to (71):
(1) A cell into which a double-stranded RNA comprising an RNA selected from the following (a) or (b) and its complementary RNA are introduced:
   (a) an RNA comprising the nucleotide sequence represented by SEQ ID NO:37, 57 or 58;
   (b) an RNA consisting of a nucleotide sequence in which one or a several nucleotide(s) is/are deleted, substituted, inserted and/or added in the nucleotide sequence represented by SEQ ID NO:37, 57 or 58 and having activity of suppressing the function of an enzyme catalyzing a reaction which converts GDP-mannose into GDP-4-keto,6-deoxy-GDP-mannose.
(2) The cell according to (1), wherein the enzyme catalyzing a dehydration reaction which converts GDP-mannose into GDP-4-keto,6-deoxy-GDP-mannose is GDP-mannose 4,6-dehydratase.
(3) The cell according to (2), wherein the GDP-mannose 4,6-dehydratase is a protein encoded by a DNA selected from the group consisting of the following (a) to (f):
   (a) a DNA comprising the nucleotide sequence represented by SEQ ID NO:8;
   (b) a DNA comprising the nucleotide sequence represented by SEQ ID NO:9;
   (c) a DNA comprising the nucleotide sequence represented by SEQ ID NO:10;
   (d) a DNA which hybridizes with a DNA consisting of the nucleotide sequence represented by SEQ ID NO:8 under stringent conditions and encodes a protein having GDP-mannose 4,6-dehydratase activity;
   (e) a DNA which hybridizes with a DNA consisting of the nucleotide sequence represented by SEQ ID NO:9 under stringent conditions and encodes a protein having GDP-mannose 4,6-dehydratase activity;
   (f) a DNA which hybridizes with a DNA consisting of the nucleotide sequence represented by SEQ ID NO:10 under stringent conditions and encodes a protein having GDP-mannose 4,6-dehydratase activity.
(4) The cell according to (2), wherein the GDP-mannose 4,6-dehydratase is a protein selected from the group consisting of the following (a) to.(i):
   (a) a protein comprising the amino acid sequence represented by SEQ ID NO:11;
   (b) a protein comprising the amino acid sequence represented by SEQ ID NO: 12;
   (c) a protein comprising the amino acid sequence represented by SEQ ID NO:13;
   (d) a protein consisting of an amino acid sequence in which one or a several amino acid(s) is/are deleted, substituted, inserted and/or added in the amino acid sequence represented by SEQ ID NO:11 and having GDP-mannose 4,6-dehydratase activity;
   (e) a protein consisting of an amino acid sequence in which one or a several amino acid(s) is/are deleted, substituted, inserted and/or added in the amino acid sequence represented by SEQ ID NO: 12 and having GDP-mannose 4,6-dehydratase activity;
   (f) a protein consisting of an amino acid sequence in which one or a several amino acid(s) is/are deleted, substituted, inserted and/or added in the amino acid sequence represented by SEQ ID NO:13 and having GDP-mannose 4,6-dehydratase activity;
   (g) a protein consisting of an amino acid sequence which has 80% or more homology to the amino acid sequence represented by SEQ ID NO:11 and having GDP-mannose 4,6-dehydratase activity;
   (h) a protein consisting of an amino acid sequence which has 80% or more homology to the amino acid sequence represented by SEQ ID NO:12 and having GDP-mannose 4,6-dehydratase activity;
   (i) a protein consisting of an amino acid sequence which has 80% or more homology to the amino acid sequence represented by SEQ ID NO:13 and having GDP-mannose 4,6-dehydratase activity.
(5) A double-stranded RNA comprising an RNA selected from the following (a) or (b) and its complementary RNA:
   (a) an RNA comprising the nucleotide sequence represented by SEQ ID NO:37, 57 or 58;
   (b) an RNA consisting of a nucleotide sequence in which one or a several nucleotide(s) is/are deleted, substituted, inserted and/or added in the nucleotide sequence represented by SEQ ID NO:37, 57 or 58 and having activity of suppressing the function of an enzyme catalyzing a reaction which converts GDP-mannose into GDP-4-keto,6-deoxy-GDP-mannose.
(6) A DNA corresponding to the RNA described in (5) and its complementary DNA
(7) A vector comprising a DNA corresponding to the RNA described in (5).
(8) A cell into which the vector described in (7) is introduced.
(9) A cell into which an RNA capable of suppressing the function of an enzyme relating to modification of a sugar chain in which 1-position of fucose is bound to 6-position ofN-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, or an RNA capable of suppressing the function of a protein relating to transport of an intracellular sugar nucleotide, GDP-fucose, to the Golgi body are introduced.
(10) A cell into which an RNA capable of suppressing the function of an enzyme relating to modification of a sugar chain in which 1-position of fucose is bound to 6-position ofN-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, are introduced.
(11) The cell according to (9) or (10), wherein the enzyme relating to synthesis of an intracellular sugar nucleotide, GDP-fucose, is an enzyme catalyzing a reaction which converts GDP-mannose into GDP-4-keto,6-deoxy-GDP-mannose.
(12) The cell according to any one of (9) to (11), wherein the enzyme relating to modification of a sugar chain in which 1-position of fucose is bound to 6-position of N-acetylglucosamine in the reducing end through α-bond in the complex type N-glycoside-linked sugar chain is α1,6-fucosyltransferase.
(13) The cell according to (12), wherein the α1,6-fucosyltransferase is a protein encoded by a DNA selected from the group consisting of (a) to (h):
   (a) a DNA comprising the nucleotide sequence represented by SEQ ID NO:1;
   (b) a DNA comprising the nucleotide sequence represented by SEQ ID NO:2;
   (c) a DNA comprising the nucleotide sequence represented by SEQ ID NO:3;
   (d) a DNA comprising the nucleotide sequence represented by SEQ ID NO:4;
   (e) a DNA which hybridizes with a DNA consisting of the nucleotide sequence represented by SEQ ID NO:1 under stringent conditions and encodes a protein having α1,6-fucosyltransferase activity;
   (f) a DNA which hybridizes with a DNA consisting of the nucleotide sequence represented by SEQ ID NO:2 under stringent conditions and encodes a protein having α1,6-fucosyltransferase activity;
   (g) a DNA which hybridizes with a DNA consisting of the nucleotide sequence represented by SEQ ID NO:3 under stringent conditions and encodes a protein having α1,6-fucosyltransferase activity;
   (h) a DNA which hybridizes with a DNA consisting of the nucleotide sequence represented by SEQ ID NO:4 under stringent conditions and encodes a protein having α1,6-fucosyltransferase activity.
(14) The cell according to (12), wherein the α1,6-fucosyltransferase is a protein selected from the group consisting of the following (a) to (1):
   (a) a protein comprising the amino acid sequence represented by SEQ ID NO: 5;
   (b) a protein comprising the amino acid sequence represented by SEQ ID NO:6;
   (c) a protein comprising the amino acid sequence represented by SEQ ID NO:7;
   (d) a protein comprising the amino acid sequence represented by SEQ ID NO:84;
   (e) a protein consisting of an amino acid sequence in which one or more amino acid(s) is/are deleted, substituted, inserted and/or added in the amino acid sequence represented by SEQ ID NO:5 and having α1,6-fucosyltransferase activity;
   (f) a protein consisting of an amino acid sequence in which one or more amino acid(s) is/are deleted, substituted, inserted and/or added in the amino acid sequence represented by SEQ ID NO:6 and having α1,6-fucosyltransferase activity;
   (g) a protein consisting of an amino acid sequence in which one or more amino acid(s) is/are deleted, substituted, inserted and/or added in the amino acid sequence represented by SEQ ID NO:7 and having α1,6-fucosyltransferase activity;
   (h) a protein consisting of an amino acid sequence in which one or more amino acid(s) is/are deleted, substituted, inserted and/or added in the amino acid sequence represented by SEQ ID NO:84 and having α1,6-fucosyltransferase activity;
   (i) a protein consisting of an amino acid sequence which has 80% or more homology to the amino acid sequence represented by SEQ ID NO:5 and having α1,6-fucosyltransferase activity;
   (j) a protein consisting of an amino acid sequence which has 80% or more homology to the amino acid sequence represented by SEQ ID NO:6 and having α1,6-fucosyltransferase activity;
   (k) a protein consisting of an amino acid sequence which has 80% or more homology to the amino acid sequence represented by SEQ ID NO:7 and having α1,6-fucosyltransferase activity;
   (l) a protein consisting of an amino acid sequence which has 80% or more homology to the amino acid sequence represented by SEQ ID NO:84 and having α1,6-fucosyltransferase activity.
(15) The cell according to any one of (9) to (14), wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is a double-stranded RNA comprising an RNA selected from the group consisting of the following (a) to (d) and its complementary RNA:
   (a) an RNA corresponding to a DNA comprising a nucleotide sequence represented by a sequence of continued 10 to 40 bases in the nucleotide sequence represented by SEQ ID NO:1;
   (b) an RNA corresponding to a DNA comprising a nucleotide sequence represented by a sequence of continued 10 to 40 bases in the nucleotide sequence represented by SEQ ID NO:2;
   (c) an RNA corresponding to a DNA comprising a nucleotide sequence represented by a sequence of continued 10 to 40 bases in the nucleotide sequence represented by SEQ ID NO:3;
   (d) an RNA corresponding to a DNA comprising a nucleotide sequence represented by a sequence of continued 10 to 40 bases in the nucleotide sequence represented by SEQ ID NO :4.
(16) The cell according to any one of (9) to (14), wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is a double-stranded RNA comprising an RNA selected from the group consisting of the following (a) and (b) and its complementary RNA:
   (a) an RNA comprising the nucleotide sequence represented by any one of SEQ ID NO:14 to 35 or 85 to 89;
   (b) an RNA consisting of a nucleotide sequence in which one or a several nucleotide(s) is/are deleted, substituted, inserted and/or added in the nucleotide sequence represented by any one of SEQ ID NO:14 to 35 or 85 to 89 and having activity of suppressing the function of α1,6-fucosyltransferase activity.
(17) The cell according to any one of (11) to (16), wherein the enzyme catalyzing a reaction which converts GDP-mannose into GDP-4-keto,6-deoxy-GDP-mannose is GDP-mannose 4,6-dehydratase.
(18) The cell according to (17), wherein the GDP-mannose 4,6-dehydratase is a protein encoded by a DNA selected from the group consisting of the following (a) to (f):
   (a) a DNA comprising the nucleotide sequence represented by SEQ ID NO:8;
   (b) a DNA comprising the nucleotide sequence represented by SEQ ID NO:9;
   (c) a DNA comprising the nucleotide sequence represented by SEQ ID NO:10;
   (d) a DNA which hybridizes with a DNA consisting of the nucleotide sequence represented by SEQ ID NO:8 under stringent conditions and encodes a protein having GDP-mannose 4,6-dehydratase activity;
   (e) a DNA which hybridizes with a DNA consisting of the nucleotide sequence represented by SEQ ID NO:9 under stringent conditions and encodes a protein having GDP-mannose 4,6-dehydratase activity;
   (f) a DNA which hybridizes with a DNA consisting of the nucleotide sequence represented by SEQ ID NO:10 under stringent conditions and encodes a protein having GDP-mannose 4,6-dehydratase activity.
(19) The cell according to (17), wherein the GDP-mannose 4,6-dehydratase is a protein selected from the group consisting of the following (a) to (i):
   (a) a protein comprising the amino acid sequence represented by SEQ ID NO:11;
   (b) a protein comprising the amino acid sequence represented by SEQ ID NO:12;
   (c) a protein comprising the amino acid sequence represented by SEQ ID NO:13;
   (d) a protein consisting of an amino acid sequence in which one or more amino acid(s) is/are deleted, substituted, inserted and/or added in the amino acid sequence represented by SEQ ID NO:11 and having GDP-mannose 4,6-dehydratase activity;
   (e) a protein consisting of an amino acid sequence in which one or more amino acid(s) is/are deleted, substituted, inserted and/or added in the amino acid sequence represented by SEQ ID NO:12 and having GDP-mannose 4,6-dehydratase activity;
   (f) a protein consisting of an amino acid sequence in which one or more amino acid(s) is/are deleted, substituted, inserted and/or added in the amino acid sequence represented by SEQ ID NO:13 and having GDP-mannose 4,6-dehydratase activity;
   (g) a protein consisting of an amino acid sequence which has 80% or more homology to the amino acid sequence represented by SEQ ID NO:11 and having GDP-mannose 4,6-dehydratase activity;
   (h) a protein consisting of an amino acid sequence which has 80% or more homology to the amino acid sequence represented by SEQ ID NO: 12 and having GDP-mannose 4,6-dehydratase activity;
   (i) a protein consisting of an amino acid sequence which has 80% or more homology to the amino acid sequence represented by SEQ ID NO:13 and having GDP-mannose 4,6-dehydratase activity.
(20) The cell according to any one of (11) to (19), wherein the RNA capable of suppressing the function of an enzyme catalyzing a reaction which converts GDP-mannose into GDP-4-keto,6-deoxy-GDP-mannose is a double-stranded RNA comprising an RNA selected from the group consisting of the following (a) to (c) and its complementary RNA:
   (a) an RNA corresponding to a DNA consisting of a nucleotide sequence represented by a sequence of continued 10 to 40 bases in the nucleotide sequence represented by SEQ ID NO:8;
   (b) an RNA corresponding to a DNA consisting of a nucleotide sequence represented by a sequence of continued 10 to 40 bases in the nucleotide sequence represented by SEQ ID NO:9;
   (c) an RNA corresponding to a DNA consisting of a nucleotide sequence represented by a sequence of continued 10 to 40 bases in the nucleotide sequence represented by SEQ ID NO:10.
(21) The cell according to any one of (11) to (19), wherein the RNA capable of suppressing the function of an enzyme catalyzing a reaction which converts GDP-mannose into GDP-4-keto,6-deoxy-GDP-mannose is a double-stranded RNA comprising an RNA selected from the group consisting of the following (a) and (b) and its complementary RNA:
   (a) an RNA comprising the nucleotide sequence represented by any one of SEQ ID NO:37, 57 or 58;
   (b) an RNA consisting of a nucleotide sequence in which one or a several nucleotide(s) is/are deleted, substituted, inserted and/or added in the nucleotide sequence represented by any one of SEQ ID NO:37, 57 or 58 and having activity of suppressing the function of GDP-mannose 4,6-dehydratase.
(22) A DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme relating to synthesis of an intracellular sugar nucleotide, GDP-fucose, and its complementary DNA or a DNA corresponding to an RNA capable of suppressing the function of a protein relating to transport of an intracellular sugar nucleotide, GDP-fucose, to the Golgi body and its complementary DNA.
(23) A DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme relating to synthesis of an intracellular sugar nucleotide, GDP-fucose, and its complementary DNA.
(24) The DNA according to (22) or (23), wherein the enzyme relating to synthesis of an intracellular sugar nucleotide, GDP-fucose, is an enzyme catalyzing a reaction which converts GDP-mannose into GDP-4-keto,6-deoxy-GDP-mannose.
(25) The DNA according to any one of (22) to (24), wherein the enzyme relating to modification of a sugar chain in which 1-position of fucose is bound to 6-position of N-acetylglucosamine in the reducing end through α-bond in the complex type N-glycoside-linked sugar chain is α1,6-fucosyltransferase.
(26) The DNA according to (25), wherein the α1,6-fucosyltransferase is a protein encoded by a DNA selected from the group consisting of (a) to (h):
   (a) a DNA comprising the nucleotide sequence represented by SEQ ID NO:1;
   (b) a DNA comprising the nucleotide sequence represented by SEQ ID NO:2;
   (c) a DNA comprising the nucleotide sequence represented by SEQ ID NO:3;
   (d) a DNA comprising the nucleotide sequence represented by SEQ ID NO:4;
   (e) a DNA which hybridizes with a DNA consisting of the nucleotide sequence represented by SEQ ID NO:1 under stringent conditions and encodes a protein having α1,6-fucosyltransferase activity;
   (f) a DNA which hybridizes with a DNA consisting of the nucleotide sequence represented by SEQ ID NO:2 under stringent conditions and encodes a protein having α1,6-fucosyltransferase activity;
   (g) a DNA which hybridizes with a DNA consisting of the nucleotide sequence represented by SEQ ID NO:3 under stringent conditions and encodes a protein having α1,6-fucosyltransferase activity;
   (h) a DNA which hybridizes with a DNA consisting of the nucleotide sequence represented by SEQ ID NO:4 under stringent conditions and encodes a protein having α1,6-fucosyltransferase activity.
(27) The DNA according to (25), wherein the α1,6-fucosyltransferase is a protein selected from the group consisting of the following (a) to (1):
   (a) a protein comprising the amino acid sequence represented by SEQ ID NO:5;
   (b) a protein comprising the amino acid sequence represented by SEQ ID NO:6;
   (c) a protein comprising the amino acid sequence represented by SEQ ID NO:7;
   (d) a protein comprising the amino acid sequence represented by SEQ ID NO:84;
   (e) a protein consisting of an amino acid sequence in which one or more amino acid(s) is/are deleted, substituted, inserted and/or added in the amino acid sequence represented by SEQ ID NO:5 and having α1,6-fucosyltransferase activity;
   (f) a protein consisting of an amino acid sequence in which one or more amino acid(s) is/are deleted, substituted, inserted and/or added in the amino acid sequence represented by SEQ ID NO:6 and having α1,6-fucosyltransferase activity;
   (g) a protein consisting of an amino acid sequence in which one or more amino acid(s) is/are deleted, substituted, inserted and/or added in the amino acid sequence represented by SEQ ID NO:7 and having α1,6-fucosyltransferase activity;
   (h) a protein consisting of an amino acid sequence in which one or more amino acid(s) is/are deleted, substituted, inserted and/or added in the amino acid sequence represented by SEQ ID NO:84 and having α1,6-fucosyltransferase activity;
   (i) a protein consisting of an amino acid sequence which has 80% or more homology to the amino acid sequence represented by SEQ ID NO:5 and having α1,6-fucosyltransferase activity;
   (j) a protein consisting of an amino acid sequence which has 80% or more homology to the amino acid sequence represented by SEQ ID NO:6 and having α1,6-fucosyltransferase activity;
   (k) a protein consisting of an amino acid sequence which has 80% or more homology to the amino acid sequence represented by SEQ ID NO:7 and having α1,6-fucosyltransferase activity;
   (l) a protein consisting of an amino acid sequence which has 80% or more homology to the amino acid sequence represented by SEQ ID NO:84 and having α1,6-fucosyltransferase activity.
(28) The DNA according to any one of (22) to (27), wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is an RNA selected from the group consisting of the following (a) to (d):
   (a) an RNA corresponding to a DNA comprising a nucleotide sequence represented by a sequence of continued 10 to 40 bases in the nucleotide sequence represented by SEQ ID NO:1;
   (b) an RNA corresponding to a DNA comprising a nucleotide sequence represented by a sequence of continued 10 to 40 bases in the nucleotide sequence represented by SEQ ID NO:2;
   (c) an RNA corresponding to a DNA comprising a nucleotide sequence represented by a sequence of continued 10 to 40 bases in the nucleotide sequence represented by SEQ ID NO:3;
   (d) an RNA corresponding to a DNA comprising a nucleotide sequence represented by a sequence of continued 10 to 40 bases in the nucleotide sequence represented by SEQ ID NO:4.
(29) The DNA according to any one of (22) to (27), wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is an RNA selected from the group consisting of the following (a) and (b):
   (a) an RNA comprising the nucleotide sequence represented by any one of SEQ ID NO:14 to 35 or 85 to 89;
   (b) an RNA consisting of a nucleotide sequence in which one or a several nucleotide(s) is/are deleted, substituted, inserted and/or added in the nucleotide sequence represented by any one of SEQ ID NO:14 to 35 or 85 to 89 and having activity of suppressing the function of α1,6-fucosyltransferase activity.
(30) The DNA according to any one of (24) to (29), wherein the enzyme catalyzing a reaction which converts GDP-mannose into GDP-4-keto, 6-deoxy-GDP-mannose is GDP-mannose 4,6-dehydratase.
(31) The DNA according to (30), wherein the GDP-mannose 4,6-dehydratase is a protein encoded by a DNA selected from the group consisting of the following (a) to (f):
   (a) a DNA comprising the nucleotide sequence represented by SEQ ID NO: 8;
   (b) a DNA comprising the nucleotide sequence represented by SEQ ID NO: 9;
   (c) a DNA comprising the nucleotide sequence represented by SEQ ID NO:10;
   (d) a DNA which hybridizes with a DNA consisting of the nucleotide sequence represented by SEQ ID NO:8 under stringent conditions and encodes a protein having GDP-mannose 4,6-dehydratase activity;
   (e) a DNA which hybridizes with a DNA consisting of the nucleotide sequence represented by SEQ ID NO:9 under stringent conditions and encodes a protein having GDP-mannose 4,6-dehydratase activity;
   (f) a DNA which hybridizes with a DNA consisting of the nucleotide sequence represented by SEQ ID NO:10 under stringent conditions and encodes a protein having GDP-mannose 4,6-dehydratase activity.
(32) The DNA according to (30), wherein the GDP-mannose 4,6-dehydratase is a protein selected from the group consisting of the following (a) to (i):
   (a) a protein comprising the amino acid sequence represented by SEQ ID NO:11;
   (b) a protein comprising the amino acid sequence represented by SEQ ID NO:12;
   (c) a protein comprising the amino acid sequence represented by SEQ ID NO:13;
   (d) a protein consisting of an amino acid sequence in which one or more amino acid(s) is/are deleted, substituted, inserted and/or added in the amino acid sequence represented by SEQ ID NO:11 and having GDP-mannose 4,6-dehydratase activity;
   (e) a protein consisting of an amino acid sequence in which one or more amino acid(s) is/are deleted, substituted, inserted and/or added in the amino acid sequence represented by SEQ ID NO:12 and having GDP-mannose 4,6-dehydratase activity;
   (f) a protein consisting of an amino acid sequence in which one or more amino acid(s) is/are deleted, substituted, inserted and/or added in the amino acid sequence represented by SEQ ID NO:13 and having GDP-mannose 4,6-dehydratase activity;
   (g) a protein consisting of an amino acid sequence which has 80% or more homology to the amino acid sequence represented by SEQ ID NO:11 and having GDP-mannose 4,6-dehydratase activity;
   (h) a protein consisting of an amino acid sequence which has 80% or more homology to the amino acid sequence represented by SEQ ID NO:12 and having GDP-mannose 4,6-dehydratase activity;
   (i) a protein consisting of an amino acid sequence which has 80% or more homology to the amino acid sequence represented by SEQ ID NO:13 and having GDP-mannose 4,6-dehydratase activity.
(33) The DNA according to any one of (24) to (32), wherein the RNA capable of suppressing the function of an enzyme catalyzing a reaction which converts GDP-mannose into GDP-4-keto,6-deoxy-GDP-mannose is an RNA selected from the group consisting of the following (a) to (c):
   (a) an RNA corresponding to a DNA consisting of a nucleotide sequence represented by a sequence of continued 10 to 40 bases in the nucleotide sequence represented by SEQ ID NO: 8;
   (b) an RNA corresponding to a DNA consisting of a nucleotide sequence represented by a sequence of continued 10 to 40 bases in the nucleotide sequence represented by SEQ ID NO:9;
   (c) an RNA corresponding to a DNA consisting of a nucleotide sequence represented by a sequence of continued 10 to 40 bases in the nucleotide sequence represented by SEQ ID NO:10.
(34) The DNA according to any one of (24) to (32), wherein the RNA capable of suppressing the function of an enzyme catalyzing a reaction which converts GDP-mannose into GDP-4-keto,6-deoxy-GDP-mannose is an RNA selected from the group consisting of the following (a) and (b):
   (a) an RNA comprising the nucleotide sequence represented by any one of SEQ ID NO:37, 57 or 58;
   (b) an RNA consisting of a nucleotide sequence in which one or a several nucleotide(s) is/are deleted, substituted, inserted and/or added in the nucleotide sequence represented by any one of SEQ ID NO:37, 57 or 58 and having activity of suppressing the function of GDP-mannose 4,6-dehydratase.
(35) A vector comprising the DNA described in any one of (22) to (34).
(36) The vector according to (35), which comprises the DNA represented by SEQ ID NO:90 and the DNA represented by SEQ ID NO:92.
(37) The vector according to (35), which comprises the DNA represented by SEQ ID NO:91 and the DNA represented by SEQ ID NO:92.
(38) The vector according to (35), which comprises the DNA represented by SEQ ID NO:90 and the DNA represented by SEQ ID NO:93.
(39) The vector according to (35), which comprises the DNA represented by SEQ ID NO:91 and the DNA represented by SEQ ID NO:93.
(40) A cell into which the vector described in any one of (35) to (39) is introduced.
(41) A cell into which a vector comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a vector comprising a DNA corresponding to an RNA capable of suppressing the function of an enzyme relating to synthesis of an intracellular sugar nucleotide, GDP-fucose, and its complementary DNA or a vector comprising a DNA corresponding to an RNA capable of suppressing the function of a protein relating to transport of an intracellular sugar nucleotide, GDP-fucose, to the Golgi body and its complementary DNA are introduced.
(42) A cell into which a vector comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a vector comprising a DNA corresponding to an RNA capable of suppressing the function of an enzyme relating to synthesis of an intracellular sugar nucleotide, GDP-fucose, and its complementary DNA are introduced.
(43) The cell according to (41) or (42), wherein the RNA capable of suppressing the function of an enzyme protein relating to synthesis of an intracellular sugar nucleotide, GDP-fucose, is an RNA capable of suppressing the function of an enzyme catalyzing a reaction which converts GDP-mannose into GDP-4-keto,6-deoxy-GDP-mannose.
(44) The cell according to any one of (41) to (43), wherein the enzyme relating to modification of a sugar chain in which 1-position of fucose is bound to 6-position of N-acetylglucosamine in the reducing end through α-bond in the complex type N-glycoside-linked sugar chain is α1,6-fucosyltransferase.
(45) The cell according to (44), wherein the α1,6-fucosyltransferase is a protein encoded by a DNA selected from the group consisting of (a) to (h):
   (a) a DNA comprising the nucleotide sequence represented by SEQ ID NO:1;
   (b) a DNA comprising the nucleotide sequence represented by SEQ ID NO:2;
   (c) a DNA comprising the nucleotide sequence represented by SEQ ID NO:3;
   (d) a DNA comprising the nucleotide sequence represented by SEQ ID NO:4;
   (e) a DNA which hybridizes with a DNA consisting of the nucleotide sequence represented by SEQ ID NO:1 under stringent conditions and encodes a protein having α1,6-fucosyltransferase activity;
   (f) a DNA which hybridizes with a DNA consisting of the nucleotide sequence represented by SEQ ID NO:2 under stringent conditions and encodes a protein having α1,6-fucosyltransferase activity;
   (g) a DNA which hybridizes with a DNA consisting of the nucleotide sequence represented by SEQ ID NO:3 under stringent conditions and encodes a protein having α1,6-fucosyltransferase activity;
   (h) a DNA which hybridizes with a DNA consisting of the nucleotide sequence represented by SEQ ID NO:4 under stringent conditions and encodes a protein having α1,6-fucosyltransferase activity.
(46) The cell according to (44), wherein the α1,6-fucosyltransferase is a protein selected from the group consisting of the following (a) to (l):
   (a) a protein comprising the amino acid sequence represented by SEQ ID NO:5;
   (b) a protein comprising the amino acid sequence represented by SEQ ID NO:6;
   (c) a protein comprising the amino acid sequence represented by SEQ ID NO:7;
   (d) a protein comprising the amino acid sequence represented by SEQ ID NO:84;
   (e) a protein consisting of an amino acid sequence in which one or more amino acid(s) is/are deleted, substituted, inserted and/or added in the amino acid sequence represented by SEQ ID NO:5 and having α1,6-fucosyltransferase activity;
   (f) a protein consisting of an amino acid sequence in which one or more amino acid(s) is/are deleted, substituted, inserted and/or added in the amino acid sequence represented by SEQ ID NO:6 and having α1,6-fucosyltransferase activity;
   (g) a protein consisting of an amino acid sequence in which one or more amino acid(s) is/are deleted, substituted, inserted and/or added in the amino acid sequence represented by SEQ ID NO:7 and having α1,6-fucosyltransferase activity;
   (h) a protein consisting of an amino acid sequence in which one or more amino acid(s) is/are deleted, substituted, inserted and/or added in the amino acid sequence represented by SEQ ID NO:84 and having α1,6-fucosyltransferase activity;
   (i) a protein consisting of an amino acid sequence which has 80% or more homology to the amino acid sequence represented by SEQ ID NO:5 and having α1,6-fucosyltransferase activity;
   (j) a protein consisting of an amino acid sequence which has 80% or more homology to the amino acid sequence represented by SEQ ID NO:6 and having α1,6-fucosyltransferase activity;
   (k) a protein consisting of an amino acid sequence which has 80% or more homology to the amino acid sequence represented by SEQ ID NO:7 and having α1,6-fucosyltransferase activity;
   (l) a protein consisting of an amino acid sequence which has 80% or more homology to the amino acid sequence represented by SEQ ID NO:84 and having α1,6-fucosyltransferase activity.
(47) The cell according to any one of (41) to (46), wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is a double-stranded RNA comprising an RNA selected from the group consisting of the following (a) to (d) and its complementary RNA:
   (a) an RNA corresponding to a DNA comprising a nucleotide sequence represented by a sequence of continued 10 to 40 bases in the nucleotide sequence represented by SEQ ID NO:1;
   (b) an RNA corresponding to a DNA comprising a nucleotide sequence represented by a sequence of continued 10 to 40 bases in the nucleotide sequence represented by SEQ ID NO:2;
   (c) an RNA corresponding to a DNA comprising a nucleotide sequence represented by a sequence of continued 10 to 40 bases in the nucleotide sequence represented by SEQ ID NO:3;
   (d) an RNA corresponding to a DNA comprising a nucleotide sequence represented by a sequence of continued 10 to 40 bases in the nucleotide sequence represented by SEQ ID NO:4.
(48) The cell according to any one of (41) to (46), wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is a double-stranded RNA comprising an RNA selected from the group consisting of the following (a) and (b) and its complementary RNA:
   (a) an RNA comprising the nucleotide sequence represented by any one of SEQ ID NO:14 to 35 or 85 to 89;
   (b) an RNA consisting of a nucleotide sequence in which one or a several nucleotide(s) is/are deleted, substituted, inserted and/or added in the nucleotide sequence represented by any one of SEQ ID NO:14 to 35 or 85 to 89 and having activity of suppressing the function of α1,6-fucosyltransferase activity.
(49) The cell according to any one of (43) to (48), wherein the enzyme catalyzing a reaction which converts GDP-mannose into GDP-4-keto,6-deoxy-GDP-mannose is GDP-mannose 4,6-dehydratase.
(50) The cell according to (49), wherein the GDP-mannose 4,6-dehydratase is a protein encoded by a DNA selected from the group consisting of the following (a) to (f):
   (a) a DNA comprising the nucleotide sequence represented by SEQ ID NO: 8;
   (b) a DNA comprising the nucleotide sequence represented by SEQ ID NO:9;
   (c) a DNA comprising the nucleotide sequence represented by SEQ ID NO:10;
   (d) a DNA which hybridizes with a DNA consisting of the nucleotide sequence represented by SEQ ID NO:8 under stringent conditions and encodes a protein having GDP-mannose 4,6-dehydratase activity;
   (e) a DNA which hybridizes with a DNA consisting of the nucleotide sequence represented by SEQ ID NO:9 under stringent conditions and encodes a protein having GDP-mannose 4,6-dehydratase activity;
   (f) a DNA which hybridizes with a DNA consisting of the nucleotide sequence represented by SEQ ID NO:10 under stringent conditions and encodes a protein having GDP-mannose 4,6-dehydratase activity.
(51) The cell according to (49), wherein the GDP-mannose 4,6-dehydratase is a protein selected from the group consisting of the following (a) to (i):
   (a) a protein comprising the amino acid sequence represented by SEQ ID NO:11;
   (b) a protein comprising the amino acid sequence represented by SEQ ID NO:12;
   (c) a protein comprising the amino acid sequence represented by SEQ ID NO:13;
   (d) a protein consisting of an amino acid sequence in which one or more amino acid(s) is/are deleted, substituted, inserted and/or added in the amino acid sequence represented by SEQ ID NO: 1 and having GDP-mannose 4,6-dehydratase activity;
   (e) a protein consisting of an amino acid sequence in which one or more amino acid(s) is/are deleted, substituted, inserted and/or added in the amino acid sequence represented by SEQ ID NO:12 and having GDP-mannose 4,6-dehydratase activity;
   (f) a protein consisting of an amino acid sequence in which one or more amino acid(s) is/are deleted, substituted, inserted and/or added in the amino acid sequence represented by SEQ ID NO: 13 and having GDP-mannose 4,6-dehydratase activity;
   (g) a protein consisting of an amino acid sequence which has 80% or more homology to the amino acid sequence represented by SEQ ID NO:11 and having GDP-mannose 4,6-dehydratase activity;
   (h) a protein consisting of an amino acid sequence which has 80% or more homology to the amino acid sequence represented by SEQ ID NO:12 and having GDP-mannose 4,6-dehydratase activity;
   (i) a protein consisting of an amino acid sequence which has 80% or more homology to the amino acid sequence represented by SEQ ID NO:13 and having GDP-mannose 4,6-dehydratase activity.
(52) The cell according to any one of (43) to (51), wherein the RNA capable of suppressing the function of an enzyme catalyzing a reaction which converts GDP-mannose into GDP-4-keto,6-deoxy-GDP-mannose is a double-stranded RNA comprising an RNA selected from the group consisting of the following (a) to (c) and its complementary RNA:
   (a) an RNA corresponding to a DNA consisting of a nucleotide sequence represented by a sequence of continued 10 to 40 bases in the nucleotide sequence represented by SEQ ID NO:8;
   (b) an RNA corresponding to a DNA consisting of a nucleotide sequence represented by a sequence of continued 10 to 40 bases in the nucleotide sequence represented by SEQ ID NO:9;
   (c) an RNA corresponding to a DNA consisting of a nucleotide sequence represented by a sequence of continued 10 to 40 bases in the nucleotide sequence represented by SEQ ID NO:10.
(53) The cell according to any one of (43) to (51), wherein the RNA capable of suppressing the function of an enzyme catalyzing a reaction which converts GDP-mannose into GDP-4-keto,6-deoxy-GDP-mannose is a double-stranded RNA comprising an RNA selected from the group consisting of the following (a) and (b) and its complementary RNA:
   (a) an RNA comprising the nucleotide sequence represented by any one of SEQ ID NO:37, 57 or 58;
   (b) an RNA consisting of a nucleotide sequence in which one or a several nucleotide(s) is/are deleted, substituted, inserted and/or added in the nucleotide sequence represented by any one of SEQ ID NO:37, 57 or 58 and having activity of suppressing the function of GDP-mannose 4,6-dehydratase.
(54) The cell according to any one of (1) to (4), (8) to (21) and (40) to (53), which is resistant to a lectin which recognizes a sugar chain structure in which 1-position of fucose is bound to 6-position of N-acetylglucosamine in the reducing end through α-bond in an N-glycoside-linked sugar chain.
(55) The cell according to (54), wherein the lectin is selected from the group consisting of the following (a) to (d):
   (a) a *Lens culinaris* agglutinin LCA (lentil agglutinin derived from *Lens culinaris*);
   (b) a *Pisum sativum* agglutinin PSA (pea lectin derived from *Pisum sativum*);
   (c) a *Vicia faba* agglutinin VFA (agglutinin derived from *Vicia faba*);
   (d) an *Aleuria aurantia* lectin AAL (lectin derived from *Aleuria aurantia*).
(56) The cell according to any one of (1) to (4), (8) to (21) and (40) to (55), which is a cell selected from the group consisting of a yeast, an animal cell, an insect cell and a plant cell.
(57) The cell according to (56), wherein the animal cell is selected from the group consisting of the following (a) to (k):
   (a) a CHO cell derived from a Chinese hamster ovary tissue;
   (b) a rat myeloma cell line YB2/3HL.P2.G11.16Ag.20 cell;
   (c) a mouse myeloma cell line NS0 cell;
   (d) a mouse myeloma cell line SP2/0-Ag14 cell;
   (e) a BHK cell derived from a Syrian hamster kidney tissue;
   (f) a hybridoma cell which produces an antibody;
   (g) a human leukemic cell line Namalwa cell;
   (h) a human leukemic cell line NM-F9 cell;
   (i) a human embryonic retinal cell line PER.C6 cell;
   (j) an embryonic stem cell;
   (k) a fertilized egg cell.
(58) The cell according to any one of (1) to (4), (8) to (21) and (40) to (57), which comprises a gene encoding a glycoprotein.
(59) The cell according to (58), wherein the glycoprotein is an antibody molecule.
(60) The cell according to (59), wherein the antibody molecule is selected from the group consisting of the following (a) to (d):
   (a) a human antibody;
   (b) a humanized antibody;
   (c) an antibody fragment comprising the Fc region of (a) or (b);
   (d) a fusion protein comprising the Fc region of (a) or (b).
(61) The cell according to (59) or (60), wherein the antibody molecule belongs to an IgG class.
(62) A process for producing a glycoprotein composition, which comprises using the cell described in (58).
(63) A process for producing a glycoprotein composition, which comprises culturing the cell described in (58) in a medium to form and accumulate the glycoprotein composition in the culture; and recovering and purifying the glycoprotein composition from the culture.
(64) A process for producing an antibody composition, which comprises using the cell described in any one of (59) to (61).
(65) A process for producing an antibody composition, which comprises culturing the cell described in any one of (59) to (61) in a medium to form and accumulate the antibody composition in the culture; and recovering and purifying the antibody composition from the culture.
(66) The process according to (64) or (65), wherein the antibody composition is an antibody composition having a higher antibody-dependent cell-mediated cytotoxic activity than an antibody composition produced by its parent cell.
(67) The process according to (66), wherein the antibody composition having a higher antibody-dependent cell-mediated cytotoxic activity has a higher ratio of a sugar chain in which fucose is not bound to N-acetylglucosamine in the reducing end in the sugar chain among total complex type N-glycoside-linked sugar chains bound to the Fc region in the antibody composition than an antibody composition produced by its parent cell.
(68) The process according to (67), wherein the sugar chain in which fucose is not bound is a sugar chain in which 1-position of the fucose is not bound to 6-position of N-acetylglucosamine in the reducing end through α-bond in a complex type N-glycoside-linked sugar chain.
(69) A glycoprotein composition produced by the process described in (62) or (63).
(70) An antibody composition produced by the process described in any one of (64) to (68).
(71) A medicament comprising the composition described in (69) or (70) as an active ingredient.

### Effect of the invention

The present invention provides a cell into which an RNA capable of suppressing the function of an enzyme catalyzing a reaction which converts GDP-mannose into GDP-4-keto,6-deoxy-GDP-mannose is introduced; a process for producing a glycoprotein using the cell; an RNA used for preparing the cell; a DNA corresponding to the RNA; and a vector comprising the DNA and its complementary DNA. Also, the present invention provides a cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme protein relating to synthesis of an intracellular sugar nucleotide, GDP-fucose, or an RNA capable of suppressing the function of a protein relating to transport of an intracellular sugar nucleotide, GDP-fucose, to the Golgi body are introduced; and a process for producing a glycoprotein composition using the cell. Furthermore, the present invention provides a DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme relating to synthesis of an intracellular sugar nucleotide, GDP-fucose, and or an RNA capable of suppressing the function of a protein relating to transport of an intracellular sugar nucleotide, GDP-fucose, to the Golgi body and its complementary DNA; a vector comprising the DNA; a cell into which the vector is introduced; a cell into which a vector comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a vector comprising a DNA corresponding to an RNA capable of suppressing the function of an enzyme relating to synthesis of an intracellular sugar nucleotide, GDP-fucose, and or an RNA capable of suppressing the function of a protein relating to transport of an intracellular sugar nucleotide, GDP-fucose, to the Golgi body and its complementary DNA are introduced; and a process for producing a glycoprotein composition using the cell.

The present invention is described below in detail. This application is filed by claiming the priorities of Japanese application No. 2004-228928 filed on August 5, 2004, Japanese application No. 2004-252682 filed on August 31, 2004 and Japanese application No. 2005-136410 filed on May 9, 2005, the description and drawings of which are incorporated hereinto by reference.

### BRIEF DESCRIPTION OF THE DRAWINGS

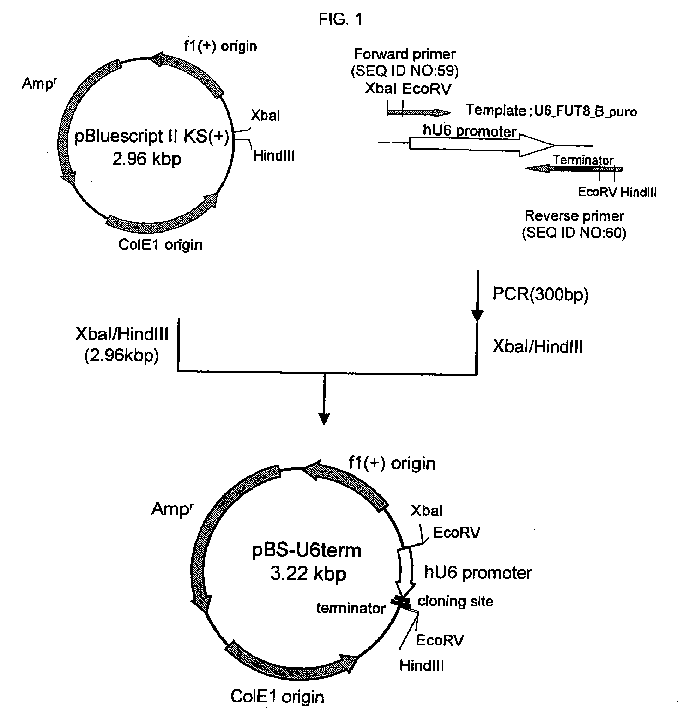

Fig. 1 shows the construction of plasmid pBS-U6term.

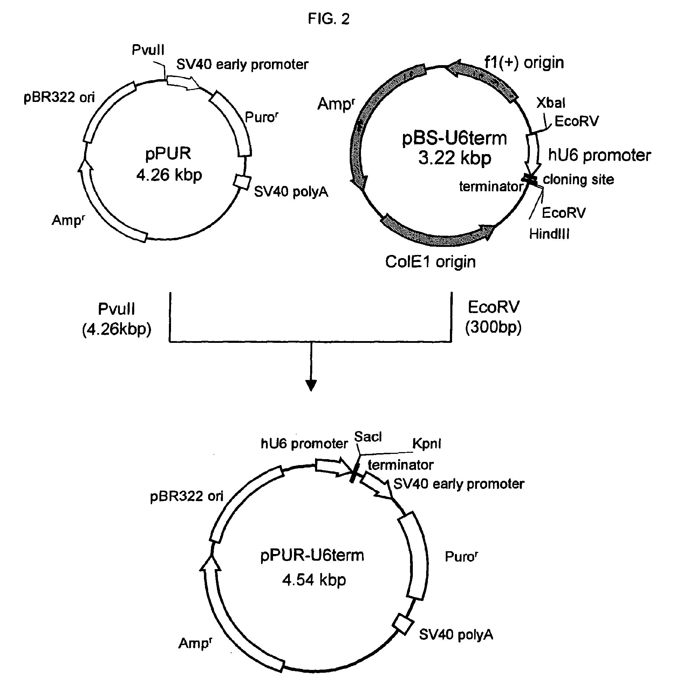

Fig. 2 shows the construction of plasmid pPUR-U6term.

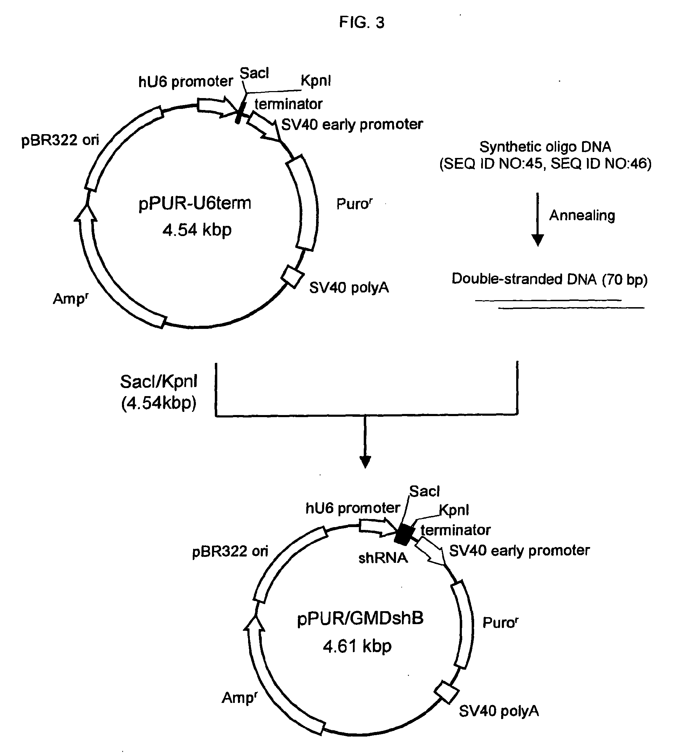

Fig. 3 shows the construction of plasmid pPUR/GMDshB.

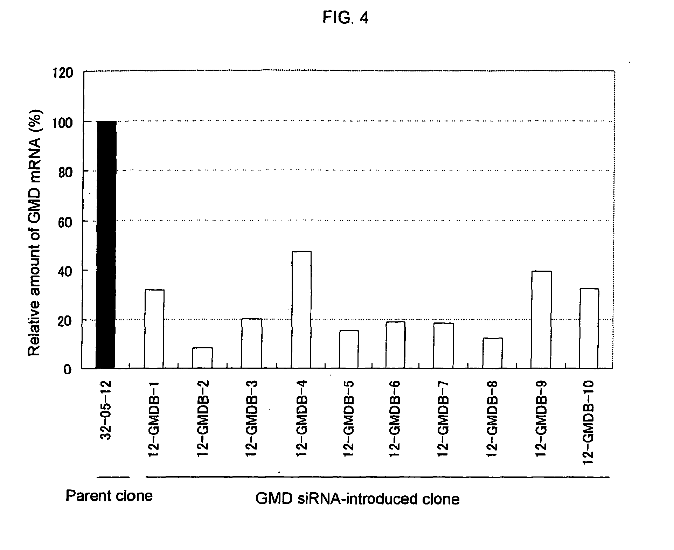

Fig. 4 shows the amount of GMD mRNA in each clone. The abscissa indicates the relative amount of GMD mRNA to the amount of β-actin mRNA when the amount of the parent clone 32-05-12 was assumed to be 100, and the ordinate indicates each clones. In the drawing, the black bar indicates the parent clone into which siRNA was not introduced, and the outline bars indicate the clones into which the GMD-targeting siRNA expression vector was introduced alone.

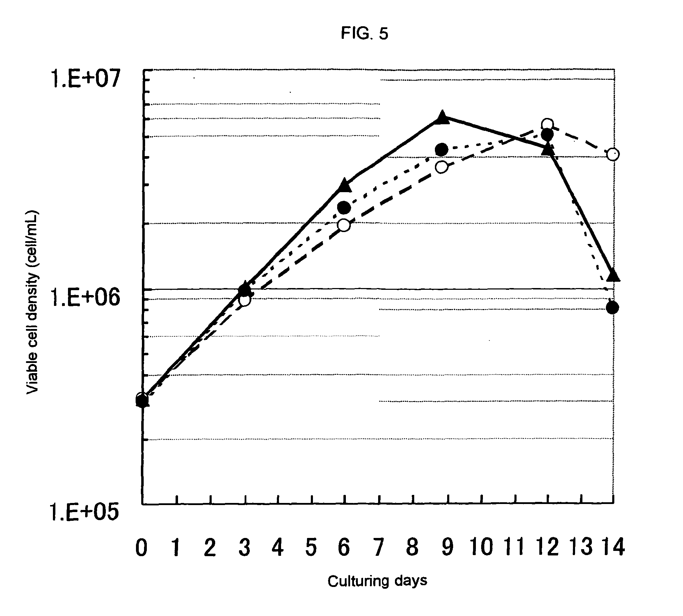

Fig. 5 shows changes of viable cell densities of clone 32-05-12AF which was CHO/DG44 cell-derived anti-CCR4 antibody-producing clone, and lectin-resistant clones, 12-GMDB-2AF and 12-GMDB-SAF, into which the GMD-targeting siRNA expression plasmid was introduced through serum-free fed-batch culture. The abscissa and the ordinate indicate the culturing days and the viable cell density, respectively. In the drawing, closed triangles, open circles and closed circles indicate clone 32-05-12AF, clone 12-GMDB-2AF and clone 12-GMDB-5, respectively.

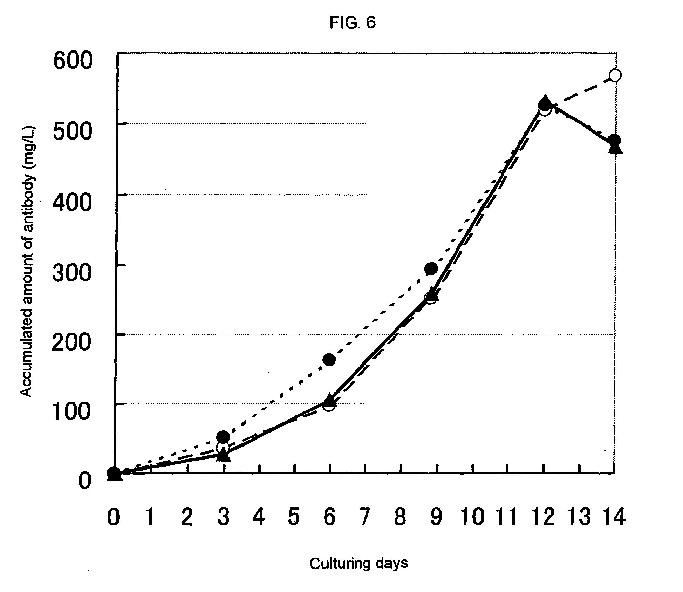

Fig. 6 shows changes of the amount of antibody accumulated in culture supernatant of serum-free fed-batch culture of clone 32-05-12AF which was CHO/DG44 cell-derived anti-CCR4 antibody-producing clone, and lectin-resistant clones, 12-GMDB-2AF and 12-GMDB-5AF, introduced with the GMD-targeting siRNA expression plasmid. The abscissa and the ordinate indicate the culturing days and the amount of antibody accumulated, respectively. In the drawing, closed triangles, open circles and closed circles indicate clone 32-05-12AF, clone 12-GMDB-2AF and clone 12-GMDB-5, respectively.

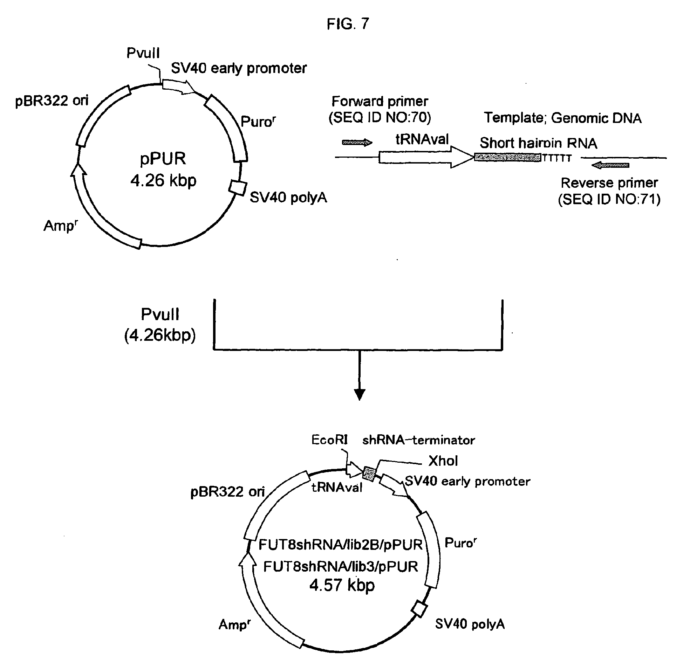

Fig. 7 shows the constructions of plasmids FUT8shRNA/lib2B/pPUR and FUT8shRNA/lib3/pPUR.

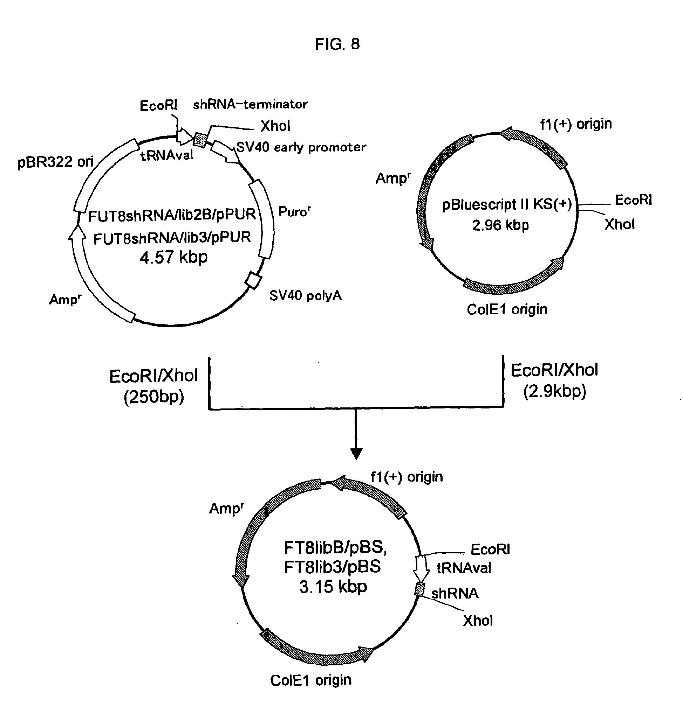

Fig. 8 shows the constructions of plasmids FT8libB/pBS and FT8lib3/pBS.

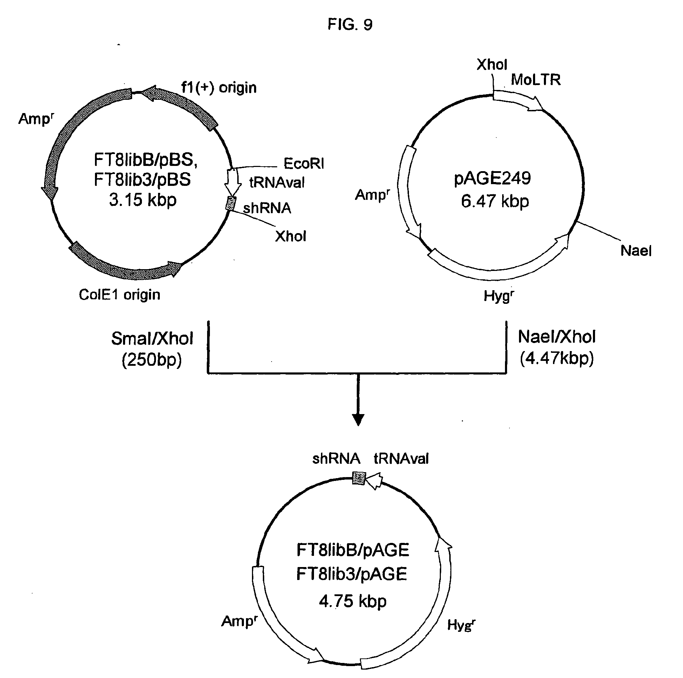

Fig. 9 shows the constructions of plasmids FT8libB/pAGE and FT8lib3/pAGE.

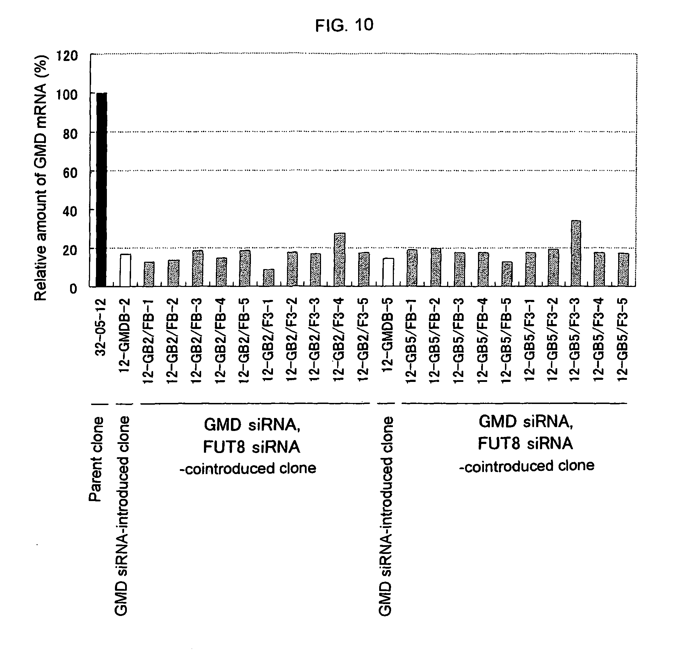

Fig. 10 shows the amount of GMD mRNA in each clone introduced with the GMD-targeting siRNA expression vector and the FUT8-targeting siRNA expression vector or with the GMD-targeting siRNA expression vector alone, and the parent clone. The abscissa indicates the relative amount of GMD mRNA to the amount of β-actin mRNA when the amount of the parent clone 32-05-12 was assumed to be 100, and the ordinate indicates each clones. In the drawing, black and outline bars indicate the parent clone without siRNA introduction and clones introduced with GMD-targeting siRNA expression vector alone, respectively.

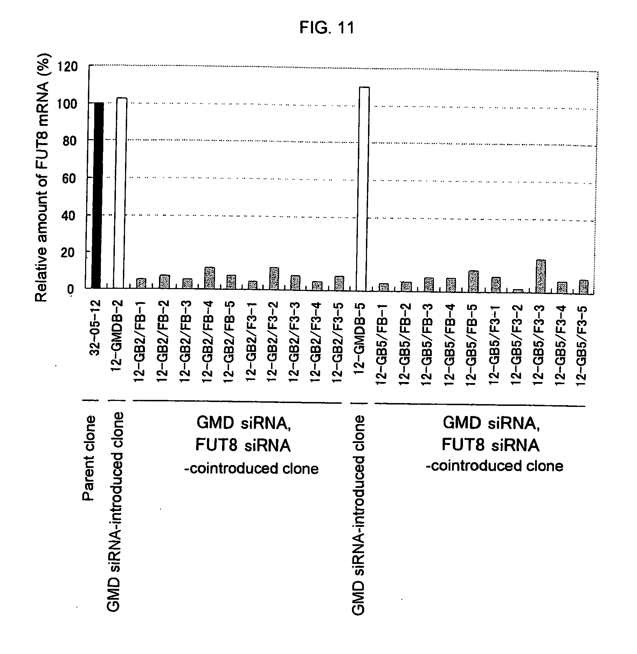

Fig. 11 shows the amount of FUT8 mRNA in each clone introduced with the GMD-targeting siRNA expression vector and the FUT8-targeting siRNA expression vector or with the GMD-targeting siRNA expression vector alone, and the parent clone. The abscissa indicates the relative amount of FUT8 mRNA to the amount of β-actin mRNA when the amount in the parent clone 32-05-12 was assumed to be 100, and the ordinate indicates each clones. In the drawing, black and outline bars indicate parent clone without siRNA introduction and clones introduced with the GMD-targeting siRNA expression vector alone, respectively.

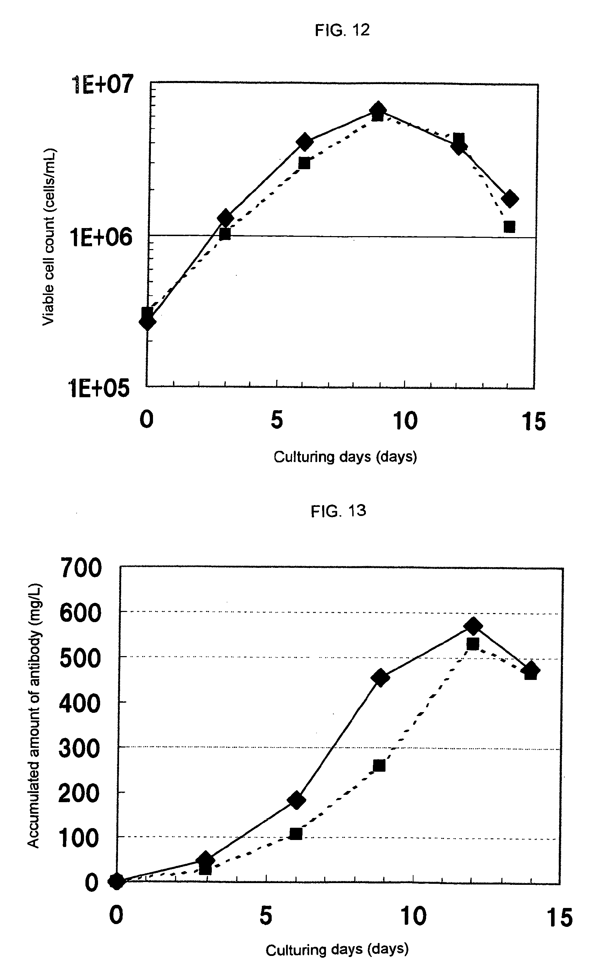

Fig. 12 shows changes of viable cell densities of clone 32-05-12AF which was CHO/DG44 cell-derived anti-CCR4 antibody-producing clone and lectin-resistant Wi23-5AF clone introduced with the GMD-targeting siRNA expression vector and the FUT8-targeting siRNA expression vector in serum-free fed-batch culture. The abscissa and the ordinate indicate culturing days and viable cell density, respectively. In the drawing, dotted and solid lines indicate 32-05-12AF and Wi23-5AF clones, respectively.
Fig. 13 shows changes of the amount of antibody accumulated in culture supernatant of serum-free fed-batch culture of clone 32-05-12AF which was CHO/DG44 cell-derived anti-CCR4 antibody-producing clone and lectin-resistant Wi23-5AF clone introduced with the GMD-targeting siRNA expression vector and the FUT8-targeting siRNA expression vector. The abscissa and the ordinate indicate culturing days and the amount of antibody accumulated, respectively. In the drawing, dotted and solid lines indicate 32-05-12AF and Wi23-5AF clones, respectively.

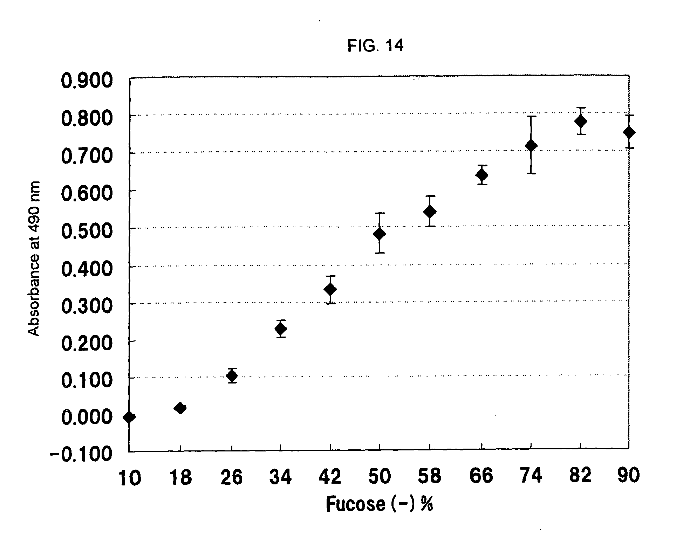

Fig. 14 shows shFcγRIIIa binding activity of a standard sample in which fucose(-)% of an anti-CCR4 chimeric antibody was known. The abscissa and the ordinate indicate fucose(-)% and absorbance at 490 nm showing shFcγRIIIa binding activity, respectively.

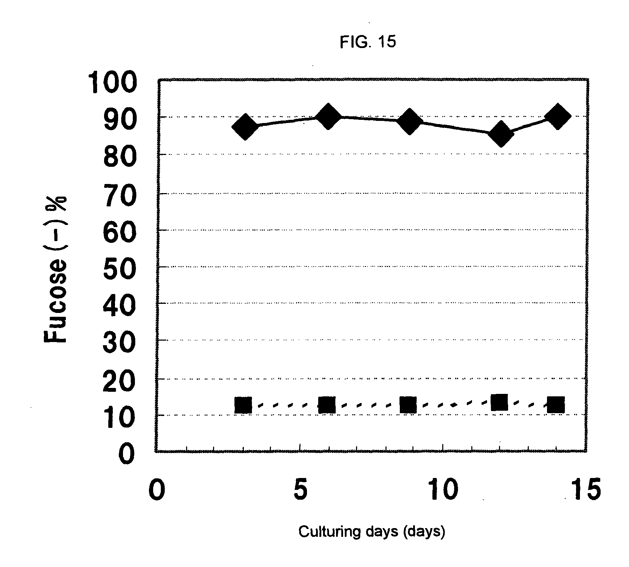

Fig. 15 shows fucose(-)% calculated from the shFcγRIIIa binding activity of anti-CCR4 chimeric antibody contained in culture supernatants in serum-free fed-batch medium of clone 32-05-12AF which was CHO/DG44 cell-derived anti-CCR4 antibody-producing clone or lectin-resistant Wi23-5AF clone introduced with the GMD-targeting siRNA expression vector and the FUT8-targeting siRNA expression vector. The abscissa and the ordinate indicate culturing days and fucose(-)%, respectively. In the drawing, dotted and solid lines indicate 32-05-12AF and Wi23-5AF clones, respectively.

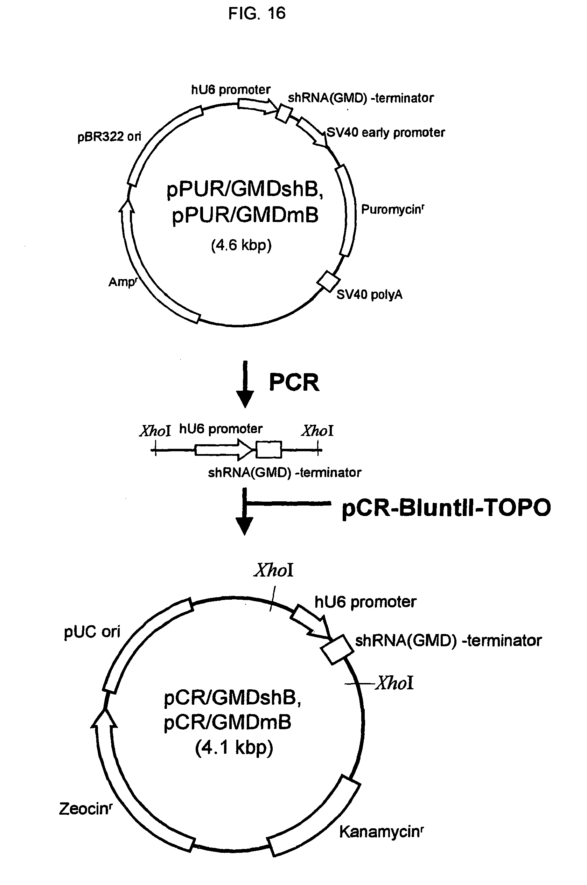

Fig. 16 shows the constructions of plasmids pCR/GMDshB and pCR/GMDmB.

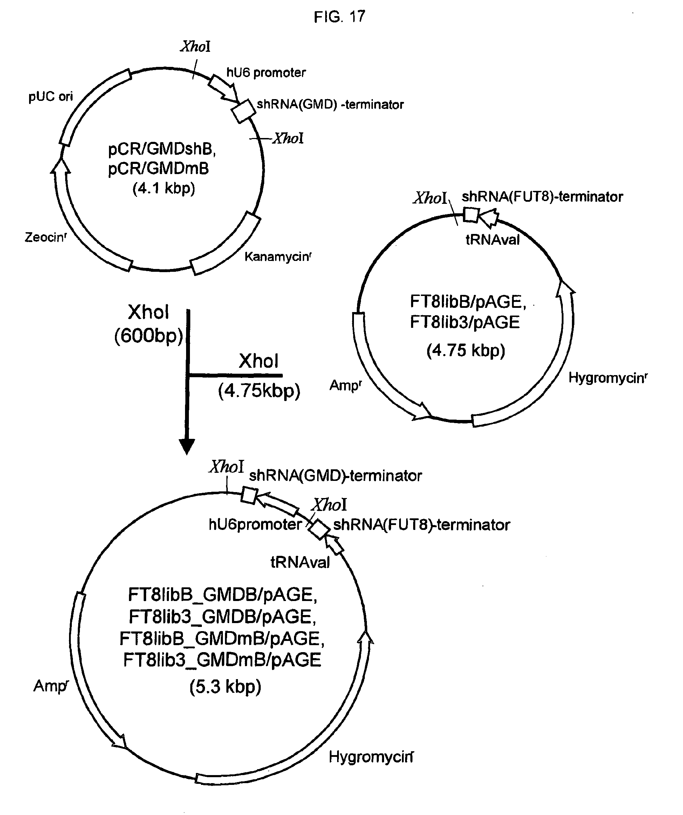

Fig. 17 shows the constructions of plasmids FT8libB_GMDB/pAGE, FT8lib3_GMDB/pAGE, FT8libB_GMDmB/pAGE and FT8lib3_GMDmB/pAGE.

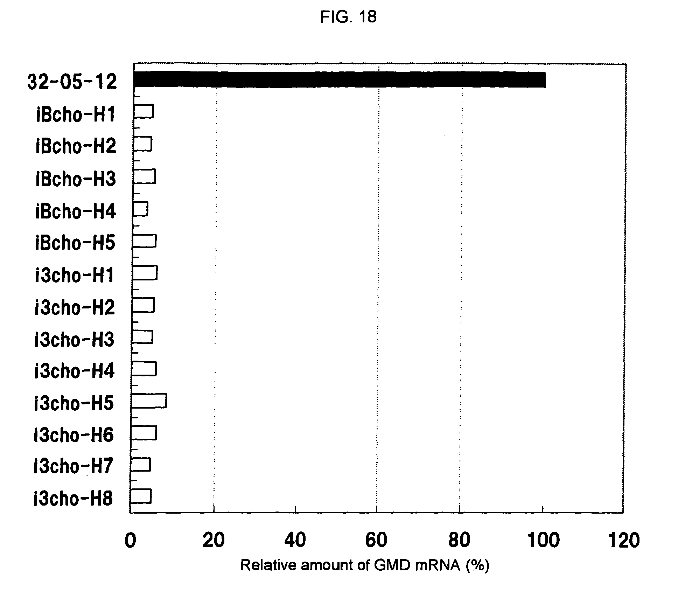

Fig. 18 shows the relative amount of GMD mRNA expressed in each clone obtained by introducing the GMD-targeting siRNA and FUT8-targeting siRNA co-expression vector into clone 32-05-12 which was CHO/DG44 cell-derived anti-CCR4 antibody-producing clone. The abscissa indicates the relative amount of GMD mRNA to the amount of β-actin mRNA when the amount in the parent clone 32-05-12 was assumed to be 100, and the ordinate indicates each clones. In the drawing, black and outline bars indicate the relative amount of GMD mRNA of the parent clone and of each clones obtained by introducing the GMD- and FUT8-targeting siRNA co-expression vector, respectively, when the amount in the parent clone 32-05-12 was assumed to be 100.

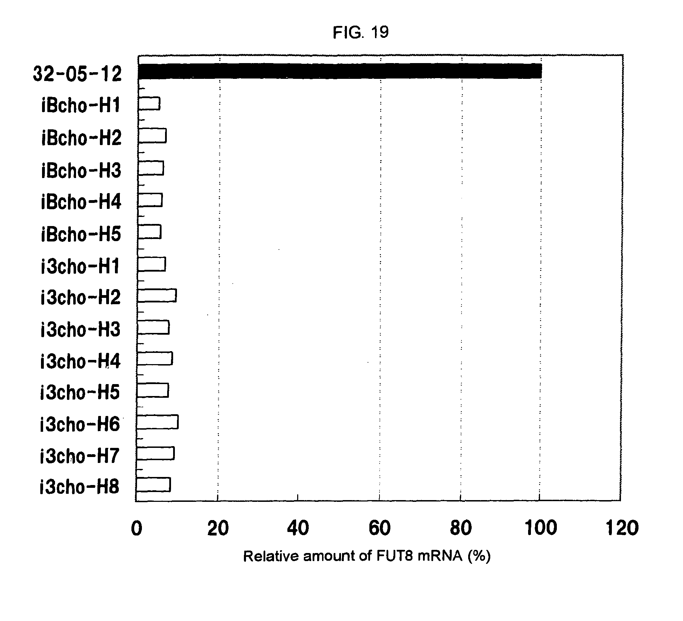

Fig. 19 shows the relative amount of FUT8 mRNA expressed in each clone obtained by introducing the GMD-targeting siRNA and FUT8-targeting siRNA co-expression vector into clone 32-05-12 which was CHO/DG44 cell-derived anti-CCR4 antibody-producing clone. The abscissa indicates the relative amount of FUT8 mRNA to the amount of β-actin mRNA when the amount in the parent clone 32-05-12 was assumed to be 100, and the ordinate indicates each clones. In the drawing, black and outline bars indicate the relative amount of FUT8 mRNA of the parent clone and of each clones obtained by introducing the GMD-targeting siRNA and FUT8-targeting siRNA co-expression vector, respectively, when the amount in the parent clone was assumed to be 100.

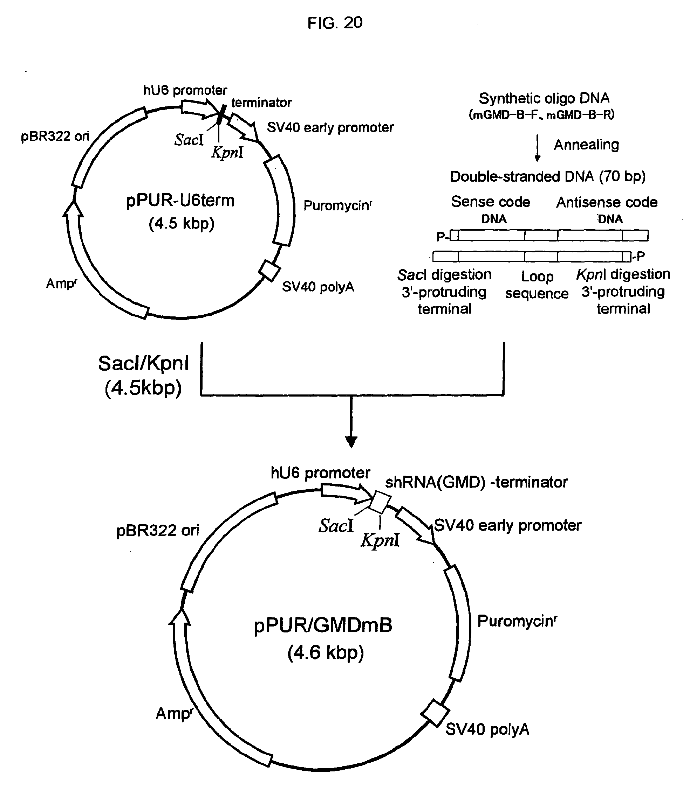

Fig. 20 shows the construction of plasmid pPUR/GMDmB.

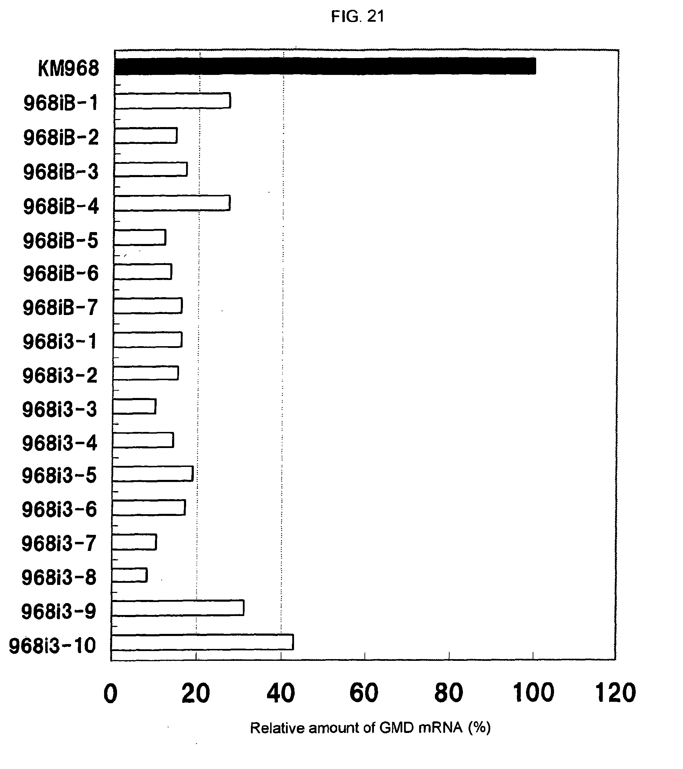

Fig. 21 shows the relative amount of GMD mRNA expressed in each clone obtained by introducing the mouse GMD-targeting siRNA and FUT8-targeting siRNA co-expression vector into clone KM968 which was SP2/0 cell-derived anti-GM₂ antibody-producing clone. The abscissa indicates the relative amount of GMD mRNA to the amount of β-actin mRNA when the amount in the parent clone KM968 was assumed to be 100, and the ordinate indicates each clones. In the drawing, black and outline bars indicate the relative amount of GMD mRNA of the parent clone and of each clones obtained by introducing the GMD-targeting siRNA and FUT8-targeting siRNA co-expression vector, respectively, when the amount in the parent clone KM968 was assumed to be 100.

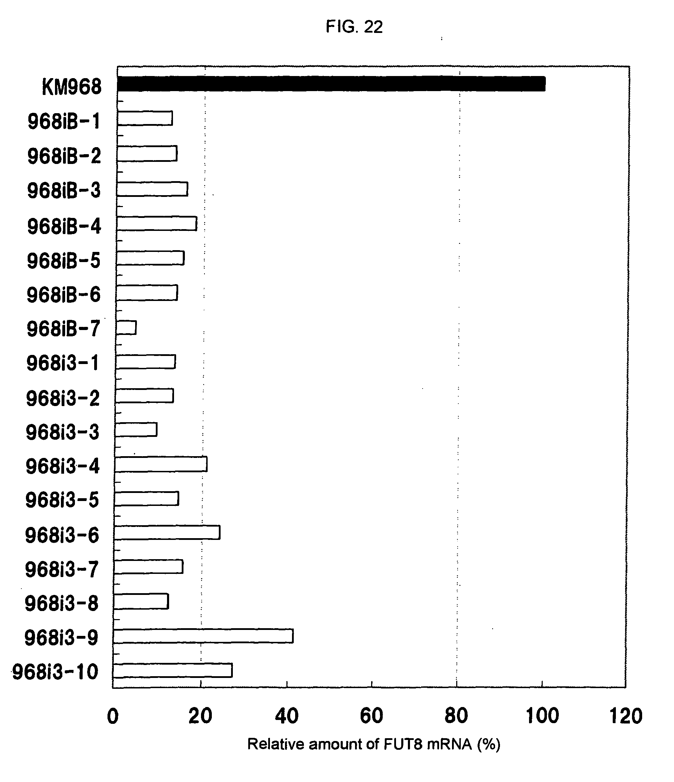

Fig. 22 shows the relative amount of FUT8 mRNA expressed in each clone obtained by introducing the mouse GMD-targeting siRNA and FUT8-targeting siRNA co-expression vector into clone KM968 which was SP2/0 cell-derived anti-GM₂ antibody-producing clone. The abscissa indicates the relative amount of FUT8 mRNA to the amount of β-actin mRNA when the amount in the parent clone KM968 was assumed to be 100, and the ordinate indicates each clones. In the drawing, black and outline bars indicate the relative amount of FUT8 mRNA of the parent clone and of each clones obtained by introducing the GMD-targeting siRNA and FUT8-targeting siRNA co-expression vector, respectively, when the amount in the parent clone KM968 was assumed to be 100.

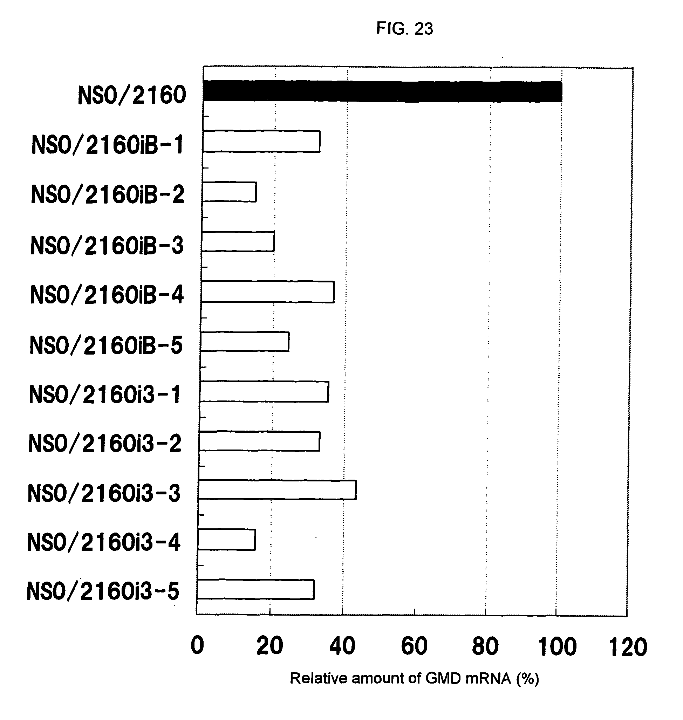

Fig. 23 shows the relative amount of GMD mRNA expressed in each clone obtained by introducing the mouse GMD-targeting siRNA and FUT8-targeting siRNA co-expression vector into clone NS0/2160 which was NS0 cell-derived anti-CCR4 antibody-producing clone. The abscissa indicates the relative amount of GMD mRNA to the amount of β-actin mRNA when that amount in the parent clone NS0/2160 was assumed to be 100, and the ordinate indicates each clones. In the drawing, black and outline bars indicate the relative amount of GMD mRNA of the parent clone and of each clones obtained by introducing the GMD-targeting siRNA and FUT8-targeting siRNA co-expression vector, respectively, when the amount in the parent clone NS0/2160 was assumed to be 100.

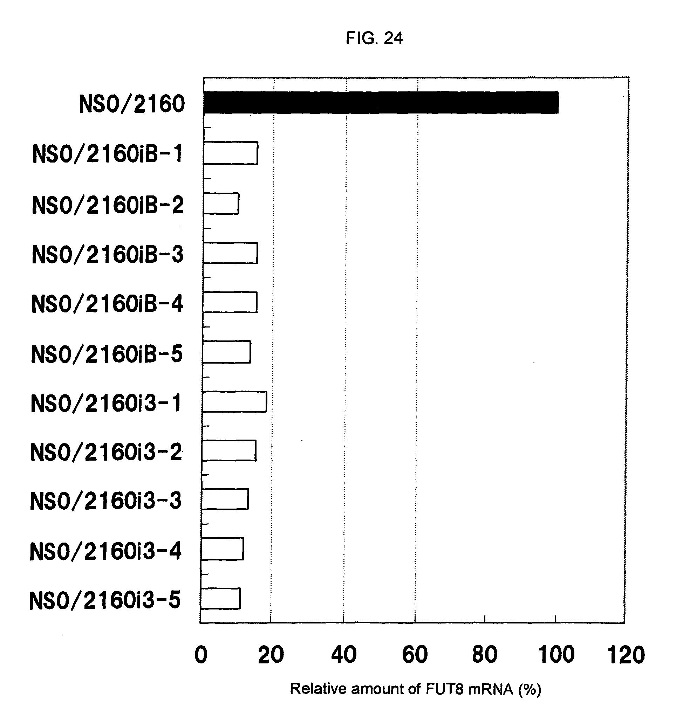

Fig. 24 shows the relative amount of FUT8 mRNA expressed in each clone obtained by introducing mouse GMD-targeting siRNA and FUT8-targeting siRNA co-expression vector into clone NS0/2160 which was NS0 cell-derived anti-CCR4 antibody-producing clone. The abscissa indicates the relative amount of GMD mRNA to the amount of β-actin mRNA when the amount in the parent clone NS0/2160 was assumed to be 100 and the ordinate indicates each clones. In the drawing, black and outline bars indicate the relative amount of FUT8 mRNA of the parent clone and of each clones obtained by introducing the GMD-targeting siRNA and FUT8-targeting siRNA co-expression vector, respectively, when the amount in the parent clone NS0/2160 was assumed to be 100.

### BEST MODE FOR CARRYING OUT THE INVENTION

In the present invention, the enzyme relating to synthesis of an intracellular sugar nucleotide, GDP-fucose (hereinafter also referred to as "GDP-fucose synthase") may be any enzyme, so long as it is an enzyme relating to synthesis of an intracellular sugar nucleotide, GDP-fucose, as a supply source of fucose to a sugar chain in a cell. Also, the GDP-fucose synthase in the present invention includes an enzyme which has influence on synthesis of an intracellular sugar nucleotide, GDP-fucose, and the like.

The intracellular GDP-fucose is supplied by a *de novo* synthesis pathway or a salvage synthesis pathway. Thus, all enzymes and proteins relating to the synthesis pathways are included in the GDP-fucose synthase.

The GDP-fucose synthase relating to the *de novo* synthesis pathway includes an enzyme catalyzing a reaction which converts GDP-mannose into GDP-4-keto,6-deoxy-GDP-mannose, an enzyme catalyzing a reaction which converts GDP-4-keto,6-deoxy-GDP-mannose into GDP-fucose, and the like.

As the enzyme catalyzing a reaction which converts GDP-mannose into GDP-4-keto,6-deoxy-GDP-mannose in the present invention, an enzyme catalyzing a dehydration reaction which converts GDP-mannose into GDP-4-keto,6-deoxy-GDP-mannose is preferably used. The enzyme catalyzing a dehydration reaction which converts GDP-mannose into GDP-4-keto,6-deoxy-GDP-mannose includes GDP-mannose 4,6-dehydratase.

The enzyme catalyzing a reaction which converts GDP-4-keto,6-deoxy-GDP-mannose into GDP-fucose in the present invention includes an enzyme catalyzing a reaction which converts GDP-4-keto,6-deoxy-GDP-mannose into GDP-4-keto,6-deoxy-GDP-fucose, an enzyme catalyzing a reaction which reduces the 4-position of GDP-4-keto,6-deoxy-GDP-fucose and the like. Specific examples include GDP-keto-6-deoxymannose 3,5-epimerase, 4-reductase having enzyme activity of catalyzing a relation which converts GDP-4-keto,6-deoxy-GDP-mannose into GDP-4-keto,6-deoxy-GDP-fucose and catalyzing a reaction which reduces the 4-position of GDP-4-keto,6-deoxy-GDP-fucose, and the like.

The GDP-fucose synthase relating to the salvage synthesis pathway includes GDP-beta-L-fucose pyrophosphorylase, fucokinase and the like.

As the enzyme which has influence on the synthesis of an intracellular sugar nucleotide, GDP-fucose, an enzyme which has influence on the activity of the above GDP-fucose synthase and an enzyme which has influence on the structure of substances as the substrate of the enzyme are also included.

The protein relating to transport of an intracellular sugar nucleotide, GDP-fucose, to the Golgi body in the present invention may be any protein, so long as it relates to transport of an intracellular sugar nucleotide, GDP-fucose, to the Golgi body. Specific examples include GDP-fucose transporter and the like.

The cell into which an RNA capable of suppressing the function of an enzyme catalyzing a reaction which converts GDP-mannose into GDP-4-keto,6-deoxy-GDP-mannose is introduced in the present invention may be any cell, so long as it is a cell into which an RNA capable of suppressing the activity or expression of an enzyme catalyzing a reaction which converts GDP-mannose into GDP-4-keto,6-deoxy-GDP-mannose (hereinafter also referred to as "GDP-mannose converting enzyme").

In the present invention, GDP-mannose 4,6-dehydratase is preferably used as the GDP-mannose converting enzyme.

In the present invention, the GDP-mannose 4,6-dehydratase includes a protein encoded by a DNA of the following (a) to (f), a protein of the following (g) to (o) and the like:
(a) a DNA comprising the nucleotide sequence represented by SEQ ID NO:8;
(b) a DNA comprising the nucleotide sequence represented by SEQ ID NO:9;
(c) a DNA comprising the nucleotide sequence represented by SEQ ID NO:10;
(d) a DNA which hybridizes with a DNA consisting of the nucleotide sequence represented by SEQ ID NO:8 under stringent conditions and encodes a protein having GDP-mannose 4,6-dehydratase activity;
(e) a DNA which hybridizes with a DNA consisting of the nucleotide sequence represented by SEQ ID NO:9 under stringent conditions and encodes a protein having GDP-mannose 4,6-dehydratase activity;
(f) a DNA which hybridizes with a DNA consisting of the nucleotide sequence represented by SEQ ID NO:10 under stringent conditions and encodes a protein having GDP-mannose 4,6-dehydratase activity;
(g) a protein comprising the amino acid sequence represented by SEQ ID NO:11;
(h) a protein comprising the amino acid sequence represented by SEQ ID NO:12;
(i) a protein comprising the amino acid sequence represented by SEQ ID NO:13;
(j) a protein consisting of an amino acid sequence in which one or more amino acid(s) is/are deleted, substituted, inserted and/or added in the amino acid sequence represented by SEQ ID NO:11 and having GDP-mannose 4,6-dehydratase activity;
(k) a protein consisting of an amino acid sequence in which one or more amino acid(s) is/are deleted, substituted, inserted and/or added in the amino acid sequence represented by SEQ ID NO:12 and having GDP-mannose 4,6-dehydratase activity;
(l) a protein consisting of an amino acid sequence in which one or more amino acid(s) is/are deleted, substituted, inserted and/or added in the amino acid sequence represented by SEQ ID NO:13 and having GDP-mannose 4,6-dehydratase activity;
(m) a protein consisting of an amino acid sequence which has 80% or more homology to the amino acid sequence represented by SEQ ID NO:11 and having GDP-mannose 4,6-dehydratase activity;
(n) a protein consisting of an amino acid sequence which has 80% or more homology to the amino acid sequence represented by SEQ ID NO:12 and having GDP-mannose 4,6-dehydratase activity;
(o) a protein consisting of an amino acid sequence which has 80% or more homology to the amino acid sequence represented by SEQ ID NO:13 and having GDP-mannose 4,6-dehydratase activity.

Also, the DNA encoding the amino acid sequence of GDP-mannose 4,6-dehydratase includes a DNA comprising the nucleotide sequence represented by any one of SEQ ID NOs:8 to 10, a DNA which hybridizes with a DNA consisting of the nucleotide sequence represented by any one of SEQ ID NOs:8 to 10 under stringent conditions and encodes an amino acid sequence having GDP-mannose 4,6-dehydratase activity, and the like.

Also, the present invention relates to a cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, or an RNA capable of suppressing the function of a protein relating to transport of an intracellular sugar nucleotide, GDP-fucose, to the Golgi body are introduced.

The cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, or an RNA capable of suppressing the function of a protein relating to transport of an intracellular sugar nucleotide, GDP-fucose, to the Golgi body are introduced in the present invention may be any cell, so long as it is a cell into which an RNA capable of suppressing the activity or expression of an enzyme relating to modification of a sugar chain in which 1-position of fucose is bound to 6-position of N-acetylglucosamine in the reducing end through α-bond in the complex type N-glycoside-linked sugar chain (hereinafter also referred to as "α1,6-fucose modifying enzyme"), and an RNA capable of suppressing the activity or expression of an enzyme relating to synthesis of an intracellular sugar nucleotide, GDP-fucose, or an RNA capable of suppressing the activity or expression of a protein relating to transport of an intracellular sugar nucleotide, GDP-fucose, to the Golgi body are introduced. A cell into which an RNA capable of suppressing the activity or expression of an α1,6- fucose modifying enzyme and an RNA capable of suppressing the activity or expression of an enzyme relating to synthesis of an intracellular sugar nucleotide, GDP-fucose, are introduced is preferred, and a cell into which an RNA capable of suppressing the activity or expression of an α1,6- fucose modifying enzyme and an RNA capable of suppressing the activity or expression of an enzyme catalyzing a reaction which converts GDP-mannose into GDP-4-keto,6-deoxy-GDP-mannose (GDP-mannose converting enzyme) is more preferred.

In the present invention, the α1,6-fucose modifying enzymes include any enzyme, so long as it is an enzyme relating to modification of a sugar chain in which 1-position of fucose is bound to 6-position of N-acetylglucosamine in the reducing end through α-bond in the complex type N-glycoside-linked sugar chain.

Specific examples of the α1,6-fucose modifying enzyme include α1,6-fucosyltransferase, α-L-fucosidase and the like.

The α1,6-fucosyltransferase includes a protein encoded by a DNA of the following (a) to (h), a protein of the following (i) to (t), and the like:
(a) a DNA comprising the nucleotide sequence represented by SEQ ID NO:1;
(b) a DNA comprising the nucleotide sequence represented by SEQ ID NO:2;
(c) a DNA comprising the nucleotide sequence represented by SEQ ID NO:3;
(d) a DNA comprising the nucleotide sequence represented by SEQ ID NO:4;
(e) a DNA which hybridizes with a DNA consisting of the nucleotide sequence represented by SEQ ID NO:1 under stringent conditions and encodes a protein having α1,6-fucosyltransferase activity;
(f) a DNA which hybridizes with a DNA consisting of the nucleotide sequence represented by SEQ ID NO:2 under stringent conditions and encodes a protein having α1,6-fucosyltransferase activity;
(g) a DNA which hybridizes with a DNA consisting of the nucleotide sequence represented by SEQ ID NO:3 under stringent conditions and encodes a protein having α1,6-fucosyltransferase activity;
(h) a DNA which hybridizes with a DNA consisting of the nucleotide sequence represented by SEQ ID NO:4 under stringent conditions and encodes a protein having α1,6-fucosyltransferase activity;
(i) a protein comprising the amino acid sequence represented by SEQ ID NO: 5;
(j) a protein comprising the amino acid sequence represented by SEQ ID NO:6;
(k) a protein comprising the amino acid sequence represented by SEQ ID NO:7;
(l) a protein comprising the amino acid sequence represented by SEQ ID NO:84;
(m) a protein consisting of an amino acid sequence in which one or more amino acid(s) is/are deleted, substituted, inserted and/or added in the amino acid sequence represented by SEQ ID NO:5 and having α1,6-fucosyltransferase activity;
(n) a protein consisting of an amino acid sequence in which one or more amino acid(s) is/are deleted, substituted, inserted and/or added in the amino acid sequence represented by SEQ ID NO:6 and having α1,6-fucosyltransferase activity;
(o) a protein consisting of an amino acid sequence in which one or more amino acid(s) is/are deleted, substituted, inserted and/or added in the amino acid sequence represented by SEQ ID NO:7 and having α1,6-fucosyltransferase activity;
(p) a protein consisting of an amino acid sequence in which one or more amino acid(s) is/are deleted, substituted, inserted and/or added in the amino acid sequence represented by SEQ ID NO:84 and having α1,6-fucosyltransferase activity;
(q) a protein consisting of an amino acid sequence which has 80% or more homology to the amino acid sequence represented by SEQ ID NO:5 and having α1,6-fucosyltransferase activity;
(r) a protein consisting of an amino acid sequence which has 80% or more homology to the amino acid sequence represented by SEQ ID NO:6 and having α1,6-fucosyltransferase activity;
(s) a protein consisting of an amino acid sequence which has 80% or more homology to the amino acid sequence represented by SEQ ID NO:7 and having α1,6-fucosyltransferase activity;
(t) a protein consisting of an amino acid sequence which has 80% or more homology to the amino acid sequence represented by SEQ ID NO:84 and having α1,6-fucosyltransferase activity.

Also, the DNA encoding the amino acid sequence of the α1,6-fucosyltransferase includes a DNA comprising the nucleotide sequence represented by any one of SEQ ID NOs:1 to 4, a DNA which hybridizes with a DNA consisting of the nucleotide sequence represented by any one of SEQ ID NOs:1 to 4 under stringent conditions and encodes an amino acid sequence having α1,6-fucosyltransferase activity, and the like.

In the present invention, a DNA which can hybridize under stringent conditions is a DNA obtained, e.g., by a method such as colony hybridization, plaque hybridization or Southern blot hybridization using a DNA such as the DNA having the nucleotide sequence represented by any one of SEQ ID NOs:1 to 4 and 8 to 10 or a partial fragment thereof as the probe, and specifically includes a DNA which can be identified by carrying out hybridization at 65°C in the presence of 0.7 to 1.0 mol/L sodium chloride using a filter to which colony- or plaque-derived DNA are immobilized, and then washing the filter at 65°C using 0.1 to 2 × SSC solution (composition of the 1 × SSC solution comprising 150 mmol/L sodium chloride and 15 mmol/L sodium citrate). The hybridization can be carried out in accordance with the methods described, e.g., in Molecular Cloning, A Laboratory Manual, Second Edition, Cold Spring Harbor Laboratory Press (1989) (hereinafter referred to as "Molecular Cloning, Second Edition"), Current Protocols in Molecular Biology, John Wiley & Sons, 1987-1997 (hereinafter referred to as "Current Protocols in Molecular Biology"); DNA Cloning 1: Core Techniques, A Practical Approach, Second Edition, Oxford University (1995); and the like. The hybridizable DNA includes a DNA having at least 60% or more, preferably 70% or more, more preferably 80% or more, still more preferably 90% or more, far more preferably 95% or more, and most preferably 98% or more, homology with the nucleotide sequence represented by any one of SEQ ID NOs:1 to 4 and 8 to 10.

In the present invention, the protein which comprises an amino acid sequence in which one or more amino acid(s) is/are deleted, substituted, inserted and/or added in the amino acid sequence represented by any one of SEQ ID NOs:5 to 7 and 84 and has α1,6-fucosyltransferase activity or the protein which comprises an amino acid sequence in which one or more amino acid(s) is/are deleted, substituted, inserted and/or added in the amino acid sequence represented by any one of SEQ ID NOs:11 to 13 and has GDP-mannose 4,6-dehydratase activity can be obtained, e.g., by introducing a site-directed mutation into a DNA encoding a protein having the amino acid sequence represented by any one of SEQ ID NOs: 5 to 7 and 84 or any one of SEQ ID NOs:11 to 13, using the site-directed mutagenesis described, e.g., in *Molecular Cloning,* Second Edition; *Current Protocols in Molecular Biology;* Nucleic Acids Research, 10, 6487 (1982); Proc. Natl. Acad. Sci. USA, 79, 6409 (1982); Gene, 34, 315 (1985); Nucleic Acids Research, 13, 4431 (1985); Proc. Natl. Acad. Sci. USA, 82, 488 (1985); and the like. The number of amino acids to be deleted, substituted, inserted and/or added is one or more, and the number is not particularly limited, but is a number which can be deleted, substituted or added by a known technique such as the site-directed mutagenesis, e.g., it is 1 to several tens, preferably 1 to 20, more preferably 1 to 10, and most preferably 1 to 5.

In the present invention, the protein which comprises an amino acid sequence having 80% or more homology with the amino acid sequence represented by any one of SEQ ID NOs: 11 to 13 and has GDP-mannose 4,6-dehydratase activity is a protein having at least 80% or more, preferably 85% or more, more preferably 90% or more, still more preferably 95% or more, far more preferably 97% or more, and most preferably 99% or more, homology with the amino acid sequence represented by any one of SEQ ID NOs:11 to 13 and having GDP-mannose 4,6-dehydratase activity.

Also, in the present invention, the protein which comprises an amino acid sequence having 80% or more homology with the amino acid sequence represented by any one of SEQ ID NOs:5 to 7 and 84 and has α1,6-fucosyltransferase activity is a protein having at least 80% or more, preferably 85% or more, more preferably 90% or more, still more preferably 95% or more, far more preferably 97% or more, and most preferably 99% or more, homology with the amino acid sequence represented by any one of SEQ ID NOs:5 to 7 and 84 and having α1,6-fucosyltransferase activity.

The number of the homology described in the present invention may be a number calculated by using a known homology search program, unless otherwise indicated. Regarding the nucleotide sequence, the number may be calculated by using a default parameter in BLAST [J. Mol., Biol., 215, 403 (1990)] or the like, and regarding the amino acid sequence, the number may be calculated by using a default parameter in BLAST2 [Nucleic Acids Res., 25, 3389 (1997); Genome Res., 7, 649 (1997); http://www.ncbi.nlm.nih.gov/Education/BLASTinfo/information3.html] or the like.

As the default parameter, G (cost to open gap) is 5 for the nucleotide sequence and 11 for the amino acid sequence; -E (cost to extend gap) is 2 for the nucleotide sequence and 1 for the amino acid sequence; -q (penalty for nucleotide mismatch) is -3; -r (reward for nucleotide match) is 1; -e (expect value) is 10; -W (wordsize) is 11 residues for the nucleotide sequence and 3 residues for the amino acid sequence; -y (dropoff (X) for blast extensions in bits) is 20 for blastn and 7 for a program other than blastn; -X (X dropoff value for gapped alignment in bits) is 15; and -Z (final X dropoff value for gapped alignment in bits) is 50 for blastn and 25 for a program other than blastn (http://www.ncbi.nlm.nih.gov/blast/html/blastcgihelp.html). Additionally, the analysis software of the amino acid sequence also includes FASTA [Methods in Enzymology, 183, 63(1990)], and the like.

The cell used in the present invention may be any cell, so long as it can express a glycoprotein such as an antibody molecule. Examples include an yeast, an animal cell, an insect cell, a plant cell and the like, and an animal cell is preferred. Preferred examples of the animal cell include a CHO cell derived from a Chinese hamster ovary tissue, a rat myeloma cell line YB2/3HL.P2.G11.16Ag.20 cell, a mouse myeloma cell line NS0 cell, a mouse myeloma SP2/0-Ag14 cell, a BHK cell derived from a syrian hamster kidney tissue, an antibody-producing-hybridoma cell, a human leukemia cell line Namalwa cell, an embryonic stem cell, a fertilized egg cell, and the like.

The cell into which an RNA capable of suppressing the function of a GDP-mannose converting enzyme is introduced or the cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, or an RNA capable of suppressing the function of a protein relating to transport of an intracellular sugar nucleotide, GDP-fucose, to the Golgi body are introduced in the present invention (hereinafter both being referred to as "the cell of the present invention" as a whole) is resistant to a lectin which recognizes a sugar chain structure in which 1-position of fucose is bound to 6-position of N-acetylglucosamine in the reducing end through α-bond in a N-glycoside-linked sugar chain. The parent cell into which the RNA is not introduced is not resistant to the lectin.

Accordingly, in the present invention, the cell of the present invention includes a cell such as a yeast, an animal cell, an insect cell or a plant cell which can produce a glycoprotein composition and is resistant to a lectin which recognizes a sugar chain structure in which 1-position of fucose is bound to 6-position of N-acetylglucosamine in the reducing end through α-bond in a complex type N-glycoside-linked sugar chain. Examples include a hybridoma cell, a host cell for producing a human antibody or a humanized antibody, an embryonic stem cell and fertilized egg cell for producing a transgenic non-human animal which produces a human antibody, a plant callus cell for producing a transgenic plant which produces a human antibody, a myeloma cell, a cell derived from a transgenic non-human animal and the like which are resistant to lectin which recognizes a sugar chain structure in which 1-position of fucose is bound to 6-position of N-acetylglucosamine in the reducing end through α-bond in a complex type N-glycoside-linked sugar chain. The myeloma cell can be used as a fusion cell for producing a hybridoma cell. Also, a hybridoma cell can be produced by immunizing a transgenic non-human animal with an antigen and using antibody-producing cells such as spleen cells of the animal.

The cell resistant to a lectin is a cell of which growth is not inhibited when the cell is cultured by applying the lectin to a culture medium at an effective concentration.

In the present invention, the effective concentration of the lectin which does not inhibit the growth can be adjusted depending on the cell line used as the parent cell, and is generally 10 µg/ml to 10.0 mg/ml, preferably 0.5 to 2.0 mg/ml. When an RNA capable of suppressing the function of a GDP-mannose converting enzyme is introduced into a parent cell, or when an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, or an RNA capable of suppressing the function of a protein relating to transport of an intracellular sugar nucleotide, GDP-fucose, to the Golgi body are introduced into a parent cell, the effective concentration of the lectin is a concentration in which the parent cell cannot normally grow or higher than the concentration, and is a concentration which is preferably the same degree, more preferably 2 to 5 times, still more preferably at least 10 times, and most preferably at least 20 times, higher than the concentration in which the parent cell cannot normally grow.

The parent cell means a cell before introduction of an RNA capable of suppressing the function of a GDP-mannose converting enzyme or a cell before introduction of an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, or an RNA capable of suppressing the function of a protein relating to transport of an intracellular sugar nucleotide, GDP-fucose, to the Golgi body.

Although the parent cell is not particularly limited, the following cells are exemplified.

The parent cell of NS0 cell includes NS0 cells described in literatures such as BIO/TECHNOLOGY, 10, 169 (1992) and Biotechnol. Bioeng., 73, 261 (2001). Also, it includes cell line NS0 (RCB 0213) registered at RIKEN Cell Bank, The Institute of Physical and Chemical Research, sub-cell lines obtained by adapting these cell lines to various media in which they can grow, and the like.

The parent cell of SP2/0-Ag14 cell includes SP2/0-Ag14 cells described in literatures such as J. Immunol., 126, 317 (1981), Nature, 276, 269 (1978) and Human Antibodies and Hybridomas, 3, 129 (1992). Also, it includes SP2/0-Ag14 cell (ATCC CRL-1581) registered at ATCC, sub-cell lines obtained by adapting these cell lines to various media in which they can grow (ATCC CRL-1581.1), and the like.

The parent cell of CHO cell derived from Chinese hamster ovary tissue includes CHO cells described in literatures such as Journal of Experimental Medicine, 108, 945 (1958), Proc. Natl. Acad. Sci. USA, 60, 1275 (1968), Genetics, 55, 513 (1968), Chromosoma, 41, 129 (1973), Methods in Cell Science, 18, 115 (1996), Radiation Research, 148, 260 (1997), Proc. Natl. Acad Sci. USA, 17, 4216 (1980), Proc. Natl. Acad Sci. USA, 60, 1275 (1968), Cell, 6, 121 (1975) and Molecular Cell Genetics, Appendix I, II (p. 883-900). Also, it includes cell line CHO-K1 (ATCC CCL-61), cell line DUXB11 (ATCC CRL-9096) and cell line Pro-5 (ATCC CRL-1781) registered at ATCC, commercially available cell line CHO-S (Cat # 11619 of Lifetechnologies), sub-cell lines obtained by adapting these cell lines to various media in which they can grow, and the like.

The parent cell of a rat myeloma cell line YB2/3HL.P2.G11.16Ag.20 cell includes cell lines established from Y3/Agl.2.3 cell (ATCC CRL-1631) such as YB2/3HL.P2.G11.16Ag.20 cell described in literatures such as J. Cell. Biol., 93, 576 (1982) and Methods Enzymol., 73B, 1 (1981). Also, it includes YB2/3HL.P2.G11.16Ag.20 cell (ATCC CRL-1662) registered at ATCC, sub-lines obtained by adapting these cell lines to various media in which they can grow, and the like.

In addition to the above, the parent cell used in the present invention includes a human leukemia cell line NM-F9 cell (DSM ACC2605, WO05/17130), a human embryonic retinal cell line PER.C6 cell (ECACC No. 96022940, US6855544), sub-lines obtained by adapting these cell lines to various media in which they can grow, and the like.

The lectin which recognizes a sugar chain structure in which 1-position of fucose is bound to 6-position of N-acetylglucosamine in the reducing end through α-bond in the complex type N-glycoside-linked sugar chain may be any lectin, so long as it can recognize the sugar chain structure. Examples include a *Lens culinaris* agglutinin LCA (lentil agglutinin derived from *Lens culinaris*), *a Pisum sativum* agglutinin PSA (pea lectin derived from *Pisum sativum*), *a Vicia faba* agglutinin VFA (agglutinin derived from *Vicia faba*), an *Aleuria aurantia* lectin AAL (lectin derived from *Aleuria aurantia)* and the like.

In the present invention, the RNA capable of suppressing the function of an enzyme relating to modification of a sugar chain in which 1-position of fucose is bound to 6-position ofN-acetylglucosamine in the reducing end through α-bond in the complex type N-glycoside-linked sugar chain, and the RNA capable of suppressing the function of an enzyme relating to synthesis of an intracellular sugar nucleotide, GDP-fucose, or the RNA capable of suppressing the function of a protein relating to transport of an intracellular sugar nucleotide, GDP-fucose, to the Golgi body may be any RNAs, so long as they comprise an RNA and its complementary RNA and is a double-stranded RNA capable of decreasing the amount of mRNA of an α1,6-fucose modifying enzyme and a double-stranded RNA capable of decreasing the amount of mRNA of an enzyme relating to synthesis of an intracellular sugar nucleotide, GDP-fucose, or the amount of mRNA of a protein relating to transport of an intracellular sugar nucleotide, GDP-fucose, to the Golgi body, respectively. Regarding the length of the RNA, a continuous RNA of 10 to 40, preferably 10 to 30, and more preferably 15 to 30, is exemplified.

The RNA capable of suppressing the function of an enzyme relating to synthesis of an intracellular sugar nucleotide, GDP-fucose, or the RNA capable of suppressing the function of a protein relating to transport of an intracellular sugar nucleotide, GDP-fucose, to the Golgi body is preferably an RNA capable of decreasing the amount of mRNA of a GDP-mannose converting enzyme.

Examples include:
(a) an RNA corresponding to a DNA consisting of a nucleotide sequence represented by a sequence of continued 10 to 40 bases in the nucleotide sequence represented by SEQ ID NO:8;
(b) an RNA corresponding to a DNA consisting of a nucleotide sequence represented by a sequence of continued 10 to 40 bases in the nucleotide sequence represented by SEQ ID NO:9; and
(c) an RNA corresponding to a DNA consisting of a nucleotide sequence represented by a sequence of continued 10 to 40 bases in the nucleotide sequence represented by SEQ ID NO:10.

Preferred examples include:
(1) an RNA comprising the nucleotide sequence represented by SEQ ID NO:37;
(2) an RNA consisting of a nucleotide sequence in which one or a several nucleotide(s) is/are deleted, substituted, inserted and/or added in the nucleotide sequence represented by SEQ ID NO:37 and having activity of suppressing the function of a GDP-mannose converting enzyme;
(3) an RNA comprising the nucleotide sequence represented by SEQ ID NO:57;
(4) an RNA consisting of a nucleotide sequence in which one or a several nucleotide(s) is/are deleted, substituted, inserted and/or added in the nucleotide sequence represented by SEQ ID NO:57 and having activity of suppressing the function of a GDP-mannose converting enzyme;
(5) an RNA comprising the nucleotide sequence represented by SEQ ID NO: 58;
(6) an RNA consisting of a nucleotide sequence in which one or a several nucleotide(s) is/are deleted, substituted, inserted and/or added in the nucleotide sequence represented by SEQ ID NO:58 and having activity of suppressing the function of a GDP-mannose converting enzyme.

In the RNAs of the above-described (a) to (c) and (1) to (6), it is preferred that the RNAs in (a), (1) and (2) are used in a parent cell derived from a hamster, the RNAs in (b), (3) and (4) are used in a parent cell derived from a human, and the RNAs in (c), (5) and (6) are used in a parent cell line derived from a mouse, as an RNA capable of suppressing the function of a GDP-mannose converting enzyme.

In the present invention, the RNA capable of suppressing the function of an α1,6-fucose modifying enzyme is preferably an RNA capable of decreasing the amount of mRNA of an α1,6-fucose modifying enzyme. The length of the RNA is, for example, 10 to 40, preferably 10 to 30 and more preferably 15 to 30.

Examples include:
(d) an RNA corresponding to a DNA consisting of a nucleotide sequence represented by a sequence of continued 10 to 40 bases in the nucleotide sequence represented by SEQ ID NO:1;
(e) an RNA corresponding to a DNA consisting of a nucleotide sequence represented by a sequence of continued 10 to 40 bases in the nucleotide sequence represented by SEQ ID NO:2;
(f) an RNA corresponding to a DNA consisting of a nucleotide sequence represented by a sequence of continued 10 to 40 bases in the nucleotide sequence represented by SEQ ID NO:3;
(g) an RNA corresponding to a DNA consisting of a nucleotide sequence represented by a sequence of continued 10 to 40 bases in the nucleotide sequence represented by SEQ ID NO:4.

Preferred examples include:
(7) an RNA comprising the nucleotide sequence represented by SEQ ID NO:14;
(8) an RNA consisting of a nucleotide sequence in which one or a several nucleotide(s) is/are deleted, substituted, inserted and/or added in the nucleotide sequence represented by SEQ ID NO:14 and having activity of suppressing the function of an α1,6-fucose modifying enzyme;
(9) an RNA comprising the nucleotide sequence represented by SEQ ID NO:15;
(10) an RNA consisting of a nucleotide sequence in which one or a several nucleotide(s) is/are deleted, substituted, inserted and/or added in the nucleotide sequence represented by SEQ ID NO:15 and having activity of suppressing the function of an α1,6-fucose modifying enzyme;
(11) an RNA comprising the nucleotide sequence represented by SEQ ID NO:16;
(12) an RNA consisting of a nucleotide sequence in which one or a several nucleotide(s) is/are deleted, substituted, inserted and/or added in the nucleotide sequence represented by SEQ ID NO:16 and having activity of suppressing the function of an α1,6-fucose modifying enzyme;
(13) an RNA comprising the nucleotide sequence represented by SEQ ID NO:17;
(14) an RNA consisting of a nucleotide sequence in which one or a several nucleotide(s) is/are deleted, substituted, inserted and/or added in the nucleotide sequence represented by SEQ ID NO:17 and having activity of suppressing the function of an α1,6-fucose modifying enzyme;
(15) an RNA comprising the nucleotide sequence represented by SEQ ID NO:18;
(16) an RNA consisting of a nucleotide sequence in which one or a several nucleotide(s) is/are deleted, substituted, inserted and/or added in the nucleotide sequence represented by SEQ ID NO:18 and having activity of suppressing the function of an α1,6-fucose modifying enzyme;
(17) an RNA comprising the nucleotide sequence represented by SEQ ID NO:19;
(18) an RNA consisting of a nucleotide sequence in which one or a several nucleotide(s) is/are deleted, substituted, inserted and/or added in the nucleotide sequence represented by SEQ ID NO:19 and having activity of suppressing the function of an α1,6-fucose modifying enzyme;
(19) an RNA comprising the nucleotide sequence represented by SEQ ID NO:20;
(20) an RNA consisting of a nucleotide sequence in which one or a several nucleotide(s) is/are deleted, substituted, inserted and/or added in the nucleotide sequence represented by SEQ ID NO:20 and having activity of suppressing the function of an α1,6-fucose modifying enzyme;
(21) an RNA comprising the nucleotide sequence represented by SEQ ID NO:21;
(22) an RNA consisting of a nucleotide sequence in which one or a several nucleotide(s) is/are deleted, substituted, inserted and/or added in the nucleotide sequence represented by SEQ ID NO:21 and having activity of suppressing the function of an α1,6-fucose modifying enzyme;
(23) an RNA comprising the nucleotide sequence represented by SEQ ID NO:22;
(24) an RNA consisting of a nucleotide sequence in which one or a several nucleotide(s) is/are deleted, substituted, inserted and/or added in the nucleotide sequence represented by SEQ ID NO:22 and having activity of suppressing the function of an α1,6-fucose modifying enzyme;
(25) an RNA comprising the nucleotide sequence represented by SEQ ID NO:23;
(26) an RNA consisting of a nucleotide sequence in which one or a several nucleotide(s) is/are deleted, substituted, inserted and/or added in the nucleotide sequence represented by SEQ ID NO:23 and having activity of suppressing the function of an α1,6-fucose modifying enzyme;
(27) an RNA comprising the nucleotide sequence represented by SEQ ID NO:24;
(28) an RNA consisting of a nucleotide sequence in which one or a several nucleotide(s) is/are deleted, substituted, inserted and/or added in the nucleotide sequence represented by SEQ ID NO:24 and having activity of suppressing the function of an α1,6-fucose modifying enzyme;
(29) an RNA comprising the nucleotide sequence represented by SEQ ID NO:25;
(30) an RNA consisting of a nucleotide sequence in which one or a several nucleotide(s) is/are deleted, substituted, inserted and/or added in the nucleotide sequence represented by SEQ ID NO:25 and having activity of suppressing the function of an α1,6-fucose modifying enzyme;
(31) an RNA comprising the nucleotide sequence represented by SEQ ID NO:26;
(32) an RNA consisting of a nucleotide sequence in which one or a several nucleotide(s) is/are deleted, substituted, inserted and/or added in the nucleotide sequence represented by SEQ ID NO:26 and having activity of suppressing the function of an α1,6-fucose modifying enzyme;
(33) an RNA comprising the nucleotide sequence represented by SEQ ID NO:27;
(34) an RNA consisting of a nucleotide sequence in which one or a several nucleotide(s) is/are deleted, substituted, inserted and/or added in the nucleotide sequence represented by SEQ ID NO:27 and having activity of suppressing the function of an α1,6-fucose modifying enzyme;
(35) an RNA comprising the nucleotide sequence represented by SEQ ID NO:28;
(36) an RNA consisting of a nucleotide sequence in which one or a several nucleotide(s) is/are deleted, substituted, inserted and/or added in the nucleotide sequence represented by SEQ ID NO:28 and having activity of suppressing the function of an α1,6-fucose modifying enzyme;
(37) an RNA comprising the nucleotide sequence represented by SEQ ID NO:29;
(38) an RNA consisting of a nucleotide sequence in which one or a several nucleotide(s) is/are deleted, substituted, inserted and/or added in the nucleotide sequence represented by SEQ ID NO:29 and having activity of suppressing the function of an α1,6-fucose modifying enzyme;
(39) an RNA comprising the nucleotide sequence represented by SEQ ID NO:30;
(40) an RNA consisting of a nucleotide sequence in which one or a several nucleotide(s) is/are deleted, substituted, inserted and/or added in the nucleotide sequence represented by SEQ ID NO:30 and having activity of suppressing the function of an α1,6-fucose modifying enzyme;
(41) an RNA comprising the nucleotide sequence represented by SEQ ID NO:31;
(42) an RNA consisting of a nucleotide sequence in which one or a several nucleotide(s) is/are deleted, substituted, inserted and/or added in the nucleotide sequence represented by SEQ ID NO:31 and having activity of suppressing the function of an α1,6-fucose modifying enzyme;
(43) an RNA comprising the nucleotide sequence represented by SEQ ID NO:32;
(44) an RNA consisting of a nucleotide sequence in which one or a several nucleotide(s) is/are deleted, substituted, inserted and/or added in the nucleotide sequence represented by SEQ ID NO:32 and having activity of suppressing the function of an α1,6-fucose modifying enzyme;
(45) an RNA comprising the nucleotide sequence represented by SEQ ID NO:33;
(46) an RNA consisting of a nucleotide sequence in which one or a several nucleotide(s) is/are deleted, substituted, inserted and/or added in the nucleotide sequence represented by SEQ ID NO:33 and having activity of suppressing the function of an α1,6-fucose modifying enzyme;
(47) an RNA comprising the nucleotide sequence represented by SEQ ID NO:34;
(48) an RNA consisting of a nucleotide sequence in which one or a several nucleotides are more nucleotide(s) is/are deleted, substituted, inserted and/or added in the nucleotide sequence represented by SEQ ID NO:34 and having activity of suppressing the function of an α1,6-fucose modifying enzyme;
(49) an RNA comprising the nucleotide sequence represented by SEQ ID NO:35;
(50) an RNA consisting of a nucleotide sequence in which one or a several nucleotide(s) is/are deleted, substituted, inserted and/or added in the nucleotide sequence represented by SEQ ID NO:35 and having activity of suppressing the function of an α1,6-fucose modifying enzyme;
(51) an RNA comprising the nucleotide sequence represented by SEQ ID NO:85;
(52) an RNA consisting of a nucleotide sequence in which one or a several nucleotide(s) is/are deleted, substituted, inserted and/or added in the nucleotide sequence represented by SEQ ID NO:85 and having activity of suppressing the function of an α1,6-fucose modifying enzyme;
(53) an RNA comprising the nucleotide sequence represented by SEQ ID NO:86;
(54) an RNA consisting of a nucleotide sequence in which one or a several nucleotide(s) is/are deleted, substituted, inserted and/or added in the nucleotide sequence represented by SEQ ID NO:86 and having activity of suppressing the function of an α1,6-fucose modifying enzyme;
(55) an RNA comprising the nucleotide sequence represented by SEQ ID NO:87;
(56) an RNA consisting of a nucleotide sequence in which one or a several nucleotide(s) is/are deleted, substituted, inserted and/or added in the nucleotide sequence represented by SEQ ID NO:87 and having activity of suppressing the function of an α1,6-fucose modifying enzyme;
(57) an RNA comprising the nucleotide sequence represented by SEQ ID NO:88;
(58) an RNA consisting of a nucleotide sequence in which one or a several nucleotide(s) is/are deleted, substituted, inserted and/or added in the nucleotide sequence represented by SEQ ID NO:88 and having activity of suppressing the function of an α1,6-fucose modifying enzyme;
(59) an RNA comprising the nucleotide sequence represented by SEQ ID NO:89;
(60) an RNA consisting of a nucleotide sequence in which one or a several nucleotide(s) is/are deleted, substituted, inserted and/or added in the nucleotide sequence represented by SEQ ID NO:89 and having activity of suppressing the function of an α1,6-fucose modifying enzyme/

In the RNAs of the above-described (d) to (g) and (7) to (60), it is preferred that the RNAs of (d) and (7) to (26) are used as a parent cell derived from a hamster, the RNAs of (e), (11), (12), (23) to (28), (33) to (39), (41), (42), (45), (46), (51), (52), (57) and (58) are used as a parent cell derived from a mouse, the RNA of (f), (7), (8), (11), (12), (19), (20), (23) to (26), (33), (34), (39) to (42), (49), (50), (55) and (56) are used as a parent cell derived from a rat, and the RNAs of (g), (23) to (26), (29) to (34), (37), (38), (43), (44), (47), (48), (53), (54), (59) and (60) are used as a parent cell derived from a human, as an RNA capable of suppressing the function of an α1,6-fucose modifying enzyme.

The nucleotide sequence in which one or a several nucleotide(s) is/are deleted, substituted, inserted and/or added is, for example, a nucleotide sequence in which one or a several nucleotide(s) is/are deleted, substituted, inserted and/or added in the nucleotide sequence represented by any one of SEQ ID NOs:14 to 37, 57, 58 and 85 to 89. A double-stranded RNA formed by the deletion, substitution, insertion and/or addition of the nucleotide may be an RNA in which the nucleotide is deleted, substituted, inserted and/or added in only one of the strands. That is, the double-stranded RNA may not always be a complete complementary strand, so long as the effect of the present invention is obtained.

Also, the present invention relates to a vector comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme relating to synthesis of an intracellular sugar nucleotide, GDP-fucose, and its complementary DNA or a DNA corresponding to an RNA capable of suppressing the function of a protein relating to transport of an intracellular sugar nucleotide, GDP-fucose, to the Golgi body and its complementary DNA; and a vector comprising the DNA.

The DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme relating to synthesis of an intracellular sugar nucleotide, GDP-fucose, and its complementary DNA or a DNA corresponding to an RNA capable of suppressing the function of a protein relating to transport of an intracellular sugar nucleotide, GDP-fucose, to the Golgi body and its complementary DNA in the present invention may be any DNA, so long as it is a DNA comprising a DNA corresponding to an RNA capable of suppressing the activity or expression of an α1,6-fucose modifying enzyme and its complementary DNA, and a DNA corresponding to an RNA capable of suppressing the activity or expression of an enzyme relating to synthesis of an intracellular sugar nucleotide, GDP-fucose, and its complementary DNA or the DNA corresponding to an RNA capable of suppressing the activity or expression of a protein relating to transport of an intracellular sugar nucleotide, GDP-fucose, to the Golgi body and its complementary DNA. A DNA comprising a DNA corresponding to an RNA capable of suppressing the activity or expression of an α1,6-fucose modifying enzyme and its complementary DNA, and a DNA corresponding to an RNA capable of suppressing the activity or expression of an enzyme relating to synthesis of an intracellular sugar nucleotide, GDP-fucose, and its complementary DNA is preferred, and a DNA comprising a DNA corresponding to an RNA capable of suppressing the activity or expression of an α1,6-fucose modifying enzyme and its complementary DNA, and a DNA corresponding to an RNA capable of suppressing the function of a GDP-mannose converting enzyme and its complementary DNA is more preferred.

The vector of the present invention may be any vector, so long as it is a vector comprising the above-described DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex N-glycoside-linked sugar chain and its complementary DNA, and a DNA corresponding to an RNA capable of suppressing the function of an enzyme relating to synthesis of an intracellular sugar nucleotide, GDP-fucose, and its complementary DNA or a DNA corresponding to an RNA capable of suppressing the function of a protein relating to transport of an intracellular sugar nucleotide, GDP-fucose, to the Golgi body and its complementary DNA in the present invention.

The vector of the present invention may be constructed, for example, by inserting, into a commercially available siRNA expression vector, the above-described DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme relating to synthesis of an intracellular sugar nucleotide, GDP-fucose, and its complementary DNA or a DNA corresponding to an RNA capable of suppressing the function of a protein relating to transport of an intracellular sugar nucleotide, GDP-fucose, to the Golgi body and its complementary DNA.

The vector of the present invention may be constructed, as one vector, by inserting, into a vector, a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA so as to be transcribed by a respectively independent promoter, and by inserting, into a vector, a DNA corresponding to an RNA capable of suppressing the function of an enzyme relating to synthesis of an intracellular sugar nucleotide, GDP-fucose, and its complementary DNA or a DNA corresponding to an RNA capable of suppressing the function of a protein relating to transport of an intracellular sugar nucleotide, GDP-fucose, to the Golgi body and its complementary DNA so as to be transcribed by a respectively independent promoter. However, in order to easily form a double-stranded RNA, it is preferred that a DNA in which a DNA corresponding to an RNA capable of suppressing the function of an α1,6-fucose modifying enzyme and its complementary DNA are linked via a linker sequence is prepared, the DNA is inserted into a vector so as to be transcribed by an independent promoter, a DNA in which a DNA corresponding to an RNA capable of suppressing the function of an enzyme relating to synthesis of an intracellular sugar nucleotide, GDP-fucose, and its complementary DNA are linked via a linker sequence or a DNA in which a DNA corresponding to an RNA capable of suppressing the function of a protein relating to transport of an intracellular sugar nucleotide, GDP-fucose, to the Golgi body and its complementary DNA are linked via a linker sequence is prepared, and the DNA is inserted into the vector so as to be transcribed by an independent promoter to thereby construct the vector of the present invention as one vector.

Furthermore, a DNA in which a DNA corresponding to an RNA capable of suppressing the function of an α1,6-fucose modifying enzyme and its complementary DNA are linked via a linker sequence, and a DNA in which a DNA corresponding to an RNA capable of suppressing the function of an enzyme relating to synthesis of an intracellular sugar nucleotide, GDP-fucose, and its complementary DNA are linked via a linker or a DNA in which a DNA corresponding to an RNA capable of suppressing the function of a protein relating to transport of an intracellular sugar nucleotide, GDP-fucose, to the Golgi body and its complementary DNA are linked via a linker may be inserted into a vector under one promoter so as to be transcribed to one continuous RNA.

The two independent promoters are either promoters of the same kind or promoters of different kinds. The directions of the two independent promoters are either the same direction or opposite directions. As the two independent promoters in the vector of the present invention, it is preferred that promoters of different kinds are positioned toward the same direction.

The promoter may be any promoter, so long as it is a promoter capable of functioning in a parent cell. When an animal cell is used as a parent cell, for example, a polymerase III promoter such as U6 promoter, H1 promoter and tRNA promoter can be used.

The nucleotide sequence used as the linker may be any sequence, so long as it is a sequence used for forming a double-stranded RNA. A sequence capable of expressing a hairpin-type siRNA in which an RNA and its complementary RNA are linked via a loop of about 2 to 10 nucleotides to thereby form a double-stranded RNA is preferred.

The DNA in which a DNA corresponding to an RNA capable of suppressing the function of an α1,6-fucose modifying enzyme and its complementary RNA and its complementary DNA are linked via a linker sequence includes a DNA selected from the nucleotide sequences represented by SEQ ID NOs:90, 91, 94 and 95.

The DNA in which a DNA and its complementary DNA corresponding to an RNA capable of suppressing the function of an enzyme relating to synthesis of an intracellular sugar nucleotide, GDP-fucose, and its complementary RNA, respectively, are linked via a linker sequence is preferably a DNA in which a DNA and its complementary DNA corresponding to an RNA capable of suppressing the function of a GDP-mannose converting enzyme and its complementary RNA, respectively, are linked via a linker sequence. The DNA in which a DNA and its complementary DNA corresponding to an RNA capable of suppressing the function of a GDP-mannose converting enzyme and its complementary RNA, respectively, are linked via a linker sequence is a DNA selected from the nucleotide sequences represented by SEQ ID NOs:92, 93 and 96.

In the vector of the present invention, the DNA in which a DNA and its complementary DNA corresponding to an RNA capable of suppressing the function of an α1,6-fucose modifying enzyme and its complementary RNA, respectively, are linked via a linker sequence and the DNA in which a DNA and its complementary DNA corresponding to an RNA capable of suppressing the function of an enzyme relating to synthesis of an intracellular sugar nucleotide, GDP-fucose, and its complementary RNA, respectively, are linked via a linker sequence or the DNA in which a DNA and its complementary DNA corresponding to an RNA capable of suppressing the function of a protein relating to transport of an intracellular sugar nucleotide, GDP-fucose, to the Golgi body and its complementary RNA, respectively, are linked via a linker sequence are preferably selected as an effective combination depending on the animal species of the parent cell to be introduced. Specifically, when the parent cell is derived from a hamster, a combination of a DNA represented by SEQ ID NO:90 and a DNA represented by SEQ ID NO:92 or a combination of a DNA represented by SEQ ID NO:91 and a DNA represented by SEQ ID NO:92 is preferably used.

When the parent cell is derived from a mouse, a combination of a DNA represented by SEQ ID NO:90 and a DNA represented by SEQ ID NO:93 or a combination of a DNA represented by SEQ ID N0:91 and a DNA represented by SEQ ID NO:93 is preferably used.

When the parent cell is derived from a human, a combination of a DNA represented by SEQ ID NO:94 and a DNA represented by SEQ ID NO:96 or a combination of a DNA represented by SEQ ID NO:95 and a DNA represented by SEQ ID NO:96 is preferably used.

The cell into which the vector of the present invention is introduced is included in the cell of the present invention.

Also, the cell of the present invention includes a cell obtained by inserting, into two kinds of independent vectors, the above-described DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and the DNA corresponding to an RNA capable of suppressing the function of an enzyme relating to synthesis of an intracellular sugar nucleotide, GDP-fucose, and its complementary DNA or the DNA corresponding to an RNA capable of suppressing the function of a protein relating to transport of an intracellular sugar nucleotide, GDP-fucose, to the Golgi body and its complementary DNA and co-introducing the two kinds of independent vectors into a cell.

The cell of the present invention is capable of producing a glycoprotein having a sugar chain structure having no fucose and having high physiological activity. That is, the present invention can provide a process for producing a glycoprotein having higher physiological activity than a glycoprotein produced by its parent cell.

Examples of the glycoprotein having high physiological activity due to the sugar chain structure having no fucose include a glycoprotein having improved affinity with a receptor, a glycoprotein having improved half-life in blood, a glycoprotein in which its tissue distribution after administration into blood is changed, and a glycoprotein in which its interaction with a protein necessary for expressing pharmacological activity is improved, due to the sugar chain structure having no fucose.

Accordingly, the glycoprotein in the present invention may be any glycoprotein, so long as it is a glycoprotein in which a produced protein has a sugar chain structure to which fucose binds when it is produced by the parent cell. Examples include an antibody, erythropoietin, thrombopoietin, tissue type plasminogen activator, prourokinase, thrombomodulin, antithrombin III, protein C, blood coagulation factor VII, blood coagulation factor VIII, blood coagulation factor IX, blood coagulation factor X, gonadotropic hormone, thyroid-stimulating hormone, epidermal growth factor (EGF), hepatocyte growth factor (HGF), keratinocyte growth factor, activin, bone morphogenetic factor, stem cell factor (SCF), interferon-α, interferon-β, interferon-γ, interleukin-2, interleukin-6, interleukin-10, interleukin-11, soluble interleukin-4 receptor, tumor necrosis factor-α, DNase I, galactosidase, α-glucosidase, glucocerebrosidase and the like.

Examples of a glycoprotein having remarkably improved physiological activity due to the sugar chain structure to which no fucose binds include an antibody composition.

That is, the cell of the present invention can produce an antibody composition having higher ADCC activity than that of an antibody composition produced by a parent cell.

Furthermore, the cell of the present invention can produce an antibody composition wherein among the total complex type N-glycoside-linked sugar chains bound to the Fc region in the antibody composition, the ratio of sugar chains in which fucose is not bound to N-acetylglucosamine in the reducing end in the sugar chain is higher than that of an antibody composition produced by a parent cell.

In the present invention, the antibody composition is a composition which comprises an antibody molecule having a complex type N-glycoside-linked sugar chain in the Fc region.

The antibody is a tetramer in which two molecules of each of two polypeptide chains, a heavy chain and a light chain (hereinafter referred to as "H chain" and "L chain", respectively) are respectively associated. Each of about a quarter of the N-terminal side of the H chain and about a half of the N-terminal side of the L chain (more than 100 amino acids for each) is called a variable region (hereinafter referred to as "V region") which is rich in diversity and directly relates to the binding with an antigen. The greater part of the moiety other than the V region is called a constant region (hereinafter referred to as "C region"). Based on homology with the C region, antibody molecules are classified into classes IgG, IgM, IgA, IgD and IgE.

Also, the IgG class is further classified into subclasses IgG1 to IgG4 based on homology of the C region.

The H chain is divided into four immunoglobulin domains, an antibody H chain V region (hereinafter referred to as "VH"), an antibody H chain C region 1 (hereinafter referred to as "CH1"), an antibody H chain C region 2 (hereinafter referred to as "CH2") and an antibody H chain C region 3 (hereinafter referred to as "CH3 "), from its N-terminal, and a highly flexible peptide region called hinge region is present between CH1 and CH2 to divide CH1 and CH2. A structural unit comprising CH2 and CH3 in the downstream of the hinge region is called Fc region to which a complex type N-glycoside-linked sugar chain is bound. The Fc region is a region to which an Fc receptor, a complement and the like are bound (Immunology Illustrated, the Original, 5th edition, published on February 10, 2000, by Nankodo; Handbook of Antibody Technology (Kotai Kogaku Nyumon), 1st edition on January 25, 1994, by Chijin Shokan).

Sugar chains of glycoproteins such as an antibody are roughly classified into two types, namely a sugar chain which binds to asparagine (N-glycoside-linked sugar chain) and a sugar chain which binds to serine, threonine or the like (O-glycoside-linked sugar chain), based on the binding form to the protein moiety. The N-glycoside-linked sugar chains have a basic common core structure shown by the following formula (I) [Biochemical Experimentation Method 23 -Method for Studying Glycoprotein Sugar Chain (Gakujutsu Shuppan Center), edited by Reiko Takahashi (1989)]:

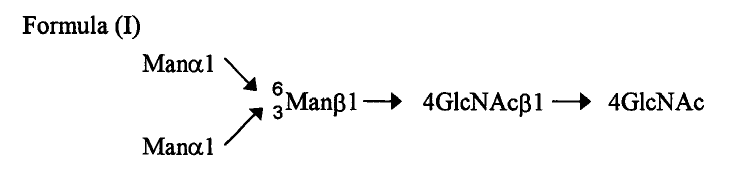

In formula (I), the sugar chain terminus which binds to asparagine is called a reducing end, and the opposite side is called a non-reducing end.

The N-glycoside-linked sugar chain may be any N-glycoside-linked sugar chain, so long as it comprises the core structure of formula (I). Examples include a high mannose type in which mannose alone binds to the non-reducing end of the core structure; a complex type in which the non-reducing end side of the core structure comprises one or plurality of parallel branches of galactose-N-acetylglucosamine (hereinafter referred to as "Gal-GlcNAc") and the non-reducing end side of Gal-GlcNAc further comprises a structure of sialic acid, bisecting N-acetylglucosamine or the like; a hybrid type in which the non-reducing end side of the core structure comprises branches of both of the high mannose type and complex type; and the like.

Since the Fc region in the antibody molecule comprises regions to which N-glycoside-linked sugar chains are separately bound, two sugar chains are bound per one antibody molecule. Since the N-glycoside-linked sugar chain which binds to an antibody molecule includes any sugar chain having the core structure represented by formula (I), there are a number of combinations of sugar chains for the two N-glycoside-linked sugar chains which bind to the antibody.

Accordingly, in the present invention, an antibody composition prepared by using a cell into which an RNA capable of suppressing the function of a GDP-mannose converting enzyme or an antibody composition prepared by using a cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, or an RNA capable of suppressing the function of a protein relating to transport of an intracellular sugar nucleotide, GDP-fucose, to the Golgi body are introduced may comprise an antibody molecule having the same sugar chain structure or an antibody molecule having different sugar chain structures, so long as the effect of the present invention can be obtained.

The ratio of sugar chains in which fucose is not bound to N-acetylglucosamine in the reducing end in the sugar chain among the total complex type N-glycoside-linked sugar chains bound to the Fc region contained in the antibody composition (hereinafter referred to the "ratio of sugar chains of the present invention") is a ratio of the number of sugar chains in which fucose is not bound to N-acetylglucosamine in the reducing end in the sugar chains to the total number of the complex type N-glycoside-linked sugar chains bound to the Fc region contained in the composition.

The sugar chain in which fucose is not bound to N-acetylglucosamine in the reducing end in the complex type N-glycoside-linked sugar chain is a sugar chain in which fucose is not bound to N-acetylglucosamine in the reducing end through α-bond in the complex type N-glycoside-linked sugar chain. Specifically, sugar chain in which fucose is not bound to N-acetylglucosamine in the reducing end through α-bond in the complex type N-glycoside-linked sugar chain is mentioned.

The higher the ratio of sugar chains of the present invention is, the higher the ADCC activity of the antibody composition is. The antibody composition having higher ADCC activity includes an antibody composition in which the ratio of sugar chains of the present invention is preferably 20% or more, more preferably 30% or more, still more preferably 40% or more, particularly preferably 50% or more, and most preferably 100%.

Furthermore, the present invention relates to a process for producing an antibody composition having higher ADCC activity than an antibody composition produced by its parent cell.

When the ratio of sugar chain of the present invention is higher than that of an antibody composition produced by its parent cell, the antibody composition has higher ADCC activity than the antibody composition produced by its parent cell.

The ADCC activity is a cytotoxic activity in which an antibody bound to a cell surface antigen on a tumor cell in the living body activates an effector cell through the binding of the antibody Fc region and an Fc receptor existing on the effector cell surface and thereby injuries the tumor cell and the like [Monoclonal Antibodies: Principles and Applications, Wiley-Liss, Inc., Chapter 2.1 (1995)]. The effector cell includes a killer cell, a natural killer cell, an activated macrophage and the like.

The ratio of sugar chains in which fucose is not bound to N-acetylglucosamine in the reducing end in the sugar chains contained in the composition which comprises an antibody molecule having complex type N-glycoside-linked sugar chains in the Fc region can be determined by releasing the sugar chains from the antibody molecule using a known method such as hydrazinolysis or enzyme digestion [Biochemical Experimentation Methods 23 Method for Studying Glycoprotein Sugar Chain (Japan Scientific Societies Press), edited by Reiko Takahashi (1989)], carrying out fluorescence labeling or radioisotope labeling of the released sugar chains and then separating the labeled sugar chains by chromatography. Also, the released sugar chains can also be determined by analyzing it with the HPAED-PAD method [J. Liq. Chromatogr., 6, 1577 (1983)].

The antibody molecule may be any antibody molecule, so long as it comprises the Fc region of an antibody. Examples include an antibody, an antibody fragment, a fusion protein comprising an Fc region, and the like.

Examples of the antibody include an antibody secreted by a hybridoma cell prepared from a spleen cell of an animal immunized with an antigen; an antibody prepared by a genetic engineering technique, namely an antibody obtained by introducing an antibody gene-inserted antibody expression vector into a host cell; and the like. Specific examples include an antibody produced by a hybridoma, a humanized antibody, a human antibody and the like.

A hybridoma is a cell which is obtained by cell fusion between a B cell obtained by immunizing a non-human mammal with an antigen and a myeloma cell derived from a mouse, a rat or the like and which can produce a monoclonal antibody having the antigen specificity of interest.

The humanized antibody includes a human chimeric antibody, a human CDR-grafted antibody and the like.

A human chimeric antibody is an antibody which comprises antibody VH and an antibody L chain V region (hereinafter referred to as "VL"), both of a non-human animal antibody, a human antibody H chain C region (hereinafter also referred to as "CH") and a human antibody L chain C region (hereinafter also referred to as "CL"). The non-human animal may be any animal such as a mouse, a rat, a hamster or a rabbit, so long as a hybridoma can be prepared therefrom.

The human chimeric antibody can be produced by obtaining cDNAs encoding VH and VL from a monoclonal antibody-producing hybridoma, inserting them into an expression vector for host cell comprising genes encoding human antibody CH and human antibody CL to thereby construct a human chimeric antibody expression vector, and then introducing the vector into a host cell to express the antibody.

As the CH of human chimeric antibody, any CH can be used, so long as it belongs to human immunoglobulin (hereinafter referred to as "hIg") can be used, and those belonging to the hIgG class are preferred, and any one of the subclasses belonging to the hIgG class, such as hIgG1, hIgG2, hIgG3 and hIgG4, can be used. As the CL of human chimeric antibody, any CL can be used, so long as it belongs to the hIg class, and those belonging to the κ class or λ class can be used.

A human CDR-grafted antibody is an antibody in which amino acid sequences of CDRs of VH and VL of a non-human animal antibody are grafted into appropriate positions of VH and VL of a human antibody.

The human CDR-grafted antibody can be produced by constructing cDNAs encoding V regions in which the amino acid sequences of CDRs of VH and VL of a non-human animal antibody are grafted into the amino acid sequences of CDRs of VH and VL of a human antibody, inserting them into an expression vector for host cell comprising genes encoding human antibody CH and human antibody CL to thereby construct a human CDR-grafted antibody expression vector, and then introducing the expression vector into a host cell to express the human CDR-grafted antibody.

As the CH of human CDR-grafted antibody, any CH can be used, so long as it belongs to the hIg, and those of the hIgG class are preferred and any one of the subclasses belonging to the hIgG class, such as hIgG1, hIgG2, hIgG3 and hIgG4, can be used. As the CL of human CDR-grafted antibody, any CL can be used, so long as it belongs to the hIg class, and those belonging to the κ class or λ class can be used.

A human antibody is originally an antibody naturally existing in the human body, but it also includes antibodies obtained from a human antibody phage library, a human antibody-producing transgenic non-transgenic animal or a human antibody-producing transgenic plant, which are prepared based on the recent advance in genetic engineering, cell engineering and embryological engineering techniques.

The antibody existing in the human body can be prepared, for example by isolating a human peripheral blood lymphocyte, immortalizing it by its infection with EB virus or the like and then cloning it to thereby obtain lymphocytes capable of producing the antibody, culturing the lymphocytes thus obtained, and purifying the antibody from the culture.

The human antibody phage library is a library in which antibody fragments such as Fab and single chain antibody are expressed on the phage surface by inserting a gene encoding an antibody prepared from a human B cell into a phage gene. A phage expressing an antibody fragment having the desired antigen binding activity can be recovered from the library, using its activity to bind to an antigen-immobilized substrate as the marker. The antibody fragment can be converted further into a human antibody molecule comprising two full H chains and two full L chains by genetic engineering techniques.

A human antibody-producing transgenic non-human animal is an animal in which a human antibody gene is introduced into cells. Specifically, a human antibody-producing transgenic non-human animal can be prepared by introducing a human antibody gene into ES cell of a mouse, grafting the ES cell into an early stage embryo of other mouse and then developing it. By introducing a human antibody gene into a fertilized egg and developing it, the human antibody-producing transgenic non-human animal can be also prepared. A human antibody is prepared from the human antibody-producing transgenic non-human animal by obtaining a human antibody-producing hybridoma by a hybridoma preparation method usually carried out in non-human mammals, culturing the obtained hybridoma and accumulating the human antibody in the culture.

The transgenic non-human animal includes cattle, sheep, goat, pig, horse, mouse, rat, fowl, monkey, rabbit and the like.

In the present invention, as the antibody, preferred are an antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen, and a human antibody which belongs to the IgG class is preferred.

An antibody fragment is a fragment which comprises the Fc region of an antibody. The antibody fragment may be any fragment, so long as it comprise the Fc region of the above-described antibody. The antibody fragment includes an H chain monomer, an H chain dimmer and the like.

A fusion protein comprising the Fc region is a protein which is obtained by fusing an antibody comprising the Fc region of an antibody or the antibody fragment with a protein such as an enzyme or a cytokine.

The present invention is explained below in detail.

### 1. Preparation of cell of the present invention

### (1) Preparation of cell into which an RNA capable of suppressing the function of a GDP-mannose converting enzyme

The cell into which an RNA capable of suppressing the function of a GDP-mannose converting enzyme in the present invention can be prepared, for example, as follows.

A cDNA or a genomic DNA of a GDP-mannose converting enzyme is prepared.

The nucleotide sequence of the prepared cDNA or genomic DNA is determined.

Based on the determined DNA sequence, a construct of an RNAi gene comprising a coding region or a non-coding region of the GDP-mannose converting enzyme at an appropriate length is designed.

In order to express the RNAi gene in a cell, a recombinant vector is prepared by inserting a fragment or full length of the prepared DNA into downstream of the promoter of an appropriate expression vector.

A transformant is obtained by introducing the recombinant vector into a host cell suitable for the expression vector.

The cell of the present invention can be obtained by selecting a transformant based on the activity of the introduced GDP-mannose converting enzyme or the sugar chain structure of the produced antibody molecule or the glycoprotein on the cell surface.

As the host cell, any cell such as an yeast, an animal cell, an insect cell or a plant cell can be used, so long as it has a gene of the target GDP-mannose converting enzyme. Examples include host cells described in the following item 2.

As the expression vector, a vector which is autonomously replicable in the host cell or can be integrated into the chromosome and comprises a promoter at such a position that the designed RNAi gene can be transcribed is used. Examples include the expression vectors transcribed by polymerase III or the expression vectors described in the following item 2.

As the method for introducing a gene into various host cells, the methods for introducing recombinant vectors suitable for various host cells described in the following item 2 can be used.

As a method for obtaining a cDNA or genomic DNA of the GDP-mannose converting enzyme, the following method is exemplified.

### Preparation method of cDNA:

A total RNA or mRNA is prepared from various host cells.

A cDNA library is prepared from the prepared total RNA or mRNA.

Degenerative primers are produced based on a known amino acid sequence of the GDP-mannose converting enzyme, such as an amino acid sequence of the enzyme in human, and a gene fragment encoding the GDP-mannose converting enzyme is obtained by PCR using the prepared cDNA library as the template.

A cDNA of the GDP-mannose converting enzyme can be obtained by screening the cDNA library using the obtained gene fragment as a probe.

The mRNA of various host cells may be a commercially available product (e.g., manufactured by Clontech) or may be prepared from various host cells as follows. The method for preparing a total mRNA from various host cells include the guanidine thiocyanate-cesium trifluoroacetate method [Methods in Enzymology, 154, 3 (1987)], the acidic guanidine thiocyanate phenol chloroform (AGPC) method [Analytical Biochemistry, 162, 156 (1987); Experimental Medicine (Jikken Igaku), 9, 1937 (1991)] and the like.

Furthermore, a method for preparing mRNA as poly(A)⁺ RNA from a total RNA includes the oligo(dT)-immobilized cellulose column method (Molecular Cloning, Second Edition).

In addition, mRNA can be prepared using a kit such as Fast Track mRNA Isolation Kit (manufactured by Invitrogen) or Quick Prep mRNA Purification Kit (manufactured by Pharmacia).

A cDNA library is prepared from the prepared mRNA of various host cells. The method for preparing cDNA libraries includes the methods described in Molecular Cloning, Second Edition; Current Protocols in Molecular Biology, A Laboratory Manual, 2nd Ed. (1989); and the like, or methods using commercially available kits such as SuperScript Plasmid System for cDNA Synthesis and Plasmid Cloning (manufactured by Life Technologies) and ZAP-cDNA Synthesis Kit (manufactured by STRATAGENE).

As the cloning vector for preparing the cDNA library, any vector such as a phage vector or a plasmid vector can be used, so long as it is autonomously replicable in *Escherichia coli* K12. Examples include ZAP Express [manufactured by STRATAGENE, Strategies, 5, 58 (1992)], pBluescript II SK(+) [Nucleic Acids Research, 17, 9494 (1989)], Lambda ZAP II (manufactured by STRATAGENE), λgt10 and λgt11 [DNA Cloning, A Practical Approach, 1, 49 (1985)], λTriplEx (manufactured by Clontech), λExCell (manufactured by Pharmacia), pT7T318U (manufactured by Pharmacia), pcD2 [Mol. Cell. Biol., 3, 280 (1983)], pUC18 [Gene, 33, 103 (1985)] and the like.

Any microorganism can be used as the host microorganism for preparing the cDNA library, and *Escherichia coli* is preferably used. Examples include *Escherichia coli* XL1-Blue MRF' [manufactured by STRATAGENE, Strategies, 5, 81 (1992)], *Escherichia coli* C600 [Genetics, 39, 440 (1954)], *Escherichia coli* Y1088 [Science, 222, 778 (1983)], *Escherichia coli* Y1090 [Science, 222, 778 (1983)], *Escherichia coli* NM522 [J. Mol. Biol., 166, 1 (1983)], *Escherichia coli* K802 [J. Mol. Biol., 16, 118 (1966)], *Escherichia coli* JM105 [Gene, 38, 275 (1985)] and the like

The cDNA library can be used as such in the subsequent analysis, but in order to obtain a full length cDNA as efficient as possible by decreasing the ratio of an infull length cDNA, a cDNA library prepared according to the oligo cap method developed by Sugano et al. [Gene, 138, 171 (1994); Gene, 200, 149 (1997); Protein, Nucleic Acid Koso (Tanpakushitu, Kakusan, Koso), 41, 603 (1996); Experimental Medicine (Jikken Igaku), 11, 2491 (1993); cDNA Cloning (Yodo-sha) (1996); Methods for Preparing Gene Libraries (Yodo-sha) (1994)] can be used in the following analysis.

Based on the amino acid sequence of the GDP-mannose converting enzyme, degenerative primers specific for the 5'-terminal and 3'-terminal nucleotide sequences of a nucleotide sequence presumed to encode the amino acid sequence are prepared, and DNA is amplified by PCR [PCR Protocols, Academic Press (1990)] using the prepared cDNA library as the template to obtain a gene fragment encoding the GDP-mannose converting enzyme.

It can be confirmed that the obtained gene fragment is a DNA encoding the GDP-mannose converting enzyme by a method generally used for analyzing a nucleotide such as the dideoxy method of Sanger et al. [Proc. Natl. Acad Sci. USA, 74, 5463 (1977)] or a nucleotide sequence analyzer such as ABIPRISM 377 DNA Sequencer (manufactured by PE Biosystems).

A cDNA encoding the GDP-mannose converting enzyme can be obtained by carrying out colony hybridization or plaque hybridization *(Molecular Cloning,* Second Edition) for the cDNA or cDNA library synthesized from the mRNA contained in various host cells, using the gene fragment as a DNA probe.

Also, a cDNA encoding the GDP-mannose converting enzyme can also be obtained by carrying out screening by PCR using the cDNA or cDNA library synthesized from the mRNA contained in various host cells as the template and using the primers used for obtaining the gene fragment encoding the GDP-mannose converting enzyme.

The nucleotide sequence of the obtained DNA encoding the GDP-mannose converting enzyme is analyzed from its terminus and determined by a method generally used for analyzing a nucleotide such as the dideoxy method of Sanger et al. [Proc. Natl. Acad. Sci. USA, 74, 5463 (1977)] or a nucleotide sequence analyzer such as ABI PRISM 377 DNA Sequencer (manufactured by PE Biosystems).

A gene encoding the GDP-mannose converting enzyme can also be determined from genes in databases by searching nucleotide sequence databases such as GenBank, EMBL and DDBJ using a homology retrieving program such as BLAST based on the determined cDNA nucleotide sequence.

The nucleotide sequence of a gene encoding the GDP-mannose converting enzyme obtained by the above method includes the nucleotide sequence represented by any one of SEQ ID NOs:8 to 10.

The cDNA of the GDP-mannose converting enzyme can also be obtained by chemically synthesizing it with a DNA synthesizer such as DNA Synthesizer model 392 manufactured by Perkin Elmer using the phosphoamidite method, based on the determined DNA nucleotide sequence.

As an example of the method for preparing a genomic DNA of the GDP-mannose converting enzyme, the method described below is exemplified.

### Preparation method of genomic DNA:

The method for preparing genomic DNA includes known methods described in Molecular Cloning, Second Edition; *Current Protocols in Molecular Biology;* and the like. In addition, a genomic DNA of the GDP-mannose converting enzyme can also be isolated using Genome DNA Library Screening System (manufactured by Genome Systems), Universal GenomeWalker^{™} Kits (manufactured by CLONTECH), or the like.

The following method can be exemplified as the method for selecting a transformant based on the activity of the GDP-mannose converting enzyme.

### Method for selecting transformant:

The method for selecting a cell in which the activity of the GDP-mannose converting enzyme is decreased includes biochemical methods or genetic engineering techniques described in New Biochemical Experimentation Series 3-Saccharides I, Glycoprotein (Tokyo Kagaku Dojin), edited by Japanese Biochemical Society (1988); Cell Engineering, Supplement, Experimental Protocol Series, Glycobiology Experimental Protocol, Glycoprotein, Glycolipid and Proteoglycan (Shujun-sha), edited by Naoyuki Taniguchi, Akemi Suzuki, Kiyoshi Furukawa and Kazuyuki Sugawara (1996); Molecular Cloning, Second Edition; *Current Protocols in Molecular Biology;* and the like. The biochemical method includes a method in which the enzyme activity is evaluated using an enzyme-specific substrate. The genetic engineering techniques include the Northern analysis, RT-PCR and the like which measures the amount of mRNA of a gene encoding the GDP-mannose converting enzyme.

Furthermore, the method for selecting a cell based on morphological change caused by decrease of the activity of the GDP-mannose converting enzyme includes a method for selecting a transformant based on the sugar chain structure of a produced glycoprotein molecule, a method for selecting a transformant based on the sugar chain structure of a glycoprotein on a cell surface, and the like. The method for selecting a transformant using the sugar chain structure of a glycoprotein-producing molecule includes method described in the item 5 below. The method for selecting a transformant using the sugar chain structure of a glycoprotein on a cell surface includes a method in which a clone resistant to a lectin which recognizes a sugar chain structure wherein 1-position of fucose is bound to 6-position of N-acetylglucosamine in the reducing end through α-bond in the N-glycoside-linked sugar chain is selected. Examples include a method using a lectin described in Somatic Cell Mol. Genet., 12, 51 (1986).

As the lectin, any lectin can be used, so long as it is a lectin which recognizes a sugar chain structure in which 1-position of fucose is bound to 6-position of N-acetylglucosamine in the reducing end through α-bond in the N-glycoside-linked sugar chain. Examples include a *Lens culinaris* agglutinin LCA (lentil agglutinin derived from *Lens culinaris), a Pisum sativum* agglutinin PSA (pea lectin derived from *Pisum sativum*), a *Vicia faba* agglutinin VFA (agglutinin derived from *Vicia faba*)*,* an *Aleuria aurantia* lectin AAL (lectin derived from *Aleuria aurantia*) and the like.

Specifically, the cell of the present invention can be selected by culturing cells for 1 day to 2 weeks, preferably from 3 days to 1 week, in a medium containing the above lectin at a concentration of 10 µg/ml to 10 mg/ml, preferably 0.5 to 2 mg/ml, subculturing surviving cells or picking up a colony and transferring it into a culture vessel, and subsequently continuing the culturing in the lectin-containing medium.

The RNAi gene for suppressing the mRNA amount of a gene encoding the GDP-mannose converting enzyme can be prepared in the usual method or by using a DNA synthesizer.

The construct of the RNAi gene can be designed according to the description in Nature, 391, 806 (1998); Proc. Natl. Acad Sci. USA, 95, 15502 (1998); Nature, 395, 854 (1998); Proc. Natl. Acad. Sci. USA, 96, 5049 (1999); Cell, 95, 1017 (1998); Proc. Natl. Acad Sci. USA, 96, 1451 (1999); Proc. Natl. Acad Sci. USA, 95, 13959 (1998); Nature Cell Biol., 2, 70 (2000) and the like.

In addition, the cell of the present invention can also be obtained without using an expression vector, by directly introducing a double-stranded RNA which is designed based on the nucleotide sequence of the GDP-mannose converting enzyme into a host cell.

The double-stranded RNA can be prepared in the usual method or by using a DNA synthesizer. Specifically, it can be prepared based on the sequence information of an oligonucleotide having a corresponding sequence of 10 to 40 bases, preferably 10 to 30 bases, and more preferably 15 to 30 bases, among complementary RNA nucleotide sequences of a cDNA and a genomic DNA of a GDP-mannose converting enzyme by synthesizing an oligonucleotide which corresponds to a sequence complementary to the oligonucleotide (antisense oligonucleotide). The oligonucleotide and the antisense oligonucleotide may be independently synthesized or may be linked via a spacer nucleotide which does not obstruct the formation of the double-stranded RNA.

The oligonucleotide includes an oligo RNA and derivatives of the oligonucleotide (hereinafter referred to as "oligonucleotide derivatives").

The oligonucleotide derivatives includes oligonucleotide derivatives in which a phosphodiester bond in the oligonucleotide is converted into a phosphorothioate bond, an oligonucleotide derivative in which a phosphodiester bond in the oligonucleotide is converted into an N3'-P5' phosphoamidate bond, an oligonucleotide derivative in which ribose and a phosphodiester bond in the oligonucleotide are converted into a peptide-nucleic acid bond, an oligonucleotide derivative in which uracil in the oligonucleotide is substituted with C-5 propynyluracil, an oligonucleotide derivative in which uracil in the oligonucleotide is substituted with C-5 thiazoleuracil, an oligonucleotide derivative in which cytosine in the oligonucleotide is substituted with C-5 propynylcytosine, an oligonucleotide derivative in which cytosine in the oligonucleotide is substituted with phenoxazine-modified cytosine, an oligonucleotide derivative in which ribose in the oligonucleotide is substituted with 2'-O-propylribose and an oligonucleotide derivative in which ribose in the oligonucleotide is substituted with 2'-methoxyethoxyribose [Cell Technology (Saibo Kogaku), 16, 1463 (1997)].

### (2) Preparation of cell into which an RNA capable of suppressing the function of an α1,6-fucose modifying enzyme and an RNA capable of suppressing the function of a GDP-fucose synthase or an RNA capable of suppressing the function of a protein relating to transport of an intracellular sugar nucleotide, GDP-fucose, to the Golgi body are introduced

A process for producing the cell into which an RNA capable of suppressing the function of an α1,6-fucose modifying enzyme and an RNA capable of suppressing the function of a GDP-fucose synthase or an RNA capable of suppressing the function of a protein relating to transport of an intracellular sugar nucleotide, GDP-fucose, to the Golgi body are introduced is explained below based on, as an example, a cell into which an RNA capable of suppressing the function of an α1,6-fucose modifying enzyme and an RNA capable of suppressing the function of a GDP-fucose synthase are introduced. The cell into which an RNA capable of suppressing the function of an α1,6-fucose modifying enzyme and an RNA capable of suppressing the function of a protein relating to transport of an intracellular sugar nucleotide, GDP-fucose, to the Golgi body are introduced can be prepared similarly.

A cDNA or a genomic DNA of each of an α1,6-fucose modifying enzyme and a GDP-fucose synthase is prepared.

The nucleotide sequence of the prepared cDNA or genomic DNA is determined.

Based on the determined DNA sequence, a construct of an RNAi gene comprising a coding region or a non-coding region of the α1,6-fucose modifying enzyme and the GDP-fucose synthase at an appropriate length is designed.

In order to express the RNAi gene in a cell, a recombinant vector is prepared by inserting a fragment or full length of the prepared DNA into downstream of the promoter of an appropriate expression vector.

A transformant is obtained by introducing the recombinant vector into a host cell suitable for the expression vector.

The cell of the present invention can be obtained by selecting a transformant based on the activity of the introduced α1,6-fucose modifying enzyme or GDP-fucose synthase or the sugar chain structure of the produced antibody molecule or the glycoprotein on the cell surface.

As the host cell, any cell such as an yeast, an animal cell, an insect cell or a plant cell can be used, so long as it has the target genes of the α1,6-fucose modifying enzyme and the GDP-fucose synthase. Examples include cells described in the following item 2.

As the expression vector, a vector which is autonomously replicable in the above host cell or can be integrated into the chromosome and comprises a promoter at such a position that the designed RNAi gene can be transcribed is used. Examples include the expression vector transcribed by polemerase III or the expression vectors described in the following item 2.

As the method for introducing a gene into various host cells, the methods for introducing recombinant vectors suitable for various host cells described in the following item 2 can be used.

A cDNA and a genomic DNA of the α1,6-fucose modifying enzyme or the GDP-fucose synthase can be obtained, for example, in the same manner as the method described in (1).

As a method for selecting a transformant based on the activity of the α1,6-fucose modifying enzyme or the GDP-fucose synthase, the following method is exemplified.

### Method for selecting transformant

The method for selecting a cell in which the activity of the α1,6-fucose modifying enzyme or the GDP-fucose synthase is decreased includes the biochemical method and genetic engineering method described in the above (1).

Also, the method for selecting a cell based on morphological change caused by a result in which the activity of α1,6-fucose modifying enzyme or the GDP-fucose synthase is decreased include the method described in the above (1).

The RNAi gene for suppressing the amount of the mRNA of the α1,6-fucose modifying enzyme gene or the GDP-fucose synthase gene can be prepared in the usual method or by using a DNA synthesizer.

A construct of the RNAi can be designed in the same manner as in the method described in the above (1).

In addition, the cell of the present invention can also be obtained without using an expression vector, by directly introducing a double-stranded RNA which is designed based on the nucleotide sequence of the α1,6-fucose modifying enzyme and a double-stranded RNA which is designed based on the nucleotide sequence of the GDP-fucose synthase into a host cell.

The double-stranded RNA can be prepared in the same manner as in the method described in the above (1).

### 2. Method for producing glycoprotein referring to antibody composition as an example.

A method for producing a glycoprotein using the cell of the present invention is explained below by referring to an antibody composition as an example.

The antibody composition can be expressed in a cell of the present invention and obtained by the method described in Molecular Cloning, Second Edition; Current Protocols in Molecular Biology; Antibodies, A Laboratory Manual, Cold Spring Harbor Laboratory, 1988 (hereinafter referred to as *"Antibodies"*); Monoclonal Antibodies: Principles and Practice, Third Edition, Acad. Press, 1993 (hereinafter referred to as *"Monoclonal Antibodies"*); or Antibody Engineering, A Practical Approach, IRL Press at Oxford University Press (hereinafter referred to as *"Antibody Engineering"*), for example, as follows.

A cDNA encoding an antibody molecule is prepared.

Based on the prepared full length cDNA of antibody molecule, a DNA fragment of an appropriate length comprising a coding region of the protein is prepared, if necessary.

A recombinant vector is prepared by inserting the DNA fragment or the full length cDNA into downstream of the promoter of an appropriate expression vector.

A transformant producing the antibody molecule can be obtained by introducing the recombinant vector into a host cell suitable for the expression vector.

In the present invention, as the host cell for producing the antibody composition, any cell such as a yeast, an animal cell, an insect cell or a plant cell can be used, so long as it is the cell of the present invention prepared in the above item 1 and can express the gene interest. An animal cell is preferred.

A cell such as an yeast, an animal cell, an insect cell or a plant cell into which an enzyme relating to the modification of an N-glycoside-linked sugar chain which binds to the Fc region of the antibody molecule is introduced by a genetic engineering technique can also be used as the host cell.

As the expression vector, a vector which is autonomously replicable in the above host cell or can be integrated into the chromosome and comprises a promoter at such a position that the DNA encoding the antibody molecule of interest can be transcribed is used.

The cDNA can be prepared from a human or non-human animal tissue or cell by using a probe primer specific for the antibody molecule of interest according to "Preparation method of cDNA" described in the above item 1.

When an yeast is used as the host cell, the expression vector includes YEP13 (ATCC 37115), YEp24 (ATCC 37051), YCp50 (ATCC 37419) and the like.

Any promoter can be used, so long as it can function in an yeast. Examples include a promoter of a gene of the glycolytic pathway such as a hexose kinase, PHO5 promoter, PGK promoter, GAP promoter, ADH promoter, gal 1 promoter, gal 10 promoter, heat shock protein promoter, MF α1 promoter, CUP 1 promoter and the like.

The host cell includes yeasts belonging to the genus *Saccharomyces,* the genus *Schizosaccharomyces,* the genus *Kluyveromyces,* the genus *Trichosporon,* the genus *Schwanniomyces* and the like, such as *Saccharomyces cerevisiae, Schizosaccharomyces pombe, Kluyveromyces lactis, Trichosporon pullulans* and *Schwanniomyces alluvius.*

As the method for introducing the recombinant vector, any method can be used, so long as it can introduce DNA into yeast. Examples include electroporation [Methods in Enzymology, 194, 182 (1990)], the spheroplast method *[*Proc. Natl. Acad Sci. USA, 84, 1929 (1978)], the lithium acetate method [J. Bacteriol., 153, 163 (1983)], the method described *in* Proc. Natl. Acad Sci. USA, 75, 1929 (1978) and the like.

When an animal cell is used as the host cell, the expression vector includes pcDNAI, pcDM8 (available from Funakoshi), pAGE107 [Japanese Published Unexamined Patent Application No. 22979/91; Cytotechnology, 3, 133 (1990)], pAS3-3 (Japanese Published Unexamined Patent Application No. 227075/90), pCDM8 [Nature, 329, 840 (1987)], pcDNAI/Amp (manufactured by Invitrogen), pREP4 (manufactured by Invitrogen), pAGE103 [J. Biochemistry, 101, 1307 (1987)], pAGE210 and the like.

Any promoter can be used, so long as it can function in an animal cell. Examples include a promoter of IE (immediate early) gene of cytomegalovirus (CMV), an early promoter of SV40, a promoter of retrovirus, a promoter of metallothionein, a heat shock promoter, an SRα promoter and the like. Also, an enhancer of the IE gene of human CMV can be used together with the promoter.

The host cell includes a human cell such as Namalwa cell, NM-F9 cell and PER.C6 cell, a monkey cell such as COS cell, a Chinese hamster cell such as CHO cell and HBT5637 (Japanese Published Unexamined Patent Application No. 299/88), a rat myeloma cell, a mouse myeloma cell, a cell derived from syrian hamster kidney, an embryonic stem cell, a fertilized egg cell and the like.

As the method for introducing the recombinant vector, any method can be used, so long as it can introduce a DNA into an animal cell. Examples include electroporation [Cytotechnology, 3, 133 (1990)], the calcium phosphate method (Japanese Published Unexamined Patent Application No. 227075/90), the lipofection method [Proc. Natl. Acad Sci. USA, 84, 7413 (1987)], the injection method [Manipulating the Mouse Embryo, A Laboratory Manual, Second Edition, Cold Spring Harbor Laboratory Press (1994), hereinafter also referred to as *"Manipulating the Mouse Embryo,* Second Edition"], a method using a particle gun (gene gun) (Japanese Patent No. 2606856, Japanese Patent No. 2517813), the DEAE-dextran method [Biomanual Series 4-Gene Transfer and Expression Analysis (Yodo-sha), edited by Takashi Yokota and Kenichi Arai (1994)], the virus vector method [Manipulating Mouse Embryo, Second Edition] and the like.

When an insect cell is used as the host cell, the protein can be expressed by the method described in Current Protocols in Molecular Biology, Baculovirus Expression Vectors, A Laboratory Manual, W.H. Freeman and Company, New York (1992), Bio/Technology, 6, 47 (1988) or the like.

That is, the protein can be expressed by co-introducing a recombinant gene-introducing vector and a baculovirus into an insect cell to obtain a recombinant virus in an insect cell culture supernatant and then infecting the insect cell with the recombinant virus.

The gene-introducing vector used in the method includes pVL1392, pVL1393, pBlueBacIII (all manufactured by Invitrogen) and the like.

The baculovirus includes *Autographa californica* nuclear polyhedrosis virus which is infected by an insect of the family *Barathra.*

The insect cell includes *Spodoptera frugiperda* oocytes Sf9 and Sf21 [Current Protocols in Molecular Biology, Baculovirus Expression Vectors, A Laboratory Manual, W.H. Freeman and Company, New York (1992)], a *Trichoplusia ni* oocyte High 5 (manufactured by Invitrogen) and the like.

The method for the co-introducing the above-mentioned recombinant gene-introducing vector and the above-mentioned baculovirus for preparing the recombinant virus to an insect cell includes the calcium phosphate method (Japanese Published Unexamined Patent Application No. 227075/90), the lipofection method [Proc. Natl. Acad Sci. USA, 84, 7413 (1987)] and the like.

When a plant cell is used as the host cell, the expression vector includes Ti plasmid, tobacco mosaic virus vector and the like.

As the promoter, any promoter can be used, so long as it can function in a plant cell. Examples include cauliflower mosaic virus (CaMV) 35S promoter, rice actin 1 promoter and the like.

The host cell includes plant cells of tobacco, potato, tomato, carrot, soybean, rape, alfalfa, rice, wheat, barley, and the like.

As the method for introducing the recombinant vector, any method can be used, so long as it can introduce a DNA into a plant cell. Examples include a method using *Agrobacterium* (Japanese Published Unexamined Patent Application No. 140885/84, Japanese Published Unexamined Patent Application No. 70080/85, WO 94/00977), electroporation (Japanese Published Unexamined Patent Application No. 251887/85), a method using a particle gun (gene gun) (Japanese Patent No. 2606856, Japanese Patent No. 2517813) and the like.

As the method for expressing an antibody gene, secretion production, expression of a fusion protein and the like can be carried out in accordance with the method described in Molecular Cloning, Second Edition or the like, in addition to the direct expression.

The antibody composition can be produced by culturing the thus obtained transformant in a medium to form and accumulate the antibody molecule in the culture and recovering the antibody composition from the culture. The method for culturing the transformant can be carried out by a conventional method used for the culturing of a host cell.

As the medium for culturing a transformant obtained using a eukaryote, such as an yeast, as the host cell, the medium may be either a natural medium or a synthetic medium, so long as it comprises materials such as a carbon source, a nitrogen source, an inorganic salt and the like which can be assimilated by the organism and culturing of the transformant can be efficiently carried out.

As the carbon source, those which can be assimilated by the organism can be used. Examples include carbohydrates such as glucose, fructose, sucrose, molasses containing them, starch and starch hydrolysate; organic acids such as acetic acid and propionic acid; alcohols such as ethanol and propanol; and the like.

The nitrogen source includes ammonia; ammonium salts of inorganic acid or organic acid such as ammonium chloride, ammonium sulfate, ammonium acetate and ammonium phosphate; other nitrogen-containing compounds; peptone; meat extract; yeast extract; corn steep liquor; casein hydrolysate; soybean meal; soybean meal hydrolysate; various fermented cells and hydrolysates thereof; and the like.

The inorganic salt includes potassium dihydrogen phosphate, dipotassium hydrogen phosphate, magnesium phosphate, magnesium sulfate, sodium chloride, ferrous sulfate, manganese sulfate, copper sulfate, calcium carbonate, and the like.

The culture is carried out generally under aerobic conditions such as shaking culture or submerged-aeration stirring culture. The culturing temperature is preferably 15 to 40°C, and the culturing time is generally 16 hours to 7 days. In the culture, the pH is maintained at 3 to 9. The pH is adjusted using an inorganic or organic acid, an alkali solution, urea, calcium carbonate, ammonia or the like.

If necessary, an antibiotic such as ampicillin or tetracycline can be added to the medium in the culture.

When an yeast transformed with a recombinant vector obtained using an inducible promoter as the promoter is cultured, an inducer can be added to the medium, if necessary. For example, when an yeast transformed with a recombinant vector obtained using *lac* promoter is cultured, isopropyl-β-D-thiogalactopyranoside can be added to the medium, and when an yeast transformed with a recombinant vector obtained using *trp* promoter is cultured, indoleacrylic acid can be added to the medium.

When a transformant obtained using an animal cell as the host cell is cultured, the medium includes generally used RPMI 1640 medium [The Journal of the American Medical Association, 199, 519 (1967)], Eagle's MEM medium [Science, 122, 501 (1952)], Dulbecco's modified MEM medium *[*Virology, 8, 396 (1959)], 199 medium [Proceeding of the Society for the Biological Medicine, 73, 1 (1950)] and Whitten's medium [Developmental Engineering Experimentation Manual-Preparation of Transgenic Mice (Kodan-sha), edited by Motoya Katsuki (1987)], the medium obtained by adding fetal bovine serum, *etc.* to these medium, and the like.

The culture is carried out generally at a pH of 6 to 8 and 30 to 40°C for 1 to 7 days in the presence of 5% CO₂. Culturing can also be carried out by culturing using a method such as fed-batch culture or hollow-fiber culture for 1 day to several months.

If necessary, an antibiotic such as kanamycin or penicillin can be added to the medium in the culture.

The medium for use in the culture of a transformant obtained using an insect cell as the host cell includes generally used TNM-FH medium (manufactured by Pharmingen), Sf-900 II SFM medium (manufactured by Life Technologies), ExCell 400 and ExCell 405 (both manufactured by JRH Biosciences), Grace's Insect Medium [Nature, 195, 788 (1962)] and the like.

The culture is carried out generally at a pH of 6 to 7 and 25 to 30°C for 1 to 5 days.

In addition, antibiotics such as gentamicin can be added to the medium in the culture, if necessary.

A transformant obtained using a plant cell as the host cell can be cultured as a cell or after differentiating it into a plant cell or organ. The medium for culturing the transformant includes generally used Murashige and Skoog (MS) medium and White medium, the medium obtained by adding a plant hormone such as auxin or cytokinin to these medium, and the like.

The culture is carried out generally at a pH of 5 to 9 and 20 to 40°C for 3 to 60 days.

Also, an antibiotic such as kanamycin or hygromycin can be added to the medium in the culture, if necessary.

Thus, an antibody composition can be produced by culturing a transformant derived from yeast, an animal cell or a plant cell which comprises a recombinant vector into which a DNA encoding an antibody molecule is inserted, in accordance with a general culturing method, to thereby produce and accumulate the antibody composition, and then recovering the antibody composition from the culture.

The process for producing an antibody composition includes a method of intracellular expression in a host cell, a method of extracellular secretion from a host cell, and a method of production on a host cell membrane outer envelope. The method can be selected by changing the host cell used or the structure of an antibody molecule produced.

When the antibody composition is produced in a host cell or on an outer membrane of a host cell, it can be positively secreted extracellularly in accordance with the method of Paulson et al. [J. Biol. Chem., 264, 17619 (1989)], the method of Lowe et al. [Proc. Natl. Acad Sci. USA, 86, 8227 (1989), Genes Develop., 4, 1288 (1990)], the methods described in Japanese Published Unexamined Patent Application No. 336963/93 and Japanese Published Unexamined Patent Application No. 823021/94 and the like.

That is, an antibody molecule of interest can be positively secreted extracellularly from a host cell by inserting a DNA encoding the antibody molecule and a DNA encoding a signal peptide suitable for the expression of the antibody molecule into an expression vector using a gene recombination technique, and introducing the expression vector into the host cell to express the antibody molecule.

Also, the production can be increased in accordance with the method described in Japanese Published Unexamined Patent Application No. 227075/90 using a gene amplification system using a dihydrofolate reductase gene.

In addition, the antibody composition can also be produced using a gene-introduced animal individual (transgenic non-human animal) or a plant individual (transgenic plant) which is constructed by the redifferentiation of an animal or plant cell into which the gene is introduced.

When the transformant is an animal individual or a plant individual, an antibody composition can be produced in accordance with a general method by rearing or cultivating it to thereby produce and accumulate the antibody composition and then recovering the antibody composition from the animal or plant individual.

The process for producing an antibody composition using an animal individual includes a method in which the antibody composition of interest is produced in an animal constructed by introducing a gene in accordance with a known method [American Journal of Clinical Nutrition, 63, 639S (1996); American Journal of Clinical Nutrition, 63, 627S (1996); BiolTechnology, 9, 830 (1991)].

In the case of an animal individual, an antibody composition can be produced by rearing a transgenic non-human animal into which a DNA encoding an antibody molecule is introduced to thereby produce and accumulate the antibody composition in the animal, and then recovering the antibody composition from the animal. The place in the animal where the composition is produced and accumulated includes milk (Japanese Published Unexamined Patent Application No. 309192/88) and eggs of the animal. As the promoter used in this case, any promoter can be used, so long as it can function in an animal. Preferred examples include mammary gland cell-specific promoters such as α casein promoter, β casein promoter, β lactoglobulin promoter, whey acidic protein promoter and the like.

The process for producing an antibody composition using a plant individual includes a method in which an antibody composition is produced by cultivating a transgenic plant into which a DNA encoding an antibody molecule is introduced by a known method *[*Tissue Culture (Soshiki Baiyo), 20 (1994); Tissue Culture (Soshiki Baiyo), 21 (1995); Trends in Biotechnology, 15, 45 (1997)] to produce and accumulate the antibody composition in the plant, and then recovering the antibody composition from the plant.

Regarding purification of an antibody composition produced by a transformant into which a gene encoding an antibody molecule is introduced, for example, when the antibody composition is intracellularly expressed in a dissolved state, the cells after culturing are recovered by centrifugation, suspended in an aqueous buffer and then disrupted using ultrasonicator, French press, Manton Gaulin homogenizer, dynomill or the like to obtain a cell-free extract, which is centrifuged to obtain a supernatant, and a purified preparation of the antibody composition can be obtained by subjecting the supernatant to a general enzyme isolation and purification techniques such as solvent extraction; salting out with ammonium sulfate etc.; desalting; precipitation with an organic solvent; anion exchange chromatography using a resin such as diethylaminoethyl (DEAE)-sepharose, DIAION HPA-75 (manufactured by Mitsubishi Chemical); cation exchange chromatography using a resin such as S-Sepharose FF (manufactured by Pharmacia); hydrophobic chromatography using a resin such as butyl-Sepharose or phenyl-Sepharose; gel filtration using a molecular sieve; affinity chromatography; chromatofocusing; electrophoresis such as isoelectric focusing; and the like which may be used alone or in combination.

When the antibody composition is expressed intracellularly by forming an inclusion body, the cells are recovered, disrupted and centrifuged in the same manner, and the inclusion body of the antibody composition is recovered as a precipitation fraction. The recovered inclusion body of the antibody composition is solubilized with a protein denaturing agent. The antibody composition is made into a normal three-dimensional structure by diluting or dialyzing the solubilized solution, and then a purified preparation of the antibody composition is obtained by the same isolation purification method as above.

When the antibody composition is secreted extracellularly, the antibody composition can be recovered from the culture supernatant. That is, the culture is treated by a technique such as centrifugation in the same manner as above to obtain a soluble fraction, and a purified preparation of the antibody composition can be obtained from the soluble fraction by the same isolation purification method as above.

The antibody composition thus obtained includes an antibody, the fragment of the antibody, a fusion protein comprising the Fc region of the antibody, and the like.

As examples for obtaining the antibody composition, processes for producing a composition of humanized antibody composition and Fc fusion protein are described below in detail, but other antibody compositions can also be obtained in accordance with the methods mentioned above and the said method.

### A. Preparation of humanized antibody composition

### (1) Construction of vector for expression of humanized antibody

A vector for expression of humanized antibody is an expression vector for animal cell into which genes encoding CH and CL of a human antibody are inserted, which can be constructed by cloning each of genes encoding CH and CL of a human antibody into an expression vector for animal cell.

The C regions of a human antibody may be CH or CL of any human antibody. Examples include the C region belonging to IgGl subclass in the H chain of a human antibody (hereinafter referred to as "hCγl"), the C region belonging to κ class in the L chain of a human antibody (hereinafter referred to as "hCκ"), and the like.

As the genes encoding CH and CL of a human antibody, a chromosomal DNA comprising an exon and an intron can be used, and a cDNA can also be used.

As the expression vector for animal cell, any vector can be used, so long as a gene encoding the C region of a human antibody can be inserted thereinto and expressed therein. Examples include pAGE107 [Cytotechnology, 3, 133 (1990)], pAGE103 [J. Biochem., 101, 1307 (1987)], pHSG274 [Gene, 27, 223 (1984)], pKCR [Proc. Natl. Acad. Sci. USA, 78, 1527 (1981), pSG1 β d2-4 [Cytotechnology, 4, 173 (1990)] and the like. The promoter and enhancer in the expression vector for animal cell include SV40 early promoter and enhancer [J. Biochem., 101, 1307 (1987)], Moloney mouse leukemia virus LTR [Biochem. Biophys. Res. Commun., 149, 960 (1987)], immunoglobulin H chain promoter [Cell, 41, 479 (1985)] and enhancer [Cell, 33, 717 (1983)], and the like.

The vector for expression of humanized antibody may be either of a type in which genes encoding the H chain and L chain of an antibody exist on separate vectors or of a type in which both genes exist on the same vector (hereinafter referred to as "tandem type"). In respect of easiness of construction of a vector for expression of humanized antibody, easiness of introduction into animal cells, and balance between the expression amounts of the H and L chains of an antibody in animal cells, a tandem type of the vector for expression of humanized antibody is more preferred [J. Immunol. Methods, 167, 271 (1994)].

The constructed vector for expression of humanized antibody can be used for expression of a human chimeric antibody and a human CDR-grafted antibody in animal cells.

### (2) Obtaining of cDNA encoding V region of non-human animal antibody

cDNAs encoding VH and VL of a non-human animal antibody such as a mouse antibody can be obtained in the following manner.

A cDNA is synthesized from mRNA extracted from a hybridoma cell which produces the mouse antibody of interest. The synthesized cDNA is cloned into a vector such as a phage or a plasmid to obtain a cDNA library. Each of a recombinant phage or recombinant plasmid comprising a cDNA encoding VH and a recombinant phage or recombinant plasmid comprising a cDNA encoding VL is isolated from the library by using a C region part or a V region part of an existing mouse antibody as the probe. Full nucleotide sequences of VH and VL of the mouse antibody of interest on the recombinant phage or recombinant plasmid are determined, and full length amino acid sequences of VH and VL are deduced from the nucleotide sequences.

As the non-human animal, any animal such as mouse, rat, hamster or rabbit can be used so long as a hybridoma cell can be prepared therefrom.

The method for preparing total RNA from a hybridoma cell includes the guanidine thiocyanate-cesium trifluoroacetate method [Methods in Enzymology, 154, 3 (1987)] and the like, and the method for preparing mRNA from total RNA includes an oligo(dT)-immobilized cellulose column method [Molecular Cloning, A Laboratory Manual, Cold Spring Harbor Lab., Press New York (1989)] and the like. In addition, a kit for preparing mRNA from a hybridoma cell includes Fast Track mRNA Isolation Kit (manufactured by Invitrogen), Quick Prep mRNA Purification Kit (manufactured by Pharmacia) and the like.

The method for synthesizing a cDNA and preparing a cDNA library includes the usual methods [Molecular Cloning, A Laboratory Manual, Cold Spring Harbor Lab., Press New York (1989), Current Protocols in Molecular Biology, Supplement 1-34], methods using a commercially available kit such as SuperScript^{™}, Plasmid System for cDNA Synthesis and Plasmid Cloning (manufactured by GIBCO BRL) or ZAP-cDNA Synthesis Kit (manufactured by Stratagene), and the like.

In preparing the cDNA library, the vector into which a cDNA synthesized by using mRNA extracted from a hybridoma cell as the template is inserted may be any vector so long as the cDNA can be inserted. Examples include ZAP Express *[*Strategies, 5. 58 (1992)], pBluescript II SK(+) [Nucleic Acids Research, 17, 9494 (1989)], λZAPII (manufactured by Stratagene), λgt10 and λgt11 [DNA Cloning, A Practical Approach, I, 49 (1985)], Lambda BlueMid (manufactured by Clontech), λExCell, pT7T3 18U (manufactured by Pharmacia), pcD2 [Mol. Cell. Biol., 3, 280 (1983)], pUC18 [Gene, 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [Strategies, 5, 81 (1992)], C600 [Genetics, 39, 440 (1954)], Y1088 and Y1090 [Science, 222, 778 (1983)], NM522 [J. Mol. Biol., 166, 1 (1983)], K802 [J. Mol. Biol., 16, 118 (1966)], JM105 [Gene, 38, 275 (1985)] and the like.

As the method for selecting a cDNA clone encoding VH and VL of a non-human animal antibody from the cDNA library, a colony hybridization or a plaque hybridization using an isotope- or fluorescence-labeled probe can be used [Molecular Cloning, A Laboratory Manual, Cold Spring Harbor Lab., Press New York (1989)]. The cDNA encoding VH and VL can also be prepared by preparing primers and carrying out polymerase chain reaction (hereinafter referred to as "PCR"; Molecular Cloning, A Laboratory Manual, Cold Spring Harbor Lab., Press New York (1989); Current Protocols in Molecular Biology, Supplement 1-34) using a cDNA synthesized from mRNA or a cDNA library as the template.

The nucleotide sequences of the cDNAs can be determined by digesting the selected cDNAs with appropriate restriction enzymes, cloning the fragments into a plasmid such as pBluescript SK(-) (manufactured by Stratagene), carrying out the reaction of a generally used nucleotide sequence analyzing method such as the dideoxy method of Sanger et al. [Proc. Natl. Acad. Sci. USA, 74, 5463 (1977)] or the like and then analyzing the clones using an automatic nucleotide sequence analyzer such as A.L.F. DNA Sequencer (manufactured by Pharmacia) or the like.

Whether or not the obtained cDNAs encode the full length amino acid sequences of VH and VL of the antibody containing a secretory signal sequence can be confirmed by deducing the full length amino acid sequences of VH and VL from the determined nucleotide sequence and comparing them with the full length amino acid sequences of VH and VL of known antibodies [Sequences of Proteins of Immunological Interest, US Dep. Health and Human Services (1991)].

### (3) Analysis of amino acid sequence of V region of non-human animal antibody

Regarding the full length amino acid sequences of VH and VL of the antibody comprising a secretory signal sequence, the length of the secretory signal sequence and the N-terminal amino acid sequences can be deduced and subgroups to which they belong can also be found, by comparing them with the full length amino acid sequences of VH and VL of known antibodies [Sequences of Proteins of Immunological Interest, US Dep. Health and Human Services (1991)]. In addition, the amino acid sequences of each CDR of VH and VL can also be found by comparing them with the amino acid sequences of VH and VL of known antibodies [Sequences of Proteins of Immunological Interest, US Dep. Health and Human Services (1991)].

### (4) Construction of human chimeric antibody expression vector

A human chimeric antibody expression vector can be constructed by cloning cDNAs encoding VH and VL of a non-human animal antibody into upstream of genes encoding CH and CL of a human antibody in the vector for expression of humanized antibody described in the item 2(1). For example, a human chimeric antibody expression vector can be constructed by ligating each of cDNAs encoding VH and VL of a non-human animal antibody to a synthetic DNA comprising nucleotide sequences at the 3'-terminals of VH and VL of a non-human animal antibody and nucleotide sequences at the 5'-terminals of CH and CL of a human antibody and also having a recognition sequence of an appropriate restriction enzyme at both terminals, and by cloning them into upstream of genes encoding CH and CL of a human antibody contained in the vector for expression of humanized antibody described in the item 2(1) in such a manner that they can be expressed in a suitable form.

### (5) Construction ofcDNA encoding V region of human CDR-grafted antibody

cDNAs encoding VH and VL of a human CDR-grafted antibody can be constructed as follows. First, amino acid sequences of the frameworks (hereinafter referred to as "FR") of VH and VL of a human antibody for grafting CDR of VH and VL of a non-human animal antibody of interest is selected. As the amino acid sequences of FRs of VH and VL of a human antibody, any amino acid sequences can be used so long as they are derived from a human antibody. Examples include amino acid sequences of FRs of VH and VL of human antibodies registered at databases such as Protein Data Bank, amino acid sequences common in each subgroup of FRs of VH and VL of human antibodies [Sequences of Proteins of Immunological Interest, US Dep. Health and Human Services (1991)] and the like. In order to produce a human CDR-grafted antibody having enough activities, it is preferred to select an amino acid sequence having a homology as high as possible (at least 60% or more) with amino acid sequences of VH and VL of a non-human animal antibody of interest.

Next, the amino acid sequences of CDRs of VH and VL of the non-human animal antibody of interest are grafted to the selected amino acid sequences of FRs of VH and VL of a human antibody to design amino acid sequences of VH and VL of the human CDR-grafted antibody. The designed amino acid sequences are converted into DNA sequences by considering the frequency of codon usage found in nucleotide sequences of antibody genes [Sequences of Proteins of Immunological Interest, US Dep. Health and Human Services (1991)], and the DNA sequences encoding the amino acid sequences of VH and VL of the human CDR-grafted antibody are designed. Based on the designed DNA sequences, several synthetic DNAs having a length of about 100 bases are synthesized, and PCR is carried out by using them. In this case, it is preferred in each of the H chain and the L chain that 6 synthetic DNAs are designed in view of the reaction efficiency of PCR and the lengths of DNAs which can be synthesized.

Also, they can be easily cloned into the vector for expression of humanized antibody described in the item 2(1) by introducing recognition sequences of an appropriate restriction enzyme into the 5'-terminals of the synthetic DNA present on both terminals. After the PCR, the amplified product is cloned into a plasmid such as pBluescript SK(-) (manufactured by Stratagene) and the nucleotide sequences are determined by the method in the item 2(2) to thereby obtain a plasmid having DNA sequences encoding the amino acid sequences of VH and VL of the desired human CDR-grafted antibody.

### (6) Modification of amino acid sequence of V region of human CDR-grafted antibody

It is known that when a human CDR-grafted antibody is prepared by simply grafting only CDRs in VH and VL of a non-human animal antibody into FRs in VH and VL of a human antibody, its antigen-binding activity is lower than that of the original non-human animal antibody [BIO/TECHNOLOGY, 9, 266 (1991)]. As the reason, it is considered that several amino acid residues of FRs other than CDRs directly or indirectly relate to antigen-binding activity in VH and VL of the original non-human animal antibody, and that they are changed to different amino acid residues of FRs in VH and VL of a human antibody. In order to solve the problem, in human CDR-grafted antibodies, among the amino acid sequences of FRs in VH and VL of a human antibody, an amino acid residue which directly relates to binding to an antigen, or an amino acid residue which indirectly relates to binding to an antigen by interacting with an amino acid residue in CDR or by maintaining the three-dimensional structure of an antibody is identified and modified to an amino acid residue which is found in the original non-human animal antibody to thereby increase the antigen binding activity which has been decreased [BIO/TECHNOLOGY, 9, 266 (1991)].

In the preparation of a human CDR-grafted antibody, it is the most important to efficiently identify the amino acid residues relating to the antigen binding activity in FR. For identifying the amino acid residues of FR relating to the antibody-antigen binding activity, the three-dimensional structure of an antibody is constructed, and analyzed by X-ray crystallography [J. Mol. Biol., 112, 535 (1977)], computer-modeling [Protein Engineering, 7, 1501 (1994)] or the like. Although the information of the three-dimensional structure of antibodies has been useful in the production of a human CDR-grafted antibody, method for producing a human CDR-grafted antibody which can be applied to all antibodies has not been established yet. Therefore, various attempts must be currently be necessary, for example, several modified antibodies of each antibody are produced and the relationship between each of the modified antibodies and its antibody binding activity is examined.

The amino acid sequence of FRs in VH and VL of a human antibody can be modified using various synthetic DNA for modification according to PCR as described in the item 2(5). With regard to the amplified product obtained by the PCR, the nucleotide sequence is determined according to the method as described in the item 2(2) to thereby confirm whether the objective modification has been carried out.

### (7) Construction of human CDR-grafted antibody expression vector

A human CDR-grafted antibody expression vector can be constructed by cloning the cDNAs encoding VH and VL of the human CDR-grafted antibody constructed in the items 2(5) and (6) into upstream of the gene encoding CH and CL of a human antibody in the vector for expression of humanized antibody described in the item 2(1). For example, a human CDR-grafted antibody expression vector can be constructed to be cloned by introducing recognizing sequences of an appropriate restriction enzyme into the 5'-terminal of synthetic DNAs positioned at both terminals, among the synthetic DNAs which are used in the items 2(5) and (6) for constructing the VH and VL of the human CDR-grafted antibody, so that they are expressed in an appropriate form in upstream of the genes encoding CH and CL of a human antibody in the vector for expression of humanized antibody described in the item 2(1).

### (8) Stable production of humanized antibody

A transformant capable of stably producing a human chimeric antibody and a human CDR-grafted antibody (both hereinafter referred to as "humanized antibody") can be obtained by introducing the vector for humanized antibody expression described in the items 2(4) and (7) into an appropriate animal cell.

The method for introducing a humanized antibody expression vector into an animal cell includes electroporation [Japanese Published Unexamined Patent Application No. 257891/90, Cytotechnology, 3, 133 (1990)] and the like.

As the animal cell into which a humanized antibody expression vector is introduced, any cell can be used so long as it is the cell of the present invention produced in the above item 1 and an animal cell which can produce the humanized antibody.

Examples include mouse myeloma cells such as NS0 cell and SP2/0 cell, Chinese hamster ovary cells such as CHO/dhfr⁻ cell and CHO/DG44 cell, rat myeloma such as YB2/0 cell and IR983F cell, BHK cell derived from a syrian hamster kidney, a human myeloma cell such as Namalwa cell, and the like. Chinese hamster ovary cell CHO/DG44 cell and rat myeloma YB2/0 cell are preferred.

After introduction of the humanized antibody expression vector, a transformant capable of stably producing the humanized antibody can be selected using a medium for animal cell culture comprising an agent such as G418 sulfate (hereinafter referred to as "G418"; manufactured by SIGMA) and the like in accordance with the method disclosed in Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum (hereinafter referred to as "FBS") to these media, and the like. The humanized antibody can be produced and accumulated in the culture supernatant by culturing the obtained transformant in a medium. The amount of production and antigen binding activity of the humanized antibody in the culture supernatant can be measured by a method such as enzyme-linked immunosorbent assay [hereinafter referred to as "ELISA"; Antibodies: A Laboratory Manual, Cold Spring Harbor Laboratory, Chapter 14 (1988); Monoclonal Antibodies: Principles and Practice, Academic Press Limited (1996)] or the like. Also, the amount of the humanized antibody produced by the transformant can be increased by using a DHFR gene amplification system in accordance with the method disclosed in Japanese Published Unexamined Patent Application No. 257891/90.

The humanized antibody can be purified from a culture supernatant of the transformant using a protein A column [Antibodies: A Laboratory Manual, Cold Spring Harbor Laboratory, Chapter 8 (1988); Monoclonal Antibodies: Principles and Practice, Academic Press Limited (1996)]. In addition, purification methods generally used for the purification of proteins can also be used. For example, the purification can be carried out through the combination of gel filtration, ion exchange chromatography, ultrafiltration, and the like. The molecular weight of the H chain, L chain or antibody molecule as a whole of the purified humanized antibody can be measured, e.g., by polyacrylamide gel electrophoresis [hereinafter referred to as "SDS-PAGE"; Nature, 227, 680 (1970)], Western blotting [Antibodies: A Laboratory Manual, Cold Spring Harbor Laboratory, Chapter 12 (1988); Monoclonal Antibodies: Principles and Practice, Academic Press Limited (1996)] or the like.

### B. Preparation of Fc fusion protein

### (1) Construction of Fc fusion protein expression vector

An Fc fusion protein expression vector is an expression vector for animal cell into which genes encoding the Fc region of a human antibody and a protein to be fused are inserted, which can be constructed by cloning each of genes encoding the Fc region of a human antibody and the protein to be fused into an expression vector for animal cell.

The Fc region of a human antibody includes those containing a part of a hinge region and/or CH1 in addition to regions containing CH2 and CH3 regions. Also, it can be any Fc region so long as at least one amino acid of CH2 or CH3 may be deleted, substituted, added or inserted, and substantially has the binding activity to the Fcγ receptor.

As the genes encoding the Fc region of a human antibody and the protein to be fused, a chromosomal DNA comprising an exon and an intron can be used, and a cDNA can also be used. The method for linking the genes and the Fc region includes PCR using each of the gene sequences as the template (*Molecular Cloning,* Second Edition; Current Protocols in Molecular Biology, Supplement 1-34).

As the expression vector for animal cell, any vector can be used, so long as a gene encoding the C region of a human antibody can be inserted thereinto and expressed therein. Examples include pAGE107 [Cytotechnology, 3, 133 (1990)], pAGE103 [J. Biochem., 101, 1307 (1987)], pHSG274 [Gene, 27, 223 (1984)], pKCR [Proc. Natl. Acad. Sci. USA, 78, 1527 (1981), pSG1 β d2-4 [Cytotechnology, 4, 173 (1990)] and the like. The promoter and enhancer in the expression vector for animal cell include SV40 early promoter and enhancer [J. Biochem., 101, 1307 (1987)], Moloney mouse leukemia virus LTR promoter [Biochem. Biophys. Res. Commun., 149, 960 (1987)], immunoglobulin H chain promoter [Cell, 41, 479 (1985)] and enhancer [Cell, 33, 717 (1983)], and the like.

### (2) Preparation of DNA encoding Fc region of human antibody and protein to be fused

A DNA encoding the Fc region of a human antibody and the protein to be fused can be obtained in the following manner.

A cDNA is synthesized from mRNA extracted from a cell or tissue which expresses the protein of interest to be fused with Fc. The synthesized cDNA is cloned into a vector such as a phage or a plasmid to obtain a cDNA library. A recombinant phage or recombinant plasmid comprising a cDNA encoding the protein of interest is isolated from the library by using the gene sequence part of the protein of interest as the probe. A full nucleotide sequence of the protein of interest on the recombinant phage or recombinant plasmid is determined, and a full length amino acid sequence is deduced from the nucleotide sequence.

As the non-human animal, any animal such as mouse, rat, hamster or rabbit can be used, so long as a cell or tissue can be removed therefrom.

The method for preparing a total RNA from a cell or tissue includes the guanidine thiocyanate-cesium trifluoroacetate method [Methods in Enzymology, 154, 3 (1987)] and the like, and the method for preparing mRNA from total RNA includes an oligo (dT)-immobilized cellulose column method (Molecular Cloning, Second Edition) and the like. In addition, a kit for preparing mRNA from a cell or tissue includes Fast Track mRNA Isolation Kit (manufactured by Invitrogen), Quick Prep mRNA Purification Kit (manufactured by Pharmacia) and the like.

The method for synthesizing a cDNA and preparing a cDNA library includes the usual methods (*Molecular Cloning,* Second Edition; *Current Protocols in Molecular Biology,* Supplement 1-34); methods using a commercially available kit such as SuperScript^{™}, Plasmid System for cDNA Synthesis and Plasmid Cloning (manufactured by GIBCO BRL) or ZAP-cDNA Synthesis Kit (manufactured by Stratagene); and the like.

In preparing the cDNA library, the vector into which a cDNA synthesized by using mRNA extracted from a cell or tissue as the template is inserted may be any vector so long as the cDNA can be inserted. Examples include ZAP Express [Strategies, 5, 58 (1992)], pBluescript II SK(+) [Nucleic Acids Research, 17, 9494 (1989)], λZAPII (manufactured by Stratagene), λgt10 and λgt11 [DNA Cloning, A Practical Approach, I, 49 (1985)], Lambda BlueMid (manufactured by Clontech), λExCell, pT7T3 18U (manufactured by Pharmacia), pcD2 [Mol. Cell. Biol., 3, 280 (1983)], pUC18 [Gene, 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [Strategies, 5, 81 (1992)], C600 [Genetics, 39, 440 (1954)], Y1088 and Y1090 [Science, 222, 778 (1983)], NM522 [J. Mol. Biol., 166, 1 (1983)], K802 [J. Mol. Biol., 16, 118 (1966)], JM105 [Gene, 38, 275 (1985)] and the like.

As the method for selecting a cDNA clone encoding the protein of interest from the cDNA library, a colony hybridization or a plaque hybridization using an isotope-or fluorescence-labeled probe can be used (Molecular Cloning, Second Edition). The cDNA encoding the protein of interest can also be prepared by preparing primers and using a cDNA synthesized from mRNA or a cDNA library as the template according to PCR.

The method for fusing the protein of interest with the Fc region of a human antibody includes PCR. For example, any synthesized oligo DNAs (primers) are designed at the 5'-terminal and 3'-terminal of the gene sequence encoding the protein of interest, and PCR is carried out to prepare a PCR product. In the same manner, any primers are designed for the gene sequence encoding the Fc region of a human antibody to be fused to prepare a PCR product. At this time, the primers are designed in such a manner that the same restriction enzyme site or the same gene sequence is present between the 3'-terminal of the PCR product of the protein to be fused and the 5'-terminal of the PCR product of the Fc region. When it is necessary to modify the amino acids around the linked site, mutation is introduced by using the primer into which the mutation is introduced. PCR is further carried out by using the two kinds of the obtained PCR fragments to link the genes. Also, they can be linked by carrying out ligation after treatment with the same restriction enzyme.

The nucleotide sequence of the DNA can be determined by digesting the gene sequence linked by the above method with appropriate restriction enzymes, cloning the fragments into a plasmid such as pBluescript SK(-) (manufactured by Stratagene), carrying out analysis by using a generally used nucleotide sequence analyzing method such as the dideoxy method of Sanger et al. [Proc. Natl. Acad Sci. USA, 74, 5463 (1977)] or an automatic nucleotide sequence analyzer such as A.L.F. DNA Sequencer (manufactured by Pharmacia).

Whether or not the obtained cDNA encodes the full length amino acid sequences of the Fc fusion protein containing a secretory signal sequence can be confirmed by deducing the full length amino acid sequence of the Fc fusion protein from the determined nucleotide sequence and comparing it with the amino acid sequence of interest.

### (3) Stable production of Fc fusion protein

A transformant capable of stably producing an Fc fusion protein can be obtained by introducing the Fc fusion protein expression vector described in the item 2.B.(1) into an appropriate animal cell.

The method for introducing the Fc fusion protein expression vector into an animal cell include electroporation [Japanese Published Unexamined Patent Application No. 257891/90, Cytotechnology, 3, 133 (1990)] and the like.

As the animal cell into which the Fc fusion protein expression vector is introduced, any cell can be used, so long as it is the cell of the present invention prepared in the above item 1 and an animal cell which can produce the Fc fusion protein.

Examples include mouse myeloma cells such as NS0 cell and SP2/0 cell, Chinese hamster ovary cells such as CHO/dhfr cell and CHO/DG44 cell, rat myeloma such as YB2/0 cell and IR983F cell, BHK cell derived from a syrian hamster kidney, a human myeloma cell such as Namalwa cell, and the like, and a Chinese hamster ovary cell CHO/DG44 cell, a rat myeloma YB2/0 cell and the like are preferred.

After introduction of the Fc fusion protein expression vector, a transformant capable of stably producing the Fc fusion protein expression vector can be selected using a medium for animal cell culture comprising an agent such as G418 and the like in accordance with the method disclosed in Japanese Published Unexamined Patent Application No. 257891/90. The medium to culture animal cells includes RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum to these media, and the like. The Fc fusion protein can be produced and accumulated in the culture supernatant by culturing the obtained transformant in a medium. The amount of production and antigen binding activity of the Fc fusion protein in the culture supernatant can be measured by a method such as ELISA. Also, the amount of the Fc fusion protein produced by the transformant can be increased by using a *dhfr* gene amplification system in accordance with the method disclosed in Japanese Published Unexamined Patent Application No. 257891/90.

The Fc fusion protein can be purified from a culture supernatant culturing the transformant by using a protein A column or a protein G column (*Antibodies,* Chapter 8; *Monoclonal Antibodies*). In addition, purification methods generally used for the purification of proteins can also be used. For example, the purification can be carried out through the combination of gel filtration, ion exchange chromatography, ultrafiltration and the like. The molecular weight as a whole of the purified Fc fusion protein molecule can be measured by SDS-PAGE [Nature, 227, 680 (1970)], Western blotting (Antibodies, Chapter 12, Monoclonal Antibodies) or the like.

Thus, methods for producing an antibody composition using an animal cell as the host cell have been described, but, as described above, the antibody composition can also be produced by yeast, an insect cell, a plant cell, an animal individual or a plant individual as described above.

When a host cell has a gene capable of expressing glycoprotein such as antibody molecule in the host cell, the host cell is prepared according to the method described in the item 1, the cell is cultured, and the glycoprotein of interest is purified from the culture to obtain the glycoprotein.

### 3. Activity evaluation of glycoprotein

Methods for measuring the amount of the purified glycoprotein, affinity to its receptor, half-life in blood, distribution in tissue after administration into blood and change of interactions between the proteins necessary for expression of pharmacological activity are measured by known methods described in Current Protocols In Protein Science, John Wiley & Sons Inc., (1995); New Biochemical Experimentation Series 19-Animal Experimental Test, Tokyo Kagaku Dojin, edited by Japanese Biochemical Society (1991); New Biochemical Experimentation Series 8-Intracellular Information and Cell Response, Tokyo Kagaku Dojin, edited by Japanese Biochemical Society (1990); New Biochemical Experimentation Series 9-Hormone I, Peptide hormone, Tokyo Kagaku Dojin, edited by Japanese Biochemical Society (1991); Experimental Biological Course 3-Isotope Experimental Test, Maruzen (1982); Monoclonal Antibodies: Principles and Applications, Wiley-Liss, Inc., (1995); Enzyme-Linked Immuno Adsorbent Assay, 3rd Ed., Igaku Shoin (1987); Revised Enzyme Immunoassay, Gakusai Kikaku (1985); and the like.

Specific examples include a method in which a purified glycoprotein is labeled with a compound such as a radioisotope and binding activity to a receptor of the labeled glycoprotein or an interacted protein is quantitatively measured. Furthermore, interaction between the proteins can be measured by using various apparatus such as BIAcore Series manufactured by Biacore [J. Immnunol. Methods, 145, 229 (1991); Experimental Medicine Supplement, Biomanual UP Series, Experimental Test of Intermolecular Interaction Experimental Test, Yodo-sha (1996)].

By administration of the labeled glycoprotein into the living body, the half-life in blood and the distribution in tissue after administered into the living body can be observed. Detection of the labeled body is preferably carried out by a detection method in which a method for detecting a labeled substance is combined with an antigen-antibody reaction using an antibody specific to the glycoprotein which is to be detected.

### 4. Activity evaluation of antibody composition

As methods for measuring the amount of the protein, the affinity to an antigen and the effector function of the purified antibody composition, the known methods described in *Monoclonal Antibodies, Antibody Engineering* and the like can be used.

As the examples, when the antibody composition is a humanized antibody, the binding activity with an antigen and the binding activity with an antigen-positive cultured cell clone can be measured by ELISA, the immunofluorescent method [Cancer Immunol. Immunother., 36, 373 (1993)] or the like. The cytotoxic activity against an antigen-positive cultured cell clone can be evaluated by measuring CDC activity, ADCC activity [Cancer Immunol. Immunother., 36, 373 (1993)] and the like.

Also, safety and therapeutic effect of the antibody composition in human can be evaluated using an appropriate model of animal species relatively close to human, such as *Macaca fascicularis.*

### 5. Analysis of sugar chains in glycoprotein

The sugar chain structure of the glycoprotein expressed in a host cell can be analyzed in accordance with the general analysis of the sugar chain structure of a glycoprotein. For example, the sugar chain which is bound to the IgG molecule comprises a neutral sugar such as galactose, mannose or fucose, an amino sugar such as N-acetylglucosamine and an acidic sugar such as sialic acid, and can be analyzed according to a method such as sugar chain structure analysis by using sugar composition analysis, two dimensional sugar chain mapping method or the like.

Hereinafter, the analysis methods of the sugar chain in the antibody composition are specifically described, but other glycoproteins can be analyzed in the same manner.

### (1) Composition analysis of neutral sugar and amino sugar

The composition of the sugar chain in an antibody molecule can be analyzed by carrying out acid hydrolysis of sugar chains with an acid such as trifluoroacetic acid to release a neutral sugar or an amino sugar and measuring the composition ratio.

Examples include a method using a sugar composition analyzer (BioLC) manufactured by Dionex. The BioLC is an apparatus which analyzes a sugar composition by HPAEC-PAD (high performance anion-exchange chromatography-pulsed amperometric detection) method [J. Liq. Chromatogr., 6, 1577 (1983)].

The composition ratio can also be analyzed by a fluorescence labeling method using 2-aminopyridine. Specifically, the composition ratio can be calculated in accordance with a known method [Agric. Biol. Chem., 55(1). 283-284 (1991)], by labeling an acid-hydrolyzed sample with a fluorescence with 2-aminopyridylation and then analyzing the composition by HPLC.

### (2) Analysis of sugar chain structure

The sugar chain structure in an antibody molecule can be analyzed by the two dimensional sugar chain mapping method [Anal. Biochem., 171, 73 (1988), Biochemical Experimentation Methods 23 - Methods for Studying Glycoprotein Sugar Chains (Japan Scientific Societies Press) edited by Reiko Takahashi (1989)]. The two dimensional sugar chain mapping method is a method for deducing a sugar chain structure by, e.g., plotting the retention time or elution position of a sugar chain by reverse phase chromatography as the X-axis and the retention time or elution position of the sugar chain by normal phase chromatography as the Y-axis, respectively, and comparing them with those of known sugar chains.

Specifically, sugar chains are released from an antibody by subjecting the antibody to hydrazinolysis, and the released sugar chains are subjected to fluorescence labeling with 2-aminopyridine (hereinafter referred to as "PA") [J. Biochem., 95, 197 (1984)], and then the sugar chains are separated from an excess PA-treating reagent by gel filtration, and subjected to reverse phase chromatography. Thereafter, each peak of the separated sugar chains is subjected to normal phase chromatography. The sugar chain structure can be deduced by plotting the results on a two dimensional sugar chain map and comparing them with the spots of a sugar chain standard (manufactured by Takara Shuzo) or a literature [Anal. Biochem., 171, 73 (1988)].

The structure deduced by the two dimensional sugar chain mapping method can be confirmed by further carrying out mass spectrometry such as MALDI-TOF-MS of each sugar chain.

### 6. Analysis of side effect of introduced RNA

The introduced RNA might affect the level of expression, translation and the like of a gene having high homology, in addition to the inhibition of the function of a target enzyme [Nature Biotechnol., 21, 635 (2003), Proc. Natl. Acad Sci. USA, 101, 1892 (2004)]. Accordingly, when a glycoprotein such as an antibody composition is produced, whether or not growth of a transformant or expression of a produced glycoprotein is influenced by side effect of the introduction of RNA should be analyzed.

Specifically, regarding the expression cell of a glycoprotein such as an antibody composition, a parent cell into which the RNA of the present invention is not introduced and the introduced cell are cultured simultaneously to confirm that there is no change in the growth curve of the cells and the level of expression of the glycoprotein. By analyzing the various properties of the produced glycoproteins, it is confirmed that the produced glycoprotein have no difference except for the biological activity due to the difference in the sugar chain structures.

The sugar chain structure of the produced glycoprotein can be analyzed by the method described in the above item 5. Various properties of the glycoprotein can be analyzed by any known analysis methods of proteins. The analysis methods of proteins include physicochemical analyses such as electrophoresis, gel filtration, isoelectric point and amino acid sequence analyses, affinity to an antigen in the case where the produced glycoprotein is an antibody, enzyme activity in the case where the produced glycoprotein is an enzyme, and affinity to a respective ligand or receptor in the case where the glycoprotein is a ligand or a receptor.

### 7. Application of glycoprotein

A glycoprotein such as an antibody composition has a sugar chain structure with which no fucose is modified and has high biological activity so that effects such as improvement of affinity to its receptor, improvement of half-life in blood, improvement of distribution in tissue after administration in blood and improvement of interaction between a protein necessary for expression of pharmacological activity are expected. Particularly, the antibody composition has high effector function, i.e., high antibody-dependent cell-mediated cytotoxic activity. The glycoprotein having high physiological activity or the antibody composition having high ADCC activity is useful for preventing and treating various diseases including cancers, inflammatory diseases, immune diseases such as autoimmune diseases and allergies, cardiovascular diseases and viral or bacterial infections.

In the case of cancers, namely malignant tumors, cancer cells grow. General anti-tumor agents inhibit the growth of cancer cells. In contrast, an antibody having high ADCC activity can treat cancers by injuring cancer cells through its cell killing effect, and therefore, it is more effective as a therapeutic agent than the general anti-tumor agents. At present, in the therapeutic agent for cancers, an anti-tumor effect of an antibody medicament alone is insufficient in many cases, so that combination therapy with chemotherapy has been carried out [Science, 280, 1197 (1998)]. If higher anti-tumor effect is found by the antibody composition of the present invention alone, the dependency on chemotherapy will be decreased and side effects will be reduced.

In immune diseases such as inflammatory diseases, autoimmune diseases and allergies, *in vivo* reactions of the diseases are induced by the release of a mediator molecule by immunocytes, so that the allergic reaction can be inhibited by eliminating immunocytes using an antibody having high ADCC activity.

The cardiovascular diseases include arteriosclerosis and the like. The arteriosclerosis is treated using balloon catheter at present, but cardiovascular diseases can be prevented and treated by suppressing growth of arterial cells in restricture after treatment using an antibody having high ADCC activity.

Various diseases including viral and bacterial infections can be prevented and treated by suppressing proliferation of cells infected with a virus or bacterium using an antibody having high ADCC activity.

An antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen are exemplified below.

The antibody which recognizes a tumor-related antigen includes anti- CA125 antibody, anti-17-1A antibody, anti-integrin αvβ3 antibody, anti-CD33 antibody, anti-CD22 antibody, anti-HLA antibody, anti-HLA-DR antibody, anti-CD20 antibody, anti-CD19 antibody, anti-EGF receptor antibody *[*Immunology Today, 21, 403 (2000)], anti-CD10 antibody [American Journal of Clinical Pathology, 113, 374 (2000); Proc. Natl. Acad. Sci. USA, 79, 4386 (1982)], anti-GD₂ antibody [Anticancer Res., 13, 331 (1993)], anti-GD₃ antibody [Cancer Immunol. Immunother., 36, 260 (1993)], anti-GM₂ antibody [Cancer Res., 54, 1511 (1994)], anti-HER2 antibody [Proc. Natl. Acad Sci. USA, 89, 4285 (1992)], anti-CD52 antibody [Nature, 332, 323 (1988)], anti-MAGE antibody [British J. Cancer, 83, 493 (2000)], anti-HM1.24 antibody [Molecular Immunol., 36, 387 (1999)], anti-parathyroid hormone-related protein (PTHrP) antibody [Cancer, 88, 2909 (2000)], anti-FGF8 antibody [Proc. Natl. Acad. Sci. USA, 86, 9911 (1989)], anti-basic fibroblast growth factor antibody, anti-FGF8 receptor antibody [J. Biol. Chem., 265, 16455 (1990)], anti-basic fibroblast growth factor receptor antibody, anti-insulin-like growth factor antibody, anti-insulin-like growth factor receptor antibody [J. Neurosci. Res., 40, 647 (1995)], anti-PMSA antibody [J. Urology, 160, 2396 (1998)], anti-vascular endothelial cell growth factor antibody [Cancer Res., 57, 4593 (1997)], anti-vascular endothelial cell growth factor receptor antibody [Oncogene, 19, 2138 (2000)] and the like.

The antibody which recognizes an allergy- or inflammation-related antigen includes anti-IgE antibody, anti-CD23 antibody, anti-CD 11a antibody [Immunology Today, 21, 403 (2000)], anti-CRTH2 antibody [J. Immunol., 162, 1278 (1999)], anti-CCR8 antibody (WO99/25734), anti-CCR3 antibody (US6207155), anti-interleukin 6 antibody [Immunol. Rev., 127, 5 (1992)], anti-interleukin 6 receptor antibody [Molecular Immunol., 31, 371 (1994)], anti-interleukin 5 antibody [Immunol. Rev., 127, 5 (1992)], anti-interleukin 5 receptor antibody, anti-interleukin 4 antibody [Cytokine, 3, 562 (1991)], anti-interleukin 4 receptor antibody [J. Immunol. Meth., 217, 41 (1998)], anti-tumor necrosis factor antibody [Hybridoma, 13, 183 (1994)], anti-tumor necrosis factor receptor antibody [Molecular Pharmacol., 58, 237 (2000)], anti-CCR4 antibody [Nature, 400, 776 (1999)], anti-chemokine antibody [J. Immuno. Meth., 174, 249 (1994)], anti-chemokine receptor antibody [J. Exp. Med, 186, 1373 (1997)] and the like.

The antibody which recognizes a cardiovascular disease-related antigen includes anti-GpIIb/IIIa antibody [J. Immunol., 152, 2968 (1994)], anti-platelet-derived growth factor antibody [Science, 253, 1129 (1991)], anti-platelet-derived growth factor receptor antibody [J. Biol. Chem., 272, 17400 (1997)], anti-blood coagulation factor antibody [Circulation, 101, 1158 (2000)] and the like.

The antibody which recognizes an antigen relating to autoimmune diseases such as psoriasis, rheumatoid arthritis, crohn' disease, uncreative colitis, systemic lupus erythema tosus, and multiple sclerosis includes an anti-auto-DNA antibody [Immunol. Letters, 72, 61 (2000)], anti-CD11a antibody, anti-ICAM3 antibody, anti-CD80 antibody, anti-CD2 antibody, anti-CD3 antibody, anti-CD4 antibody, anti-integrin α4β7 antibody, anti-CD40L antibody, anti-IL-2 receptor antibody [Immunology Today, 21, 403 (2000)] and the like.

The antibody which recognizes a viral or bacterial infection-related antigen includes anti-gp120 antibody [Structure, 8, 385 (2000)], anti-CD4 antibody [J. Rheumatology, 25, 2065 (1998)], anti-CCR5 antibody and anti-Vero toxin antibody [J. Clin. Microbiol., 37, 396 (1999)] and the like.

These antibodies can be obtained from public organizations such as ATCC (The American Type Culture Collection), RIKEN Gene Bank at The Institute of Physical and Chemical Research and National Institute of Bioscience and Human Technology, Agency of Industrial Science and Technology, or private reagent sales companies such as Dainippon Pharmaceutical, R & D SYSTEMS, PharMingen, Cosmo Bio and Funakoshi.

Hereinafter, examples of the Fc region fusion protein of the present invention are described below.

Examples of a fusion protein of an binding protein relating to inflammatory diseases and immune diseases such as autoimmune diseases and allergies with the Fc region of an antibody include etanercept which is a fusion protein of sTNFRII with the Fc region (USP5605690), alefacept which is a fusion protein of LFA-3 expressed on antigen presenting cells with the Fc region (USP5914111), a fusion protein of Cytotoxic T Lymphocyte-associated antigen-4 (CTLA-4) with the Fc region [J. Exp. Med., 181, 1869 (1995)], a fusion protein of interleukin 15 with the Fc region [J. Immunol., 160, 5742 (1998)], a fusion protein of factor VII with the Fc region [Proc. Natl. Acad Sci. USA, 98, 12180 (2001)], a fusion protein of interleukin 10 with the Fc region [J. Immunol., 154, 5590 (1995)], a fusion protein of interleukin 2 with the Fc region [J. Immunol., 146, 915 (1991)]; a fusion protein of CD40 with the Fc region [Surgery, 132, 149 (2002)], a fusion protein of Flt-3 (fms-like tyrosine kinase) with the antibody Fc region *[*Acta. Haemato., 95, 218 (1996)], a fusion protein of OX40 with the antibody Fc region [J. Leu. Biol., 72, 522 (2002)] and the like. In addition, many fusion proteins have been reported, such as various human CD molecules [CD2, CD30 (TNFRSF8), CD95 (Fas), CD106 (VCAM-1), CD137], adhesion molecules [ALCAM (activated leukocyte cell adhesion molecule), cadherins, ICAM (intercellular adhesion molecule)-1, ICAM-2, ICAM-3], cytokine receptors (hereinafter "receptor" being referred to as "R"), (interleukin-4R, interleukin-5R, interleukin-6R, interleukin-9R, interleukin-10R, interleukin-12R, interleukin-13Rα1, interleukin-13Rα2, interleukin-15R, interleukin-21R), chemokines, cell death-inducing signal molecules [B7-H1, DR6 (Death receptor 6), PD-1 (Programmed death-1), TRAIL R1], costimulating molecules [B7-1, B7-2, B7-H2, ICOS (inducible co-stimulator)], growth factors (ErbB2, ErbB3, ErbB4, HGFR), diffentiation-inducing factors (B7-H3), activating factors (NKG2D), signal transfer molecules (gp130), or receptors or ligands of these binding proteins with the antibody Fc region.

The medicament comprising the glycoprotein such as the antibody composition can be administered as a therapeutic agent alone, but generally, it is preferred to provide it as a pharmaceutical formulation produced by an appropriate method well known in the technical field of pharmaceutics, by mixing it with at least one pharmaceutically acceptable carrier.

It is preferred to select a route of administration which is most effective in treatment. Examples include oral administration and parenteral administration, such as buccal, tracheal, rectal, subcutaneous, intramuscular or intravenous administration. In the case of a glycoprotein preparation, intravenous administration is preferred.

The dosage form includes sprays, capsules, tablets, granules, syrups, emulsions, suppositories, injections, ointments, tapes and the like.

The pharmaceutical preparation suitable for oral administration includes emulsions, syrups, capsules, tablets, powders, granules and the like.

Liquid preparations such as emulsions and syrups can be produced using, as additives, water; sugars such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like.

Capsules, tablets, powders, granules and the like can be produced using, as additives, fillers such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid ester; plasticizers such as glycerin; and the like.

The pharmaceutical preparation suitable for parenteral administration includes injections, suppositories, sprays and the like.

Injections can be prepared using a carrier such as a salt solution, a glucose solution or a mixture of both thereof Also, powdered injections can be prepared by freeze-drying the glycoprotein in the usual way and adding sodium chloride thereto.

Suppositories can be prepared using a carrier such as cacao butter, hydrogenated fat or carboxylic acid.

Sprays can be prepared using the glycoprotein as such or using it together with a carrier which does not stimulate the buccal or airway mucous membrane of the patient and can facilitate absorption of the glycoprotein by dispersing it as fine particles.

The carrier includes lactose, glycerol and the like. Depending on the properties of the glycoprotein and the carrier, it is possible to produce pharmaceutical preparations such as aerosols and dry powders. In addition, the components exemplified as additives for oral preparations can also be added to the parenteral preparations.

Although the dose or the frequency of administration varies depending on the objective therapeutic effect, administration method, treating period, age, body weight and the like, it is usually 10 µg/kg to 20 mg/kg per day and per adult.

Also, as the method for examining antitumor effect of the antibody composition against various tumor cells, *in vitro* tests include CDC activity measuring method, ADCC activity measuring method and the like, and *in vivo* tests include antitumor experiments using a tumor system in an experimental animal such as a mouse.

CDC activity and ADCC activity and antitumor experiments can be carried out in accordance with the methods described in literature [Cancer Immunology Immunotherapy, 36, 373 (1993); Cancer Research, 54, 1511 (1994)] and the like.

The present invention is described below in detail based on Examples; however, Examples are only simple illustrations, and the scope of the present invention is not limited thereto.

### Example 1

### Preparation of lectin-resistant CHO/DG44 cell by introducing GMD-targeting small interfering RNA (siRNA) expression plasmid:

### 1. Construction of GMD-targeting siRNA expression vector

### (1) Cloning of "human U6 promoter-cloning site-terminator" sequence expression cassette

A "human U6 promoter-cloning site-terminator" sequence expression cassette was obtained according to the following procedure (Fig. 1).

First, a forward primer in which recognition sequences of restriction enzymes *Hind*III and *Eco*RV were added to the 5'-terminal of a nucleotide sequence which binds to human U6 promoter sequence [GenBank Acc. No. M14486] (hereinafter referred to as "hU6p-F-*Hind*III/*Eco*RV", represented by SEQ ID NO:59) and a reverse primer in which recognition sequences of restriction enzymes *Xba*I and *Eco*RV*,* continued 6 adenines bases corresponding to a terminator sequence, and recognition sequences of restriction enzymes *Kpn*I and *Sac*I for insertion of a different synthetic oligonucleotide DNA to the 5'-terminal of a nucleotide sequence which binds to human U6 promoter sequence (hereinafter referred to as "hU6p-R-term-*Xba*I*lEco*RV"*,* represented by SEQ ID NO:60) were designed.

Then, after preparing 50 µL of a reaction solution [KOD buffer #1 (manufactured by TOYOBO), 0.1 mmol/L dNTPs, 1 mmol/L MgCl₂, 0.4 µmol/L primer hU6p-F-*Hin*dIII/*Eco*RV, and 0.4 µmol/L primer hU6p-R-term-*Xba*I/*Eco*RV] containing 40 ng of U6_FUT8_B_puro plasmid, described in Example 12 of WO03/085118, as a template, polymerase chain reaction (hereinafter referred to as "PCR") was carried out using DNA polymerase KOD polymerase (manufactured by TOYOBO). After heating at 94°C for 2 minutes, PCR was carried out by 30 cycles, one cycle consisting of reaction at 94°C for 15 seconds, reaction at 65°C for 5 seconds and reaction at 74°C for 30 seconds.

After the PCR, the reaction solution was subjected to agarose gel electrophoresis, and a specifically amplified fragment (about 300 bp) was recovered using RECOCHIP (manufactured by TAKARA BIO). The DNA fragment was dissolved in 30 µL of NEBuffer 2 (manufactured by New England Biolabs), and digested for 2 hours at 37°C with 10 units of restriction enzymes *Xba*I (manufactured by New England Biolabs) and *Hind*III (manufactured by New England Biolabs). The reaction solution was purified by phenol/chloroform extraction and ethanol precipitation, and the recovered DNA fragments digested with the restriction enzymes were dissolved in 20 µL of sterilized water.

Also, 1 µg of plasmid pBluescript II KS(+) (manufactured by STRATAGENE) was dissolved in 30 µL of NEBuffer 2 (manufactured by New England Biolabs) containing 100 µg/mL BSA (manufactured by New England Biolabs), and digested for 8 hours at 37°C with 10 units of restriction enzymes *Hind*III and *Xba*I (manufactured by New England Biolabs). After the digestion reaction, 22µL of sterilized water, 6 µL of 10×alkaline phosphatase buffer, and 1 unit of alkaline phosphatase *E. coli* C75 (manufactured by TAKARA BIO) were added to the reaction solution for carrying out dephosphorylation reaction at 37°C for 1 hour. The reaction solution was subjected to agarose gel electrophoresis, and *Hind*III-*Xba*I fragment (about 2.9 kb) derived from plasmid pBluescript II KS(+) was recovered using RECOCHIP (manufactured by TAKARA BIO).

Then, 8 µL of the DNA fragment (about 300 bp) and 2 µL of *Hind*III-*Xba*I fragment (about 2.9 kb) derived from plasmid pBluescript II KS(+) obtained above were mixed with 10 µL of Ligation High (manufactured by TOYOBO), and were allowed to react for 2 hours at 16°C. *E. coli* DH5α (manufactured by TOYOBO) was transformed with the reaction solution, and a plasmid was isolated from the resulting ampicillin-resistant clones using QIAprep spin Mini prep Kit (manufactured by Qiagen). The plasmid is hereinafter referred to as "pBS-U6term".

### (2) Ligation of "human U6 promoter-cloning site-terminator" sequence expression cassette to pPUR

The "human U6 promoter-cloning site-terminator" sequence expression cassette in the plasmid pBS-U6term obtained in the above (1) was excised, and ligated to expression vector pPUR (manufactured by CLONTECH) according to the following procedure (Fig. 2).

First, 1 µg of plasmid pBS-U6term prepared in the above (1) above was dissolved in 20 µL of NEBuffer 2 (manufactured by New England Biolabs) containing 100µg/mL BSA (manufactured by New England Biolabs), and digested with 10 units of a restriction enzyme *Eco*RV (manufactured by New England Biolabs) for 2 hours at 37°C. The reaction solution was subjected to agarose gel electrophoresis, and a DNA fragment containing "human U6 promoter-cloning site-terminator" sequence expression cassette (about 350 bp) was recovered using RECOCHIP (manufactured by TAKARA BIO).

Also, 6 µg of plasmid pPUR (manufactured by CLONTECH) was dissolved in 20 µL of NEBuffer 2 (manufactured by New England Biolabs), and digested with 10 units of a restriction enzyme *Pvu*II (manufactured by New England Biolabs) for 2 hours at 37°C. After the reaction, 5µL of sterilized water, 3 µL of 10×alkaline phosphatase buffer, and 1 unit of alkaline phosphatase *E. coli* C75 (manufactured by TAKARA BIO) were added to the reaction solution for carrying out dephosphorylation reaction at 37°C for 1 hour. The reaction solution was subjected to agarose gel electrophoresis, and a *Pvu*II fragment (about 4.3 kb) derived from plasmid pPUR was recovered using RECOCHIP (manufactured by TAKARA BIO).

Then, 8 µL of the DNA fragment (about 350 bp) containing human U6 promoter-cloning site-terminator sequence expression cassette and 2 µL of the *Pvu*II fragment (about 4.3 kb) derived from plasmid pPUR obtained above were mixed with 10 µL of Ligation High (manufactured by TOYOBO) and allowed to react for 3 hours at 16°C. *E. coli* DH5α (manufactured by TOYOBO) was transformed with the reaction solution, and plasmid DNAs were isolated from the resulting ampicillin-resistant clones using QIAprep spin Mini prep Kit (manufactured by Qiagen). Approximately 0.5 µg of the plasmid DNA was dissolved in 10 µL of NEBuffer 2 (manufactured by New England Biolabs), and digested with 10 units of restriction enzymes *Sac*I and *Hind*III (manufactured by New England Biolabs) for 2 hours at 37°C. The reaction solution was subjected to agarose gel electrophoresis to confirm the presence and inserted direction of the inserted fragment of interest. In addition, nucleotide sequences of the DNA inserted into each plasmid were determined using DNA sequencer 377 (manufactured by Perkin Elmer) and BigDye Terminator v3.0 Cycle Sequencing Kit (manufactured by Applied Biosystems) according to the manufacturer's instruction. pPUR *Pvu*II-seq-F (SEQ ID NO:61) and pPUR *Pvu*II-seq-R (SEQ ID NO:62) were used as primers for sequence analysis to confirm that the inserted DNA fragments had the identical human U6 promoter sequence to GenBank Acc. No. M14486 and that primer site sequences used to amplify the "human U6 promoter-cloning site-terminator" sequence expression cassette and ligation site sequences were correct, and a plasmid in which the inserted hU6 promoters was in the same direction as the puromycin-resistant gene expression unit selected from the resulting plasmids. The plasmid is hereinafter referred to as "pPUR-U6term".

### (3) Selection of target sequence and design of synthetic oligo DNA

Synthetic oligo DNAs which form a double-stranded DNA cassette containing siRNA target sequence against the GMD gene of Chinese hamster ovary CHO/DG44 cell were prepared as follows.

First, seven target sequences which satisfied the conditions described below were selected from Chinese hamster-derived GMD cDNA sequence (GenBank Acc. No. AF525364; SEQ ID NO: 8). Selected target sequences are represented by SEQ ID NOs: 36 to 42.
- Condition 1:: A consensus sequence consisting of "NAR(N17)YNN" is included, wherein N represents A, G, U or C; R represents A or G, and Y represents U or C, respectively.
- Condition 2:: Condition 1 is satisfied, and a sequence of continued 3 or more same bases is not included.
- Condition 3:: Conditions 1 and 2 are satisfied, and GC content is more preferably 35 to 45%, and preferably 45 to 55%.
- Condition 4:: A sequence of 29 bases in which 6 bases are added to the 5'- or 3'-terminal of 23 bases which satisfy the conditions 1-3 satisfies the condition 2.
- Condition 5:: The condition 4 is satisfied, and GC content is more preferably 35 to 45%, and preferably 45 to 55%.

In addition, double-stranded DNA cassettes were designed for the selected target sequences according to the following procedure. Sequentially from 5'-terminal, the double-stranded DNA cassettes have 3'-cohesive end generated by digestion with a restriction enzyme *Sac*I*,* sense DNA that corresponds to the SEQ ID NOs:36 to 42, loop sequence of human miR-23-precursor-19 micro RNA consisting 10 bases (GenBank Acc. No. AF480558), an antisense DNA complementary to the DNA sequences of SEQ ID NOs: 36 to 42, and 3'-cohesive end generated by a restriction enzyme *Kpn*I*.* The 5'-terminal of the double-stranded DNA was phosphorylated.

The nucleotide sequence of the sense strand of the synthetic oligo DNA (hereinafter referred to as "GMD-dsRNA-A-F") which was designed based on the target sequence represented by SEQ ID NO:36 is represented by SEQ ID NO:43; the nucleotide sequence of the antisense strand (hereinafter referred to as "GMD-dsRNA-A-R") is represented by SEQ ID NO:44; the nucleotide sequence of the sense strand sequence of the synthetic oligo DNA (hereinafter referred to as "GMD-dsRNA-B-F") which was designed based on the target sequence represented by SEQ ID NO:37 is represented by SEQ ID NO:45; the nucleotide sequence of the antisense strand (hereinafter referred to as "GMD-dsRNA-B-R") is represented by SEQ ID NO:46; the nucleotide sequence of the sense strand of the synthetic oligo DNA (hereinafter referred to as "GMD-dsRNA-C-F") which was designed based on the target sequence represented by SEQ ID NO:38 is represented by SEQ ID NO:47; the nucleotide sequence of the antisense strand (hereinafter referred to as "GMD-dsRNA-C-R") is represented by SEQ ID NO:48; the nucleotide sequence of the sense strand of the synthetic oligo DNA (hereinafter referred to as "GMD-dsRNA-D-F") which was designed based on the target sequence represented by SEQ ID NO:39 is represented by SEQ ID NO:49; the nucleotide sequence of the antisense strand (hereinafter referred to as "GMD-dsRNA-D-R") is represented by SEQ ID NO:50; the nucleotide sequence of the sense strand of the synthetic oligo DNA (hereinafter referred to as "GMD-dsRNA-E-F") which was designed based on the target sequence represented by SEQ ID NO:40 is represented by SEQ ID NO:51; the nucleotide sequence of the antisense strand (hereinafter referred to as "GMD-dsRNA-E-R") is represented by SEQ ID NO:52; the nucleotide sequence of the sense strand of the synthetic oligo DNA (hereinafter referred to as "GMD-dsRNA-F-F") which was designed based on the target sequence represented by SEQ ID NO:41 is represented by SEQ ID NO:53; the nucleotide sequence of the antisense strand (hereinafter referred to as "GMD-dsRNA-F-R") in SEQ ID NO:54; the nucleotide sequence of the sense strand of the synthetic oligo DNA (hereinafter referred to as "GMD-dsRNA-F-F") which was designed based on the target sequence represented by SEQ ID NO:42 is represented by SEQ ID NO:55; and the nucleotide sequence of the antisense strand (hereinafter referred to as "GMD-dsRNA-F-R") is represented by SEQ ID NO:56, respectively. Designed synthetic oligo DNAs were synthesized according to the conventional procedure (Molecular Cloning, Second Edition).

### (4) Insertion of synthetic oligo DNA into plasmid pPUR-U6term

The synthetic oligo DNAs synthesized in the above (3) were inserted into the cloning site of pPUR-U6term obtained in the above (2) (Fig. 3).

First, synthetic oligo DNAs were annealed according to the following procedure. In 10 µL of an annealing buffer [10 mmol/L Tris (pH 7.5)-50 mmol/L NaCl-1 mmol/L EDTA], 200 pmol each of sense and antisense strands of the synthetic oligo DNAs were dissolved, followed by boiling for 2 minutes. Then, they were cooled gradually to room temperature over approximately 3 hours. Subsequently, annealed synthetic oligo DNAs were diluted 15-fold with sterilized water.

Also, 3 µg of plasmid pPUR-U6term was dissolved in 40 µL of NEBuffer 1 (manufactured by New England Biolabs) containing 100 µg/mL BSA (manufactured by New England Biolabs), and digested with 20 units of restriction enzymes *Kpn*I and *Sac*I (manufactured by New England Biolabs) for 4 hours at 37°C. After the digestion reaction, 12 µL of sterilized water, 6 µL of 10×alkaline phosphatase buffer, and 1 unit of alkaline phosphatase *E. coli* C75 (manufactured by TAKARA BIO) were added to the reaction solution for carrying out dephosphorylation reaction at 37°C for 1 hour. The reaction solution was subjected to agarose gel electrophoresis, and a *Kpn*I*-Sac*I fragment (about 4.5 kb) derived from plasmid pPUR-U6term was recovered using RECOCHIP (manufactured by TAKARA BIO).

Then, 1 µL of the double-stranded synthetic oligo solution and 1 µL of the *Kpn*I*-Sac*I fragment derived from plasmid pPUR-U6term obtained above were mixed with 8 µL of sterilized water and 10 µL of Ligation High (manufactured by TOYOBO), and allowed to react overnight at 16°C. *E. coli* DH5α (manufactured by TOYOBO) was transformed with the reaction solution, and plasmid DNAs were isolated from the resulting ampicillin-resistant clones using QIAprep spin Mini prep Kit (manufactured by Qiagen).

The nucleotide sequences of the DNA inserted into each plasmid were determined using DNA sequencer 377 (manufactured by Perkin Elmer) and BigDye Terminator v3.0 Cycle Sequencing Kit (manufactured by Applied Biosystems) according to the manufacturer's instruction. A plasmid DNA which was boiled for approximately 1 minute and cooled rapidly was used as a template, and pPUR *Pvu*II-seq-F (SEQ ID NO:61), hU6p Tsp45I/seq-F (SEQ ID NO:63) and pPUR *Pvu*II-seq-R (SEQ ID NO:62) were used as primers for sequence analysis, and the inserted synthetic oligo DNA sequences and ligation sites were confirmed. Hereinafter, a plasmid into which a double-stranded DNA consisting of synthetic oligo DNA GMD-dsRNA-A-F and GMD-dsRNA-A-R are introduced is referred to as "pPUR/GMDshA"; a plasmid into which a double-stranded DNA consisting of synthetic oligo DNA GMD-dsRNA-B-F and GMD-dsRNA-B-R are introduced is referred to as "pPUR/GMDshB"; a plasmid into which double-stranded DNA consisting of synthetic oligo DNA GMD-dsRNA-C-F and GMD-dsRNA-C-R are introduced is referred to as "pPUR/GMDshC"; a plasmid into which a double-stranded DNA consisting of synthetic oligo DNA GMD-dsRNA-D-F and GMD-dsRNA-D-R are introduced is referred to as "pPUR/GMDshD"; a plasmid into which a double-stranded DNA consisting of synthetic oligo DNA GMD-dsRNA-E-F and GMD-dsRNA-E-R are introduced is referred to as "pPUR/GMDshE"; a plasmid into which a double-stranded DNA consisting of synthetic oligo DNA GMD-dsRNA-F-F and GMD-dsRNA-F-R are introduced is referred to as "pPUR/GMDshF"; and a plasmid into which a double-stranded DNA consisting of synthetic oligo DNA GMD-dsRNA-G-F and GMD-dsRNA-G-R are introduced is referred to as "pPUR/GMDshG".

### 2. Obtaining and culture of lectin-resistant clones by introducing GMD-targeting siRNA expression vector

### (1) Obtaining of lectin-resistant clones by introducing GMD-targeting siRNA expression vector

Each of plasmids pPUR/GMDshA, pPUR/GMDshB, pPUR/GMDshC, pPUR/GMDshD, pPUR/GMDshE, pPUR/GMDshF and pPUR/GMDshG constructed in the item 1 of this Example was introduced into CHO/DG44 cell-derived anti-CCR4 chimeric antibody-producing clone, clone 35-02-12 (hereinafter referred to as "clone 32-05-12"), which was obtained by the same method as described in Reference Example 1 of WO03/85118, and clones resistant to a lectin which recognizes a sugar chain structure in which 1-position of fucose is bound to 6-position of N-acetylglucosamine in the reducing end through α-bond in a N-glycoside-linked sugar chain, a *Lens culinaris agglutinin* (hereinafter referred to as "LCA") were obtained as described below.

Transfection of various siRNA expression vector plasmids into clone 32-05-12 was carried out by electroporation [Cytotechnology, 3, 133 (1990)] according to the following procedure. First, 10 µg of each of siRNA expression vector plasmids was dissolved in 30 µL of NEBuffer 4 (manufactured by New England Biolabs), and digested to be linearized with 10 units of a restriction enzyme *Fsp*I (manufactured by New England Biolabs) overnight at 37°C. After the linearized plasmid was confirmed by agarose gel electrophoresis using a part of the reaction solution, the remaining reaction solution was purified by phenol/chloroform extraction and ethanol precipitation, and the recovered linearized plasmid was dissolved in 10 µL, of sterilized water.

Also, clone 32-05-12 was suspended in a K-PBS buffer (137 mmol/L KCl, 2.7 mmol/L NaCl, 8.1 mmol/L Na₂HPO₄, 1.5 mmol/KH₂PO₄, and 4.0 mmol/L MgCl₂) at 8×10⁶ cells/mL. After 200 µL of the cell suspension (1.6×10⁶) was mixed with 10 µL of the above linearized plasmid solution, all of the cell/DNA mixture was transferred to Gene Pulser Cuvette (Electrode interval: 2 mm) (manufactured by BIO-RAD), and transfection was carried out under conditions of 350V pulse voltage and 250 µF capacitance using a cell fusion device Gene Pulser (manufactured by BIO-RAD). After the transfection, the cell suspension was suspended in a basal medium [Iscove's Modified Dulbecco's Medium (hereinafter referred to as "IMDM", manufactured by Invitrogen) containing 10% fetal bovine dialyzed serum (manufactured by Invitrogen), 50 µg/mL gentamicin (manufactured by Nacalai Tesque), and 500 nmol/L MTX (manufactured by SIGMA)], and inoculated into four 10 cm-dishes for adherent cell culture (manufactured by Falcon). After culturing under conditions of 5% CO₂ and 37°C for 24 hours, the culture supernatant was removed, and a basal medium supplemented with 12 µg/mL puromycin (manufactured by SIGMA) was added thereto, followed by culturing for further 7 days. Subsequently, the culture supernatant was removed from one of the dishes, a basal medium containing 12 µg/mL puromycin (manufactured by SIGMA) was added thereto, followed by culturing for further 6 to 8 days, and appeared puromycin-resistant colonies were counted. Also, the culture supernatant was removed from the remaining dishes, and a basal medium supplemented with 12 µg/mL puromycin (manufactured by SIGMA) and 0.5 mg/mL LCA (manufactured by VECTOR) was added thereto, followed by culturing for further 7 days. As a result, lectin-resistant clones were obtained when pPUR/GMDshB was introduced.

### (2) Expansion culture of lectin-resistant clones

Lectin-resistant clones obtained in the above (1) by introducing pPUR/GMDshB were expansion cultured according to the following procedure.

First, the number of appeared colonies in each dish was counted. Then, lectin-resistant colonies were scraped and sucked up with a pipetteman (manufactured by GILSON) under observation with a stereoscopic microscope, and collected onto a U-shaped-bottom 96-well plate for adherent cells (manufactured by ASAHI TECHNOGLASS). After trypsin treatment, each clone was dispensed onto a flat-bottom 96-well plate for adherent cells (manufactured by Greiner), and cultured in a basal medium containing 12 µg/mL puromycin (manufactured by SIGMA) under conditions of 5% CO₂ and 37°C for a week. After the culture, 10 clones were expansion cultured in a basal medium containing 12 µg/mL puromycin (manufactured by SIGMA). Clones used in the expansion culture were respectively named "12-GMDB-1", "12-GMDB-2", "12-GMDB-3", "12-GMDB-4", "12-GMDB-5", "12-GMDB-6", "12-GMDB-7", "12-GMDB-8", "12-GMDB-9" and "12-GMDB-10", and used in the analysis described in the item 3 below. Also, clone 12-GMDB-5 has been deposited to International Patent Organism Depositary, National Institute of Advanced Industrial Science and Technology (Tsukuba Central 6, 1, Higashi 1-chome, Tsukuba-shi, Ibaraki-ken, Japan) as FERM BP-10051 on July 1, 2004.

### 3. Determination of the amount of GMD mRNA in lectin-resistant clone into which GMD-targeting siRNA expression vector was introduced

### (1) Preparation of total RNA

A total RNA was prepared from clone 32-05-12 and lectin-resistant clones which were obtained in the item 2 of this Example according to the following procedure. Clone 32-05-12 was suspended in a basal medium and lectin-resistant clones were suspended in a basal medium supplemented with 12 µg/mL puromycin (manufactured by SIGMA) at a density of 3×10⁵ cells/mL, and they inoculated at 4 ml into 6 cm-dishes for adherent cells (manufactured by Falcon). Cells were statically cultured under conditions of 5% CO₂ and 37°C for 3 days, and each cell suspension was collected after trypsin treatment, and centrifuged at 1,000 rpm and 4°C for 5 minutes to remove the supernatant. After the cells were suspended in Dulbecco's PBS buffer (manufactured by Invitrogen) and centrifuged again at 1,000 rpm and 4°C for 5 minutes to remove the supernatant, a total RNA was extracted using RNeasy (manufactured by QIAGEN). The method was carried out according to the manufacturer's instruction, and the prepared total RNA was dissolved in 40 µL of sterilized water.

### (2) Synthesis of single-stranded cDNA

A single-stranded cDNA was synthesized from 3 µg of each of the total RNA obtained in the item (1) by reverse transcription reaction with oligo (dT) primer in 20 µL reaction system using SUPERSCRIPT^{™} Preamplification System for First Strand cDNA Synthesis (manufactured by Invitrogen) according to the manufacturer's instruction. Subsequently, the reaction solution was treated with RNase and the final reaction volume was adjusted to 40 µL. In addition, each of the reaction solutions was diluted 50-fold with sterilized water, and used for determination of the amount of gene transcription described below.

### (3) Determination of the amount of gene transcription by SYBR-PCR

The amount of mRNA transcribed from GMD gene and β-actin gene were determined using For Real Time PCR TaKaRa Ex Taq R-PCR Version (manufactured by TAKARA BIO) according to the following procedure.

In this connection, plasmid pAGE249GMD containing CHO cell-derived GMD cDNA described in Example 15 of WO02/31140, each diluted at concentration of 0.0512 fg/µL, 0.256 fg/µL, 1.28 fg/µL, 6.4 fg/µL, 32 fg/µL and 160 fg/µL were used as internal controls of GMD determination; β-actin standard plasmid described in Example 9 of WO02/31140, each diluted at concentration of 1.28 fg/µL, 6.4 fg/µL, 32 fg/µL, 160 fg/µL, 800 fg/µL, and 4,000 fg/µL were used as internal controls of β-actin determination. As PCR primers, forward and reverse primers represented by SEQ ID NOs: 64 and 65, respectively, were used to amplify GMD, and forward and reverse primers represented by SEQ ID NOs: 66 and 67, respectively, were used to amplify β-actin gene.

Then, 20 µL of reaction solution [R-PCR buffer (manufactured by TAKARA BIO), 2.5 mmol/L Mg²⁺ Solution for R-PCR (manufactured by TAKARA BIO), 0.3 mmol/L dNTP mixture (manufactured by TAKARA BIO), 0.3 µmol/L forward primer, 0.3 µmol/L reverse primer, 2×10⁻⁵ diluted SYBR GreenI, 1 unit TaKaRa Ex Taq R-PCR] containing 5 µL of the single-stranded cDNA prepared in the item (2) or each concentration of internal control plasmid solution was prepared using For Real Time PCR TaKaRa Ex Taq R-PCR Version (manufactured by TAKARA BIO). The prepared reaction solutions were dispensed into each well of a 96-well Polypropylene PCR Plate (manufactured by Falcon), and the plate was sealed with Plate Sealer (manufactured by Edge Biosystems). ABI PRISM 7700 Sequence Detection System was used for PCR and analysis, and the amount of GMD mRNA and the amount of β-actin mRNA were determined according to the manufacturer's instruction.

A calibration curve was made based upon the measurements with the internal control plasmid, and the amount of GMD mRNA and the amount of β-actin mRNA were converted into numerical terms. In addition, assuming that the amount of mRNA transcribed from β-actin gene are uniform among the clones, the relative amount of GMD mRNA to the amount of β-actin mRNA were calculated and compared, and the results are shown in Fig. 4.

It was shown that the amount of GMD mRNA in all the clones obtained by introducing the GMD-targeting siRNA expression plasmid were reduced to 8% to 50% in that of the parent cell.

### Example 2

### Production of antibody composition using lectin-resistant CHO/DG44 cell into which GMD-targeting siRNA expression plasmid was introduced:

### 1. Obtaining of antibody compositions produced by lectin-resistant clone into which GMD-targeting siRNA expression plasmid was introduced

Anti-CCR4 chimeric antibodies produced by lectin-resistant clone 12-GMDB-2 and clone 12-GMDB-5 into which the GMD-targeting siRNA expression plasmid was introduced obtained in Example 1 were obtained according to the following procedure.

Clone 32-05-12 was suspended in a basal medium and clones 12-GMDB-2 and 12-GMDB-5 were suspended in a basal medium supplemented with 12 µg/mL puromycin (manufactured by SIGMA) at a density of 3×10⁵ cells/mL, and they were inoculated at 15 mL into a T75 flask for adherent cells (manufactured by Greiner). After culturing under conditions of 5% CO₂ and 37°C for 6 days, the culture supernatant was removed, and after washing twice with 10 mL of Dulbecco's PBS (manufactured by Invitrogen), 20 mL of EXCELL301 medium (manufactured by JRH Bioscience) was added. After culturing under conditions of 5% CO₂ and 37°C for 7 days, the culture supernatant was recovered, and anti-CCR4 chimeric antibodies were purified using a MabSelect column (manufactured by Amersham Bioscience) according to the manufacturer's instruction. After exchange with 10 mmol/L KH₂PO₄ buffer using Econo-Pac 10DG (manufactured by Bio Rad), anti-CCR4 chimeric antibodies purified from culture supernatant of various clones were subjected to sterile filtration by using Millex GV (manufactured by MILLIPORE) of 0.22 mm pore size.

### 2. Composition analysis of monosaccharide of antibody compositions produced by lectin-resistant clone into which GMD-targeting siRNA expression plasmid was introduced

Composition analysis of monosaccharide was carried out on the anti-CCR4 chimeric antibodies obtained in the item 1 of this Example according to the known method [Journal of Liquid Chromatography, 6, 1577 (1983)].

**Table 1**

| Clone | Ratio of sugar chains in which fucose is not bound |
|---|---|
| 32-05-12 | 3% |
| 12-GMDB-2 | 78% |
| 12-GMDB-5 | 79% |

The composition ratio of complex type sugar chains in which fucose is not bound among the total complex sugar chains calculated from the composition of monosaccharide ratio of each antibody is shown in Table 1. Among antibody compositions produced from parent clone 32-05-12, which was used to introduce the GMD-targeting siRNA expression vector, the ratio of sugar chains in which fucose is not bound was 3%, while those of 12-GMDB-2 and 12-GMDB-5 lectin-resistant clones into which siRNA was introduced were 78% and 79%, respectively, demonstrating that the ratios of sugar chains in which fucose is not bound are greatly increased in comparison with the parent cell.

The above results demonstrate that the α1,6-fucose content in the antibodies produced by the host cells can be controlled by the introduction of GMD-targeting siRNA.

### Example 3

### Serum-free fed-batch culture of lectin-resistant CHO/DG44 cell into which GMD-targeting siRNA expression plasmid was introduced:

### 1. Adaptation of lectin-resistant CHO/DG44 cell into which GMD-targeting siRNA expression plasmid was introduced to serum-free medium

The parent clone 32-05-12 before vector introduction, and lectin-resistant clones 12-GMDB-2 and 12-GMDB-5 into which the GMD-targeting siRNA expression plasmid were introduced obtained in Example 1 were adapted to a serum-free medium according to the following procedure.

Clone 32-05-12 was suspended in a basal medium and clones 12-GMDB-2 and 12-GMDB-5 were suspended in a basal medium supplemented with 12 µg/mL puromycin (manufactured by SIGMA) at a density of 3×10⁵ cells/mL, and each was inoculated at 5 ml into a T75 flask for adherent cells (manufactured by Greiner). After culturing under conditions of 5% CO₂ and 37°C for 3 days, cell suspension was obtained by trypsin treatment, and cells were recovered by centrifugation at 1,000 rpm for 5 minutes. Clone 32-05-12 was suspended in EX-CELL302 medium (manufactured by JRH) containing 500 nmol/L MTX (manufactured by SIGMA), 6 mmol/L L-glutamine (manufactured by Invitrogen), 50 µg/mL gentamicin (manufactured by Nacalai Tesque) and 100 nmol/L 3,3,5-triiodo-L-thyronine (manufactured by SIGMA) (hereinafter referred to as "serum-free medium") and clones 12-GMDB-2 and 12-GMDB-5 were suspended in the serum-free medium supplemented with 12 µg/mL puromycin (manufactured by SIGMA) at a density of 5×10⁵ cells/mL, and 15 mL of the cell suspension was inoculated into a 125 mL conical flask (manufactured by Corning). After ventilating the flask with 5% CO₂ (at least 4-fold volume of culture vessel) and sealing the flask, suspension rotation culture was carried out at 90-100 rpm and 35°C. Passage was repeated at 3 to 4 day intervals, and finally, clones which could grow in the serum-free medium were obtained. Hereinafter, clones 32-05-12, 12-GMDB-2 and 12-GMDB-5 adapted to the serum-free medium were referred to as "32-05-12AF", and 12-GMDB-2 and 12-GMDB-5 adapted to the serum-free medium were referred to as "12-GMDB-2AF" and "12-GMDB-5AF", respectively.

### 2. Serum-free fed-batch culture of lectin-resistant CHO/DG44 cell into which GMD-targeting siRNA expression plasmid was introduced and adapted to serum-free medium

### (1) Serum-free fed-batch culture in conical flask

Serum-free fed-batch culture was carried out using clones 32-05-12AF, 12-GMDB-2AF, and 12-GMDB-5AF adapted to the serum-free medium in the item 1 of this Example according to the following procedure.

EX-CELL302 medium (manufactured by JRH) containing 500 nmol/L MTX (manufactured by SIGMA), 6 mmol/L L-glutamine (manufactured by Invitrogen), 100 nmol/L 3,3,5-triiodo-L-thyronine (manufactured by SIGMA), 0.1% Pluronic F-68 (manufactured by Invitrogen), and 5,000 mg/L D(+)-glucose (manufactured by Nacalai Tesque) (hereinafter referred to as "serum-free fed-batch medium") was used for fed-batch culture, and a medium containing amino acids prepared at higher concentrations than usual addition (0.177 g/L L-alanine, 0.593 g/L L-arginine monohydrochloride, 0.177 g/L L-asparagine monohydrate, 0.212 g/L L-asparatic acid, 0.646 g/L L-cystine dihydrochloride, 0.530 g/L L-glutamic acid, 5.84 g/L L-glutamine, 0.212 g/L glycine, 0.297 g/L L-histidine monohydrochloride dihydrate, 0.742 g/L L-isoleucine, 0.742 g/L L-leucine, 1.031 g/L L-lysine monohydrochloride, 0.212 g/L L-methionine, 0.466 g/L L-phenylalanine, 0.283 g/L L-proline, 0.297 g/L L-serine, 0.671 g/L L-threonine, 0.113 g/L L-tryptophan, 0.73 5 g/L L-tyrosine disodium dihydrate, and 0.664 g/L L-valine), vitamins (0.0918 mg/L d-biotin, 0.0283 g/L D-calcium pantothenate, 0.0283 g/L choline chloride, 0.0283 g/L folic acid, 0.0509 g/L myo-inositol, 0.0283 g/L niacinamide, 0.0283 g/L pyridoxal hydrochloride, 0.00283 g/L riboflavin, 0.0283 g/L thiamine hydrochloride, and 0.0918 mg/L cyanocobalamin) and 0.314 g/L insulin (hereinafter referred to as "feed medium") was used as a medium for feeding.

Clones 32-05-12AF, 12-GMDB-2AF and 12-GMDB-SAF were suspended in the serum-free fed-batch medium at a density of 3×10⁵ cells/mL, and 40 mL each of the cell suspension was inoculated into a 250 mL conical flask (manufactured by Corning). After ventilating the flask with 5% CO₂ (at least 4-fold volume of culture vessel), and sealing the flask, suspension rotation culture was carried out at 90 to 100 rpm and 35°C. On days 3, 6, 9 and 12 after starting the culture, 3.3 mL of feed medium was added to supplement the consumption of amino acids and the like, and 20% (w/v) glucose solution was added at a final concentration of 5,000 mg/L to adjust the glucose concentration. On days 0, 3, 6, 9, 12 and 14 after starting the culture, 2-4 mL each of the culture was collected, and used for the analyses described in the items (2) to (4). In addition, the fed-batch culture was finished on day 14 after starting the culture, and the whole culture was collected and used for the analysis described in the item (4).

### (2) Measurement of the viable cell number

Viable cell number and viability of the culture in the item (1), collected on days 0, 3, 6, 9, 12 and 14 after starting the culture, were measured by trypan blue staining. Viable cell number at each point of time after starting the culture of clones 32-05-12AF, 12-GMDB-2AF and 12-GMDB-5AF are shown in Fig. 5. Clone 12-GMDB-2 grew slower in comparison with clone 32-05-12AF, and retained a high viability even on day 14. Also, the viable cell number of clone 12-GMDB-5AF at each point of time after starting the culture was similar to those of clone 32-05-12. Therefore, it was demonstrated that the target sequences of GMD-targeting siRNA had no significant effects on the cell growth.

### (3) Determination of antibody concentration

Antibody concentrations contained in the culture supernatants on days 0, 3, 6, 9, 12 and 14 after starting the culture obtained in the item (1) were determined according to the following procedure.

In 750 mL of Dulbecco's PBS (manufactured by Invitrogen), 1 mL of anti-human IgG (H+L) antibody (manufactured by American Qualex) was dissolved, and the mixture was dispensed at 50 µl onto each well of an ELISA plate. After leaving overnight at 4°C, the solution was removed, and 100 µL of PBS containing 1% BSA (bovine serum albumin) (hereinafter referred to as "BSA-PBS") was added to each well, and the plate was allowed to stand for approximately 1 hour at room temperature, and stored at -20°C. On measuring the amount of antibody, the plate was thawed at room temperature, and after removing the BSA-PBS in wells, 50 µL of the culture supernatant diluted with BSA-PBS was added to each well. After the plate was allowed to stand for 1 to 2 hours at room temperature, the wells were washed with PBS containing 0.05% Tween20^{™} (hereinafter referred to as "Tween-PBS"). After removing the washing liquid, 50 µL of goat anti-human IgG (H&L)-HRP (manufactured by American Qualex) diluted 2000-fold with BSA-PBS, was added to each well as a second antibody. After the plate was allowed to stand for 1 to 2 hours at room temperature, wells were washed with Tween-PBS and then with resin water. After washing, 50 µL of an ABTS substrate solution supplemented with 0.1% H₂O₂ [0.55 g of 2,2'-azinobis(3-ethylbenzothiazoline-6-sulfonic acid)ammonium was dissolved in 1 L of 0.1 mol/L citrate buffer (pH 4.2), and supplemented with oxygenated water at 1 µL/mL before use] was added to each well for color development. After the plate was allowed to stand at room temperature for approximately 15 minutes, when appropriate color developed, 50 µL of 5% SDS solution was added to each well to stop the reaction. Absorption at 490 nm was measured with that of at 415 nm as reference using a microplate reader. Antibody concentrations of each diluted sample were calculated using the linear area of the sigmoid curve of the calibration curve prepared with a standard of purified antibody preparation. Each antibody concentration of culture supernatants was calculated by multiplying the antibody concentrations of the obtained diluted samples by the dilution rate. Determination results of the antibody concentrations in the culture supernatants at each point of time after starting culture of clones, 32-05-12AF, 12-GMDB-2AF and 12-GMDB-5AF, are shown in Fig. 6. The antibody composition concentrations in the culture supernatants after start of culture were similar among clones 32-05-12AF, 12-GMDB-2AF and 12-GMDB-SAF. Therefore, it was demonstrated that the GMD-targeting siRNA sequence does not affect cellular antibody productivity.

### (4) Sugar chain structural analysis of antibody compositions

Anti-CCR4 chimeric antibody compositions were purified from culture supernatants of the serum-free fed-batch culture on day 14 of clone 32-05-12AF and those of the serum-free fed-batch culture on days 6, 12 and 14 of clones 12-GMDB-2AF and 12-GMDB-5AF obtained in the item (1) by using a MabSelect column (manufactured by Amersham Biosciences) according to the manufacturer's instruction. After exchange with 10 mmol/L KH₂PO₄ buffer using Econo-Pac 10DG (manufactured by Bio Rad), anti-CCR4 chimeric antibody compositions purified from culture supernatants of various clones were subjected to sterile filtration by using Millex GV (manufactured by MILLIPORE) of 0.22 mm pore size. Composition analysis of monosaccharide was carried out with the anti-CCR4 chimeric antibody compositions obtained from culture supernatants of each clones adapted to the serum-free medium according to the known method [Journal of Liquid Chromatography, 6, 1577 (1983)]. The ratio of sugar chains in which fucose is not bound among the total complex sugar chains (hereinafter referred to as "fucose(-)%") calculated from composition ratio of monosaccharide of each antibody compositions is shown in Table 2.

**Table 2**

| Clone | Culturing days | Fucose(-)% |
|---|---|---|
| 32-05-12AF | 14 | 7% |
| 12-GMDB-2AF | 6 | 88% |
| | 12 | 87% |
| | 14 | 85% |
| 12-GMDB-5AF | 6 | 84% |
| | 12 | 82% |
| | 14 | 81% |

On day 14 when the culture was finished, fucose(-)% of antibody compositions produced by clone 32-05-12AF was 7%, while those of lectin-resistant clones 12-GMDB-2AF and 12-GMDB-5AF into which the GMD-targeting siRNA expression plasmid was introduced were 81 to 85%. Therefore, it was demonstrated that, also in the serum-free fed-batch medium, lectin-resistant clones into which the GMD-targeting siRNA expression plasmid was introduced could produce antibody compositions having higher fucose(-)% than the parent clone. In addition, fucose(-)% of antibody compositions produced by clones 12-GMDB-2AF and 12-GMDB-5AF showed roughly constant values on days 6, 12, and 14. Therefore, it was demonstrated that inhibitory effects on α1,6-fucose addition to complex sugar chains of antibody compositions by introduction of the GMD-targeting siRNA expression plasmid were stable in the serum-free fed-batch culture.

### Example 4

### Screening of siRNA target sequence effective for obtaining lectin-resistant clone using α1,6-fucosyltransferase (FUT8)-targeting siRNA expression vector library and construction of effective FUT8-targeting siRNA expression vector:

### 1. Construction of α1,6-fucosyltransferase (FUT8)-targeting siRNA expression vector library (FUT8shRNAlib/pPUR)

### (1) Obtaining of CHO cell-derived α1,6-fucosyltransferase (FUT8) cDNA sequence

A cDNA encoding α1,6-fucosyltransferase (FUT8) was cloned from a single-stranded cDNA prepared from Chinese hamster ovary-derived CHO/DG44 cell according to the procedure described in WO00/61739.

First, 5'-untranslated region-specific forward primer (SEQ ID NO: 68) and 3'-untranslated region-specific reverse primer (SEQ ID NO: 69) were designed based upon the nucleotide sequence of mouse FUT8 cDNA (GenBank Acc. No. AB025198).

Then, after preparing 25 µL of a reaction solution [ExTaq buffer (manufactured by TaKaRa), 0.2 mmol/L dNTPs, 4% DMSO, and 0.5 µmol/L specific primers described above (SEQ ID NOs: 68 and 69)] containing 1 µL of CHO/DG44 cell-derived single-stranded cDNA, PCR was carried out using DNA polymerase ExTaq (manufactured by TaKaRa). After heating at 94°C for 1 minute, the PCR was carried out by 30 cycles, one cycle consisting of reaction at 94°C for 30 seconds, reaction at 55°C for 30 seconds and reaction at 72°C for 2 minutes, followed by reaction at 72°C for 10 minutes.

After the PCR, the reaction solution was subjected to 0.8% agarose gel electrophoresis, and a specifically amplified fragment (about 2 kb) was recovered. The DNA fragment was ligated to plasmid pCR2.1 using TOPO TA cloning Kit (manufactured by Invitrogen) according to the manufacturer's instruction, and *E. coli* DH5α was transformed with the ligation solution. Among the resulting kanamycin-resistant colonies, plasmid DNAs were isolated from 8 clones with which cDNA was inserted according to the known method.

After reaction using BigDye Terminator Cycle Sequencing FS Ready Reaction Kit (manufactured by Applied Biosystems) according to the manufacturer's instruction, the sequence of cDNA inserted in each plasmid were analyzed using DNA sequencer ABI PRISM 377 manufactured by Applied Biosystems. By this method, it was confirmed that all the cDNAs inserted in the plasmids were cDNA encoding full-length Chinese hamster FUT8 ORF. Among cDNA inserted in the plasmid DNAs whose sequences were determined, a plasmid DNA, free of readout errors of nucleotide resulting from PCR, was selected. Hereinafter, the plasmid is referred to as "CHfFUT8-pCR2.1". The nucleotide sequence of Chinese hamster FUT8 cDNA, determined in this manner, is represented by SEQ ID NO: 1.

### (2) Preparation of FUT8-targeting siRNA expression vector library

Human tRNA-val promoter type FUT8-targeting siRNA expression vector library was constructed using CHfFUT8-pCR2.1 obtained in the item (1), based upon the method described in Example 13 of WO03/46186. Also, pPUR (manufactured by CLONTECH) was used as a vector, using a recognition sequence of a restriction enzyme *Bam*HI as a loop sequence between antisense and sense DNAs. Hereinafter, the prepared library is referred to as "FUTBshRNAlib/pPUR/DH10B".

Plasmid vectors were prepared by amplifying the siRNA expression vector library, FUT8shRNA1ib/pPUR/DH10B. LB agar medium containing 100 µg/mL ampicillin was prepared using sterilized dishes [243 mm × 243 mm × 18 mm (manufactured by Nalgenunc)], and 50 µL/dish FUT8shRNAlib/pPUR/DH10B glycerol stock was plated. After stationary culture overnight at 37°C, the *E. coli* on the plates were collected in suspension with sterilized water, and a plasmid DNA was recovered according to the known method. Hereinafter, the recovered plasmid is referred to as "FUT8shRNAlib/pPUR" .

### 2. Obtaining of lectin-resistant clone into which α1,6-fucosyltransferase (FUT8)-targeting siRNA expression library was introduced

FUT8-targeting siRNA expression library plasmid, FUT8shRNAlib/pPUR obtained in the item 1 of this Example was introduced into clone 32-05-12, and clones resistant to LCA, a lectin which specifically recognizes α1,6-fucose, were isolated as follows.

Plasmid FUT8shRNAlib/pPUR obtained in the item 1 of this Example was digested with a restriction enzyme *Fsp*I (manufactured by New England Biolabs) to be linearized, and after 10 µg of the linearized plasmid FUT8shRNAlib/pPUR was introduced into 1.6×10⁶ cells of clone 32-05-12 by electroporation [Cytotechnology, 3, 133 (1990)], the cells were suspended in a basal medium [IMDM (manufactured by Invitrogen) containing 10% fetal bovine serum (manufactured by Invitrogen), 50 µg/mL gentamicin (manufactured by Nacalai Tesque), and 500 nmol/L MTX (manufactured by SIGMA)], and inoculated at 8 mL into 3 dishes of 10 cm for adherent cell culture (manufactured by Falcon). Also, transfection was carried out 10 times under the same conditions, and the cells were cultured in a total of 30 culture dishes of 10 cm. After culturing in a 5% CO₂ incubator at 37°C for 24 hours, the medium was exchanged with 8 mL of a basal medium containing 12 µg/mL puromycin (manufactured by SIGMA). After culturing in a 5% CO₂ incubator at 37°C for 7 days, the medium was exchanged with 8 mL of a basal medium containing 12 µg/mL puromycin (manufactured by SIGMA) and 0.5 mg/mL LCA (manufactured by VECTOR), and the culture was continued for further 6 to 8 days to isolate lectin-resistant clones.

### 3. Analysis of target sequence of α1,6-fucosyltransferase (FUT8)-targeting siRNA expression plasmid

### (1) Isolation of siRNA expression cassette on genomic DNA of lectin-resistant clone

siRNA expression cassette was isolated from genomic DNA of lectin-resistant clones obtained in the item 2 of this Example as follows (Fig. 7).

Lectin-resistant clones were collected into a flat-bottom plate for adherent cells (manufactured by Greiner) according to the known method [Gene Targeting, Oxford University Press (1993)], and cultured in a basal medium containing 12 µg/mL puromycin (manufactured by SIGMA) at 37°C for 1 week in the 5% CO₂ incubator.

After culturing, each clone of the plate was treated with trypsin, and dispensed onto 2 flat-bottom 96-well plates for adherent cells (manufactured by Greiner). One plate was used as a replica plate, and another was freeze-stored as a master plate. After the replica plate was cultured in a basal medium containing 12 µg/mL puromycin (manufactured by SIGMA) at 37°C for 1 week in a 5% CO₂ incubator, genomic DNA was prepared from each clone according to the known method [Analytical Biochemistry, 201, 331 (1992)], and dissolved in 30 µL each of TE-RNase buffer (pH 8.0) [10 mmol/L Tris-HCl, 1 mmol/L EDTA, and 200 µg/mL RNase A] overnight, then diluted at 0.05 µg/µL with sterilized water.

In addition, a forward primer which binds to the upstream of the tRNA-val promoter region of the siRNA expression cassette (SEQ ID NO:70) and a reverse primer which binds to the downstream of the terminator sequence of the siRNA expression cassette (SEQ ID NO:71) were each designed for FUT8-targeting siRNA expression plasmid, FUT8shRNAlib/pPUR.

Polymerase chain reaction (PCR) was carried out with DNA polymerase KOD polymerase (manufactured by TOYOBO), using the genomic DNA prepared from each clone as a template. A reaction solution (50 µL) [KOD Buffer1 (manufactured by TOYOBO), 0.2 mmol/L dNTPs, 1 mmol/L MgCl₂, and 0.4 µmol/L of the above primers (SEQ ID NOs:70 and 71)] containing 5 µL, of the genomic DNA solution described above was prepared for each clone, and after heating at 94°C for 1 minute, the PCR was carried out by 25 cycles, one cycle consisting of reaction at 97°C for 10 seconds and reaction at 68°C for 30 seconds. After the PCR, the reaction solution was subjected to agarose gel electrophoresis, and the amplified fragment (about 300 bp) containing the siRNA expression cassette region was recovered.

Also, 2 µg of plasmid pPUR (manufactured by CLONTECH) was digested with a restriction enzyme *Pvu*II (manufactured by New England Biolabs) at 37°C overnight. After the digestion reaction, the reaction solution was subjected to agarose gel electrophoresis, and a digested DNA fragment (about 4.3 kb) was recovered.

The PCR-amplified fragment (about 300 bp) obtained above was ligated to a *Pvu*II fragment derived from plasmid pPUR using Ligation High (manufactured by TOYOBO). *E. coli* DH5α was transformed with the reaction solution. Plasmid DNAs were isolated from a number of obtained ampicillin-resistant colonies according to the known method.

### (2) Analysis of target sequence contained in the siRNA expression unit

FUT8-targeting sequences contained in the siRNA expression cassette of the plasmids obtained in the item (1) were analyzed

First, after reaction with BigDye Terminator v3.0 Cycle sequencing Kit (manufactured by Applied Biosystems) according to the manufacturer's instruction, nucleotide sequences of siRNA expression cassette which were inserted into each plasmid DNA obtained in the item (1) were analyzed using DNA sequencer ABI PRISM 377 (manufactured by Applied Biosystems). Among nucleotide sequences determined for 159 clones, homology of target sequences against FUT8 was compared with the sequence of CHO cell FUT8 cDNA (SEQ ID NO:1), and the start and end points of each target sequence, which corresponded to SEQ ID NO:1, are shown in Table 3.

**Table 3**

| Clone No. | Start point of target sequence | End point of target sequence | Length of target sequence (bp) | | Clone No. | Start point of target sequence | End point of target sequence | Length of target sequence (bp) |
|---|---|---|---|---|---|---|---|---|
| 1 | 1 | 19 | 19 | | 41 | 539 | 565 | 27 |
| 2 | 1 | 20 | 20 | | 42 | 543 | 567 | 25 |
| 3 | 1 | 22 | 22 | | 43 | 543 | 570 | 28 |
| 4 | 2 | 31 | 30 | | 44 | 545 | 569 | 25 |
| 5 | 5 | 30 | 26 | | 45 | 561 | 589 | 29 |
| 6 | 29 | 53 | 25 | | 46 | 567 | 589 | 23 |
| 7 | 35 | 60 | 26 | | 47 | 603 | 629 | 27 |
| 8 | 35 | 62 | 28 | | 48 | 608 | 640 | 33 |
| 9 | 76 | 103 | 28 | | 49 | 642 | 660 | 19 |
| 10 | 78 | 105 | 28 | | 50 | 642 | 663 | 22 |
| 11 | 83 | 112 | 30 | | 51 | 642 | 670 | 29 |
| 12 | 87 | 112 | 26 | | 52 | 650 | 679 | 30 |
| 13 | 95 | 120 | 26 | | 53 | 663 | 689 | 27 |
| 14 | 96 | 120 | 25 | | 54 | 682 | 708 | 27 |
| 15 | 97 | 121 | 25 | | 55 | 710 | 736 | 27 |
| 16 | 109 | 133 | 25 | | 56 | 711 | 741 | 31 |
| 17 | 121 | 146 | 26 | | 57 | 713 | 740 | 28 |
| 18 | 144 | 170 | 27 | | 58 | 774 | 801 | 28 |
| 19 | 148 | 174 | 27 | | 59 | 789 | 816 | 28 |
| 20 | 150 | 174 | 25 | | 60 | 802 | 836 | 35 |
| 21 | 175 | 200 | 26 | | 61 | 824 | 850 | 27 |
| 22 | 216 | 242 | 27 | | 62 | 824 | 852 | 29 |
| 23 | 221 | 260 | 40 | | 63 | 824 | 854 | 31 |
| 24 | 230 | 256 | 27 | | 64 | 824 | 857 | 34 |
| 25 | 245 | 267 | 23 | | 65 | 827 | 858 | 32 |
| 26 | 268 | 296 | 29 | | 66 | 828 | 853 | 26 |
| 27 | 275 | 300 | 26 | | 67 | 834 | 858 | 25 |
| 28 | 276 | 306 | 31 | | 68 | 834 | 858 | 25 |
| 29 | 278 | 308 | 31 | | 69 | 834 | 860 | 27 |
| 30 | 279 | 306 | 28 | | 70 | 880 | 906 | 27 |
| 31 | 283 | 309 | 27 | | 71 | 886 | 913 | 28 |
| 32 | 301 | 326 | 26 | | 72 | 898 | 926 | 29 |
| 33 | 302 | 328 | 27 | | 73 | 900 | 922 | 23 |
| 34 | 330 | 361 | 32 | | 74 | 905 | 930 | 26 |
| 35 | 334 | 359 | 26 | | 75 | 907 | 934 | 28 |
| 36 | 372 | 398 | 27 | | 76 | 912 | 937 | 26 |
| 37 | 401 | 428 | 28 | | 77 | 917 | 946 | 30 |
| 38 | 534 | 563 | 30 | | 78 | 932 | 952 | 21 |
| 39 | 534 | 566 | 33 | | 79 | 950 | 968 | 19 |
| 40 | 536 | 563 | 28 | | 80 | 986 | 1013 | 28 |
| 81 | 990 | 1019 | 30 | | 121 | 1555 | 1578 | 24 |
| 82 | 1015 | 1042 | 28 | | 122 | 1584 | 1612 | 29 |
| 83 | 1022 | 1049 | 28 | | 123 | 1588 | 1615 | 28 |
| 84 | 1046 | 1071 | 26 | | 124 | 1591 | 1615 | 25 |
| 85 | 1062 | 1089 | 28 | | 125 | 1591 | 1619 | 29 |
| 86 | 1073 | 1102 | 30 | | 126 | 1602 | 1626 | 25 |
| 87 | 1095 | 1124 | 30 | | 127 | 1602 | 1629 | 28 |
| 88 | 1112 | 1137 | 26 | | 128 | 1610 | 1637 | 28 |
| 89 | 1122 | 1145 | 24 | | 129 | 1613 | 1637 | 25 |
| 90 | 1138 | 1169 | 32 | | 130 | 1619 | 1645 | 27 |
| 91 | 1149 | 1174 | 26 | | 131 | 1622 | 1647 | 26 |
| 92 | 1149 | 1182 | 34 | | 132 | 1680 | 1707 | 28 |
| 93 | 1150 | 1181 | 32 | | 133 | 1687 | 1713 | 27 |
| 94 | 1157 | 1181 | 25 | | 134 | 1729 | 1746 | 18 |
| 95 | 1166 | 1191 | 26 | | 135 | 1730 | 1746 | 17 |
| 96 | 1180 | 1207 | 28 | | 136 | 1730 | 1746 | 17 |
| 97 | 1211 | 1237 | 27 | | 137 | 1744 | 1758 | 15 |
| 98 | 1254 | 1278 | 25 | | 138 | 1744 | 1768 | 25 |
| 99 | 1340 | 1365 | 26 | | 139 | 1744 | 1773 | 30 |
| 100 | 1340 | 1370 | 31 | | 140 | 1765 | 1796 | 32 |
| 101 | 1416 | 1445 | 30 | | 141 | 1786 | 1811 | 26 |
| 102 | 1422 | 1448 | 27 | | 142 | 1821 | 1839 | 19 |
| 103 | 1425 | 1453 | 29 | | 143 | 1821 | 1842 | 22 |
| 104 | 1428 | 1460 | 33 | | 144 | 1821 | 1844 | 24 |
| 105 | 1441 | 1468 | 28 | | 145 | 1863 | 1890 | 28 |
| 106 | 1451 | 1480 | 30 | | 146 | 1927 | 1951 | 25 |
| 107 | 1463 | 1491 | 29 | | 147 | 1940 | 1965 | 26 |
| 108 | 1464 | 1489 | 26 | | 148 | 1948 | 1984 | 37 |
| 109 | 1465 | 1490 | 26 | | 149 | 1949 | 1976 | 28 |
| 110 | 1498 | 1517 | 20 | | 150 | 1951 | 1979 | 29 |
| 111 | 1498 | 1517 | 20 | | 151 | 1957 | 1982 | 26 |
| 112 | 1499 | 1526 | 28 | | 152 | 1957 | 1982 | 26 |
| 113 | 1501 | 1534 | 34 | | 153 | 1963 | 1987 | 25 |
| 114 | 1502 | 1529 | 28 | | 154 | 1963 | 1989 | 27 |
| 115 | 1504 | 1529 | 26 | | 155 | 1963 | 1990 | 28 |
| 116 | 1504 | 1530 | 27 | | 156 | 1964 | 1987 | 24 |
| 117 | 1504 | 1534 | 31 | | 157 | 1965 | 1990 | 26 |
| 118 | 1508 | 1526 | 19 | | 158 | 1974 | 2000 | 27 |
| 119 | 1532 | 1557 | 26 | | 159 | 1978 | 2008 | 31 |
| 120 | 1535 | 1563 | 29 | | | | | |

Among target sequences of 159 clones, RNA sequences corresponding to the representative target region are represented by SEQ ID NOs:14 to 23

### (3) Search of mouse, rat and human sequences homologous to the target sequence contained in the siRNA expression unit

Sequences corresponding to the target sequences represented by SEQ ID NOs:14 to 23 obtained in the item (2) were searched in mouse, rat and human FUT8 sequences as follows.

SEQ ID NOs:2, 3, and 4 show mouse, rat and human FUT8 sequences, respectively. Among the sequences, sequences corresponding to the target sequences represented by SEQ ID NOs:14 to 23 obtained in the item (2) were searched. In this search, completely matched sequences were excluded.

Each sequence number of the selected sequences is shown below. Mouse FUT8 sequence corresponding to SEQ ID NO:14 is represented by SEQ ID NO:85; human FUT8 sequence corresponding to SEQ ID NO:14 is represented by SEQ ID NO: 86; mouse FUT8 sequence corresponding to SEQ ID NO:15 is represented by SEQ ID N0:24; human FUT8 sequence corresponding to SEQ ID NO:1 is represented by SEQ ID NO:25; rat FUT8 sequence corresponding to SEQ ID NO:15 is represented by SEQ ID NO:87; human FUT8 sequence corresponding to SEQ ID NO:16 is represented by SEQ ID NO:26; human, mouse, and rat FUT8 sequences corresponding to SEQ ID NO:17 are represented by SEQ ID NO:27; mouse FUT8 sequence corresponding to SEQ ID NO:18 is represented by SEQ ID NO:28; human FUT8 sequence corresponding to SEQ ID NO:18 is represented by SEQ ID NO: 29; rat FUT8 sequence corresponding to SEQ ID NO:18 is represented by SEQ ID NO:30; mouse and rat FUT8 sequences corresponding to SEQ ID NO:19 are represented by SEQ ID NO:31; human FUT8 sequence corresponding to SEQ ID NO:19 is represented by SEQ ID NO:32; mouse FUT8 sequence corresponding to SEQ ID NO:20 is represented by SEQ ID NO:33; human FUT8 sequence corresponding to SEQ ID NO:20 is represented by SEQ ID NO:34; mouse FUT8 sequence corresponding to SEQ ID NO:21 is represented by SEQ ID NO:88 ; human FUT8 sequence corresponding to SEQ ID NO:21 is represented by SEQ ID NO:89; and rat FUT8 sequence corresponding to SEQ ID NO:22 is represented by SEQ ID NO:35.

### 4. Construction of effective α1,6-fucosyltransferase (FUT8)-targeting siRNA expression vector

Among target regions of FUT8-targeting siRNA obtained in the item 3 of this Example, siRNA expression vectors which target at the sequence contained in SEQ ID NO:15 or 16 were constructed according to the following procedure (Figs. 7, 8, and 9):

Among plasmids containing the siRNA expression cassette obtained in the item 3(1) of this Example, plasmids were selected, which contain the DNA sequence represented by SEQ ID NO:72 which is a target sequence contained in SEQ ID NO:15 equivalent to clone No. 31 shown in Table 1 as a sense DNA, and a nucleotide sequence complementary to the DNA sequence represented by SEQ ID NO:72 as an antisense DNA (hereinafter referred to as "FUT8shRNA/lib2B/pPUR"), and plasmids containing a DNA sequence corresponding to SEQ ID NO: 16 equivalent to clone No. 72 shown in Table 3 as a sense DNA, and a nucleotide sequence complementary to the DNA sequence corresponding to SEQ ID NO:16 as an antisense DNA (hereinafter referred to as "FUT8shRNA/lib3/pPUR") (Fig. 7).

First, after 1 µg of FUT8shRNA/lib2B/pPUR or FUTBshRNA/lib3/pPUR plasmid was dissolved in 30 µL of NEBuffer for *Eco*RI (manufactured by New England Biolabs) and digested with 10 units of restriction enzymes *Eco*RI and *Xho*I (manufactured by New England Biolabs) for 3 hours at 37°C, the reaction solution was subjected to agarose gel electrophoresis, and a DNA fragment (about 250 bp) containing human tRNA-val promoter-short hairpin type RNA-terminator sequence expression cassette was recovered using RECOCHIP (manufactured by TAKARA BIO).

Also, 1 µg of plasmid pBluescript II KS(+) was dissolved in 30 µL of NEBuffer for *Eco*RI (manufactured by New England Biolabs) and digested with 10 units of restriction enzymes *Eco*RI and *Xho*I (manufactured by New England Biolabs) for 2 hours at 37°C. After the reaction, 13 µL of sterilized water, 5 µL of 10×alkaline phosphatase buffer, and 1 unit of alkaline phosphatase *E. coli* C75 (manufactured by TAKARA BIO) were added to the reaction solution for carrying out dephosphorylation reaction at 37°C for 1 hour, the reaction solution was subjected to agarose gel electrophoresis, and an *Eco*RI*-Xho*I fragment (about 2.9 Kb) derived from plasmid pBluescript II KS(+) was recovered using RECOCHIP (manufactured by TAKARA BIO).

Then, 8 µL of the above *Eco*RI*-Xho*I DNA fragment (about 250 bp), 2 µL of the *Eco*RI*-Xho*I fragment (about 2.9 Kb) derived from plasmid pBluescript II KS(+), and 10 µL of Ligation High (manufactured by TOYOBO) were mixed and allowed to react for 2 hours at 16°C. *E. coli* DH5α (manufactured by Invitrogen) was transformed with the reaction solution, and plasmids were isolated from the resulting ampicillin-resistant clones using QIAprep spin Mini prep Kit (manufactured by Qiagen). Hereinafter, among the above plasmids, plasmids into which DNA fragments (about 250 bp) derived from plasmids FT8libB/pPUR and FT8lib3/pPUR are inserted are referred to as "FT8libB/pBS" and "FT8lib3/pBS", respectively.

After 1 µg of plasmid FT8libB/pBS or FT8lib3/pBS was dissolved in 20 µL of NEBuffer 4 (manufactured by New England Biolabs) and digested with 10 units of a restriction enzyme *Sma*I (manufactured by New England Biolabs) at 25°C for 5 hours, the restriction enzyme *Sma*I was inactivated by heating at 65°C for 20 minutes. To the reaction solution, 14.6 µL of sterilized water, 4 µL of 10xNEBuffer 2 (manufactured by New England Biolabs), 0.4 µL of 100xBSA (manufactured by New England Biolabs), and 10 units of a restriction enzyme *Xho*I (manufactured by New England Biolabs) were added, and digested overnight at 37°C, then the reaction solution was subjected to on agarose gel electrophoresis, and an DNA fragment containing a human tRNA-val promoter-short hairpin-type RNA-terminator sequence expression cassette (about 250 bp) was recovered using RECOCHIP (manufactured by TAKARA BIO).

Also, 1 µg of plasmid pAGE249 was dissolved in 30 µL of NEBuffer 1 (manufactured by New England Biolabs) and digested with 10 units of restriction enzymes *Nae*I and *Xho*I (manufactured by New England Biolabs) for 6 hours at 37°C. After the digestion reaction, 22 µL of sterilized water, 6 µL of 10xalkaline phosphatase buffer, and 1 unit of alkaline phosphatase *E. coli* C75 (manufactured by TAKARA BIO) were added to the reaction solution for carrying out dephosphorylation reaction at 37°C for 1 hour. The reaction solution was subjected to agarose gel electrophoresis, and a *Nae*I*-Xho*I fragment (about 4.4 Kb) derived from plasmid pAGE249 was recovered using RECOCHIP (manufactured by TAKARA BIO). Also, pAGE249 is a derivative of pAGE248 [J. Biol. Chem., 269, 14730 (1994)], namely a pAGE248 vector from which a 2.7 Kb fragment containing dihydrofolate reductase (dhfr) gene expression unit, digested with *Sph*I restriction enzyme, was removed.

Then, 10 µL of the above DNA fragment (about 250 bp), 5 µL of the *Nae*I-*Xho*I fragment (about 4.4 Kb) derived from plasmid pAGE249, and 15 µL of Ligation High (manufactured by TOYOBO) were mixed and allowed to react overnight at 16°C. *E. coli* DH5α (manufactured by Invitrogen) was transformed with the reaction solution, and plasmid DNA was isolated from the resulting ampicillin-resistant clones using QIAprep spin Mini prep Kit (manufactured by Qiagen). The nucleotide sequence of the DNA inserted in each plasmid was determined with DNA sequencer 377 (manufactured by Perkin Elmer) and BigDye Terminator v3.0 Cycle sequencing Kit (manufactured by Applied Biosystems) according to the manufacturer's instruction. pAGE249-seq FW (SEQ ID NO:73) and pAGE249-seq RV (SEQ ID NO:74) were used as sequencing primers, and agreement of inserted DNA sequences with the sequence corresponding to the *Eco*RI*-Xho*I fragment which is a human tRNA-val promoter-short hairpin-type RNA expression unit contained in plasmid FUT8shRNA/lib2B/pPUR or FUT8shRNA/lib3/pPUR was confirmed. Hereinafter, among the above plasmids, plasmids into which DNA fragments (about 250 bp) derived from plasmids FUT8shRNA/lib2B/pPUR and FUT8shRNA/lib3/pPUR are inserted are referred to as "FT8libB/pAGE" and "FT8lib3/pAGE", respectively.

### Example 5

### Preparation of lectin-resistant CHO/DG44 cell in the presence of L-fucose by co-introducing FUT8-targeting siRNA expression plasmid into lectin-resistant clone introduced with GMD-targeting siRNA expression plasmid:

### 1. Obtaining and culturing of lectin-resistant clone in the presence of L-fucose by introducing FUT8-targeting siRNA expression vector into lectin-resistant clone introduced with GMD-targeting siRNA expression vector

### (1) Obtaining of lectin-resistant clone in the presence of L-fucose by introducing FUT8-targeting siRNA expression vector

Lectin-resistant clones were obtained under L-fucose-added culturing conditions by introducing FT8libB/pAGE or FT8lib3/pAGE, the FUT8-targeting siRNA expression vector constructed in Example 2, into lectin-resistant clone 12-GMDB-2 or 12-GMDB-5 introduced with the GMD-targeting siRNA expression vector obtained in Example 1.

Transfection of plasmid FT8libB/pAGE or FT8lib3/pAGE into clone 12-GMDB-2 or 12-GMDB-5 was carried out by the same method as in the item 2(1) of Example 1.

After the transfection, the cell suspension was suspended in a basal medium containing 12 µg/mL puromycin (manufactured by SIGMA) and inoculated into four 10 cm-culture dishes for adherent cells (manufactured by Falcon). After culturing under conditions of 37°C and 5% CO₂ for 24 hours, the culture supernatant was removed, and a basal medium containing 400 µg/mL hygromycin (manufactured by WAKO) and 3 µg/mL puromycin (manufactured by SIGMA) was added thereto, followed by culturing for further 8 days. Subsequently, culture supernatant was removed from one of the dishes, a basal medium containing 400 µg/mL hygromycin (manufactured by WAKO) and 3 µg/mL puromycin (manufactured by SIGMA) was added thereto, followed by culturing for further 6 to 8 days, and the appeared hygromycin-resistant colonies were counted. Also, the culture supernatant was removed from the remaining dishes, and a basal medium containing 400 µg/mL hygromycin (manufactured by WAKO), 3 µg/mL puromycin (manufactured by SIGMA), 100 µmol/L L-fucose (manufactured by Nacalai Tesque), and 0.5 mg/mL LCA (manufactured by VECTOR) was added thereto, followed by culturing for further 9 days, and lectin-resistant clones in the presence of 100 µmol/L L-fucose, namely lectin-resistant clones co-introduced with FUT8-targeting siRNA expression vector and GMD-targeting siRNA expression vector, were obtained.

### (2) Expansion culture of lectin-resistant clones

Lectin-resistant clones obtained in the item (1) by co-introducing FUT8-targeting siRNA expression vector and GMD-targeting siRNA expression vector were expansion cultured according to the following procedure.

First, the number of colonies appeared in each dish was counted. Then, lectin-resistant colonies were scraped and sucked up with a pipetteman (manufactured by GILSON) under observation with stereoscopic microscope, and collected into a U-shaped-bottom 96-well plate for adherent cells (manufactured by ASAHI TECHNOGLASS). After trypsin treatment, each clone was inoculated into a flat-bottom 96-well plate for adherent cells (manufactured by Greiner), and cultured in a basal medium containing 400 µg/mL hygromycin (manufactured by WAKO) and 3 µg/mL puromycin (manufactured by SIGMA) under conditions of 5% CO₂ and 37°C overnight. The culture supernatant was removed after the culturing, and the cells were cultured in a basal medium containing 400 µg/mL hygromycin (manufactured by WAKO), 3 µg/mL puromycin (manufactured by SIGMA), 100 µmol/L L-fucose (manufactured by Nacalai Tesque) and 0.5 mg/mL LCA (manufactured by VECTOR) for further 6 days. After the culturing, each clone of the above plate was expansion cultured in a basal medium containing 400 µg/mL hygromycin (manufactured by WAKO) and 3 µg/mL puromycin (manufactured by SIGMA). Hereinafter, among clones used in the expansion culture, lectin-resistant clones obtained by introducing FT8libB/pAGE into clone 12-GMDB-2 are referred to as "12-GB2/FB-1", "12-GB2/FB-2", "12-GB2/FB-3", "12-GB2/FB-4" and "12-GB2/FB-5"; those by introducing FT8lib3/pAGE into clone 12-GMDB-2 are referred to as "12-GB2/F3-1", "12-GB2/F3-2", "12-GB2/F3-3 ", "12-GB2/F3-4" and "12-GB2/F3-5"; those by introducing FT8libB/pAGE into clone 12-GMDB-5 are referred to as "12-GBS/FB-1", "12-GBS/FB-2", "12-GBS/FB-3", "12-GB5/FB-4" and "12-GB5/FB-5"; those by introducing FT8lib3/pAGE into clone 12-GMDB-5 are referred to as "12-GB5/F3-1", "12-GB5/F3-2", "12-GB5/F3-3", "12-GB5/F3-4" and "12-GB5/F3-5", respectively. Also, clones 12-GB5/FB-1 and 12-GB5/F3-2 have been deposited to International Patent Organism Depositary, National Institute of Advanced Industrial Science and Technology (Tsukuba Central 6, 1, Higashi 1-chome, Tsukuba-shi, Ibaraki-ken, Japan n) as FERM BP-10049 and FERM BP-10050, respectively, on July 1, 2004.

### 2. Determination of the amount of GMD mRNA and FUT8 mRNA in lectin-resistant clones into which GMD-targeting expression vector and FUT8-targeting siRNA expression vector were introduced in the presence of L-fucose

### (1) Preparation of total RNA

A total RNA was prepared from the parent clone 32-05-12 before vector introduction, lectin-resistant clones 12-GMDB-2 and 12-GMDB-5 into which the GMD-targeting siRNA expression vector was introduced obtained in Example 1, and lectin-resistant clones into which the GMD-targeting expression vector and the FUT8-targeting siRNA expression vector were co-introduced obtained in the item 1 of this Example according to the following procedure.

Clone 32-05-12 was suspended in a basal medium, clones 12-GMDB-2 and 12-GMDB-5 were suspended in basal medium supplemented with 12 µg/mL puromycin (manufactured by SIGMA), and lectin-resistant clones into which FUT8-targeting siRNA expression vector and GMD-targeting siRNA expression vector were introduced were suspended in a basal medium containing 400 µg/mL hygromycin (manufactured by WAKO) and 3 µg/mL puromycin (manufactured by SIGMA), at a density of 3×10⁵ cells/mL, and 4 mL each was inoculated into 6 cm-dishes for adherent cells (manufactured by Falcon). The cells were cultured under conditions of 5% CO₂ and 37°C for 3 days, and each cell suspension was recovered by trypsin treatment and centrifugation at 1,000 rpm at 4°C for 5 minutes. After the recovered cells were suspended in Dulbecco's PBS buffer (manufactured by Invitrogen), cells were again recovered by re-centrifugation at 1,000 rpm at 4°C for 5 minutes, and a total RNA was extracted using RNeasy (manufactured by QIAGEN). The method was carried out according to the manufacturer's instruction, and the prepared total RNA was dissolved in 40 µL of sterilized water.

### (2) Synthesis of single-stranded cDNA

A single-stranded cDNA was synthesized from 3 µg of each of the total RNA obtained in the item (1) by reverse transcription reaction with oligo (dT) primer in 20 µL reaction system using SUPERSCRIPT^{™} Preamplification System for First Strand cDNA Synthesis (manufactured by Invitrogen) according to the manufacturer's instruction. Subsequently, the reaction solution was treated with RNase and the final reaction volume was adjusted to 40 µL. In addition, each of the reaction solutions was diluted 50-fold with sterilized water, and used for determination of the amount of gene transcription described below.

### (3) Determination of the amount of GMD gene transcription by SYBR-PCR

The amount of mRNA transcribed from GMD gene and β-actin gene were quantified as described below. Also, plasmid pAGE249GMD containing CHO cell-derived GMD cDNA described in Example 15 of WO02/31140, each diluted at concentration of 0.0512 fg/µL, 0.256 fg/µL, 1.28 fg/µL, 6.4 fg/µL, 32 fg/µL and 160 fg/µL, were used as internal controls of GMD determination, and β-actin standard plasmid described in Example 9 of WO02/31140, each diluted at concentration of 1.28 fg/µL, 6.4 fg/µL, 32 fg/µL, 160 fg/µL, 800 fg/µL and 4,000 fg/µL, were used as internal controls of β-actin determination. Furthermore, as PCR primers, forward and reverse primers represented by SEQ ID NOs:64 and 65, respectively, were used to amplify GMD, and forward and reverse primers represented by SEQ ID NOs:66 and 67, respectively, were used to amplify β-actin.

Then, 20 µL of reaction solution [R-PCR buffer (manufactured by TAKARA BIO), 2.5 mmol/L Mg²⁺ Solution for R-PCR (manufactured by TAKARA BIO), 0.3 mmol/L dNTP mixture (manufactured by TAKARA BIO), 0.3 µmol/L forward primer, 0.3 µmol/L reverse primer, 2×10⁻⁵ diluted SYBR GreenI, 1 unit TaKaRa Ex Taq R-PCR] containing 5 µL of the single-stranded cDNA solution prepared in the item (2) or each concentration of internal control plasmid solution was prepared using For Real Time PCR TaKaRa Ex Taq R-PCR Version (manufactured by TAKARA BIO). The prepared reaction solution was dispensed into each well of a 96-well Polypropylene PCR Plate (manufactured by Falcon), and the plate was sealed with Plate Sealer (manufactured by Edge Biosystems). ABI PRISM 7700 Sequence Detection System was used for PCR and analysis, and the amount of GMD mRNA and the amount of β-actin mRNA were determined according to the manufacturer's instruction.

A calibration curve was obtained based upon the measurements with the internal control plasmid, and the amount of GMD mRNA and the amount of β-actin mRNA were converted into numerical terms. In addition, assuming that the amount of mRNA transcribed from β-actin gene are uniform among the clones, the relative amount of GMD mRNA to the amount of β-actin mRNA were calculated and compared, and the results are shown in Fig. 10. The amount of GMD mRNA in all the clones obtained by co-introducing FUT8-targeting siRNA expression vector and GMD-targeting siRNA expression vector were reduced to about 10% in comparison with that in the parent clone 32-05-12.

### (4) Determination of the amount of FUT8 gene transcription by SYBR-PCR

The amount mRNA transcribed from FUT8 gene and β-actin gene were determined as described below. Also, FUT8 standard plasmid described in Example 9 of WO02/31140, each diluted at concentration of 0.0512 fg/µL, 0.256 fg/µL, 1.28 fg/µL, 6.4 fg/µL, 32 fg/µL and 160 fg/µL, were used as internal controls of FUT8 determination; β-actin standard plasmid described in Example 9 of WO02/31140, each diluted at concentration of 1.28 fg/µL, 6.4 fg/µL,, 32 fg/µL, 160 fg/µL, 800 fg/µL and 4,000 fg/µL, were used as internal controls of β-actin determination. Furthermore, as PCR primer, forward and reverse primers represented by SEQ ID NOs:75 and 76, respectively, were used to amplify FUT8, and forward and reverse primers represented by SEQ ID NOs:66 and 67, respectively, were used to amplify β-actin.

Then, 20 µL of reaction solution [R-PCR buffer (manufactured by TAKARA BIO), 2.5 mmol/L Mg²⁺ Solution for R-PCR (manufactured by TAKARA BIO), 0.3 mmol/L dNTP mixture (manufactured by TAKARA BIO), 0.3 µmol/L forward primer, 0.3 µmol/L reverse primer, 2×10⁻⁵ diluted SYBR GreenI, and 1 unit TaKaRa Ex Taq R-PCR] containing 5 µL of the single-stranded cDNA solution prepared in the item (2) or each concentration of internal control plasmid solution was prepared using For Real Time PCR TaKaRa Ex Taq R-PCR Version (manufactured by TAKARA BIO). The prepared reaction solution was dispensed into each well of a 96-well Polypropylene PCR Plate (manufactured by Falcon), and the plate was sealed with Plate Sealer (manufactured by Edge Biosystems). ABI PRISM 7700 Sequence Detection System was used for PCR and analysis, and the amount of GMD mRNA and the amount of β-actin mRNA were determined according to the manufacturer's instruction.

A calibration curve was obtained based upon the measurements with the internal control plasmid, and the amount of FUT8 mRNA and the amount of β-actin mRNA were converted into numerical terms. In addition, assuming that the amount of mRNA transcribed from β-actin gene are uniform among the clones, the relative amount of FUT8 mRNA to the amount of β-actin mRNA were calculated and compared, and the results are shown in Fig. 11. The amounts of FUT8 mRNA in all the clones obtained by co-introducing FUT8-targeting siRNA expression vector and GMD-targeting siRNA expression vector were reduced to about 10% in comparison with that in the parent clone 32-05-12.

### Example 6

### Production of antibody compositions using CHO/DG44 cell into which GMD-targeting siRNA expression plasmid and FUT8-targeting siRNA expression plasmid were introduced:

### 1. Obtaining of antibody compositions under culturing in the absence of L-fucose

Anti-CCR4 chimeric antibody compositions produced by the parent clone 32-05-12 before vector introduction, lectin-resistant clones 12-GMDB-2 and 12-GMDB-5 into which GMD-targeting siRNA expression plasmid was introduced obtained in Example 1, and lectin-resistant clones 12-GB2/FB-1, 12-GB2/FB-3, 12-GB2/F3-3, 12-GB2/F3-5, 12-GB5/FB-1, 12-GB5/FB-2, 12-GB5/F3-2 and 12-GB5/F3-4 into which FUT8-targeting siRNA expression vector and GMD-targeting siRNA expression vector were co-introduced obtained in Example 3, were obtained according to the following procedure.

Clone 32-05-12 was suspended in a basal medium, clones 12-GMDB-2 and 12-GMDB-5 were suspended in a basal medium containing 12 µg/mL puromycin (manufactured by SIGMA), and clones 12-GB2/FB-1, 12-GB2/FB-3, 12-GB2/F3-3, 12-GB2/F3-5, 12-GBS/FB-1, 12-GBS/FB-2, 12-GBS/F3-2 and 12-GB5/F3-4 were suspended in a basal medium containing 400 µg/mL hygromycin (manufactured by WAKO) and 3 µg/mL puromycin (manufactured by SIGMA), at a density of 3×10⁵ cells/mL, and were inoculated at 15 mL into a T75 flask for adherent cells (manufactured by Greiner). After culturing under conditions of 5% CO₂ and 37°C for 6 days, the culture supernatant was removed, and after washing with 10 mL of Dulbecco's PBS (manufactured by Invitrogen), 20 mL of EXCELL301 medium (manufactured by JRH Bioscience) was added. After culturing under conditions of 5% CO₂ and 37°C for 7 days, the culture supernatant was recovered, and anti-CCR4 chimeric antibody compositions were purified using a MabSelect column (manufactured by Amersham Bioscience) according to the manufacturer's instruction. After exchange into 10 mmol/L KH₂PO₄ buffer using Econo-Pac 10DG (manufactured by Bio Rad), anti-CCR4 chimeric antibody compositions purified from culture supernatants of various clones were subjected to steric filtration by using Millex GV (manufactured by MILLIPORE) of 0.22 µm pore size.

### 2. Obtaining of antibody compositions under culturing in the presence of L-fucose

Anti-CCR4 chimeric antibody compositions produced under presence of L-fucose culturing conditions by the parent clone 32-05-12 before vector introduction, lectin-resistant clones 12-GMDB-2 and 12-GMDB-5 into which the GMD-targeting siRNA expression plasmid was introduced obtained in Example 1, and lectin-resistant clones 12-GB2/FB-1, 12-GB2/FB-3, 12-GB2/F3-3, 12-GB2/F3-5, 12-GB5/FB-1, 12-GB5/FB-2, 12-GB5/F3-2 and 12-GB5/F3-4 into which the GMD-targeting siRNA expression plasmid and the FUT8-targeting siRNA expression plasmid were introduced obtained in Example 3, were obtained according to the following procedure:

Clone 32-05-12 was cultured in a basal medium, clones 12-GMDB-2 and 12-GMDB-5 were suspended in a basal medium supplemented with 12 µg/mL puromycin (manufactured by SIGMA), and clones 12-GB2/FB-1, 12-GB2/FB-3, 12-GB2/F3-3, 12-GB2/F3-5, 12-GBS/FB-1, 12-GBS/FB-2, 12-GB5/F3-2 and 12-GB5/F3-4 were suspended in a basal medium supplemented with 400 µg/mL hygromycin (manufactured by WAKO) and 3 µg/mL puromycin (manufactured by SIGMA), at a density of 3×10⁵ cells/mL, and were inoculated at 15 mL into a T75 flask for adherent cells (manufactured by Greiner). After culturing under conditions of 5% CO₂ and 37°C for 3 days, the culture supernatant was removed, and the culture was replaced by a basal medium supplemented with 500 µmol/L L-fucose (manufactured by Nacalai Tesque) for clone 32-05-12, a basal medium supplemented with 500 µmol/L L-fucose (manufactured by Nacalai Tesque) and 12 µg/mL puromycin (manufactured by SIGMA) for clones 12-GMDB-2 and 12-GMDB-5, and a basal medium supplemented with 500 µmol/L L-fucose (manufactured by Nacalai Tesque), 400 µg/mL hygromycin (manufactured by WAKO), and 3 µg/mL puromycin(manufactured by SIGMA) for clones 12-GB2/FB-1, 12-GB2/FB-3, 12-GB2/F3-3, 12-GB2/F3-5, 12-GBS/FB-1, 12-GBS/FB-2, 12-GBS/F3-2 and 12-GBS/F3-4. After culturing under conditions of 5% CO₂ and 37°C for further 3 days, the culture supernatant was removed, and after washing with 10 mL of Dulbecco's PBS (manufactured by Invitrogen), 20 mL of EXCELL301 medium (manufactured by JRH Bioscience) supplemented with 500 µmol/L L-fucose (manufactured by Nacalai Tesque) was added. After culturing under conditions of 5% CO₂ and 37°C for 3 days, 1 mL of 10 mmol/L L-fucose (manufactured by Nacalai Tesque) was added to each flask, followed by culturing for further 4 days. After the culturing, the culture supernatant was recovered, and anti-CCR4 chimeric antibodies were purified using a MabSelect column (manufactured by Amersham Bioscience) according to the manufacturer's instruction. After exchange with 10 mmol/L KH₂PO₄ buffer using Econo-Pac 10DG (manufactured by Bio Rad), anti-CCR4 chimeric antibody compositions purified from culture supernatant of various clones were subjected to steric filtration by using Millex GV (manufactured by MILLIPORE) 0.22 µm pore size.

### 3. Composition analysis of monosaccharide of antibody compositions

Composition analysis of monosaccharide was carried out for each anti-CCR4 chimeric antibody compositions obtained in the items 1 and 2 of this Example according to the known method [Journal ofLiquid Chromatography, 6, 1577 (1983)].

**Table 4**

| Fucose(-)% of anti-CCR4-chimeric antibody composition produced by each clone | | |
|---|---|---|
| Clone | Fucose(-)% | |
| | Culturing in the presence of L-fucose | Culturing in the absence of L-fucose |
| 32-05-12 | 6% | 3% |
| 12-GMDB-2 | 6% | 78% |
| 12-GB2/FB-1 | 66% | 94% |
| 12-GB2/FB-3 | 67% | 92% |
| 12-GB2/F3-3 | 81% | 98% |
| 12-GB2/F3-5 | 79% | 97% |
| 12-GMDB-5 | 8% | 79% |
| 12-GB5/FB-1 | 71% | 97% |
| 12-GBS/FB-2 | 76% | 96% |
| 12-GBS/F3-2 | 81% | 95% |
| 12-GBS/F3-4 | 67% | 91% |

The ratios of antibody having sugar chains in which fucose is not bound to N-acetylglucosamine in the reducing end in the complex type N-glycoside-linked sugar chain calculated from the monosaccharide composition ratio of each antibody composition (hereinafter referred to as "fucose(-)% of antibody composition") are shown in Table 4.

Under culturing in the absence of L-fucose, fucose(-)% of the antibody composition produced from the parent clone 32-05-12 used for introduction of the GMD-targeting siRNA expression vector was 3%, while those of lectin-resistant clones 12-GMDB-2 and 12-GMDB-5 into which GMD-targeting siRNA was introduced were 78% and 79%, respectively, demonstrating significantly increased fucose(-)% of the antibody compositions in comparison with that of the parent cell. In addition, fucose(-)% of the antibody compositions produced by lectin-resistant clones 12-GB2/FB-1, 12-GB2/FB-3, 12-GB2/F3-3, 12-GB2/F3-5, 12-GB5/FB-1, 12-GB5/FB-2, 12-GB5/F3-2 and 12-GB5/F3-4 into which GMD-targeting siRNA and FUT8-targeting siRNA were co-introduced were 91 to 98%, demonstrating further increase in fucose(-)% of antibody compositions in comparison with those of lectin-resistant clones introduced with GMD-targeting siRNA alone.

Also, under culturing in the presence of L-fucose, fucose(-)% of antibody composition produced by the parent clone 32-05-12 used for introduction of the GMD-targeting siRNA expression vector was 6%, while those of lectin-resistant clones 12-GMDB-2 and 12-GMDB-5 into which GMD-targeting siRNA was introduced were 6% and 8%, respectively. On the other hand, fucose(-)% of the antibody compositions produced by lectin-resistant clones 12-GB2/FB-1, 12-GB2/FB-3, 12-GB2/F3-3, 12-GB2/F3-5, 12-GBS/FB-1, 12-GB5/FB-2, 12-GB5/F3-2 and 12-GB5/F3-4 into which GMD-targeting siRNA and FUT8-targeting siRNA were co-introduced were values between 66 to 81%, demonstrating a significant increase in fucose(-)% of the antibody compositions in comparison with those of lectin-resistant clones into which GMD-targeting siRNA was introduced alone.

As described above, fucose(-)% of the antibody compositions produced by lectin-resistant clones co-introduced with GMD-targeting and FUT8-targeting siRNA were higher than that of lectin-resistant clones into which GMD-targeting siRNA was introduced alone under culturing in the absence or presence of L-fucose, therefore, regardless of the presence or absence of L-fucose, effect of inhibiting the addition of α1,6-fucose to complex sugar chains of cell-produced antibody compositions based on co-introducing GMD-targeting siRNA and FUT8-targeting siRNA were higher than those of introducing GMD-targeting siRNA alone.

In addition, fucose(-)% of the antibody compositions produced by lectin-resistant clones into which GMD-targeting siRNA and FUT8-targeting siRNA were co-introduced were higher in culturing in the absence of L-fucose than culturing in the presence of L-fucose where effect of rising fucose(-)% of GMD-targeting siRNA are lost, it is shown that the suppressive effects on addition of α1,6-fucose to complex sugar chains of cell-produced antibody compositions by co-introducing GMD-targeting siRNA and FUT8-targeting siRNA is higher than the suppressive effect of introducing GMD-targeting siRNA alone.

### Example 7

### Serum-free fed-batch culture of CHO/DG44 cell into which GMD-targeting expression vector and FUT8-targeting siRNA expression vector were introduced:

### 1. Adaptation of CHO/DG44 cell into which GMD-targeting expression vector and FUT8-targeting siRNA expression vector were introduced to serum-free medium

The parent clone 32-05-12 before vector introduction, and lectin-resistant clones 12-GB2/FB-1, 12-GB2/FB-3, 12-GB2/F3-3, 12-GB2/F3-5, 12-GBS/FB-1, 12-GB5/FB-2, 12-GBS/F3-2 and 12-GB5/F3-4 into which the GMD-targeting siRNA expression vector and FUT8-targeting siRNA expression vector were introduced obtained in Example 3, were adapted to a serum-free medium according to the following procedure.

Clone 32-05-12 was suspended in a basal medium, and clones 12-GB2/FB-1, 12-GB2/FB-3, 12-GB2/F3-3, 12-GB2/F3-5, 12-GB5/FB-1, 12-GB5/FB-2, 12-GB5/F3-2 and 12-GB5/F3-4 were suspended in a basal medium supplemented with 400 µg/mL hygromycin (manufactured by WAKO) and 3 µg/mL puromycin (manufactured by SIGMA), at a density of 3×10⁵ cells/mL, and each was inoculated at 15 mL into a T75 flask for adherent cells (manufactured by Greiner). After culturing under conditions of 5% CO₂ and 37°C for 3 days, cells were suspended by trypsin treatment, and cells were recovered by centrifugation at 1,000 rpm for 5 minutes. Clone 32-05-12 was suspended in EX-CELL302 medium (manufactured by JRH) containing 500 nmol/L MTX (manufactured by SIGMA), 6 mmol/L L-glutamine (manufactured by Invitrogen), 50 µg/mL gentamicin (manufactured by Nacalai Tesque), and 100 nmol/L 3,3,5-Triiodo-L-thyronine (manufactured by SIGMA) (hereinafter referred to as "serum-free medium"), and clones 12-GB2/FB-1, 12-GB2/FB-3, 12-GB2/F3-3, 12-GB2/F3-5, 12-GBS/FB-1, 12-GB5/FB-2, 12-GB5/F3-2 and 12-GB5/F3-4 were suspended in a serum-free medium supplemented with 400 µg/mL hygromycin (manufactured by WAKO) and 3 µg/mL puromycin (manufactured by SIGMA), at a density of 5×10⁵ cells/mL, and 15 mL of the cell suspension was inoculated into a 125 mL conical flask (manufactured by Corning). After ventilating the flask with 5% CO₂ (at least 4-fold volume of culture vessel) and sealing the flask, suspension rotation culture was carried out at 90 to 100 rpm and 35°C. Passage was repeated at 3 to 4 day intervals, and finally, clones which could grow in the serum-free medium were obtained. Hereinafter, clone 32-05-12 adapted to the serum-free medium is referred to as "32-05-12AF"; clone 12-GB2/FB-1 adapted to the serum-free medium is referred to as "clone Wi2B-1AF"; clone 12-GB2/FB-3 adapted to the serum-free medium is referred to as "Wi2B-3AF"; clone 12-GB2/F3-3 adapted to the serum-free medium is referred to as "Wi23-3AF"; clone 12-GB2/F3-5 adapted to the serum-free medium is referred to as "Wi23-5AF"; clone 12-GB5/FB-1 adapted to the serum-free medium is referred to as "Wi5B-1AF"; clone 12-GB5/FB-2 adapted to the serum-free medium is referred to as "Wi5B-2AF"; clone 12-GB5/F3-2 adapted to the serum-free medium is referred to as "Wi53-2AF"; and clone 12-GB5/F3-4 adapted to serum-free medium is referred to as "Wi53-4AF", respectively.

### 2. Serum-free fed-batch culture using CHO/DG44 cell into which GMD-targeting expression vector and FUT8-targeting siRNA expression vector were introduced and adapted to serum-free medium

Serum-free fed-batch culture was carried out with clones, 32-05-12AF, Wi23-3AF, Wi23-5AF and Wi5B-1AF adapted to the serum-free medium in the item 1 of this Example according to the following procedure:

A serum-free fed-batch medium and a feed medium were used for the fed-batch culture.

Clones 32-05-12AF, Wi23-3AF, Wi23-5AF and Wi5B-1AF were suspended in a serum-free fed-batch medium at a density of 3×10⁵ cells/mL, and 40 mL each of the cell suspension was inoculated into a 250 mL conical flask (manufactured by Corning). After ventilating the flask with 5% CO₂ (at least 4-fold volume of culture vessel) and sealing the flask, suspension rotation culture was carried out at 90 to 100 rpm and 35°C. On days 3, 6, 9 and 12 after starting the culture, 3.3 mL of a feed medium was added to supplement the consumption of amino acids and the like, and 20% (w/v) glucose solution was added at a final concentration of 5,000 mg/L to adjust the glucose concentration. On days 0, 3, 6, 9, 12 and 14 after starting the culture, 2 to 4 mL each culture was collected, and the viable cell number and viability were measured by trypan blue staining, and antibody concentrations contained in each culture supernatant were measured by ELISA described in the item 3(1) of this Example. Viable cell number and antibody composition concentration in culture supernatant at each point of time after starting the culture of clones 32-05-12AF and Wi23-5AF are shown in Figs. 12 and 13, respectively. Viable cell numbers and antibody composition concentrations in culture supernatants at each point of time after starting the culture were similar between clones 32-05-12AF and Wi23-5AF. Therefore, it was demonstrated that the target sequences of GMD-targeting siRNA and FUT8-targeting siRNA had no significant effects on the cell growth and antibody production.

### 3. Determination of antibodies having sugar chains in which 1-position of fucose is not bound to 6-position of N-acetylglucosamine in the reducing end through α-bond using the binding activity to soluble human FcγRIIIa as an indicator

Fucose(-)% of anti-CCR4 chimeric antibody compositions contained in the serum-free fed-batch culture supernatant of clones 32-05-12AF, Wi23-3AF, Wi23-5AF and Wi5B-1AF obtained in the item 2 of this Example were measured using the binding activity to soluble human FcγRIIIa (hereinafter referred to as "shFcγRIIIa") described in Example 10 of WO03/85119 as an indicator according to the following procedure.

### (1) Determination of antibody concentration by ELISA

Antibody concentrations in the culture supernatant were determined in the same manner as in the item 2(3) of Example 3.

Antibody concentrations of each diluted sample were calculated using the linear area of the sigmoid curve of the calibration curve prepared with a standard of purified antibody preparation, and each antibody concentration of culture supernatants was calculated by multiplying antibody concentration of the obtained diluted samples by the dilution rate.

### (2) Preparation of standards with different fucose(-)%

Standards with different fucose(-)% were prepared using anti-CCR4 chimeric antibodies, YB2/0 cell-derived KM2760-1 and CHO/DG44 cell-derived KM3060, described in Example 4 of WO03/85119. Fucose(-)% was measured by composition analysis of monosaccharide described in the item 3 of Example 4 for a total of 11 standard samples including KM2760-1, KM3060, and 9 standard samples prepared by mixing KM2760-1 and KM3060; KM2760-1 was 90%; KM3060 was 10%; 9 standard preparation samples prepared were 82%, 74%, 66%, 58%, 50%, 42%, 34%, 26% and 18%, respectively.

### (3) Evaluation of the binding activity of antibody to shFcγRIIIa

BSA conjugate with human CCR4 cell extracellular peptides having the amino acid sequence represented by SEQ ID NO:77 to which an anti-CCR4 chimeric antibody reacts was prepared in the same manner as the method described in the item 2 of Example 4 of WO03/85119.

After 50 µL/well of the prepared BSA conjugate with human CCR4 extracellular peptide was dispensed onto 96-well ELISA plates (manufactured by Greiner) at a concentration of 1 µg/mL, the mixture was left overnight at 4°C to adsorb. After washing with PBS, 100 µL/well of BSA-PBS was added, and was allowed to react for 1 hour at room temperature to block remaining active groups. After washing each well with Tween-PBS, 50 µL/well of each anti-CCR4 chimeric antibody sample diluted with BSA-PBS was added at 2.5 µg/mL according to the antibody concentrations measured by ELISA described in the item (1), and was allowed to react for 1 hour at room temperature. After washing each well with Tween-PBS, 50 µL/well of shFcγRIIIa solution diluted at 5 µg/mL with BSA-PBS was added thereto, and was allowed to react for 1 hour at room temperature. After washing each well with Tween-PBS, 50 µL/well of HRP-labeled mouse antibody Penta-His HRP Conjugate (manufactured by QIAGEN) prepared with BSA-PBS at 0.1 µg/mL was added, and the mixture was allowed to react for 1 hour at room temperature. After washing with Tween-PBS, 50 µL/well of ABTS substrate solution was added, and after color development in the same manner as the method in the item (2), absorbance at 490 nm was measured with absorbance at 415 nm as reference, using a microplate reader.

Binding activities of each anti-CCR4 chimeric antibody standard preparation prepared in the item (2) with known fucose(-)% to shFcγRIIIa are shown in Fig. 14. Binding activity to shFcγRIIIa increased in proportion to fucose(-)%, and a calibration curve was obtained based on it. Also, in the measurement, calibration curves were made for each 96-well ELISA plate.

From the absorbance at 490 nm at which each anti-CCR4 chimeric antibody composition contained in the serum-free fed-batch culture supernatant obtained in the item 2 of this Example showed in binding activities to shFcγRIIIa, fucose(-)% of the anti-CCR4 chimeric antibody composition contained in culture supernatant was obtained using the calibration curve. Results of clones 32-05-12AF and Wi23-5AF are shown in Fig. 15.

Antibody composition contained in the culture supernatant of 32-05-12AF exhibited low binding activity to shFcγRIIIa in the culture, and its fucose(-)% was approximately 10%. Also, antibody composition contained in the culture supernatant of clones Wi23-3AF, Wi23-5AF, and Wi5B-1AF exhibited high binding activity to shFcγRIIIa in the culture, and their fucose(-)% were 75 to 90% or higher. Therefore, it was demonstrated that the target sequences of GMD-targeting siRNA and FUT8-targeting siRNA could stably produce antibody compositions with high fucose(-)% without affecting cell growth and antibody production.

### 4. Sugar chain structural analysis of antibody composition produced at the end of serum-free fed-batch culture

### (1) Obtaining of purified antibody upon completion of serum-free fed-batch culture

Each anti-CCR4 chimeric antibody composition was purified from culture supernatants of serum-free fed-batch culture on day 14 of clones 32-05-12AF, Wi23-3AF, Wi23-5AF, and Wi5B-1AF obtained in the item 2 of this Example, using a MabSelect column (manufactured by Amersham Biosciences) according to the manufacturer's instruction. After exchange with 10 mmol/L KH₂PO₄ buffer using Econo-Pac 10DG (manufactured by Bio Rad), each purified anti-CCR4 chimeric antibody composition was subjected to sterile filtration by using Millex GV (manufactured by MILLIPORE) of 0.22 µm pore size.

### (2) Composition analysis of Monosaccharide of antibody compositions

Composition analysis of Monosaccharide was carried out with each anti-CCR4 chimeric antibody compositions obtained from culture supernatants of clones adapted to serum-free medium in the item (1) according to the known method [Journal of Liquid Chromatography, 6, 1577 (1983)].

Fucose(-)% calculated from composition ratio of monosaccharide of each antibody composition is shown in Table 5.

**Table 5**

| Fucose(-)% of anti-CCR4-chimeric antibody composition produced by each clone | |
|---|---|
| Clone | Fucose(-)% |
| 32-05-12AF | 7% |
| Wi23-3AF | 87% |
| Wi23-5AF | 81% |
| Wi5B-1AF | 81% |

Fucose(-)% of antibody composition produced by clone 32-05-12AF was 7%, while those of lectin-resistant clones Wi23-3AF, Wi23-5AF and Wi5B-lAF into which the GMD-targeting siRNA expression vector and the FUT8-targeting siRNA expression vector were co-introduced were approximately 80 to 90%, and the comparison with the parent clone demonstrated that inhibitory effects on α1,6-fucose addition to complex sugar chains of antibody compositions were maintained in lectin-resistant clones into which the GMD-targeting expression vector and the FUT8-targeting siRNA expression vector were co-introduced even at the end of the culture.

### Example 8

### Production of antibody compositions using CHO/DG44 cell into which GMD-targeting siRNA and FUT8-targeting siRNA co-expression vector was introduced

### 1. Construction of GMD-targeting siRNA and FUT8-targeting siRNA co-expression vector

### (1) Construction of GMD-targeting siRNA expression unit in the downstream of human U6 promoter

GMD-targeting siRNA expression in the downstream of human U6 promoter was constructed using pPUR/GMDshB constructed in the item 1 of Example 1 according to the following procedure (Fig. 16):

First, PUR-FW-*Xho*I forward primer (SEQ ID NO:78) with recognition sequences of *Xho*I restriction enzyme, which binds to the upstream of the human U6 promoter sequence of pPUR/GMDshB, and PUR-RV-*Xho*I reverse primer (SEQ ID NO:79) which binds to the downstream of the terminator sequence of siRNA expression cassette were each designed.

PCR was carried out using DNA polymerase, KOD polymerase (manufactured by TOYOBO) and pPUR/GMDshB as a template. Then, 20 µL of reaction solution [KOD buffer 1 (manufactured by TOYOBO), 0.2 mmol/L dNTPs, 1 mmol/L MgCl₂, 0.4 µmol/L above primers (SEQ ID NO:78 and 79), and 1U KOD polymerase] containing 10 ng of plasmid pPUR/GMDshB was prepared, and after heating at 98°C for 1 minute, PCR was carried out by 25 cycles, one cycle consisting of reaction at 98°C for 15 seconds, reaction at 65°C for 5 seconds and reaction at 74°C for 30 seconds. After the PCR, the reaction solution was subjected to agarose gel electrophoresis, and an amplified fragment (about 600 bp) containing GMD-targeting siRNA expression cassette region in the downstream of the human U6 promoter was recovered.

The above PCR-amplified fragment (about 600 bp) was ligated to pCR-BluntII-TOPO (manufactured by Invitrogen) using Ligation High (manufactured by TOYOBO). *E. coli* DH5α was transformed with the reaction solution. A plasmid DNA was isolated from the resulting kanamycin-resistant clones using QIAprep spin Mini prep Kit (manufactured by Qiagen). Hereinafter, the plasmid is referred to as "pCR/GMDshB".

### (2) Construction of GMD-targeting siRNA and FUT8-targeting siRNA co-expression vector

GMD-targeting siRNA and FUT8-targeting siRNA co-expression vector was constructed using plasmid pCR/GMDshB constructed in the item (1), and plasmid FT8libB/pAGE or FT8lib3/pAGE constructed in the item 4 of Example 4 according to the following procedure (Fig. 17).

First, plasmid pCR/GMDshB was dissolved in 40 µL, of NEBuffer 2 (manufactured by New England Biolabs) containing 100µg/mL BSA (manufactured by New England Biolabs), and digested with 10 units of a restriction enzyme *Xho*I (manufactured by New England Biolabs) at 37°C overnight. The reaction solution was subjected to agarose gel electrophoresis, and DNA fragment (about 600 bp) containing GMD-targeting siRNA expression cassette in the downstream of the human U6 promoter was recovered using RECOCHIP (manufactured by TAKARA BIO).

Also, 1 µg of plasmid FT8libB/pAGE or FT8lib3/pAGE was dissolved in 40 µL of NEBuffer 2 (manufactured by New England Biolabs) containing 100µg/mL BSA (manufactured by New England Biolabs), and digested with 10 units of a restriction enzyme *Xho*I (manufactured by New England Biolabs) at 37°C overnight. After the reaction, 15µL of sterilized water, 4 µL of 10xalkaline phosphatase buffer, and 1 unit of alkaline phosphatase *E. coli* C75 (manufactured by TAKARA BIO) were added to 20 µL of the reaction solution for carrying out dephosphorylation reaction at 37°C for 1 hour. The reaction solution was subjected to agarose gel electrophoresis, and *Xho*I fragment (about 4.7 kb) derived from plasmid FT8libB/pAGE or FT8lib3/pAGE was recovered using RECOCHIP (manufactured by TAKARA BIO).

After 5 µL of the DNA fragment (about 600 bp) containing GMD-targeting siRNA expression cassette in the downstream of the human U6 promoter and 5 µL of the *Xho*I fragment (about 4.7 kb) derived from plasmid FT8libB/pAGE or FT8lib3/pAGE obtained above were mixed with 10 µL of Ligation High (manufactured by TOYOBO), the mixture was allowed to react at 16°C overnight. *E. coli* DH5α (manufactured by TOYOBO) was transformed with the reaction solution, and plasmid DNAs were isolated from the resulting ampicillin-resistant clones using QIAprep spin Mini prep Kit (manufactured by Qiagen). The nucleotide sequences of each plasmid were confirmed using DNA sequencer 377 (manufactured by Perkin Elmer) and BigDye Terminator v3.0 Cycle Sequencing Kit (manufactured by Applied Biosystems) according to the manufacturer's instruction. pPUR *Pvu*II-seq-F (SEQ ID NO:61), pPUR *Pvu*II-seq-R (SEQ ID NO:62), hu6pTsp45I/seq-F (SEQ ID NO:63), pAGE249-seqFW (SEQ ID NO:73) and pAGE249-seqRV (SEQ ID NO:74) were used as primers for sequence analysis. As a result of the sequence analysis, clones in which siRNA expression cassette sequence was correct and GMD-targeting siRNA expression cassette in the downstream of the human U6 promoters and FUT8-targeting siRNA expression cassette in the downstream of the human tRNA-val promoter were in the same direction was selected Hereinafter, plasmids FT8libB/pAGE and FT8lib3/pAGE inserted the GMD-targeting siRNA expression cassette in the downstream of the human hU6 promoters are referred to as "FT8libB_GMDB/pAGE" and "FT8lib3_GMDB/pAGE", respectively.

### 2. Obtaining and culturing of lectin-resistant clone CHO/DG44 by introducing GMD-targeting siRNA and FUT8-targeting siRNA co-expression vector

### (1) Obtaining of lectin-resistant clone by introducing GMD-targeting siRNA and FUT8-targeting siRNA co-expression vector

Lectin-resistant clones were obtained by introducing FT8libB_GMDB/pAGE or FT8lib3_GMDB/pAGE, the GMD-targeting siRNA and FUT8-targeting siRNA co-expression vector constructed in the item 1 of this Example into clone 32-05-12 according to the following procedure.

Transfection of plasmid FT8libB_GMDB/pAGE or FT8lib3_GMDB/pAGE into clone 32-05-12 was carried out in the same manner as in the item 2(1) of Example 1.

After the transfection, the cell suspension was suspended in a basal medium, and inoculated into 10 dishes of 10 cm for adherent cell culture (manufactured by Falcon). After culturing under conditions of 5% CO₂ and 37°C for 24 hours, the culture supernatant was removed, and a basal medium containing 500 µg/mL hygromycin (manufactured by WAKO) (hereinafter referred to as "Hyg500-IMDM medium") was added, followed by culturing for further 8 days, and appeared hygromycin-resistant colonies were counted. Furthermore, the culture supernatant was removed from some of the dishes, and LCA-IMDM medium [IMDM medium (manufactured by Invitrogen) containing 5% bovine fetal serum (manufactured by Invitrogen), 50 µg/mL gentamicin (manufactured by Nacalai Tesque), 500 nmol/L MTX (manufactured by SIGMA), 1 mmol/L L-Fucose (manufactured by Nacalai Tesque), and 500 µg/mL LCA (manufactured by VECTOR)] was added, followed by culturing for further 7 days. As a result, lectin-resistant clones were obtained at a high frequency by introducing either plasmid FT8libB_GMDB/pAGE or FT8lib3_GMDB/pAGE.

### (2) Expansion culture of lectin-resistant clones

Lectin-resistant clones into which GMD-targeting and FUT8-targeting siRNA co-expression vector was introduced, obtained in the item (1) were expansion cultured according to the following procedure.

First, the number of appeared colonies in each dish was counted. Then, lectin-resistant colonies were scraped and sucked up with a pipetteman (manufactured by GILSON) under observation with a stereoscopic microscope, and collected into a U-shaped-bottom 96-well plate for adherent cells (manufactured by ASAHI TECHNOGLASS). After trypsin treatment, each clone was inoculated into a flat-bottom 96-well plate for adherent cells (manufactured by Greiner), and cultured in an LCA-IMDM medium under conditions of 5% CO₂ and 37°C overnight. After the culturing, the culture supernatant was removed, and a new Hyg500-IMDM medium was added thereto, followed by culturing for further 9 days. After the culturing, each clone in the plate was expansion cultured in a Hyg500-IMDM medium. Hereinafter, lectin-resistant clones obtained by introducing plasmid FT8libB_GMDB/pAGE into clone 32-05-12 are referred to as "clone iBcho-H1", "clone iBcho-H2", "clone iBcho-H3", "clone iBcho-H4" or "clone iBcho-H5"; those obtained by introducing plasmid FT8lib3_GNMB/pAGE into clone 32-05-12 are referred to as "clone i3cho-H1", "clone i3cho-H2", "clone i3cho-H3", "clone i3cho-H4", "clone i3cho-H5", "clone i3cho-H6", "clone i3cho-H7" or "clone i3cho-H8", respectively.

### 3. Determination of the amount of GMD mRNA and the amount of FUT8 mRNA in lectin-resistant clone CHO/DG44 into which GMD-targeting siRNA and FUT8-targeting siRNA co-expression vector was introduced

### (1) Preparation of total RNA

A total RNA was prepared from clone 32-05-12 and lectin-resistant clones iBcho-H1, iBcho-H2, iBcho-H3, iBcho-H4, iBcho-H5, i3cho-H1, i3cho-H2, i3cho-H3, i3cho-H4, i3cho-H5, i3cho-H6, i3cho-H7 and i3cho-H8 into which the GMD-targeting siRNA and FUT8-targeting siRNA co-expression vector was introduced obtained in the item 2 of this Example according to the following procedure.

Clone 32-05-12 was suspended in a basal medium, and clones iBcho-H1, iBcho-H2, iBcho-H3, iBcho-H4, iBcho-H5, i3cho-H1, i3cho-H2, i3cho-H3, i3cho-H4, i3cho-H5, i3cho-H6, i3cho-H7 and i3cho-H8 were suspended in Hyg500-IMDM medium, at a density of 3×10⁵ cells/mL, and were inoculated at 2 mL into a 6 well plate for adherent cells (manufactured by Greiner). After culturing under conditions of 5% CO₂ and 37°C for 3 days, each cell was suspended by trypsin treatment, and collected by centrifugation at 1,000 rpm at 4°C for 5 minutes. After the cells were suspended in Dulbecco's PBS buffer (manufactured by Invitrogen) and re-centrifuged at 1,000 rpm at 4°C for 5 minutes to collect cells, a total RNA was each extracted using an RNeasy Mini Kit (manufactured by QIAGEN) according to the manufacturer's instruction. The prepared total RNA was dissolved in 40 µL of sterilized water.

### (2) Synthesis of single-stranded cDNA

A single-stranded cDNA was synthesized from 3 µg each of the total RNA obtained in the item (1) by reverse transcription reaction with oligo (dT) primer in a 20 µL, reaction system using a SuperScriptIII First-strand Synthesis System for RT-PCR (manufactured by Invitrogen) according to the manufacturer's instruction. The synthesized single-stranded cDNAs were treated with RNase and the final reaction volume was adjusted to 40 µL. In addition, each reaction solution was diluted 50-fold with sterilized water, and used for analysis of the amount of gene transcription described below.

### (3) Determination of the amount of GMD gene transcription by SYBR-PCR

The amount of mRNA transcribed from GMD and the amount of mRNA transcribed from β-actin genes were determined according to the procedure described in the item 2(3) of Example 5. As PCR primers, forward and reverse primers represented by SEQ ID NOs:80 and 81 were used to amplify GMD, and forward and reverse primers represented by SEQ ID NOs:66 and 67 were used to amplify β-actin, respectively. A calibration curve was obtained from measurements with the internal control plasmid, and the amount of GMD mRNA and the amount of β-actin mRNA were converted into numerical terms. When the relative amount of GMD mRNA to the amount of β-actin mRNA in clone 32-05-12 was assumed to be 100, the comparative results of the relative amount of GMD mRNA to the amount of β-actin mRNA are shown in Fig. 18. The amount of GMD mRNA in all the clones obtained by introducing GMD-targeting siRNA and FUT8-targeting siRNA co-expression vector were reduced to approximately 10% in comparison with that in the parent clone 32-05-12.

### (4) Determination of the amount of FUT8 gene transcription by SYBR-PCR

The amount of mRNA transcribed from FUT8 gene and the amount of mRNA transcribed from β-actin gene were determined according to the procedure described in the item 2(4) in Example 5. As PCR primers, forward and reverse primers represented by SEQ ID NOs:75 and 76 were used to amplify FUT8, and forward and reverse primers represented by SEQ ID NOs:66 and 67 were used to amplify β-actin, respectively. A calibration curve was obtained from measurements with the internal control plasmid, and the amount of FUT8 mRNA and the amount of β-actin mRNA were converted into numerical terms.

When the relative amount of GMD mRNA for the amount β-actin mRNA in clone 32-05-12 was assumed to be 100, the comparative results of the relative amount of FUT8 mRNA to β-actin mRNA are shown in Fig. 19. The amount of FUT8 mRNA in all the clones obtained by introducing GMD-targeting siRNA and FUT8-targeting siRNA co-expression vector were reduced to approximately 5% in comparison with that in the parent clone 32-05-12.

### 4. Production and analysis of antibody composition using lectin-resistant clone CHO/DG44 into which GMD-targeting siRNA and FUT8-targeting siRNA co-expression vector was introduced

### (1) Production of antibody composition by lectin-resistant clone CHO/DG44 into which GMD-targeting siRNA and FUT8-targeting siRNA co-expression vector was introduced

Anti-CCR4 chimeric antibody compositions produced by clone 32-05-12 and lectin-resistant clones iBcho-H2, iBcho-H3, i3cho-HI, i3cho-H2, i3cho-H3, i3cho-H4, i3cho-H5, i3cho-H6, i3cho-H7, and i3cho-H8 into which GMD-targeting siRNA and FUT8-targeting siRNA co-expression vector was introduced obtained in the item 2 of this Example were purified according to the following procedure.

Clone 32-05-12 was suspended in basal medium, and clones iBcho-H2, iBcho-H3, i3cho-H1, i3cho-H2, i3cho-H3, i3cho-H4, i3cho-H5, i3cho-H6, i3cho-H7, and i3cho-H8 were suspended in Hyg500-IMDM medium, at a density of 3×10⁵ cells/mL, and were inoculated at 10 mL into a T75 flask for adherent cells (manufactured by Greiner). After culturing under conditions of 5% CO₂ and 37°C for 5 days, the culture supernatant was removed, and after washing twice with 10 mL of Dulbecco's PBS (manufactured by Invitrogen), 24 mL of EXCELL301 medium (manufactured by JRH Bioscience) was added. After culturing under conditions of 5% CO₂ and 37°C for 7 days, the each culture supernatant was recovered, and anti-CCR4 chimeric antibody compositions were purified using a MabSelect column (manufactured by Amersham Bioscience) according to the manufacturer's instruction. After exchange with 10 mmol/L KH₂PO₄ buffer using Econo-Pac 10DG (manufactured by Bio Rad), anti-CCR4 chimeric antibody compositions purified from culture supernatants of various clones were subjected to sterile filtration by using Millex GV (manufactured by MILLIPORE) of 0.22µm pore size.

### (2) Composition analysis of monosaccharide of antibody compositions

Composition analysis of monosaccharide was carried out on the anti-CCR4 chimeric antibody compositions obtained in the item 4(1) of this Example according to the known method [Journal ofLiquid Chromatography, 6, 1577 (1983)].

Fucose(-)% calculated from the composition ratio of monosaccharide of each antibody are shown in Table 6. Also, when no fucose-derived peak was detected, fucose(-)% was regarded as 100%.

**Table 6**

| Fucose(-)% of anti-CCR4-chimeric antibody composition produced by each clone | |
|---|---|
| Clone | Fucose(-)% |
| 32-05-12 | 4% |
| iBcho-H2 | 100% |
| iBcho-H3 | 99% |
| i3cho-H1 | 100% |
| i3cho-H2 | 100% |
| i3cho-H3 | 100% |
| i3cho-H4 | 98% |
| i3cho-H5 | 100% |
| i3cho-H6 | 98% |
| i3cho-H7 | 100% |
| i3cho-H8 | 100% |

Fucose(-)% of antibody compositions produced by the parent clone 32-05-12 before vector introduction was 4%, while fucose(-)% of antibody compositions produced by lectin-resistant clones obtained by the introducing GMD-targeting siRNA and FUT8-targeting siRNA co-expression vector into the parent clone 32-05-12 were 98 to 100%, showing a significant increase in comparison with that of the parent clone. These results demonstrated that the introduction of the GMD-targeting siRNA and FUT8-targeting siRNA co-expression vector can convert CHO/DG44 cell into cells which produce antibody compositions with low fucose content.

### Example 9

### Production of antibody compositions using SP2/0 cell into which mouse GMD-targeting siRNA and FUT8-targeting siRNA co-expression vector was introduced

### 1. Construction of mouse GMD-targeting siRNA and mouse FUT8-targeting siRNA co-expression vector

### (1) Construction of mouse GMD-targeting siRNA expression vector in the downstream of the human U6 promoter

A mouse GMD gene-targeting siRNA expression vector was constructed according to the following procedure (Fig. 20):

First, a mouse sequence (SEQ ID NO:58) corresponding to the target sequence (SEQ ID NO:37) of the GMD-targeting siRNA expression vector (pPUR/GMDshB) which proved effective in Chinese hamster ovary-derived CHO/DG44 cell was selected as a target sequence. Then, a double-stranded DNA cassette was designed against the selected target sequence in the same manner as in the item 1(3) of Example 1. Sense (hereinafter referred to as "mGMD-B-F") and antisense (hereinafter referred to as "mGMD-B-R") strands of the designed synthetic oligo DNA were represented by SEQ ID NOs:82 and 83, respectively.

In addition, the nucleotide sequence of the plasmid DNA constructed by inserting double-stranded DNA, obtained by annealing the synthetic oligo DNA, into pPUR-U6term obtained in the item 1(2) of Example 1 was determined in the same manner as in the item 1(4) of Example 1 using DNA sequencer 377 (manufactured by Perkin Elmer) and BigDye Terminator v3.0 Cycle Sequencing Kit (manufactured by Applied Biosystems) according to the manufacturer's instruction. pPUR *Pvu*II-seq-F (SEQ ID NO:61) and pPUR *Pvu*II-seq-R (SEQ ID NO:62) were used as primers for sequence analysis, and the sequences of the inserted synthetic oligo DNA and ligation site were confirmed. Hereinafter, a plasmid inserted a double-stranded DNA obtained by annealing synthetic oligo DNA (mGMD-B-F and mGMD-B-R) are referred to as "pPUR/GMDmB".

### (2) Obtaining of mouse GMD-targeting siRNA expression unit in the downstream of the human U6 promoter

A mouse GMD-targeting siRNA expression cassette in the downstream of the human U6 promoter was obtained using pPUR/GMDmB constructed in the item (1) by the same method as in the item 1(1) of Example 6 (Fig. 16).

A plasmid DNA was isolated from a number of kanamycin-resistant colonies using QIAprep spin Mini prep Kit (manufactured by QIAGEN). Hereinafter, the plasmid is referred to as "pCR/GMDmB".

### (3) Construction of mouse GMD-targeting siRNA and FUT8-targeting siRNA co-expression vector

A mouse GMD-targeting siRNA and FUT8-targeting siRNA co-expression vector was constructed using plasmid pCR/GMDmB constructed in the item (2), and plasmid FT8libB/pAGE or FT8lib3/pAGE constructed in the item 4 of Example 2 in the same manner as in the item 1(2) of Example 6 (Fig. 17).

A plasmid DNA was isolated from the resulting ampicillin-resistant clones using QIAprep spin Mini prep Kit (manufactured by QIAGEN). The nucleotide sequence of each of the plasmid was confirmed using DNA sequencer 377 (manufactured by Perkin Elmer) and BigDye Terminator v3.0 Cycle Sequencing Kit (manufactured by Applied Biosystems) according to the manufacturer's instruction. pPUR *Pvu*II-seq-F (SEQ ID NO:61), pPUR *Pvu*II-seq-R (SEQ ID NO:62), hu6pTsp45I/seq-F (SEQ ID NO:63), pAGE249-seqFW (SEQ ID NO:73), and pAGE249-seqRV (SEQ ID NO:74) were used as primers for sequence analysis.

As a result of the sequence analysis, clones in which sequences of siRNA expression cassette were correct and mouse GMD-targeting siRNA expression cassette in the downstream of the human U6 promoter was inserted in the same direction as FUT8-targeting siRNA expression cassette in the downstream of the human tRNA-val promoter were selected. Hereinafter, plasmid FT8libB/pAGE into which mouse GMD-targeting siRNA expression cassette in the downstream of the human U6 promoter is introduced is referred to as "FT8libB_GMDmB/pAGE" and FT8lib3/pAGE inserted mouse GMD-targeting siRNA expression cassette under the human U6 promoters is referred to as "FT8lib38_GMDmB/pAGE", respectively.

### 2. Obtaining and culture of lectin-resistant cell line SP2/0 by introducing mouse GMD-targeting siRNA and mouse FUT8-targeting siRNA co-expression vector

Lectin-resistant clones were obtained by introducing FT8libB_GMDmB/pAGE or FT8lib3_GMDmB/pAGE, the mouse GMD-targeting and mouse FUT8-targeting siRNA co-expression vector obtained in the item 1 of this Example, into clone KM968 (hereinafter referred to as "clone KM968") which is a transformant of anti-GM₂ chimeric antibody-producing mouse myeloma SP2/0 cell (ATCC CRL-1581) described in Example 1 of Japanese Published Unexamined Patent Application No. 205694/94) according to the following procedure.

Transfection of various siRNA expression vector plasmids into clone KM968 was carried out according to the following procedure by electroporation [*Cytotechnology,* 3, 133 (1990)]:

First, 10 µg of each of various siRNA expression vector plasmids was dissolved in 30 µL of NEBuffer 4 (manufactured by New England Biolabs), and digested to be linearized with 10 units of a restriction enzyme *FspI* (manufactured by New England Biolabs) at 37°C overnight. After the linearized plasmid was confirmed by agarose gel electrophoresis using a part of the reaction solution, the remaining reaction solution was purified by phenol/chloroform extraction and ethanol precipitation, and the recovered linearized plasmid was dissolved in 10 µL of sterilized water.

Also, clone KM968 was suspended in a K-PBS buffer at 1.6×10⁷ cells/mL. After 200 µL of cell suspension (3.2×10⁶) was mixed with 10 µL of the above linearized plasmid solution, all of the cell/DNA mixture was transferred to Gene Pulser Cuvette (Electrode interval: 2 mm) (manufactured by BIO-RAD), and transfection was carried out under conditions of 200V pulse voltage and 250 µF capacitance using a cell fusion device Gene Pulser (manufactured by BIO-RAD).

After the transfection, the cell suspension was suspended in RPMI-FBS(10)-MTX(500) medium [RPMI1640 (manufactured by Invitrogen) containing 10% fetal bovine serum (manufactured by Invitrogen) and 500 nmol/L MTX (manufactured by SIGMA)], and inoculated into a T75 flask for suspension culture (manufactured by ASAHI TECHNOGLASS). After the culture under conditions of 5% CO₂ and 37°C for 24 hours, hygromycin (manufactured by WAKO) was added at the final concentration of 500 µg/mL, and the cells were cultured for further 3 days. After the culture, the cells were collected by centrifugation at 800 rpm for 5 minutes, and suspended in an RPMI-FBS(10)-MTX(500) medium containing 500 µg/mL hygromycin [hereinafter referred to as "RPMI-FBS(10)-MTX(500)-Hyg(500) medium"] at a density of 0.5 to 1×10⁴ viable cells/mL, and were inoculated at 100 µL into a 96-well culture plate (manufactured by Greiner). Subsequently, the cells were cultured for 2 weeks, while half the volume of the culture supernatant was routinely replaced by a new RPMI-FBS(10)-MTX(500)-Hyg(500) medium once every 2 to 3 days.

After the culture, the cells in each well of the 96-well culture plate were divided into two 96-well culture plates; one for a master plate, and another for a replica plate. The master and replica plates were each cultured for 3 days in an RPMI-FBS(10)-MTX(500)-Hyg(500) medium and RPMI-FBS(10)-MTX(500)-Hyg(500) medium containing 1 mmol/L L-fucose (manufactured by Nacalai Tesque) and 500 µg/mL LCA (manufactured by VECTOR), respectively. As a result, excellent growth in the presence of LCA was confirmed in approximately 30% of the wells of the replica plate corresponding to wells of the master plate which showed excellent growth.

Cells in the wells of the master plate, which corresponded to wells of the replica plate which showed excellent growth, were expansion cultured in an RPMI-FBS(10)-MTX(500)-Hyg(500) medium. Hereinafter, among clones used for the expansion culture, those obtained by introducing FT8libB_GMDmB/pAGE are referred to as "clone 968iB-1", "clone 968iB-2", "clone 968iB-3", "clone 968iB-4", "clone 968iB-5", "clone 968iB-6" and "clone 968iB-7"; those obtained by introducing FT8lib3_GMDmB/pAGE are referred to as "clone 968i3-1", "clone 968i3-2", "clone 968i3-3", "clone 968i3-4" ,"clone 968i3-5", "clone 968i3-6", "clone 968i3-7", "clone 968i3-8", "clone 968i3-9" and "clone 968i3-10", respectively.

### 3. Determination of the amount of GMD mRNA and the amount of FUT8 mRNA in lectin-resistant SP2/0 cell into which mouse GMD-targeting siRNA and mouse FUT8-targeting siRNA co-expression vector was introduced

### (1) Preparation of total RNA

A total RNA was prepared from clone KM968, and clones 968iB-1, 968iB-2, 968iB-3, 968iB-4, 968iB-5, 968iB-6, 968iB-7, 968i3-1, 968i3-2, 968i3-3, 968i3-4, 968i3-5, 968i3-6, 968i3-7, 968i3-8, 968i3-9 and 968i3-10 which are lectin-resistant cell line SP2/0 into which the mouse GMD-targeting siRNA and mouse FUT8-targeting siRNA co-expression vector was introduced obtained in item 2 of this Example according to the following procedure.

Clone KM968 was suspended in an RPMI-FBS(10)-MTX(500) medium, and lectin-resistant cell line SP2/0 into which the mouse GMD-targeting siRNA and mouse FUT8-targeting siRNA co-expression vector was introduced were suspended in RPMI-FBS(10)-MTX(500)-Hyg(500) medium at a density of 1×10⁵ cells/mL, and inoculated into a T75 flask for suspension culture (manufactured by ASAHI TECHNOGLASS). After the culture under conditions of 5% CO₂ and 37°C for 3 days, the cells were counted, and 5×10⁵ cells/mL each was collected. The cells were recovered by centrifugation at 800 rpm for 5 minutes. After the recovered cells were suspended in Dulbecco's PBS buffer (manufactured by Invitrogen) and re-centrifuged at 800 rpm for 5 minutes to recover cells, total RNA was each extracted using an RNeasy Micro Kit (manufactured by QIAGEN) according to the manufacturer's instruction. Each prepared total RNA was dissolved in 14 µL of sterilized water.

### (2) Synthesis of single-stranded cDNA

A single-stranded cDNA was synthesized from 3 µg of each of the total RNAs obtained in the item (1) by reverse transcription reaction with oligo (dT) primer in 20 µL reaction system using SuperScriptIII First-strand Synthesis System for RT-PCR (manufactured by Invitrogen) according to the manufacturer's instruction. The synthesized single-stranded cDNAs were each treated with RNase and the final reaction volume was adjusted to 40 µL. Then, each of the reaction solutions was diluted 50-fold with sterilized water, and used for analysis of the amount of gene transcription described below.

### (3) Determination of the amount of GMD gene transcription by SYBR-PCR

The amount of mRNA transcribed from GMD gene and the amount of mRNA transcribed from β-actin gene were determined according to the procedure described in the item 3(3) of Example 8. A calibration curve was obtained from measurements with the internal control plasmid, and the amount of GMD mRNA and the amount of β-actin mRNA were converted into numerical terms.

When the relative amount of GMD mRNA to the amount of β-actin mRNA in clone KM968 was assumed to be 100, the comparative results of the relative mRNA amounts of GMD to the mRNA amount of β-actin are shown in Fig. 21. The amount of GMD mRNA of all the clones obtained by introducing the mouse GMD-targeting siRNA and mouse FUT8-targeting siRNA co-expression vector were reduced to approximately 10% in comparison with that of the parent clone KM968.

### (4) Determination of the amount of FUT8 gene transcription by SYBR-PCR

The amount of mRNA transcribed from FUT8 and the amount of mRNA transcribed from β-actin gene were determined according to the procedure described in the item 3(4) of Example 8. A calibration curve was obtained from measurements with the internal control plasmid, and determination of the amount of FUT8 mRNA and the amount of β-actin mRNA were converted into numerical terms.

When the relative amount FUT8 mRNA to the amount of β-actin mRNA in clone KM968 was assumed to be 100, the comparative results of the amount of FUT8 mRNA relative to the amount β-actin mRNA are shown in Fig. 22. The amount of FUT8 mRNA of all the clones obtained by introducing the mouse GMD-targeting siRNA and mouse FUT8-targeting siRNA co-expression vector were reduced to approximately 10% in comparison with that in the parent clone KM968.

### 4. Production and analysis of antibody composition using lectin-resistant cell line SP2/0 into which mouse GMD-targeting siRNA and mouse FUT8-targeting siRNA co-expression vector was introduced

### (1) Production of antibody composition by lectin-resistant cell line SP2/0 into which mouse GMD-targeting siRNA and mouse FUT8-targeting siRNA co-expression vector was introduced

Anti-GM₂ chimeric antibody composition produced by clone KM968 and lectin-resistant clones 968i3-2 and 968i3-3 which is cell line SP2/0 into which the mouse GMD-targeting siRNA and mouse FUT8-targeting siRNA co-expression vector was introduced obtained in the item 2 of this Example, were each purified according to the following procedure.

Clone KM968 was suspended in SP-HSFM medium [Hybridoma-SFM medium containing 5% UltraLow-IgG FBS (manufactured by Invitrogen) and 500 nmol/L MTX (manufactured by SIGMA)], and clones 968i3-2 and 968i3-3 were suspended in SP-HSFM medium containing 500 µg/mL hygromycin (manufactured by WAKO), at a density of 1×10⁵ cells/mL, and were inoculated at 50 mL into a T225 flask for suspension culture (manufactured by ASAHI TECHNOGLASS). After the culture under conditions of 5% CO₂ and 37°C for 7 days, culture supernatants were each recovered, and anti-GM₂ chimeric antibody compositions were purified using a MabSelect column (manufactured by Amersham Bioscience) according to the manufacturer's instruction. After exchange 10 mmol/L KH₂PO₄ buffer using Econo-Pac 10DG (manufactured by Bio Rad), anti-CCR4 chimeric antibodies purified from culture supernatants of various clones were subjected to sterile filtration by using Millex GV (manufactured by MILLIPORE) of 0.22µm pore size.

### (2) Composition analysis of monosaccharide of antibody compositions

Composition analysis of monosaccharide was carried out for the anti-GM₂ chimeric antibody compositions obtained in the item 4(1) of this Example according to the known method [Journal of Liquid Chromatography, 6, 1577 (1983)].

Fucose(-)% calculated from the composition ratio of monosaccharide of each antibody are shown in Table 7.

**Table 7**

| Fucose(-)% of anti-GM₂ chimeric antibody produced by each clone | |
|---|---|
| Clone | Ratio of sugar chains to which fucose is not bound (%) |
| KM968 | 3% |
| 968i3-2 | 64% |
| 968i3-3 | 62% |

Fucose(-)% of antibody compositions produced by clone KM968 was 3%, while fucose(-)% of antibody compositions produced by lectin-resistant clones 968i3-2 and 968i3-3 obtained by introducing the mouse GMD-targeting siRNA and mouse FUT8-targeting siRNA co-expression vector into the parent clone KM968 were approximately 60%, showing a significant increase in comparison with that of the parent clone KM968. These results demonstrated that the introduction of the GMD-targeting siRNA and FUT8-targeting siRNA co-expression vector can convert SP2/0 cell into cells which produce antibody compositions with low fucose content.

### Example 10

### Production of antibody compositions using NS0 cell into which mouse GMD-targeting siRNA and mouse FUT8-targeting siRNA co-expression vector was introduced

### 1. Obtaining and culturing of cell line NS0 into which mouse GMD-targeting siRNA and mouse FUT8-targeting siRNA co-expression vector was introduced

Transformants were obtained by introducing FT8libB_GMDmB/pAGE or FT8lib3_GMDmB/pAGE which is a mouse GMD-targeting siRNA and mouse FUT8-targeting siRNA co-expression vector obtained in the item 1 of Example 7 into mouse myeloma NS0 cell (RCB0213)-derived anti-CCR4 chimeric antibody-producing clone NS0/2160 (hereinafter referred to as "clone NS0/2160") which was obtained in the same manner as in item (2) of Example 1 of WO03/85118 according to the following procedure.

Transfection of various siRNA expression vector plasmids into clone NS0/2160 was carried out according to the following procedure by electroporation [Cytotechnology, 3, 133 (1990)].

First, 10 µg of each of various siRNA expression vector plasmids was dissolved in 30 µL of NEBuffer 4 (manufactured by New England Biolabs), and digested to be linearized with 10 units of a restriction enzyme *Fsp*I (manufactured by New England Biolabs) at 37°C overnight. After the linearized plasmid was confirmed by agarose gel electrophoresis using a part of the reaction solution, the remaining reaction solution was purified by phenol/chloroform extraction and ethanol precipitation, and the recovered linearized plasmid was dissolved in 10 µL, of sterilized water.

Also, clone NS0/2160 was suspended in a K-PBS buffer at 1×10⁷ cells/mL. After 200 µL of cell suspension (2×10⁶) was mixed with 10 µL of the linearized plasmid solution, all of the cell/DNA mixture was transferred to Gene Pulser Cuvette (Electrode interval: 2 mm) (manufactured by BIO-RAD), and transfection was carried out under conditions of 200V pulse voltage and 250 µF capacitance using a cell fusion device Gene Pulser (manufactured by BIO-RAD).

After the transfection, the cell suspension were suspended in an RPMI-FBS(10)-MTX(500) medium, and inoculated into a T75 flask for suspension culture (manufactured by ASAHI TECHNOGLASS). After the culture under conditions of 5% CO₂ and 37°C for 24 hours, hygromycin (manufactured by WAKO) was added at the final concentration of 500 µg/mL, and cells were cultured for further 2 days. After the culture, cells were collected by centrifugation at 800 rpm for 5 minutes, and suspended in RPMI-FBS(10)-MTX(500)-Hyg(500) at a density of 0.5 to 1×10⁴ living cells/mL, and were inoculated at 100 µL into a 96-well culture plate (manufactured by Greiner). Subsequently, half the volume of the culture supernatant was routinely replaced by a new RPMI-FBS(10)-MTX(500)-Hyg(500) medium once every 2 to 3 days, and the cells were cultured for 2 to 3 weeks.

After the culture, culture supernatants in each well of the 96-well culture plates were collected, and the binding activities of anti-CCR4 chimeric antibody compositions, contained in each culture supernatant, to shFcγRIIIa were evaluated by the method described in the item 3 of Example 7. As a result of the analysis on 80 wells for each vector, for antibody compositions of the culture supernatants in 90% or more of the wells increased binding activities to shFcγRIIIa were confirmed in comparison with those of parent clone NS0/2160, demonstrating that cells in each well was converted into cells which produce antibody compositions with low fucose content.

Cells in 5 wells that showed increased binding activities to shFcγRIIIa were expansion cultured in the RPMI-FBS(10)-MTX(500)-Hyg(500) medium. Hereinafter, among clones used for the expansion culture, those obtained by introducing FT8libB_GMDmB/pAGE are referred to as "clone NS0/2160iB-1", "clone NS0/2160iB-2", "clone NS0/2160iB-3", "clone NS0/2160iB-4" and "clone NS0/2160iB-5"; those by introducing FT8lib3_GMDmB/pAGE are referred to as "clone NS0/2160i3-1", "clone NS0/2160i3-2", "clone NS0/2160i3-3", "clone NS0/2160i3-4" and "clone NS0/2160i3-5", respectively.

### 2. Determination of the amount of GMD mRNA and the amount of FUT8 mRNA in lectin-resistant NS0 cells into which mouse GMD-targeting siRNA and mouse FUT8-targeting siRNA co-expression vector was introduced

### (1) Preparation of total RNA

A total RNA was prepared from clone NS0/2160, and clones NS0/2160iB-1, NS0/2160iB-2, NS0/2160iB-3, NS0/2160iB-4, NS0/2160iB-5, NS0/2160i3-1, NS0/2160i3-2, NS0/2160i3-3, NS0/2160i3-4 and NS0/2160i3-5 which are cell line NS0 into which the mouse GMD-targeting siRNA and mouse FUT8-targeting siRNA co-expression vector was introduced obtained in the item 1 of this Example according to the following procedure.

Clone NS0/2160 was suspended in an RPMI-FBS(10)-MTX(500) medium, and cell line NS0 into which a mouse GMD-targeting siRNA and mouse FUT8-targeting siRNA co-expression vector was introduced was suspended in an RPMI-FBS(10)-MTX(500)-Hyg(500) medium, at a density of 1×10⁵ cells/mL, and inoculated into a T75 flask for suspension culture (manufactured by ASAHI TECHNOGLASS). After culturing under conditions of 5% CO₂ and 37°C for 3 days, the cells were counted and 5×10⁵ cells were each collected. The supernatant was removed by centrifugation at 800 rpm for 5 minutes. After the cells were suspended in Dulbecco's PBS buffer (manufactured by Invitrogen) and re-centrifuged at 800 rpm for 5 minutes to recover cells, total RNA was each extracted using an RNeasy Micro Kit (manufactured by QIAGEN) according to the manufacturer's instruction. A total RNA prepared was dissolved in 14 µL of sterilized water.

### (2) Synthesis of single-stranded cDNA

A single-stranded cDNA was synthesized from 3 µg each of the total RNA obtained in the item (1) by reverse transcription reaction with oligo (dT) primer in 20 µL reaction system using SuperScriptIII First-strand Synthesis System for RT-PCR (manufactured by Invitrogen) according to the manufacturer's instruction. The synthesized single-stranded cDNAs were treated with RNase and the final reaction volume was adjusted to 40 µL. Then, each of the reaction solutions was diluted 50-fold with sterilized water, and used for analysis of the amount of gene transcription described below.

### (3) Determination of the amount of GMD gene transcription by SYBR-PCR

The amount of mRNA transcribed from GMD gene and the amount of mRNA transcribed from β-actin gene were determined according to the procedure described in the item 3(3) of Example 8. A calibration curve was obtained from measurements with the internal control plasmid, and the amount of GMD mRNA and the amount of β-actin mRNA were converted into numerical terms.

When the relative amount of GMD mRNA to the amount of β-actin mRNA in clone NS0/2160 was assumed to be 100, the comparative results of the relative amount of GMD mRNA to the amount of β-actin mRNA are shown in Fig. 23. The amount of GMD mRNA in all the clones obtained by introducing the mouse GMD-targeting siRNA and mouse FUT8-targeting siRNA co-expression vector were reduced to approximately 20% in comparison with that in the parent clone NS0/2160.

### (4) Determination of the amount of FUT8 gene transcription by SYBR-PCR

The amount of mRNA transcribed from FUT8 gene and the amount of mRNA transcribed β-actin gene were determined according to the procedure described in the item 3(4) of Example 8. A calibration curve was obtained from measurements with the internal control plasmid, and the amount of FUT8 mRNA and the amount of β-actin mRNA were converted into numerical terms.

When the relative amount of FUT8 mRNA to the amount of β-actin mRNA in clone NS0/2160 was assumed to be 100, the comparative results of the relative amounts of FUT8 mRNA to the amount of β-actin mRNA are shown in Fig. 24. The amount of FUT8 mRNA in all the clones obtained by introducing the mouse GMD-targeting siRNA and mouse FUT8-targeting siRNA co-expression vector were reduced to approximately 10% in comparison with that in the parent clone KM968.

### 3. Production and analysis of antibody composition using NS0 cell into which mouse GMD-targeting siRNA and mouse FUT8-targeting siRNA co-expression vector was introduced

### (1) Production of antibody composition by antibody with low fucose content producing cell line NS0 into which mouse GMD-targeting siRNA and mouse FUT8-targeting siRNA co-expression vector was introduced

Anti-CCR4 chimeric antibody compositions produced by clone NS0/2160, and clones NS0/2160iB-3, NS0/2160iB-5, NS0/2160i3-1, NS0/2160i3-4 and NS0/2160i3-5 which are cell line NS0 into which the mouse GMD-targeting siRNA and mouse FUT8-targeting siRNA co-expression vector was introduced in the item 1 of this Example were purified according to the following procedure.

Clone NS0/2160 was suspended in NS-HSFM medium [Hybridoma-SFM medium (manufactured by Invitrogen) containing 0.2% bovine serum albumin (manufactured by Invitrogen) and 500 nmol/L MTX (manufactured by SIGMA)], and clones NS0/2160iB- NS0/2160iB NS0/2160i3 NS0/2160i3-4 and NS0/2160i3-5 were suspended in NS-HSFM medium containing 500 µg/mL hygromycin (manufactured by WAKO), at a density of 2×10⁵ cells/mL, and were inoculated at 40 mL into a T225 flask for suspension culture (manufactured by ASAHI TECHNOGLASS). After the culture under conditions of 5% CO₂ and 37°C for 7 days, each culture supernatant was recovered, and anti-CCR4 chimeric antibody compositions were purified using a MabSelect column (manufactured by Amersham Bioscience) according to the manufacturer's instruction. After exchange with 10 mmol/L KH₂PO₄ buffer using Econo-Pac 10DG (manufactured by Bio Rad), anti-CCR4 chimeric antibody compositions purified from culture supernatants of various clones were subjected to sterile filtration by using Millex GV (manufactured by MILLIPORE) of 0.22 µm pore size.

### (2) Composition analysis of monosaccharide of antibody compositions

Composition analysis of monosaccharide was carried out for the anti-CCR4 chimeric antibody compositions obtained in the item 3(1) of this Example according to the known method [Journal ofLiquid Chromatography, 6, 1577 (1983)].

Fucose(-)% calculated from the composition ratio of monosaccharide of each antibody composition are shown in Table 8.

**Table 8**

| Fucose(-)% of anti-CCR4 chimeric antibody composition produced by each clone | |
|---|---|
| Clone | Fucose(-)% |
| NS0/2160 | 28% |
| NS0/2160iB-3 | 93% |
| NS0/2160iB-5 | 92% |
| NS0/2160i3-1 | 93% |
| NS0/2160i3-4 | 93% |
| NS0/2160i3-5 | 92% |

Fucose(-)% of the antibody compositions produced by clone NS0/2160 was 28%, while fucose(-)% of the antibody compositions produced by clones NS0/2160iB-3, NS0/2160iB-5, NS0/2160i3-1, NS0/2160i3-4 and NS0/2160i3-5 which were obtained by introducing the mouse GMD-targeting siRNA and mouse FUT8-targeting siRNA co-expression vector into the parent clone NS0/2160 were approximately 90%, showing a significant increase in comparison with that of the parent cell. These results demonstrated that the introduction of the GMD-targeting siRNA and FUT8-targeting siRNA co-expression vector can convert NS0 cells into cells which produce antibody with low fucose content.

### INDUSTRIAL APPLICABILITY

The present invention provides a cell into which an RNA capable of suppressing the function of an enzyme catalyzing a reaction which converts GDP-mannose into GDP-4-keto,6-deoxy-GDP-mannose is introduced; a process for producing a glycoprotein using the cell; a cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme protein relating to synthesis of an intracellular sugar nucleotide, GDP-fucose, or an RNA capable of suppressing the function of a protein relating to transport of an intracellular sugar nucleotide, GDP-fucose, to the Golgi body are introduced; a process for producing a glycoprotein composition using the cell; and the like. The antibody composition produced by the process of the present invention has high effector activity and is useful as a medicament.

### Free text of sequence listing

SEQ ID NO:14 - Description of Artificial Sequence: Synthetic RNA
SEQ ID NO:15 - Description of Artificial Sequence: Synthetic RNA
SEQ ID NO:16 - Description of Artificial Sequence: Synthetic RNA
SEQ ID NO:17 - Description of Artificial Sequence: Synthetic RNA
SEQ ID NO:18 - Description of Artificial Sequence: Synthetic RNA
SEQ ID NO:19 - Description of Artificial Sequence: Synthetic RNA
SEQ ID NO:20 - Description of Artificial Sequence: Synthetic RNA
SEQ ID NO:21 - Description of Artificial Sequence: Synthetic RNA
SEQ ID NO:22 - Description of Artificial Sequence: Synthetic RNA
SEQ ID NO:23 - Description of Artificial Sequence: Synthetic RNA
SEQ ID NO:24 - Description of Artificial Sequence: Synthetic RNA
SEQ ID NO:25 - Description of Artificial Sequence: Synthetic RNA
SEQ ID NO:26 - Description of Artificial Sequence: Synthetic RNA
SEQ ID NO:27 - Description of Artificial Sequence: Synthetic RNA
SEQ ID NO:28 - Description of Artificial Sequence: Synthetic RNA
SEQ ID NO:29 - Description of Artificial Sequence: Synthetic RNA
SEQ ID NO:30 - Description of Artificial Sequence: Synthetic RNA
SEQ ID NO:31 - Description of Artificial Sequence: Synthetic RNA
SEQ ID NO:32 - Description of Artificial Sequence: Synthetic RNA
SEQ ID NO:33 - Description of Artificial Sequence: Synthetic RNA
SEQ ID NO:34 - Description of Artificial Sequence: Synthetic RNA
SEQ ID NO:35 - Description of Artificial Sequence: Synthetic RNA
SEQ ID NO:36 - Description of Artificial Sequence: Synthetic RNA
SEQ ID NO:37 - Description of Artificial Sequence: Synthetic RNA
SEQ ID NO:38 - Description of Artificial Sequence: Synthetic RNA
SEQ ID NO:39 - Description of Artificial Sequence: Synthetic RNA
SEQ ID NO:40 - Description of Artificial Sequence: Synthetic RNA
SEQ ID NO:41 - Description of Artificial Sequence: Synthetic RNA
SEQ ID N0:42 - Description of Artificial Sequence: Synthetic RNA
SEQ ID NO:43 - Description of Artificial Sequence: Synthetic DNA
SEQ ID NO:44 - Description of Artificial Sequence: Synthetic DNA
SEQ ID NO:45 - Description of Artificial Sequence: Synthetic DNA
SEQ ID NO:46 - Description of Artificial Sequence: Synthetic DNA
SEQ ID NO:47 - Description of Artificial Sequence: Synthetic DNA
SEQ ID NO:48 - Description of Artificial Sequence: Synthetic DNA
SEQ ID NO:49 - Description of Artificial Sequence: Synthetic DNA
SEQ ID NO:50 - Description of Artificial Sequence: Synthetic DNA
SEQ ID NO:51 - Description of Artificial Sequence: Synthetic DNA
SEQ ID NO:52 - Description of Artificial Sequence: Synthetic DNA
SEQ ID NO:53 - Description of Artificial Sequence: Synthetic DNA
SEQ ID NO:54 - Description of Artificial Sequence: Synthetic DNA
SEQ ID NO:55 - Description of Artificial Sequence: Synthetic DNA
SEQ ID NO:56 - Description of Artificial Sequence: Synthetic DNA
SEQ ID NO:57 - Description of Artificial Sequence: Synthetic RNA
SEQ ID NO:58 - Description of Artificial Sequence: Synthetic RNA
SEQ ID NO:59 - Description of Artificial Sequence: Synthetic DNA .
SEQ ID NO:60 - Description of Artificial Sequence: Synthetic DNA
SEQ ID NO:61 - Description of Artificial Sequence: Synthetic DNA
SEQ ID N0:62 - Description of Artificial Sequence: Synthetic DNA
SEQ ID NO:63 - Description of Artificial Sequence: Synthetic DNA
SEQ ID NO:64 - Description of Artificial Sequence: Synthetic DNA
SEQ ID NO:65 - Description of Artificial Sequence: Synthetic DNA
SEQ ID NO:66 - Description of Artificial Sequence: Synthetic DNA
SEQ ID NO:67 - Description of Artificial Sequence: Synthetic RNA
SEQ ID NO:68 - Description of Artificial Sequence: Synthetic RNA
SEQ ID NO: 69 - Description of Artificial Sequence: Synthetic DNA
SEQ ID NO:70 - Description of Artificial Sequence: Synthetic DNA
SEQ ID NO:71 - Description of Artificial Sequence: Synthetic DNA
SEQ ID NO:72 - Description of Artificial Sequence: Synthetic DNA
SEQ ID NO:73 - Description of Artificial Sequence: Synthetic DNA
SEQ ID NO:74 - Description of Artificial Sequence: Synthetic DNA
SEQ ID NO:75 - Description of Artificial Sequence: Synthetic DNA
SEQ ID NO:76 - Description of Artificial Sequence: Synthetic DNA
SEQ ID NO:77 - Description of Artificial Sequence: Synthetic Peptide
SEQ ID NO:78 - Description of Artificial Sequence: Synthetic DNA
SEQ ID NO:79 - Description of Artificial Sequence: Synthetic DNA
SEQ ID NO:80 - Description of Artificial Sequence: Synthetic DNA
SEQ ID NO:81 - Description of Artificial Sequence: Synthetic DNA
SEQ ID NO:82 - Description of Artificial Sequence: Synthetic DNA
SEQ ID NO:83 - Description of Artificial Sequence: Synthetic DNA
SEQ ID NO:85 - Description of Artificial Sequence: Synthetic RNA
SEQ ID NO:86 - Description of Artificial Sequence: Synthetic RNA
SEQ ID NO:87 - Description of Artificial Sequence: Synthetic RNA
SEQ ID NO:88 - Description of Artificial Sequence: Synthetic RNA
SEQ ID NO:89 - Description of Artificial Sequence: Synthetic RNA
SEQ ID NO:90 - Description of Artificial Sequence: Synthetic DNA
SEQ ID NO:91 - Description of Artificial Sequence: Synthetic DNA
SEQ ID NO:92 - Description of Artificial Sequence: Synthetic DNA
SEQ ID NO:93 - Description of Artificial Sequence: Synthetic DNA
SEQ ID NO:94 - Description of Artificial Sequence: Synthetic DNA
SEQ ID NO:95 - Description of Artificial Sequence: Synthetic DNA
SEQ ID NO:96 - Description of Artificial Sequence: Synthetic DNA

## Claims

1. A cell into which a double-stranded RNA comprising an RNA selected from the following (a) or (b) and its complementary RNA are introduced:
(a) an RNA comprising the nucleotide sequence represented by SEQ ID NO:37, 57 or 58;
(b) an RNA consisting of a nucleotide sequence in which one or a several nucleotide(s) is/are deleted, substituted, inserted and/or added in the nucleotide sequence represented by SEQ ID NO:37, 57 or 58 and having activity of suppressing the function of an enzyme catalyzing a reaction which converts GDP-mannose into GDP-4-keto,6-deoxy-GDP-mannose.

2. The cell according to claim 1, wherein the enzyme catalyzing a dehydration reaction which converts GDP-mannose into GDP-4-keto,6-deoxy-GDP-mannose is GDP-mannose 4,6-dehydratase.

3. The cell according to claim 2, wherein the GDP-mannose 4,6-dehydratase is a protein encoded by a DNA selected from the group consisting of the following (a) to (f):
(a) a DNA comprising the nucleotide sequence represented by SEQ ID NO:8;
(b) a DNA comprising the nucleotide sequence represented by SEQ ID NO:9;
(c) a DNA comprising the nucleotide sequence represented by SEQ ID NO:10;
(d) a DNA which hybridizes with a DNA consisting of the nucleotide sequence represented by SEQ ID NO:8 under stringent conditions and encodes a protein having GDP-mannose 4,6-dehydratase activity;
(e) a DNA which hybridizes with a DNA consisting of the nucleotide sequence represented by SEQ ID NO:9 under stringent conditions and encodes a protein having GDP-mannose 4,6-dehydratase activity;
(f) a DNA which hybridizes with a DNA consisting of the nucleotide sequence represented by SEQ ID NO:10 under stringent conditions and encodes a protein having GDP-mannose 4,6-dehydratase activity.

4. The cell according to claim 2, wherein the GDP-mannose 4,6-dehydratase is a protein selected from the group consisting of the following (a) to (i):
(a) a protein comprising the amino acid sequence represented by SEQ ID NO:11;
(b) a protein comprising the amino acid sequence represented by SEQ ID NO:12;
(c) a protein comprising the amino acid sequence represented by SEQ ID NO:13;
(d) a protein consisting of an amino acid sequence in which one or more amino acid(s) is/are deleted, substituted, inserted and/or added in the amino acid sequence represented by SEQ ID NO:11 and having GDP-mannose 4,6-dehydratase activity;
(e) a protein consisting of an amino acid sequence in which one or more amino acid(s) is/are deleted, substituted, inserted and/or added in the amino acid sequence represented by SEQ ID NO:12 and having GDP-mannose 4,6-dehydratase activity;
(f) a protein consisting of an amino acid sequence in which one or more amino acid(s) is/are deleted, substituted, inserted and/or added in the amino acid sequence represented by SEQ ID NO:13 and having GDP-mannose 4,6-dehydratase activity;
(g) a protein consisting of an amino acid sequence which has 80% or more homology to the amino acid sequence represented by SEQ ID NO:11 and having GDP-mannose 4,6-dehydratase activity;
(h) a protein consisting of an amino acid sequence which has 80% or more homology to the amino acid sequence represented by SEQ ID NO: 12 and having GDP-mannose 4,6-dehydratase activity;
(i) a protein consisting of an amino acid sequence which has 80% or more homology to the amino acid sequence represented by SEQ ID NO:13 and having GDP-mannose 4,6-dehydratase activity.

5. A double-stranded RNA comprising an RNA selected from the following (a) or (b) and its complementary RNA:
(a) an RNA comprising the nucleotide sequence represented by SEQ ID NO:37, 57 or 58;
(b) an RNA consisting of a nucleotide sequence in which one or a several nucleotide(s) is/are deleted, substituted, inserted and/or added in the nucleotide sequence represented by SEQ ID NO:37, 57 or 58 and having activity of suppressing the function of an enzyme catalyzing a reaction which converts GDP-mannose into GDP-4-keto,6-deoxy-GDP-mannose.

6. A DNA corresponding to the RNA described in claim 5 and its complementary DNA.

7. A vector comprising a DNA corresponding to the RNA described in claim 5.

8. A cell into which the vector described in claim 7 is introduced.

9. A cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, or an RNA capable of suppressing the function of a protein relating to transport of an intracellular sugar nucleotide, GDP-fucose, to the Golgi body are introduced.

10. A cell into which an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain, and an RNA capable of suppressing the function of an enzyme relating to synthesis of an intracellular sugar nucleotide, GDP-fucose, are introduced.

11. The cell according to claim 9 or 10, wherein the enzyme relating to synthesis of an intracellular sugar nucleotide, GDP-fucose, is an enzyme catalyzing a reaction which converts GDP-mannose into GDP-4-keto,6-deoxy-GDP-mannose.

12. The cell according to any one of claims 9 to 11, wherein the enzyme relating to modification of a sugar chain in which 1-position of fucose is bound to 6-position of N-acetylglucosamine in the reducing end through α-bond in the complex type N-glycoside-linked sugar chain is α1,6-fucosyltransferase.

13. The cell according to claim 12, wherein the α1,6-fucosyltransferase is a protein encoded by a DNA selected from the group consisting of (a) to (h):
(a) a DNA comprising the nucleotide sequence represented by SEQ ID NO:1;
(b) a DNA comprising the nucleotide sequence represented by SEQ ID NO:2;
(c) a DNA comprising the nucleotide sequence represented by SEQ ID NO:3;
(d) a DNA comprising the nucleotide sequence represented by SEQ ID NO:4;
(e) a DNA which hybridizes with a DNA consisting of the nucleotide sequence represented by SEQ ID NO:1 under stringent conditions and encodes a protein having α1,6-fucosyltransferase activity;
(f) a DNA which hybridizes with a DNA consisting of the nucleotide sequence represented by SEQ ID NO:2 under stringent conditions and encodes a protein having α1,6-fucosyltransferase activity;
(g) a DNA which hybridizes with a DNA consisting of the nucleotide sequence represented by SEQ ID NO:3 under stringent conditions and encodes a protein having α1,6-fucosyltransferase activity;
(h) a DNA which hybridizes with a DNA consisting of the nucleotide sequence represented by SEQ ID NO:4 under stringent conditions and encodes a protein having α1,6-fucosyltransferase activity.

14. The cell according to claim 12, wherein the α1,6-fucosyltransferase is a protein selected from the group consisting of the following (a) to (l):
(a) a protein comprising the amino acid sequence represented by SEQ ID NO:5;
(b) a protein comprising the amino acid sequence represented by SEQ ID NO:6;
(c) a protein comprising the amino acid sequence represented by SEQ ID NO:7;
(d) a protein comprising the amino acid sequence represented by SEQ ID NO:84;
(e) a protein consisting of an amino acid sequence in which one or more amino acid(s) is/are deleted, substituted, inserted and/or added in the amino acid sequence represented by SEQ ID NO:5 and having α1,6-fucosyltransferase activity;
(f) a protein consisting of an amino acid sequence in which one or more amino acid(s) is/are deleted, substituted, inserted and/or added in the amino acid sequence represented by SEQ ID NO:6 and having α1,6-fucosyltransferase activity;
(g) a protein consisting of an amino acid sequence in which one or more amino acid(s) is/are deleted, substituted, inserted and/or added in the amino acid sequence represented by SEQ ID NO:7 and having α1,6-fucosyltransferase activity;
(h) a protein consisting of an amino acid sequence in which one or more amino acid(s) is/are deleted, substituted, inserted and/or added in the amino acid sequence represented by SEQ ID NO:84 and having α1,6-fucosyltransferase activity;
(i) a protein consisting of an amino acid sequence which has 80% or more homology to the amino acid sequence represented by SEQ ID NO:5 and having α1,6-fucosyltransferase activity;
(j) a protein consisting of an amino acid sequence which has 80% or more homology to the amino acid sequence represented by SEQ ID NO:6 and having α1,6-fucosyltransferase activity;
(k) a protein consisting of an amino acid sequence which has 80% or more homology to the amino acid sequence represented by SEQ ID NO:7 and having α1,6-fucosyltransferase activity;
(l) a protein consisting of an amino acid sequence which has 80% or more homology to the amino acid sequence represented by SEQ ID NO:84 and having α1,6-fucosyltransferase activity.

15. The cell according to any one of claims 9 to 14, wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is a double-stranded RNA comprising an RNA selected from the group consisting of the following (a) to (d) and its complementary RNA:
(a) an RNA corresponding to a DNA comprising a nucleotide sequence represented by a sequence of continued 10 to 40 bases in the nucleotide sequence represented by SEQ ID NO:1;
(b) an RNA corresponding to a DNA comprising a nucleotide sequence represented by a sequence of continued 10 to 40 bases in the nucleotide sequence represented by SEQ ID NO:2;
(c) an RNA corresponding to a DNA comprising a nucleotide sequence represented by a sequence of continued 10 to 40 bases in the nucleotide sequence represented by SEQ ID NO: 3;
(d) an RNA corresponding to a DNA comprising a nucleotide sequence represented by a sequence of continued 10 to 40 bases in the nucleotide sequence represented by SEQ ID NO:4.

16. The cell according to any one of claims 9 to 14, wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is a double-stranded RNA comprising an RNA selected from the group consisting of the following (a) and (b) and its complementary RNA:
(a) an RNA comprising the nucleotide sequence represented by any one of SEQ ID NO:14 to 35 or 85 to 89;
(b) an RNA consisting of a nucleotide sequence in which one or a several nucleotide(s) is/are deleted, substituted, inserted and/or added in the nucleotide sequence represented by any one of SEQ ID NO:14 to 35 or 85 to 89 and having activity of suppressing the function of α1,6-fucosyltransferase activity.

17. The cell according to any one of claims 11 to 16, wherein the enzyme catalyzing a reaction which converts GDP-mannose into GDP-4-keto,6-deoxy-GDP-mannose is GDP-mannose 4,6-dehydratase.

18. The cell according to claim 17, wherein the GDP-mannose 4,6-dehydratase is a protein encoded by a DNA selected from the group consisting of the following (a) to (f):
(a) a DNA comprising the nucleotide sequence represented by SEQ ID NO:8;
(b) a DNA comprising the nucleotide sequence represented by SEQ ID NO:9;
(c) a DNA comprising the nucleotide sequence represented by SEQ ID NO:10;
(d) a DNA which hybridizes with a DNA consisting of the nucleotide sequence represented by SEQ ID NO:8 under stringent conditions and encodes a protein having GDP-mannose 4,6-dehydratase activity;
(e) a DNA which hybridizes with a DNA consisting of the nucleotide sequence represented by SEQ ID NO:9 under stringent conditions and encodes a protein having GDP-mannose 4,6-dehydratase activity;
(f) a DNA which hybridizes with a DNA consisting of the nucleotide sequence represented by SEQ ID NO:10 under stringent conditions and encodes a protein having GDP-mannose 4,6-dehydratase activity.

19. The cell according to claim 17, wherein the GDP-mannose 4,6-dehydratase is a protein selected from the group consisting of the following (a) to (i):
(a) a protein comprising the amino acid sequence represented by SEQ ID NO:11;
(b) a protein comprising the amino acid sequence represented by SEQ ID NO: 12;
(c) a protein comprising the amino acid sequence represented by SEQ ID NO:13;
(d) a protein consisting of an amino acid sequence in which one or more amino acid(s) is/are deleted, substituted, inserted and/or added in the amino acid sequence represented by SEQ ID NO:11 and having GDP-mannose 4,6-dehydratase activity;
(e) a protein consisting of an amino acid sequence in which one or more amino acid(s) is/are deleted, substituted, inserted and/or added in the amino acid sequence represented by SEQ ID NO:12 and having GDP-mannose 4,6-dehydratase activity;
(f) a protein consisting of an amino acid sequence in which one or more amino acid(s) is/are deleted, substituted, inserted and/or added in the amino acid sequence represented by SEQ ID NO:13 and having GDP-mannose 4,6-dehydratase activity;
(g) a protein consisting of an amino acid sequence which has 80% or more homology to the amino acid sequence represented by SEQ ID NO:11 and having GDP-mannose 4,6-dehydratase activity;
(h) a protein consisting of an amino acid sequence which has 80% or more homology to the amino acid sequence represented by SEQ ID NO:12 and having GDP-mannose 4,6-dehydratase activity;
(i) a protein consisting of an amino acid sequence which has 80% or more homology to the amino acid sequence represented by SEQ ID NO:13 and having GDP-mannose 4,6-dehydratase activity.

20. The cell according to any one of claims 11 to 19, wherein the RNA capable of suppressing the function of an enzyme catalyzing a reaction which converts GDP-mannose into GDP-4-keto,6-deoxy-GDP-mannose is a double-stranded RNA comprising an RNA selected from the group consisting of the following (a) to (c) and its complementary RNA:
(a) an RNA corresponding to a DNA consisting of a nucleotide sequence represented by a sequence of continued 10 to 40 bases in the nucleotide sequence represented by SEQ ID NO:8;
(b) an RNA corresponding to a DNA consisting of a nucleotide sequence represented by a sequence of continued 10 to 40 bases in the nucleotide sequence represented by SEQ ID NO:9;
(c) an RNA corresponding to a DNA consisting of a nucleotide sequence represented by a sequence of continued 10 to 40 bases in the nucleotide sequence represented by SEQ ID NO:10.

21. The cell according to any one of claims 11 to 19, wherein the RNA capable of suppressing the function of an enzyme catalyzing a reaction which converts GDP-mannose into GDP-4-keto,6-deoxy-GDP-mannose is a double-stranded RNA comprising an RNA selected from the group consisting of the following (a) and (b) and its complementary RNA:
(a) an RNA comprising the nucleotide sequence represented by any one of SEQ ID NO:37, 57 or 58;
(b) an RNA consisting of a nucleotide sequence in which one or a several nucleotide(s) is/are deleted, substituted, inserted and/or added in the nucleotide sequence represented by any one of SEQ ID NO:37, 57 or 58 and having activity of suppressing the function of GDP-mannose 4,6-dehydratase.

22. A DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme relating to synthesis of an intracellular sugar nucleotide, GDP-fucose, and its complementary DNA or a DNA corresponding to an RNA capable of suppressing the function of a protein relating to transport of an intracellular sugar nucleotide, GDP-fucose, to the Golgi body and its complementary DNA.

23. A DNA comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a DNA corresponding to an RNA capable of suppressing the function of an enzyme relating to synthesis of an intracellular sugar nucleotide, GDP-fucose, and its complementary DNA.

24. The DNA according to claim 22 or 23, wherein the enzyme relating to synthesis of an intracellular sugar nucleotide, GDP-fucose, is an enzyme catalyzing a reaction which converts GDP-mannose into GDP-4-keto,6-deoxy-GDP-mannose.

25. The DNA according to any one of claims 22 to 24, wherein the enzyme relating to modification of a sugar chain in which 1-position of fucose is bound to 6-position of N-acetylglucosamine in the reducing end through α-bond in the complex type N-glycoside-linked sugar chain is α1,6-fucosyltransferase.

26. The DNA according to claim 25, wherein the α1,6-fucosyltransferase is a protein encoded by a DNA selected from the group consisting of the following (a) to (h):
(a) a DNA comprising the nucleotide sequence represented by SEQ ID NO:1;
(b) a DNA comprising the nucleotide sequence represented by SEQ ID NO:2;
(c) a DNA comprising the nucleotide sequence represented by SEQ ID N0:3;
(d) a DNA comprising the nucleotide sequence represented by SEQ ID NO:4;
(e) a DNA which hybridizes with a DNA consisting of the nucleotide sequence represented by SEQ ID NO:1 under stringent conditions and encodes a protein having α1,6-fucosyltransferase activity;
(f) a DNA which hybridizes with a DNA consisting of the nucleotide sequence represented by SEQ ID NO:2 under stringent conditions and encodes a protein having α1,6-fucosyltransferase activity;
(g) a DNA which hybridizes with a DNA consisting of the nucleotide sequence represented by SEQ ID NO:3 under stringent conditions and encodes a protein having α1,6-fucosyltransferase activity;
(h) a DNA which hybridizes with a DNA consisting of the nucleotide sequence represented by SEQ ID NO:4 under stringent conditions and encodes a protein having α1,6-fucosyltransferase activity.

27. The DNA according to claim 25, wherein the α1,6-fucosyltransferase is a protein selected from the group consisting of the following (a) to (1):
(a) a protein comprising the amino acid sequence represented by SEQ ID NO:5;
(b) a protein comprising the amino acid sequence represented by SEQ ID NO:6;
(c) a protein comprising the amino acid sequence represented by SEQ ID NO:7;
(d) a protein comprising the amino acid sequence represented by SEQ ID NO:84;
(e) a protein consisting of an amino acid sequence in which one or more amino acid(s) is/are deleted, substituted, inserted and/or added in the amino acid sequence represented by SEQ ID NO:5 and having α1,6-fucosyltransferase activity;
(f) a protein consisting of an amino acid sequence in which one or more amino acid(s) is/are deleted, substituted, inserted and/or added in the amino acid sequence represented by SEQ ID NO:6 and having α1,6-fucosyltransferase activity;
(g) a protein consisting of an amino acid sequence in which one or more amino acid(s) is/are deleted, substituted, inserted and/or added in the amino acid sequence represented by SEQ ID NO:7 and having α1,6-fucosyltransferase activity;
(h) a protein consisting of an amino acid sequence in which one or more amino acid(s) is/are deleted, substituted, inserted and/or added in the amino acid sequence represented by SEQ ID NO:84 and having α1,6-fucosyltransferase activity;
(i) a protein consisting of an amino acid sequence which has 80% or more homology to the amino acid sequence represented by SEQ ID NO:5 and having α1,6-fucosyltransferase activity;
(j) a protein consisting of an amino acid sequence which has 80% or more homology to the amino acid sequence represented by SEQ ID NO:6 and having α1,6-fucosyltransferase activity;
(k) a protein consisting of an amino acid sequence which has 80% or more homology to the amino acid sequence represented by SEQ ID NO:7 and having α1,6-fucosyltransferase activity;
(l) a protein consisting of an amino acid sequence which has 80% or more homology to the amino acid sequence represented by SEQ ID NO:84 and having α1,6-fucosyltransferase activity.

28. The DNA according to any one of claims 22 to 27, wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is an RNA selected from the group consisting of the following (a) to (d):
(a) an RNA corresponding to a DNA comprising a nucleotide sequence represented by a sequence of continued 10 to 40 bases in the nucleotide sequence represented by SEQ ID NO:1;
(b) an RNA corresponding to a DNA comprising a nucleotide sequence represented by a sequence of continued 10 to 40 bases in the nucleotide sequence represented by SEQ ID NO:2;
(c) an RNA corresponding to a DNA comprising a nucleotide sequence represented by a sequence of continued 10 to 40 bases in the nucleotide sequence represented by SEQ ID NO:3;
(d) an RNA corresponding to a DNA comprising a nucleotide sequence represented by a sequence of continued 10 to 40 bases in the nucleotide sequence represented by SEQ ID NO:4.

29. The DNA according to any one of claims 22 to 27, wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is an RNA selected from the group consisting of the following (a) and (d):
(a) an RNA comprising the nucleotide sequence represented by any one of SEQ ID NO:14 to 35 or 85 to 89;
(b) an RNA consisting of a nucleotide sequence in which one or a several nucleotide(s) is/are deleted, substituted, inserted and/or added in the nucleotide sequence represented by any one of SEQ ID NO:14 to 35 or 85 to 89 and having activity of suppressing the function of α1,6-fucosyltransferase activity.

30. The DNA according to any one of claims 24 to 29, wherein the enzyme catalyzing a reaction which converts GDP-mannose into GDP-4-keto,6-deoxy-GDP-mannose is GDP-mannose 4,6-dehydratase.

31. The DNA according to claim 30, wherein the GDP-mannose 4,6-dehydratase is a protein encoded by a DNA selected from the group consisting of the following (a) to (f):
(a) a DNA comprising the nucleotide sequence represented by SEQ ID NO:8;
(b) a DNA comprising the nucleotide sequence represented by SEQ ID NO:9;
(c) a DNA comprising the nucleotide sequence represented by SEQ ID NO:10;
(d) a DNA which hybridizes with a DNA consisting of the nucleotide sequence represented by SEQ ID NO:8 under stringent conditions and encodes a protein having GDP-mannose 4,6-dehydratase activity;
(e) a DNA which hybridizes with a DNA consisting of the nucleotide sequence represented by SEQ ID NO:9 under stringent conditions and encodes a protein having GDP-mannose 4,6-dehydratase activity;
(f) a DNA which hybridizes with a DNA consisting of the nucleotide sequence represented by SEQ ID NO:10 under stringent conditions and encodes a protein having GDP-mannose 4,6-dehydratase activity.

32. The DNA according to claim 30, wherein the GDP-mannose 4,6-dehydratase is a protein selected from the group consisting of the following (a) to (i):
(a) a protein comprising the amino acid sequence represented by SEQ ID NO:11;
(b) a protein comprising the amino acid sequence represented by SEQ ID NO:12;
(c) a protein comprising the amino acid sequence represented by SEQ ID NO:13;
(d) a protein consisting of an amino acid sequence in which one or more amino acid(s) is/are deleted, substituted, inserted and/or added in the amino acid sequence represented by SEQ ID NO:11 and having GDP-mannose 4,6-dehydratase activity;
(e) a protein consisting of an amino acid sequence in which one or more amino acid(s) is/are deleted, substituted, inserted and/or added in the amino acid sequence represented by SEQ ID NO: 12 and having GDP-mannose 4,6-dehydratase activity;
(f) a protein consisting of an amino acid sequence in which one or more amino acid(s) is/are deleted, substituted, inserted and/or added in the amino acid sequence represented by SEQ ID NO:13 and having GDP-mannose 4,6-dehydratase activity;
(g) a protein consisting of an amino acid sequence which has 80% or more homology to the amino acid sequence represented by SEQ ID NO:11 and having GDP-mannose 4,6-dehydratase activity;
(h) a protein consisting of an amino acid sequence which has 80% or more homology to the amino acid sequence represented by SEQ ID NO: 12 and having GDP-mannose 4,6-dehydratase activity;
(i) a protein consisting of an amino acid sequence which has 80% or more homology to the amino acid sequence represented by SEQ ID NO:13 and having GDP-mannose 4,6-dehydratase activity.

33. The DNA according to any one of claims 24 to 32, wherein the RNA capable of suppressing the function of an enzyme catalyzing a reaction which converts GDP-mannose into GDP-4-keto,6-deoxy-GDP-mannose is an RNA selected from the group consisting of the following (a) to (c):
(a) an RNA corresponding to a DNA consisting of a nucleotide sequence represented by a sequence of continued 10 to 40 bases in the nucleotide sequence represented by SEQ ID NO:8;
(b) an RNA corresponding to a DNA consisting of a nucleotide sequence represented by a sequence of continued 10 to 40 bases in the nucleotide sequence represented by SEQ ID NO:9;
(c) an RNA corresponding to a DNA consisting of a nucleotide sequence represented by a sequence of continued 10 to 40 bases in the nucleotide sequence represented by SEQ ID NO:10.

34. The DNA according to any one of claims 24 to 32, wherein the RNA capable of suppressing the function of an enzyme catalyzing a reaction which converts GDP-mannose into GDP-4-keto,6-deoxy-GDP-mannose is an RNA selected from the group consisting of the following (a) and (b):
(a) an RNA comprising the nucleotide sequence represented by any one of SEQ ID NO:37, 57 or 58;
(b) an RNA consisting of a nucleotide sequence in which one or a several nucleotide(s) is/are deleted, substituted, inserted and/or added in the nucleotide sequence represented by any one of SEQ ID NO:37, 57 or 58 and having activity of suppressing the function of GDP-mannose 4,6-dehydratase.

35. A vector comprising the DNA described in any one of claims 22 to 34.

36. The vector according to claim 35, which comprises the DNA represented by SEQ ID NO:90 and the DNA represented by SEQ ID NO:92.

37. The vector according to claim 35, which comprises the DNA represented by SEQ ID NO:91 and the DNA represented by SEQ ID NO:92.

38. The vector according to claim 35, which comprises the DNA represented by SEQ ID NO:90 and the DNA represented by SEQ ID NO:93.

39. The vector according to claim 35, which comprises the DNA represented by SEQ ID NO:91 and the DNA represented by SEQ ID NO:93.

40. A cell into which the vector according to any one of claims 35 to 39 is introduced.

41. A cell into which a vector comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a vector comprising a DNA corresponding to an RNA capable of suppressing the function of an enzyme relating to synthesis of an intracellular sugar nucleotide, GDP-fucose, and its complementary DNA or a vector comprising a DNA corresponding to an RNA capable of suppressing the function of a protein relating to transport of an intracellular sugar nucleotide, GDP-fucose, to the Golgi body and its complementary DNA are introduced.

42. A cell into which a vector comprising a DNA corresponding to an RNA capable of suppressing the function of an enzyme relating to modification of a sugar chain in which 1-position of fucose is bound to 6-position of N-acetylglucosamine in the reducing end through α-bond in the complex type N-glycoside-linked sugar chain and its complementary DNA, and a vector comprising a DNA corresponding to an RNA capable of suppressing the function of an enzyme relating to synthesis of an intracellular sugar nucleotide, GDP-fucose, and its complementary DNA are introduced.

43. The cell according to claim 41 or 42, wherein the RNA capable of suppressing the function of an enzyme protein relating to synthesis of an intracellular sugar nucleotide, GDP-fucose, is an RNA capable of suppressing the function of an enzyme catalyzing a reaction which converts GDP-mannose into GDP-4-keto,6-deoxy-GDP-mannose.

44. The cell according to any one of claims 41 to 43, wherein the enzyme relating to modification of a sugar chain in which 1-position of fucose is bound to 6-position of N-acetylglucosamine in the reducing end through α-bond in the complex type N-glycoside-linked sugar chain is α1,6-fucosyltransferase.

45. The cell according to claim 44, wherein the α1,6-fucosyltransferase is a protein encoded by a DNA selected from the group consisting of (a) to (h):
(a) a DNA comprising the nucleotide sequence represented by SEQ ID NO:1;
(b) a DNA comprising the nucleotide sequence represented by SEQ ID NO:2;
(c) a DNA comprising the nucleotide sequence represented by SEQ ID NO:3;
(d) a DNA comprising the nucleotide sequence represented by SEQ ID NO:4;
(e) a DNA which hybridizes with a DNA consisting of the nucleotide sequence represented by SEQ ID NO: under stringent conditions and encodes a protein having α1,6-fucosyltransferase activity;
(f) a DNA which hybridizes with a DNA consisting of the nucleotide sequence represented by SEQ ID NO:2 under stringent conditions and encodes a protein having α1,6-fucosyltransferase activity;
(g) a DNA which hybridizes with a DNA consisting of the nucleotide sequence represented by SEQ ID NO:3 under stringent conditions and encodes a protein having α1,6-fucosyltransferase activity;
(h) a DNA which hybridizes with a DNA consisting of the nucleotide sequence represented by SEQ ID NO:4 under stringent conditions and encodes a protein having α1,6-fucosyltransferase activity.

46. The cell according to claim 44, wherein the α1,6-fucosyltransferase is a protein selected from the group consisting of the following (a) to (l):
(a) a protein comprising the amino acid sequence represented by SEQ ID NO:5;
(b) a protein comprising the amino acid sequence represented by SEQ ID NO:6;
(c) a protein comprising the amino acid sequence represented by SEQ ID NO:7;
(d) a protein comprising the amino acid sequence represented by SEQ ID NO: 84;
(e) a protein consisting of an amino acid sequence in which one or more amino acid(s) is/are deleted, substituted, inserted and/or added in the amino acid sequence represented by SEQ ID NO:5 and having α1,6-fucosyltransferase activity;
(f) a protein consisting of an amino acid sequence in which one or more amino acid(s) is/are deleted, substituted, inserted and/or added in the amino acid sequence represented by SEQ ID NO:6 and having α1,6-fucosyltransferase activity;
(g) a protein consisting of an amino acid sequence in which one or more amino acid(s) is/are deleted, substituted, inserted and/or added in the amino acid sequence represented by SEQ ID NO:7 and having a1,6-fucosyltransferase activity;
(h) a protein consisting of an amino acid sequence in which one or more amino acid(s) is/are deleted, substituted, inserted and/or added in the amino acid sequence represented by SEQ ID NO:84 and having α1,6-fucosyltransferase activity;
(i) a protein consisting of an amino acid sequence which has 80% or more homology to the amino acid sequence represented by SEQ ID NO:5 and having α1,6-fucosyltransferase activity;
(j) a protein consisting of an amino acid sequence which has 80% or more homology to the amino acid sequence represented by SEQ ID NO:6 and having α1,6-fucosyltransferase activity;
(k) a protein consisting of an amino acid sequence which has 80% or more homology to the amino acid sequence represented by SEQ ID NO:7 and having α1,6-fucosyltransferase activity;
(l) a protein consisting of an amino acid sequence which has 80% or more homology to the amino acid sequence represented by SEQ ID NO:84 and having α1,6-fucosyltransferase activity.

47. The cell according to any one of claims 41 to 46, wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is a double-stranded RNA comprising an RNA selected from the group consisting of the following (a) to (d) and its complementary RNA:
(a) an RNA corresponding to a DNA comprising a nucleotide sequence represented by a sequence of continued 10 to 40 bases in the nucleotide sequence represented by SEQ ID NO:1;
(b) an RNA corresponding to a DNA comprising a nucleotide sequence represented by a sequence of continued 10 to 40 bases in the nucleotide sequence represented by SEQ ID NO:2;
(c) an RNA corresponding to a DNA comprising a nucleotide sequence represented by a sequence of continued 10 to 40 bases in the nucleotide sequence represented by SEQ ID NO:3;
(d) an RNA corresponding to a DNA comprising a nucleotide sequence represented by a sequence of continued 10 to 40 bases in the nucleotide sequence represented by SEQ ID NO:4.

48. The cell according to any one of claims 41 to 46, wherein the RNA capable of suppressing the function of an enzyme relating to modification of a sugar chain in which 1-position of fucose is bound to 6-position of N-acetylglucosamine in the reducing end through α-bond in the complex type N-glycoside-linked sugar chain is a double-stranded RNA comprising an RNA selected from the group consisting of the following (a) and (b) and its complementary RNA:
(a) an RNA comprising the nucleotide sequence represented by any one of SEQ ID N0:14 to 35 or 85 to 89;
(b) an RNA consisting of a nucleotide sequence in which one or more nucleotide(s) is/are deleted, substituted, inserted and/or added in the nucleotide sequence represented by any one of SEQ ID NO:14 to 35 or 85 to 89 and having activity of suppressing the function of α1,6-fucosyltransferase activity.

49. The cell according to any one of claims 43 to 48, wherein the enzyme catalyzing a reaction which converts GDP-mannose into GDP-4-keto,6-deoxy-GDP-mannose is GDP-mannose 4,6-dehydratase.

50. The cell according to claim 49, wherein the GDP-mannose 4,6-dehydratase is a protein encoded by a DNA selected from the group consisting of the following (a) to (f):
(a) a DNA comprising the nucleotide sequence represented by SEQ ID NO:8;
(b) a DNA comprising the nucleotide sequence represented by SEQ ID NO:9;
(c) a DNA comprising the nucleotide sequence represented by SEQ ID NO:10;
(d) a DNA which hybridizes with a DNA consisting of the nucleotide sequence represented by SEQ ID NO:8 under stringent conditions and encodes a protein having GDP-mannose 4,6-dehydratase activity;
(e) a DNA which hybridizes with a DNA consisting of the nucleotide sequence represented by SEQ ID NO:9 under stringent conditions and encodes a protein having GDP-mannose 4,6-dehydratase activity;
(f) a DNA which hybridizes with a DNA consisting of the nucleotide sequence represented by SEQ ID NO:10 under stringent conditions and encodes a protein having GDP-mannose 4,6-dehydratase activity.

51. The cell according to claim 49, wherein the GDP-mannose 4,6-dehydratase is a protein selected from the group consisting of the following (a) to (i):
(a) a protein comprising the amino acid sequence represented by SEQ ID NO: 11;
(b) a protein comprising the amino acid sequence represented by SEQ ID NO:12;
(c) a protein comprising the amino acid sequence represented by SEQ ID NO:13;
(d) a protein consisting of an amino acid sequence in which one or more amino acid(s) is/are deleted, substituted, inserted and/or added in the amino acid sequence represented by SEQ ID NO:11 and having GDP-mannose 4,6-dehydratase activity;
(e) a protein consisting of an amino acid sequence in which one or more amino acid(s) is/are deleted, substituted, inserted and/or added in the amino acid sequence represented by SEQ ID NO:12 and having GDP-mannose 4,6-dehydratase activity;
(f) a protein consisting of an amino acid sequence in which one or more amino acid(s) is/are deleted, substituted, inserted and/or added in the amino acid sequence represented by SEQ ID NO: 13 and having GDP-mannose 4,6-dehydratase activity;
(g) a protein consisting of an amino acid sequence which has 80% or more homology to the amino acid sequence represented by SEQ ID NO:11 and having GDP-mannose 4,6-dehydratase activity;
(h) a protein consisting of an amino acid sequence which has 80% or more homology to the amino acid sequence represented by SEQ ID NO:12 and having GDP-mannose 4,6-dehydratase activity;
(i) a protein consisting of an amino acid sequence which has 80% or more homology to the amino acid sequence represented by SEQ ID NO:13 and having GDP-mannose 4,6-dehydratase activity.

52. The cell according to any one of claims 43 to 51, wherein the RNA capable of suppressing the function of an enzyme catalyzing a reaction which converts GDP-mannose into GDP-4-keto,6-deoxy-GDP-mannose is a double-stranded RNA comprising an RNA selected from the group consisting of the following (a) to (c) and its complementary RNA:
(a) an RNA corresponding to a DNA consisting of a nucleotide sequence represented by a sequence of continued 10 to 40 bases in the nucleotide sequence represented by SEQ ID NO:8;
(b) an RNA corresponding to a DNA consisting of a nucleotide sequence represented by a sequence of continued 10 to 40 bases in the nucleotide sequence represented by SEQ ID NO:9;
(c) an RNA corresponding to a DNA consisting of a nucleotide sequence represented by a sequence of continued 10 to 40 bases in the nucleotide sequence represented by SEQ ID NO:10.

53. The cell according to any one of claims 43 to 51, wherein the RNA capable of suppressing the function of an enzyme catalyzing a reaction which converts GDP-mannose into GDP-4-keto,6-deoxy-GDP-mannose is a double-stranded RNA comprising an RNA selected from the group consisting of the following (a) and (b) and its complementary RNA:
(a) an RNA comprising the nucleotide sequence represented by any one of SEQ ID NO:37, 57 or 58;
(b) an RNA consisting of a nucleotide sequence in which one or a several nucleotide(s) is/are deleted, substituted, inserted and/or added in the nucleotide sequence represented by any one of SEQ ID NO:37, 57 or 58 and having activity of suppressing the function of GDP-mannose 4,6-dehydratase.

54. The cell according to any one of claim 1 to 4, 8 to 21 and 40 to 53, which is resistant to a lectin which recognizes a sugar chain structure in which 1-position of fucose is bound to 6-position of N-acetylglucosamine in the reducing end through α-bond in an N-glycoside-linked sugar chain.

55. The cell according to claim 54, wherein the lectin is selected from the group consisting of the following (a) to (d):
(a) a *Lens culinaris* agglutinin LCA (lentil agglutinin derived from *Lens culinaris*);
(b) a *Pisum sativum* agglutinin PSA (pea lectin derived from *Pisum sativum*);
(c) a *Vicia faba* agglutinin VFA (agglutinin derived from *Vicia faba*);
(d) an *Aleuria aurantia* lectin AAL (lectin derived from *Aleuria aurantia*).

56. The cell according to any one of claims 1 to 4, 8 to 21 and 40 to 55, which is a cell selected from the group consisting of an yeast, an animal cell, an insect cell and a plant cell.

57. The cell according to claim 56, wherein the animal cell is selected from the group consisting of the following (a) to (k):
(a) a CHO cell derived from a Chinese hamster ovary tissue;
(b) a rat myeloma cell line YB2/3HL.P2.G11.16Ag.20 cell;
(c) a mouse myeloma cell line NS0 cell;
(d) a mouse myeloma cell line SP2/0-Ag14 cell;
(e) a BHK cell derived from a Syrian hamster kidney tissue;
(f) a hybridoma cell which produces an antibody;
(g) a human leukemic cell line Namalwa cell;
(h) a human leukemic cell line NM-F9 cell;
(i) a human embryonic retinal cell line PER.C6 cell;
(j) an embryonic stem cell;
(k) a fertilized egg cell.

58. The cell according to any one of claims 1 to 4, 8 to 21 and 40 to 57, which comprises a gene encoding a glycoprotein.

59. The cell according to claim 58, wherein the glycoprotein is an antibody molecule.

60. The cell according to claim 59, wherein the antibody molecule is selected from the group consisting of the following (a) to (d):
(a) a human antibody;
(b) a humanized antibody;
(c) an antibody fragment comprising the Fc region of (a) or (b);
(d) a fusion protein comprising the Fc region of (a) or (b).

61. The cell according to claim 59, wherein the antibody molecule belongs to an IgG class.

62. A process for producing a glycoprotein composition, which comprises using the cell described in claim 58.

63. A process for producing a glycoprotein composition, which comprises culturing the cell described in claim 58 in a medium to form and accumulate the glycoprotein composition in the culture; and recovering and purifying the glycoprotein composition from the culture.

64. A process for producing an antibody composition, which comprises using the cell described in any one of claims 59 to 61.

65. A process for producing an antibody composition, which comprises culturing the cell described in any one of claims 59 to 61 in a medium to form and accumulate the antibody composition in the culture; and recovering and purifying the antibody composition from the culture.

66. The process according to claim 64 or 65, wherein the antibody composition is an antibody composition having a higher antibody-dependent cell-mediated cytotoxic activity than an antibody composition produced by its parent cell.

67. The process according to claim 66, wherein the antibody composition having a higher antibody-dependent cell-mediated cytotoxic activity has a higher ratio of a sugar chain in which fucose is not bound to N-acetylglucosamine in the reducing end in the sugar chain among total complex type N-glycoside-linked sugar chains bound to the Fc region in the antibody composition than an antibody composition produced by its parent cell.

68. The process according to claim 67, wherein the sugar chain in which fucose is not bound is a sugar chain in which 1-position of the fucose is not bound to 6-position of N-acetylglucosamine in the reducing end through α-bond in a complex type N-glycoside-linked sugar chain.

69. A glycoprotein composition produced by the process described in claim 62 or 63.

70. An antibody composition produced by the process described in any one of claims 64 to 68.

71. A medicament comprising the composition described in claim 69 or 70 as an active ingredient.
